# EUROPEAN PATENT SPECIFICATION

(11) **EP 1 682 665 B1**
(45) Date of publication and mention of the grant of the patent: **27.04.2011**
(21) Application number: 04797676.6
(22) Date of filing: 05.11.2004
(51) Int. Cl.: C12N 15/19

(54) **USE OF FIBROBLAST GROWTH FACTOR FRAGMENTS**
VERWENDUNG VON FIBROBLASTEN-WACHSTUMSFAKTOR-FRAGMENTEN
UTILISATION DE FRAGMENTS DU FACTEUR DE CROISSANCE DES FIBROBLASTES

(30) Priority: 07.11.2003 US 518073 P; 18.05.2004 US 572247 P
(43) Date of publication of application: 26.07.2006
(73) Proprietor: Novartis AG, 4056 Basel (CH)
(72) Inventor: BOLLEKENS, Jacques, B-1150 Bruxelles (BE); CHIBOUT, Salah-Dine, F-68720 Tagolsheim (FR); VONDERSCHER, Jacky, Cambridge, MA 02140 (US); LEGAY, François, F-68300 Saint Louis (FR); CORDIER, André, CH-4054 Basel (CH); PAPOIAN, Ruben, CH-4053 Basel (CH); SCHERER, Andreas, 79618 Rheinfelden-Herten (DE)
(74) Representative: Roth, Peter Richard
(86) International application number: PCT/EP2004/012572
(87) International publication number: WO 2005/045044

(56) References cited:
- WO-A-01/66596
- WO-A-02/088358
- DATABASE Geneseq [Online] 28 March 2003 (2003-03-28), "Human fibroblast growth factor 23 polypeptide." XP002316719 retrieved from EBI accession no. GSN:ABP58110 Database accession no. ABP58110
- JONSSON K.B. ET AL: "Fibroblast growth factor 23 in oncogenic osteomalacia and X-linked hypophosphatemia" THE NEW ENGLAND JOURNAL OF MEDICINE, vol. 348, no. 17, 24 April 2003 (2003-04-24), pages 1656-1663, XP008037462

### SEQUENCE LISTING

<110> Novartis AG
<120> Use of fibroblast growth factor fragments
<130> 4-33264A
<160> 4
<170> PatentIn version 3.1
<210> 1
   <211> 251
   <212> PRT
   <213> Homo sapiens
<400> 1
<210> 2
   <211> 75
   <212> PRT
   <213> Homo sapiens
<400> 2
   His Thr Arg Ser Ala Glu Asp Asp Ser Glu Arg Asp Pro Leu Asn Val
   1 5 10 15
   Leu Lys Pro Arg Ala Arg Met Thr Pro Ala Pro Ala Ser Cys Ser Gln
   20 25 30
   Glu Leu Pro Ser Ala Glu Asp Asn Ser Pro Met Ala Ser Asp Pro Leu
   35 40 45
   Gly Val Val Arg Gly Gly Arg Val Asn Thr His Ala Gly Gly Thr Gly
   50 55 60
   Pro Glu Gly Cys Arg Pro Phe Ala Lys Phe Ile
   65 70 75
<210> 3
   <211> 756
   <212> DNA
   <213> Homo sapiens
<400> 3
   atgttggggg cccgcctcag gctctgggtc tgtgccttgt gcagcgtctg cagcatgagc      60
   gtcctcagag cctatcccaa tgcctcccca ctgctcggct ccagctgggg tggcctgatc     120
   cacctgtaca cagccacagc caggaacagc taccacctgc agatccacaa gaatggccat     180
   gtggatggcg caccccatca gaccatctac agtgccctga tgatcagatc agaggatgct     240
   ggctttgtgg tgattacagg tgtgatgagc agaagatacc tctgcatgga tttcagaggc     300
   aacatttttg gatcacacta tttcgacccg gagaactgca ggttccaaca ccagacgctg     360
   gaaaacgggt acgacgtcta ccactctcct cagtatcact tcctggtcag tctgggccgg     420
   gcgaagagag ccttcctgcc aggcatgaac ccacccccgt actcccagtt cctgtcccgg     480
   aggaacgaga tccccctaat tcacttcaac acccccatac cacggcggca cacccggagc     540
   gccgaggacg actcggagcg ggaccccctg aacgtgctga agccccgggc ccggatgacc     600
   ccggccccgg cctcctgttc acaggagctc ccgagcgccg aggacaacag cccgatggcc     660
   agtgacccat taggggtggt caggggcggt cgagtgaaca cgcacgctgg gggaacgggc     720
   ccggaaggct gccgcccctt cgccaagttc atctag                               756
<210> 4
   <211> 228
   <212> DNA
   <213> Homo sapiens
<400> 4
   cacacccgga gcgccgagga cgactcggag cgggaccccc tgaacgtgct gaagccccgg      60
   gcccggatga ccccggcccc ggcctcctgt tcacaggagc tcccgagcgc cgaggacaac     120
   agcccgatgg ccagtgaccc attaggggtg gtcaggggcg gtcgagtgaa cacgcacgct     180
   gggggaacgg gcccggaagg ctgccgcccc ttcgccaagt tcatctag                 228

## Description

### FIELD OF THE INVENTION

The invention relates generally to the *in vivo* testing of the efficacy of a compound or composition, and particularly to the testing and biologically functionalizing of classical small molecules, natural products, genes, peptides and proteins by activity *in vivo.*

The invention further relates to medical uses of fibroblast growth factor 23 (FGF-23), FGF-23 fragments, FGF-23 C-terminal polypeptides, FGF-23 homologs and/or FGF-23 variants, in particular for the manufacture of a medicament for the treatment of diseases associated with deregulated angiogenesis or cell proliferative disorders.

### BACKGROUND OF THE INVENTION

Pharmaceutical companies are interested in evaluating and understanding the function and regulation of newly discovered genes and gene products (proteins), especially newly discovered genes and proteins, which could help in the understanding of the mechanisms linked to diseases or compounds action. In addition, the genes and gene products can become potential drugs or biomarkers. Grenet O, Pharmacogenomics J. 1(1):11-2 (2001).

However, a gene sequence alone does not provide information about the actual function of the protein in the cell or organism physiology. In addition, while the genome has a relatively well defined number of genes, there is no known limit to the possible number of protein variants. The potential number of proteins encoded by these genes is estimated to be from two to at least one-hundred times higher than the number of genes, since it has recently been found that proteins can also be produced by splicing at the protein level not just at the RNA level.

The current process of drug discovery proceeds from single target to single drug product. The current process is a long process, frequently with late attrition for lack of efficacy, wrong design or false indication.

Thus, there is a need in the art for a more efficient method for discovering and identifying drug candidates, gene targets and biomarkers.

### SUMMARY OF THE INVENTION

The invention provides a discovery process for biologically functionalizing peptides, proteins, genes, small molecules and natural products using organism-wide gene expression profiling. The discovery process of the invention proceeds from single lead or drug to multiple targets and indications (as indicated by an impact on any target in the cascade chain of a pathway), and multiple drug products, thus providing rapid guidance to a correct human proof-of-concept.

The discovery process of the invention begins with administration of test substances to animals, followed by screening of the resulting gene expression in many organs obtained from the test animal. The invention can be used to biologically functionalize the entire genome of any organism where micoarray chips are available. The invention is not restricted to the type of compound to be functionalized. Small molecules, proteins, natural products, cDNA (for functionalizing any gene of interest), *etc*. are all susceptible to the strategy of the invention.

Since the discovery process of the invention is based on a non-preconceived hypothesis and whole organism multi-organ analysis, polypeptides can be selected for testing in the absence of any biological selection criteria other than peptide sequence. The resulting organism-wide pattern of the gene expression changes in the transcriptome provides an overview of the activities at the molecular and organism-wide levels. Accordingly, the unbiased approach of the invention regarding the administration of a compound can provide information about the physiological relationships throughout the entire body that are caused by the compound's administration.

The discovery process of the invention then integrates *in vivo* profiling with internal and external genomic databases to elucidate the function of unknown proteins, typically within few months. The unbiased approach of the invention in regards to the administration of a compound advantageously provides genomic signatures from multiple organs. The resulting data can be analyzed either by using tools that are known to those of skill in the art or by using mols that compare the compound signatures produced by the administration of the compound among the different organs. This multi-organ analysis is in contrast to standard approaches, which, since they do not use an unbiased approach of compound administration, do not result in a multi-organ identification of the function of the compound. Instead the standard approaches provide analysis on a case-by-case basis, which can make cross-experimental comparisons difficult. In contrast to the standard approaches, the identification of the function of the compound using the method of the invention allows for the identification of the function of the compound in many metabolic and regulatory pathways. In addition, the identification of the function of the compound using the method of the invention advantageously results in an understanding of the stability of the active compound in the body, a property of the administered compound which would otherwise not be predictable *a priori* using standard approaches.

The identification of the function of the compound using the method of the invention can be multi-step, as one step or part of the identification leads to another step or part of the identification, to provide a more complete understanding of the administered compound's activity *in vivo.* For example, the identification of compound function in one organ (such as the spleen) can lead to an understanding of the compound function in other organs. By contrast, the standard approaches, which rely on immediate access to tests on a limited number of organs, depend on anecdotal evidence from other experiments to further steps in the identification of compound function.

The invention is suitable for several stages of drug discovery, identifying both drug targets and biomarkers. The discovery process of the invention advantageously delivers an increased number of validated drug candidates and identified drug targets and biomarkers along with a savings in time, resources and animals. The discovery process of the invention advantageously integrates into one process standard exploratory tools with new genomic approaches. The discovery process of the invention can also be used for the reprofiling of safe compounds stopped after the initial stages of drug approval (*e.g.*, Phase I) for re-indication. The invention can be used for adjusting the best fit for combination therapies, by optimally matching of gene expression signatures between two compounds, cancelling of side effects and the potentiation of efficacy. The discovery process of the invention can be used for the profiling of the more advanced development portfolio to guide the later stages af drug approval process (*e.g.* Phase II and Phase III)

In several embodiments, the process can be used to analyze tissues or body fluids (such as coronary heart disease, breast cancer and another indication; each compared to healthy controls). Plasma proteins that are differentially expressed between normal. subjects and coronary artery diseased patients, with known or unknown function, are analyzed for potential target identification/validation, and biomarker identification.

In one embodiment, the discovery process of the invention begins with an *in vivo* screening of proteins, peptides and reference compounds in mice. Based on the results of the mice screening, an *in vivo* verification of selected proteins, peptides or reference compounds is then conducted in non-human primates or animal models of human pathology or disease. The comparison of the resultant information to a profile of reference drugs, with well characterized pharmacological activity, facilitates biological interpretation of the profiles of unknown compounds. In a particular embodiment, the selection rate for the proteins, peptides or reference compounds is ~20%.

In one embodiment the discovery process of the invention combines in one process: (a) pre-screening in mice; (b) verification of selected proteins/peptides/reference compounds in monkeys; (c) large number of the analyzed tissues (up to 25 in mice, up to 120 in monkeys); (d) homogeneity of the tissue sample (e) high quality mRNA; (f) a genome-wide approach with hybridization chips; (g) powerful bioinformation tools for clustering and statistics; (h) the possibility of cross-assay meta-analysis; and (i) localization at the cellular level of the affected genes or pathways by *in situ* hybridization.

By use of the discovery process of the invention, it has now surprisingly been found that polypeptides relating to FGF-23 affect key genes controlling cellular differentiation and proliferation, as well as angiogenesis.

Fibroblast growth factors (FGFs) make up a large family of polypeptide growth factors that are found in organisms ranging from nematodes to humans. During embryonic development, FGFs have diverse roles in regulating cell proliferation, migration and differentiation. In the adult organism, FGFs are homeostatic factors and function in tissue repair and response to injury. Inappropriate expression of some FGFs can contribute to the pathogenesis of cancer.

Mouse FGF-23 has been identified by homology-search in the GenBank Nucleotide Sequence Database with amino acid sequence of mousse FGF-15. Mousse IGF-23 and human FGF-23 are highly identical (~72% amino acid identity). Both, mouse and human FGF-23, cDNAs encode a protein of 251 amino acids, having a hydrophobic amino terminus (~24 amino acids) typical for secreted proteins, and a unique C-terminus having no homology to other FGF family members. In the mouse, FGF-23 mRNA is expressed in the brain, preferentially in the ventrolateral thalamic nucleus, and in the thymus at low levels.

Overexpression of FGF-23 or expression of mutated FGF-23 has been demonstrated to be associated with several pathological findings:

Recombinant FGF-23 induces hypophosphatemia *in vivo* as a result of urinary phosphate wasting (Shimada T., et al., Proc. Natl. Acad. Sci. U. S. A. 98: 6500-6505 (2001)).

FGF-23 overexpression has been observed in tumors that are responsible for oncogenic osteomalacia (OOM) (White K.E., et al., J. Clin. Endocrinol. Metab. 86: 497-500 (2001)).

Autosomal dominant hypophosphatemic rickets (ADHR) has been shown to be associated with mutations of FGF-23 within the 176-RXXXR-179 cleavage site, preventing degradation of FGF-23 (The ADHR Consortium, Nat. Genet. 26: 345-348 (2000)).

While OOM and ADHR have been demonstrated to be associated with FGF-23, a further disorder, X-linked hypophosphatemia (XLH), which is phenotypically similar to OOM and ADHR, has been shown to result from mutations in the PHEX gene. PHEX encodes a membrane-bound endopeptidase (The HYP Consortium, Nat. Genet. 11: 130-136 (1995)) and it is hypothesized that FGF-23 is a PHEX substrate, while FGF-23 ADHR mutant (FGF-23(R179Q) being undegradeable by PHEX.

Each of the above described syndromes is characterized by hypophosphatemia, decreased renal phosphate reabsorption, normal or low serum calcitriol concentrations, normal serum concentrations of calcium and parathyroid hormone, and defective skeletal mineralization (Quarles L. D. and Drezner M. K., J. Clin. Endocrino/. Metab. 86:494-496 (2001)).

Because both the overproduction and missense mutations of FGF-23 cause hypophosphatemia with renal phosphate wasting, it is concluded that FGF-23 is at least one of the causative factors of OOM and is an important regulator of phosphate and bone metabolism (Shimada T., Proc. Natl. Acad. Sci. USA 98: 6500-6505(2001)). However, the molecular targets of FGF-23 or of FGF-23 proteolytic cleavage products are so far unknown, as is the mechanism of how FGF-23 or FGF-23 derived proteins or peptides cause renal and skeletal abnormalities (Quarles L. D., Am. J. Physiol. Endocrinol. Metab. 285: E1 - 9 (2003)).

The present invention thus relates the use of a polypeptide for the manufacture of a medicament for use in the treatment of a disease associated with deregulated angiogenesis, wherein the polypeptide is selected from the groups consisting of (a) fibroblast growth factor 23 (FGF-23) (SEQ. ID No: 1) or a fragment of FGF-23; (b) a bioactive polypeptide having a percentage of identity of at least 50% with the amino acid sequence of any one of the polypeptides of (a); or (c) a bioactive variant of any one of the polypeptides of (a) or (b).

In a further aspect, the present invention relates to the use of a polypeptide as defined above for the manufacture of a medicament for use in the treatment of a cell proliferative disorder.

In a further aspect, the present invention relates to a method for the treatment of a disease associated with deregulated angiogenesis or of a cell proliferative disorder comprising administering an effective amount of a polypeptide as defined above to a mammal including a human suffering from the disease or disorder.

In another aspect, the present invention relates to a pharmaceutical composition for use in a disease associated with deregulated angiogenesis or a cell proliferative disorder comprising a polypeptide as defined above and a pharmaceutically-acceptable carrier.

### BRIEF DESCRIPTION OF THE DRAWINGS

FIG. 1 is a set of polypeptide sequences and putative polypeptide sequence correlations for GPA018, GPA019, GPA020, GPA022 and GPA023.

FIG. 2 is a box plot of the proliferation scores (Units) relating to vascular changes on retinal flatmounts of animals treated with FGF-23 C-terminal polypeptide (FGF23CTP; (right eye; column 1) and PBS (left eye; columm 2);

### DETAILED DESCRIPTION OF THE INVENTION

*Introduction and overview.* The classical discovery process in the pharmaceutical industry is based on targets (enzymes, receptors, cellular assays, animal and disease models, *etc.*). Chemicals or biological products are tested, in a high throughput mode, on a battery of pre-selected different targets. The weakness of the classical approach are the "artificially disconnected" *in vitro* target models compared to the tightly interconnected and interdependent relationship of the different targets in a whole organism and the fact that biological activity on all non selected targets is missed.

By contrast, the invention is a "non pre-conceived hypothesis" discovery process to rapidly identify and analyze the biological activity of new products in the whole organism, multi-organs and whole transcriptome. All physiological interactions between the different organs or tissues are present and any cellular pathway or any potential targets could potentially be analyzed in a non artificial system.

The drug discovery process of the invention advantageously increases the capabilities in the field of proteomics and functionalization. Proteomics involves the systematic separation, identification and characterization of the proteins present in a sample of tissue, or in a biological fluid, at a given time. All biological processes, including diseases and responses to drugs, induce changes in proteins, and the global protein profile (the "proteome") varies during the development of an organism, maturation of cell types or tissues, and progression or treatment of disease. Each cell type may express different patterns of proteins at different times. Each protein in turn may be modified chemically in an equally diverse number of ways to serve different cellular functions. As proteins derived from the same gene can be largely identical, and might differ only in small but functionally relevant details, protein identification tools not only identify a large number of proteins but also differentiate between close relatives.

The classical proteomics approach combines high resolution two-dimensional gel electrophoresis (2-DGE) with imaging software to quantitatively and qualitatively screen for proteins that differ in abundance, molecular weight (Mr) or charge between the gels. These protein differences can then be identified with high speed and sensitivity by using acombination of "state-of-the-art" mass spectrometry (MS) approaches and robotics, alongside sensitive bioinformatics search tools.

RNA transcripts represent the intermediate form between the DNA and the proteins that are among the most active molecules involved in the cellular functions. The total content of RNA is called the "transcriptome". The high-density DNA chip technology gives potential access to the analysis of all the transcripts produced by a cell population or tissue at any determined time point. Genome-scale RNA expression analysis can thus provide new insights into the cellular events induced upon administration of an animal with peptides or other chemicals. This provides a broad view of the metabolic, signalling, regulatory or other biochemical pathways in the animal being tested. The analysis of the induced perturbations in cellular transcription gives a detailed molecular description of the activity of the administered compound.

An analysis of a transcriptome has become an approachable reality with the implementation of high throughput RNA quantification system. The high-density microarrays allow collecting thousands of information points of a transcriptome at once, reaching the order of magnitude of the probable number of genes expressed and producing a broad and detailed view of the cellular events.

As the changes of the different functions inside a cell are tightly interconnected, the changes in different organs inside an organism are linked. Applying gene profiling to different organs submitted to the same treatment gives a complete overview of the effects and modifications of the physiological status. The identification of common changes in organs with originally very different transcriptomes facilitates the elimination of the experimental noise. The presence or absence of identical signals can indicate if the treatment has a pleiotropic effect or is affecting a target organ. If a compound is targeting a primary organ, the other organs will reflect the functional modifications of the first organ impacted. This type of information can be collected in correlation to the pharmacological effect or to the potential toxic effects. The organism-wide pattern of expression changes can also provide useful information on the pharmacodynamic of the compound, precisely delineating the range of organs affected.

The accumulation of information in different organs not only helps to elucidate the precise mode of action but also provides a complete reconstruction of the compound-induced modifications at the organism scale.

*Administration of compounds.* Administration of the protein or other drug compound tiggers multiple cascades of intracellular signalling events, involving complex networks (pathways) and relying on protein modifications such as phosphorylation, glycosylation, *etc.* These events eventually lead to modifications of gene expression levels. Administration of an active compound therefore leads to multiple and interdependent changes in the composition of the transcriptome.

In one embodiment, the test animal is a vertebrate. In a particular embodiment, the vertebrate is a mammal. In a more particular embodiment, the mammal is a primate, such as a cynomolgus monkey or a human. As used herein, the administration of an agent or drug to a subject or patient includes self-administration and the administration by another.

In more particular embodiments, natural or synthetic substances of biological or non-biological sources, *e.g.* amino acids, peptides, proteins, nucleotides, cDNAs, chemicals, can be administered to animals, *e.g.* mice *(Mus musculus),* rat *(Rattus norvegicus),* monkey *(Macaca fascicularis),* by methods known in the art, *e.g.* by injection, inhalation, or oral administration. Administration of those substances can be adjusted in terms of time of exposure and dosage, and combinations thereof. The "treatment group" of animals should receive a substance or a combination of substances in a vehicle compound suitable for administration of the substance or the combination of substances, while the "control" (or "baseline") group should receive the vehicle compound only. During the treatment period biological specimen such as tissue pieces (*e.g.* obtained by biopsy), or body fluids, such as blood, urine, or saliva, can be sampled. At the end of the treatment time all animals of all groups can be sacrificed and biological specimen such as whole organs or pieces thereof can be sampled. All sampled specimen can be stored as known in the art for further analysis that include, but are not limited to, RT-PCR, Northern blotting, in-situ hybridization, gene expression profiling with microarrays.

As used herein, "direct administration" is the injecting, oral gavage, feeding or other administration of a compound, such as a protein, into animals. After some time, *i.e*. hours, days or weeks, organs and tissues are collected from the animals and the geneexpression profiles determined. This procedure is commonly used in pharmacotoxigenomics, pharmacogenomics and the like.

In one embodiment, the invention begins with differentially expressed proteins in plasma between normal subjects and coronary artery diseased patients with regard to the identification and validation of potential targets and the identification of biomarkers.

The drug discovery process of the invention is particularly amenable to the analysis of the smaller proteins of a proteome (ranging from 0.5 to 20 kDa) escaping the classical detection methods. Small molecular weight proteins can be readily synthesized by commercial methods (*e.g.*, Microprot™ method, GeneProt, Geneva, Switzerland). Chemically-synthesized proteins can be rapidly produced and do not contain biological contaminants.

For mice, a minimal amount of the compound to be functionalized (only *ca.* 5 mg) is used.

As used herein, "indirect administration" is the injecting of a gene that codes for that protein (as a cDNA plasmid) and then doing the gene expression profiling. In one embodiment, the technology is the use of 'naked' DNA (a cDNA expression plasmid) injected into mice (or other animals). This technique is widely published for either DNA immunization (Kim J-M et al., Gene Ther. 10(15): 1216-24 (August 2003)) and delivery of genes for therapeutic purposes (Aliño SF et al., Gene Ther. 10(19):1672-9 (September 2003)). Among a number of techniques for gene transfer *in vivo,* intravenous injection or the direct injection of plasmid DNA into muscle are simple, inexpensive, and safe. Kim J-M et al., Gene Ther. 10(15): 1216-24 (August 2003). The important efficacy of nonviral genomic DNA opens a new avenue in the safety applications of human gene therapy. Aliño SF et al., Gene Ther. 10(19):1672-9 (September 2003).

Administration of naked DNA can be by methods known to those of skill in the art, see, U.S. Pat. Nos. 6,165,754; 6,309,370; 6,566,342; 6,620,617 and 6,651,655, and references cited therein.

*Gene expression profiles.* After a period of time (*e.g.,* two weeks) of protein administration, the treated animals are necropsied. Selected tissues (*e.g.*, 25 tissues for mice/ 120 tissues for monkeys) are dissected and rapidly snap-frozen for genomics analysis. Organ samples (*e.g.*, fifty organs samples for monkeys) can be isolated for histopathological examinations and for gene expression localizations, such as by *in situ* hybridization. Initial studies have shown that for mice, 3-10 tissues out of twenty five sampled tissues are generally sufficient to characterize a compound by gene expression and - hybridization; for monkeys, twenty tissues out of 120 sampled tissues are generally sufficient.

In more particular embodiments, the methods of detecting the level of expression of mRNA are well-known in the art and include, but are not limited to, reverse transcription PCR, real time quantitative PCR, Northern blotting and other hybridization methods. A particularly useful method for detecting the level of mRNA transcripts obtained from a plurality of genes involves hybridization of labelled mRNA to an ordered array of oligonucleotides. Such a method allows the level of transcription of a plurality of these genes to be determined simultaneously to generate gene expression profiles or patterns.

As used herein, a gene expression profile is diagnostic when the increased or decreased gene expression is an increase or decrease (*e.g.*, at least a 1.2-fold difference) over the baseline gene expression following administration of a compound. As used herein, a gene expression pattern is "higher than normal" when the gene expression (*e.g.*, in a sample from a treated subject) shows a 1.2-fold difference (*i.e.*, higher) in the level of expression compared to the baseline samples. A gene expression pattern is "lower than normal" when the gene expression (*e.g.*, in a sample from a treated subject) shows a 1.2-fold difference (*i.e.*, lower) in the level of expression compared to the baseline samples. In other embodiments, a 1.5-fold change may be used as the criteria.

Techniques for the detection of gene expression of the genes described by this invention include, but are not limited to northern blots, RT-PCT, real time PCR, primer extension, RNase protection, RNA expression profiling and related techniques. Techniques for the detection of gene expression by detection of the protein products encoded by the genes described by this invention include, but are not limited to, antibodies recognizing the protein products, western blots, immunofluorescence, immunoprecipitation, ELISAs and related techniques. These techniques are well known to those of skill in the art. Sambrook J et al., Molecular Cloning: A Laboratory Manual, Third Edition (Cold Spring Harbor Press, Cold Spring Harbor, 2000). In one embodiment, the technique for detecting gene expression includes the use of a gene chip. The construction and use of gene chips are well know in the art. See, U.S. Pat Nos. 5,202,232; 5,445,934; 5,525,464; 5,695,940; 5,744,305; 5,795,716 and 5,800,992. See also, Johnston, M. Curr Biol 8:K171-174 (1998), Iyer VR et al., Science 283:83-8T (1999) and Elias P, "New human genome 'chip' is a revolution in the offing" Los Angeles Daily News (October 3, 2003):

Gene expression profiles can be generated using *e.g.* the Affymetrix microarray technology. Briefly, total or, preferably, polyA+-RNA from a biological sample is extracted using standard procedures known in the art, *e.g.* the RNeasy® kit (Qiagen, MD, USA). In a following step, double stranded cDNA is prepared in a process termed "reverse transcription (RT)" which is known in the art, using e.g. the "SuperScript Double-Stranded cDNA Synthesis Kit" (Invitrogen, CA, USA). In a subsequent step, termed "in-vitro transcription", double stranded cDNA obtained in a previous step is labelled with a fluorochrome by methods known in the art, using e.g. the ENZO Labeling Kit (ENZO, NY, USA). Labelled RNA is hybridized to oligonucleotide microarrays. These are known in the art and consist of a surface to which probes that correspond in sequence to gene products (*e.g.* mRNAs, polypeptides, fragments thereof etc.) can be specifically hybridized or bound to a known position. Processing of the microarrays, including e.g. washing, staining, scanning, is performed according to the manufacturer's instructions. Hybridization intensity data detected by the scanner are automatically acquired and processed by analytical software components, *e.g.* the GENECHIP® software (Affymetrix, CA, USA). Raw data is normalized to expression levels using a target intensity of 200.

Two elements of value in expression profiling are the quality and homogeneity of the tissue samples and the mRNA quality. For this purpose, the location of tissues to be sampled and each sample can be carefully dissected from the other surrounding tissues using a binocular microscope.

The samples are then transferred to a molecular biology laboratory for RNA extraction. The protocol for RNA extraction can be partially automated thus increasing the reproducibility and speed of this step. The extracted RNA can be stored for long periods of time in a frozen state and kept as an archive.

An aliquot of the extracted RNA is reverse transcribed to obtain a cDNA. In a second step, cDNA is transcribed in the presence of a fluorescent label to obtain cRNA. The composition of the cRNA obtained is identical to the original composition of the RNA in the samples, but each molecule now carries a fluorescent marker. The labelled mixture of cRNA is used for the hybridization process, *e.g.* using GeneChip® assays (Affymetrix, Santa Clara, California USA). The raw data (obtained after laser-scanning of the chip) are processed by a specific algorithm condensing for each gene all available information in a unique value. This value called average difference represents the level of expression of the gene.

The information can be further refined by the use of complementary techniques. *In situ* hybridization, for example, can indicate precisely which cell type inside an organ is specifically expressing a given gene. This technique based on the detection of RNA is independent of the availability of an antibody. Quantitative PCR may also be used to confirm expression levels of particular genes of interest.

*Analysis.* Mathematical and statistical processing of the data (clustering) help to reduce the complexity and the size of the data sets. Different types of clustering can be used to separate the different genes according to their behavioural similarity across the different conditions and to establish links between genes that may be related to the same biological phenomenon. Data processing also includes statistical tests to separate significant variations from experimental noise. However, the stringency of the various filtering steps must be modulated to integrate the biological nature of the data.

The list of different affected genes is then compared to the information collected in the scientific literature. The synthesis of the available knowledge related to the different genes, points to one or several signalling, metabolic or other biochemical pathways or to known modifications. Once a coherent picture has been reconstructed, the profiles may be associated with potential indications. The discrimination between the different hypotheses follows a process closely related to differential diagnosis.

During the analysis, a constant comparison between the expression data and the current knowledge on cell signalling and regulation is established. Such a permanent bridge provides an efficient way to refine the existing models, particularly in the field of intra- and inter-cellular signalling. The interdependence of gene expression changes is assessed in different organs and under different stimuli. New players in the pathways can be identified and the link between the already described players can be refined. Even if only a part or the cellular regulation depends on the RNA' expression changes, the accumulation of expression data can help to build new and more accurate model of the cell functions. The information collected could help to identify the critical elements of the pathways to be used as target or biomarker.

Some of the expression profiles can be easily matched with existing information harvested from the general scientific knowledge. Linking this information with a potential indication or a potential side effect is then straightforward. Some combinations of expression changes are more difficult to translate into pharmacological information. In such cases, matching of the RNA expression profile of an unknown compound to the profile of a reference drug or disease may facilitate the interpretation. It may then not be necessary to reconstruct the entire cellular modifications to find a potential indication. The reference drugs and disease profiling will also help to build the critical mass of information into the database.

In more particular statistical analysis embodiments, microarray datasets can be analyzed by the use of analytical software components, such as GeneSpring® (Silicon Genetics, CA, USA). Microarray datasets consist in part of probe set identifiers that refer to an oligonucleotide sequence that is bound to the glass slide and to which a labelled cDNA (see above) with complementary sequence binds if it is present in the tissue or body fluid sample. The scanned intensity of the signal that is detected and converted into numeric values by a software, for example MAS5 (Affymetrix, CA, USA), is an indirect measure for the amount, or expression level of the cDNA present in the biological samples under investigation. The entity of gene expression levels as indicated by signal intensity values for all probe sets in a microarray dataset of a biological sample can be referred to as expression profile of that sample. In each microarray dataset, signal intensity values, cDNA or gene annotations, as well as quality parameters that can be created by software, for example MAS5 (Affymetrix, CA, USA), are informational results associated with the probe set ID.

In the cDNA microarray system, expressions of genes from the experimental cells of interest are measured relative to the expressions of the same genes in a fixed reference or control cell type. To identify statistically relevant effects of a substance on the expression profile of samples or tissue or body fluid under investigation, probe sets can be filtered based on the associated values given by the software used to create the values, for example MAS5 (Affymetrix, CA, USA). Filters can be based on quality parameters, expression level, changes of expression levels in the samples from treated versus control specimen, as well as significance. The resulting list of probe sets refers to such genes that experience a significant change in their expression level as a direct or indirect result of the treatment of the biological samples they are derived from.

The interpretation of such gene lists with regards to effects of a substance on biological systems and pathways is subject to the investigators knowledge and experience. Application of analytical software components such as GeneSifter® (VizXLabs, Seattle, WA, USA) assists in the interpretation of such gene lists.

Moreover, we have developed software for a multiorgan analysis of the data generated by the method of the invention, which compares the compound signatures produced by the administration of the compound among the different organs.

*New uses identified by the drug discovery process of the invention.* The present invention also provides for the use of a polypeptide for the manufacture of a medicament for use in the treatment of a disease associated with deregulated angiogenesis, wherein the polypeptide is selected from the groups consisting of (a) fibroblast growth factor 23 (FGF-23) (SEQ. ID No: 1) or a fragment of FGF-23; (b) a bioactive polypeptide having a percentage of identity of at least 50% with the amino acid sequence of any one of the polypeptides of (a); or (c) a bioactive variant of any one of the polypeptides of (a) or (b).

According to another aspect, the present invention provides for the use of a polypeptide as defined above for the manufacture of a medicament for use in the treatment of a cell proliferative disorder.

The term "polypeptide" as used herein, refers to a protein, peptide, oligopeptide or synthetic oligopeptide. These terms are intended to be used interchangeably. Any one of said terms refers to a chain of two or more amino acids which are linked together with peptide or amide bonds, regardless of post-translational modification such as glycosylation or phosphorylation. The polypeptides may also comprise more than one subunit, where each subunit is encoded by a separate DNA sequence.

The term "bioactive", as used herein, refers to a molecule that elicits or affects a biological event. Such biological event may for example be related to a disease associated with deregulated angiogenesis or to a cell proliferative disorder.

A "bioactive polypeptide" of the invention includes FGF-23, fragments of FGF-23 such as fragments derived from the C-terminus of FGF-23 . Also included are homologs which have an amino acid sequence having a percentage of identity of at least 50% to FGF-23 or fragments thereof and variants of FGF-23 or of FGF-23 fragments. The polypeptide according to the invention may comprise FGF-23 having the amino acid sequence of SEQ ID NO: I. A fragment of FGF 23 may comprise at least 10 amino acids, preferably at least 15, 20, 25 or 30 amino acids. More preferably a fragment of FGF-23 may comprise at least 50, 60, or 70 amino acids. Most preferably a fragment of FGF-23 comprises 75 amino acids. Alternatively, a fragment of FGF-23 may comprise at least 80 or 100 amino acids, and most preferred at least 120 or 150 amino acids. In particular, the fragment may comprise at least 180 amino acids, such as e.g. 200 amino acids.

Such polypeptide may also be a proteolytic cleavage product of FGF-23 generated by proteases such as a membrane-bound endopeptidase including PHEX. A polypeptide according to the invention may comprise a C-terminal fragment of FGF-23. Such C-terminal fragment may comprise at least 15 amino acids of the C-terminus of FGF-23, preferably at least 25, at least 35 or 45, more preferably at least 55 or at least 65, most preferred at least 70, such as e.g. 75 amino acids. The at least 15 amino acids may comprise the most C-terminal at least 15 amino acids, it may also comprise the at least 15 amino acids within the C-terminal part of FGF-23. The polypeptide may comprise the at least 75 most C-terminal amino acids of FGF-23 and it may have the amino acid sequence of SEQ ID NO: 2, designated FGF-23 C-terminal polypeptide (FGF23CTP).

The polypeptide may also have an amino acid sequence having a percentage of identity of at least 50%, preferably at least 60%, more preferred at least 70% or 80%, and most preferably at least 90% such as 95%, 97%, or 99% identity with the amino acid sequence of any one of the aforementioned polypeptides.

The bioactive polypeptides of the present invention as described above may also be referred to as FGF-23, FGF-23 protein or polypeptide, FGF-23 derived or related polypeptides, FGF-23 C-terminal derived or related polypeptides, or FGF-23 C-terminal Polypeptides.

Amino acid residues are referred to herein by their standard single-letter or three-letter notations:A (Ala) alanine; C (Cys) cysteine; D (Asp) aspartic acid; E (Glu) glutamic acid; F (Phe) phenylalanine; G (Gly) glycine; H (His) histidine; I (Ile) isoleucine; K (Lys) lysine; L (Leu) leucine; M (Met) methionine; N (Asn) asparagine; P (Pro) proline; Q (Gln) glutamine; R (Arg) arginine; S (Ser) serine; T (Thr) threonine; V (Val) valine; W (Trp) tryptophan; Y (Tyr) tyrosine.

The term "percentage (%) of identity", or like term, used in respect of the comparison of a reference sequence and another sequence (i.e. a "candidate" sequence), means that in an optimal alignment between the two sequences, the candidate sequence is identical to the reference sequence in a number of subunit positions equivalent to the indicated percentage, the subunits being nucleotides for polynucleotide comparisons or amino acids for polypeptide comparisons. As used herein, an "optimal alignment" of sequences being compared is one that maximizes matches between subunits and minimizes the number of gaps employed in constructing an alignment. Percent identities may be determined with commercially available implementations of algorithms described by Needleman and Wunsch, J. Mol. Biol. 48: 443-453 (1970)("GAP" program of Wisconsin Sequence Analysis Package, Genetics Computer Group, Madison, WI). Other software packages in the art for constructing alignments and calculating percentage identity or other measures of similarity include the "BestFit" program, based on the algorithm of Smith and Waterman, Advances in Applied Mathematics 2: 482-489 (1981) (Wisconsin Sequence Analysis Package, Genetics Computer Group, Madison, WI). The percentage of identity may also be generated by WU-BLAST-2 (Altschul et al., Methods in Enzymology 266: 460-480 (1996)). WU-BLAST-2 used several search parameters, most of which are set to the default values. The adjustable parameters are set with the following values: overlap span = 1, overlap fraction = 0.125, word threshold (T) = 11. A % amino acid sequence identity value is determined by the number of matching identical residues divided by the total number of residues in the aligned region. For example, to obtain a polypeptide having an amino acid sequence at least 95% identical to a reference amino acid sequence, up to five percent of the amino acid residues in the reference sequence may be deleted or substituted with another amino acid, or a number of amino acids up to five percent of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence of in one or more contiguous groups with in the references sequence. It is understood that in making comparisons with reference sequences of the invention that candidate sequence may be a component or segment of a larger polypeptide or polynucleotide and that such comparisons for the purpose computing percentage identity is to be carried out with respect to the relevant component or segment.

A polypeptide of the invention also includes a polypeptide fragment of a polypeptide of the invention. Such polypeptide fragment is meant to be a polypeptide having an amino acid sequence that entirely is the same in part, but not in all, of the amino acid sequence of a polypeptide of the invention. Such polypeptide fragment may be "freestanding," or may be part of a larger polypeptide of which such polypeptide fragment forms a part or region, most preferably as a single continuous region. Preferably such polypeptide or polypeptide fragment retains the biological activity of the corresponding polypeptide of the invention.

The invention also includes functionally preserved variants of the polypeptides or polypeptide fragments described herein. Such variants may be made using methods standard in the art, for example, by conservative amino acid substitutions. Typically such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5 to 10, 1 to 5, or 2 amino acids are substituted, deleted or added, in any combination.

In various other embodiments, the polypeptide (fragment) or polypeptide variant may be linear or branched, it may comprise modified amino acids, it may be interrupted by non-amino acids, and/or it may be assembled into a complex of more than one polypeptide chain. As is well understood in the art, a polypeptide may be modified naturally or by intervention; for example, disulfide bond formation, glycosylation, lipidation, acetylation phosphorylation, or any other manipulation or modification, such as conjugation with a labeling component. In some embodiments polypeptides or polypeptide fragments contain one or more analogs of an amino acid (including; for example, unnatural amino acids, etc.), as well as other modifications known in the art.

A polypeptide or a polypeptide fragment of the invention includes isolated naturally occurring polypeptides. Preferably, such a naturally occurring polypeptide has a frequency in a selected population of at least five percent, and most preferably, of at least ten percent. The selected population may be any recognized population of study in the field of population genetics. Preferably, the selected population is Caucasian, Negroid, or Asian. More preferably, the selected population is French, German, English, Spanish, Swiss, Japanese, Chinese, Korean, Singaporean of Chinese ancestry, Icelandic, North American, Israeli, Arab, Turkish, Greek, Italian, Polish, Pacific Islander, or Indian.

A polypeptide (fragment) of the invention may also include recombinantly produced polypeptides, synthetically produced polypeptides and a combination of such polypeptides of the invention, and fragments thereof. Means for preparing such polypeptides are well understood in the art. For instance, a polynucleotide fragment or a polypeptide of the invention can be isolated from body fluids including, but not limited to, serum, urine, and ascites, or synthesized by chemical or biological methods (for example, cell culture, recombinant gene expression). "Isolated", if not otherwise specified herein includes the meaning "separated from coexisting material".

Recombinant polypeptides of the present invention may be prepared by processes well known in the art from genetically engineered host cells comprising expression systems. Accordingly, in a further aspect, the present invention relates to the production of polypeptides by recombinant techniques, to expression system which comprises a nucleic acid or nucleic acids encoding the polypeptides of the present invention, to host cells which are genetically engineered with such expression systems, and to methods to isolate the polypeptides.

Another embodiment provides that a polypeptide of the invention is encoded by a nucleic acid which hybridizes under stringent conditions to SEQ. ID No: 3 or to SEQ. ID No:4. In some embodiments, the nucleic acid comprises at least 50, at least 75, at least 100, at least 125, or at least 150 nucleotides. Preferably the nucleic acid comprises at least 175 or ar least 200 nucleotides. In particular it comprises 225 or 228 nucleotides. The nucleic acid may also comprise at least 300, or at least 400 or 500 nucleotides. Preferably it may comprise at least 600 or at least 700 nucleotides. Most preferably it comprises at least 750 nucleotides. Such nucleic acids may comprise contiguous nucleotides of SEQ ID No: 3 or 4 or contiguous nucleotides able to hybridize to SEQ ID NO: 3 or 4 under stringent conditions.

The term "nucleic acid" means natural or semi-synthetic or synthetic or modified nucleic acid molecules. It refers to nucleotide sequences, oligonucleotides or polynucleotides including deoxyribonucleic acid (DNA) and/or ribonucleic acid (RNA) and/or modified nucleotides. These terms are intended to be used interchangeably. RNA may be in the form of an tRNA (transfer RNA), snRNA (small nuclear RNA), rRNA (ribosomal RNA), mRNA (messenger RNA), anti-sense RNA, and ribozymes. DNA may be in form of plasmid DNA, viral DNA, linear DNA, chromosomal or genomic DNA, cDNA, or derivatives of these groups. In addition these DNAs and RNAs may be single, double, triple, or quadruple stranded. The term also includes PNAs (peptide nucleic acids), phosphorothioates, and other variants of the phosphate backbone of native nucleic acids.

"Stringent conditions" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends upon the ability of a denatured nucleic acid to reanneal when complementary strands are present in an environment near but below their melting temperature. The higher the degree of homology between the probe and the hybridizable sequence such as SEQ. ID No: 3 or 4, the higher the relative temperature which can be used. As a result, it follows that higher relative temperatures would tend to make the reaction conditions more stringent, while lower temperatures less so. Moreover, stringency is also inversely proportional to salt concentrations. "Stringent conditions" are exemplified by reaction conditions characterized by: (1) low ionic strength and high temperature for washing, for example 0.015 M sodium Chloride/0.0015 M sodium citrate/0.1 % sodium dodecyl sulfate at 50°C; (2) the use of a denaturing agent, such as formamide, for example, 50% (vol/vol) formamide with 0.1% bovine serum albumin/0.1 % Ficoll/0.1% polyvinylpyrrolidone/50 mM sodium phosphate buffer at pH 6.5 with 750 mM sodium chloride, 75 mM sodium citrate at 42°C. Alternatively, stringent conditions can be: 50% formamide, 5x SSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42°C, with washes at 42°C in 0.2x SSC (sodium chloride/sodium citrate) and 50% formamide at 55°C, followed by a high-stringency wash consisting of 0.1x SSC containing EDTA at 55°C. For additional details and explanation of stringency of hybridization reactions, see Ausubel et al., Protocols in Molecular Biology (1995).

*Recombinant Manufacture of FGF-23 C-terminal polypeptides.* The nucleic acids described herein such as SEQ. ID No: 3 or 4 may be used in recombinant DNA molecules to direct the expression of the corresponding polypeptides in appropriate host cells. Because of the degeneracy in the genetic code, other DNA sequences may encode the equivalent amino acid sequence, and maybe used to clone and express FGF-23 or fragments thereof. Codons preferred by a particular host cell may be selected and substitued into the naturally occuring nucleotide sequences, to increase the rate and/or efficiency of expression. The nucleic acid (e.g., cDNA or genomic DNA) encoding the desired FGF-23 or FGF-23 fragments such as FGF23CTP may be inserted into a replicable vector for cloning (amplification of the DNA), and/or for expression.

*Expression Systems.* The polypeptide can be expressed recombinantly in any of a number of expression systems according to methods known in the art (Ausubel, et al., editors, Current Protocols in Molecular Biology, John Wiley Sons, New York, 1990). Such expression systems include chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids.

The expression systems may contain control regions that regulate as well as engender expression. Generally, any system or vector which is able to maintain, propagate or express a nucleic acid to produce a polypeptide in a host may be used. The appropriate nucleotide sequence may be inserted into an expression system by any of a variety of well-known and routine techniques, such as, for example, those set forth in Sambrook et al., Molecular Cloning: A Laboratory Manual, 2nd Ed., Cold Spring Harbor Laboratory Press, Cold Spring Harbor, N.Y. (1989). In general, DNA is inserted into an appropriate restriction endonuclease site using techniques known in the art.

Vector components generally include, but are not limited to, one or more of an origin of replication, one or more marker genes, an enhancer element, a promoter, a signal or secretion sequence, and a transcription termination sequence:

The expression vector may have two replication systems, thus allowing it to be maintained in two organisms, for example in mammalian or insect cells for expression and in a prokaryotic host for cloning and amplification. Such sequences are well known for a variety of bacteria, yeast strains, and viruses.

Preferably, the expression vector contains a marker gene to allow the selection of transformed host cells. Selection genes are well known in the art and will vary with the host cell used**_**Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients e.g., the D-alanine racemase gene.

Promoter sequences encode either constitutive or inducible promoters. The promoters may be either naturally occurring promoters or hybrid promoters. Hybrid promoters, which combine elements of more than one promoter, are also known in the art, and are useful in the present invention. Further, for integrating expression vectors, the expression vector contains at least one sequence homologous to the host cell genome, and preferably, two homologous sequences which flank the expression construct. The integrating vector may be directed to a specific locus in the host cell by insertion of the appropriate homologous sequence in the vector. Constructs for integrating vectors are well known in the art.

An appropriate secretion signal may be incorporated into the desired polypeptide to allow secretion of the polypeptide into the lumen of the endoplasmic reticulum, the periplasmic space or the extracellular environment. These signals may be endogenous to the polypeptide or they may be heterologous signals. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, the alpha factor leader (including Saccharomyces and Kluyveromyces α-factor leaders). In mammalian cell expression systems, mammalian signal sequences from secreted polypeptides of the same or related species, as well as viral secretory leaders may be used to direct secretion of FGF-23 or fragments thereof such as FGF23CTP.

Appropriate host cells include yeast, bacteria, archebacteria, fungi, and insect and animal cells, including mammalian cells, for example primary cells, including but not limited to stem cells. Representative examples of appropriate hosts include bacterial cells, such as E. coli. Streptococci, Staphylococci, Streptomyces, and Bacillus subtilis, fungal cells, such as Saccharomyces cerevisiae, other yeast cells orAspergillus; insect cells such as Drosophila S2 and Spodoptera Sf9 cells; animal cells such as CHO, COS, HeLa, C127, 3T3, BHK, HEK 293 and Bowes melanoma cells, and plant cells.

A host cell strain may be chosen for its ability to modulate the expression of the inserted sequences or to process the expressed polypeptide in the desired fashion. Such modifications of the polypeptide include, but are not limited to, acetylation, carboxylation, glycosylation, phosphorylation, lipidation and acylation. Post-translational processing, which cleaves a "prepro" form of the polypeptide, may also be important for correct insertion, folding and/or function.

FGF-23 or fragments thereof such as FGF23CTP may be produced by culturing a host cell transformed with an expression vector containing a nucleic acid encoding an FGF-23 or fragments thereof under the appropriate conditions to induce or cause expression of the protein or polypeptide. In a preferred embodiment of the invention a host cell is provided which is stably or transiently transfected with a nucleic acid of SEQ. ID No: 3 or 4 or transfected with a nucleic acid which hybridizes under stringent conditions to SEQ. ID No: 3 or 4. According to another embodiment of the invention said host cell is cultured to allow expression of FGF-23 or of an FGF-23 fragment, and the polypeptide is isolated from the cell culture.

Transformed host cells include, but are not limited to, microorganisms such as bacteria transformed with recombinant bacteriophage, plasmid or cosmic DNA expression vectors, yeast transformed with yeast expression vectors, and insect cells infected with a recombinant insect virus (such as baculovirus), and mammalian expression systems.

The appropriate conditions for expression of FGF-23 or fragments thereof such as FGF23CTP will vary with the choice of the expression vector and the host cell, and will be easily ascertained by one skilled in the art through routine experimentation. For example, the use of constitutive promoters in the expression vector will require optimizing the growth and proliferation of the host cell, while the use of an inducible promoter requires the appropriate growth conditions for induction. In addition, in some embodiments, the timing of the harvest is important. For example, the baculoviral systems used together with insect cells are lytic viruses, and thus harvest time selection can be crucial for product yield,

The desired FGF-23 or FGF-23 fragment may be produced recombinantly not only directly, but also as a fusion polypeptide with a heterologous polypeptide. Such heterologous polypeptide is generally placed at the amino- or carboxyl-terminus of FGF-23 or of an FGF-23 fragment and may provide for an epitope tag to which an anti-tag antibody can selectively bind. Accordingly, such epitope tag enables FGF-23 or a fragment thereof to be readily purified by using an anti-tag antibody or another type of affinity matrix that binds to the epitope tag. Examples of epitope tags are 6xHis or c-myc tag. Alternatively FGF-23 or a fragment thereof may be expressed in the form of e.g. an GST-fusion protein. Appropriate constructs are generally known in the art and are available from commercial suppliers such as Invitrogen (San Diego, Calif.), Stratagene (La Jolla, Calif.), Gibco BRL (Rockville, Md.) or Clontech (Palo Alto, Calif.).

*Evaluation of Gene Expression.* Gene expression may be evaluated in a sample directly, for example, by standard techniques known to those of skill in the art, e.g., Southern blotting for DNA detection, Northern blotting to determine the transcription of mRNA, dot blotting (DNA or RNA), or in situ hybridization, using an appropriately labeled probe, based on the sequences provided herein. Alternatively, antibodies may be used in assays for detection of nucleic acids, such as specific duplexes, including DNA duplexes, RNA duplexes, and DNA-RNA hybrid duplexes or DNA-protein duplexes. Such antibodies may be labeled and the assay carried out where the duplex is bound to a surface, so that upon the formation of duplex on the surface, the presence of antibody bound to the duplex can be detected. Gene expression, alternatively, may be measured by immunohistochemical staining of cells or tissue sections and assay of cell culture or body fluids, to directly evaluate the expression of FGF-23 or of an FGF-23 fragment. Antibodies useful for such immunological assays may be either monoclonal or polyclonal, and may be prepared against a native sequence FGF-23 or FGF-23 fragments based on the DNA sequences provided herein.

*Purification of Expressed Protein.* Expressed FGF-23 or an FGF-23 fragment such as FGF23CTP may be purified or isolated after expression, using any of a variety of methods known to those skilled in the art. The appropriate technique will vary depending upon the way of expression of FGF-23 or an FGF-23 fragment. The polypeptide may for example be recovered from culture medium in the form of a secreted potein or from host cell lysates. Cells can be disrupted by various physical or chemical means, such as freeze thaw cycling, sonication, mechanical disruption, or by use of cell lysing agents, whereas membrane-bound polypeptides may be released from the membrane using a suitable detergent solution (e.g. Triton-X100) or by enzymatic cleavage. The appropriate technique for polypeptide purification or isolation will also vary depending upon what other components are present in the sample. The degree of purification necessary will also vary depending on the use of FGF-23 or a fragment thereof. Contaminant components that are removed by isolation or purification are materials that would typically interfere with diagnostic or therapeutic uses for the polypeptide, and may include enzymes, hormones, and other solutes. The purification step(s) selected will depend, for example, on the nature of the production process used and the particular FGF-23 or FGF-23 fragment produced.

Ordinarily, isolated FGF-23 or a fragment thereof will be prepared by at least one purification step. Well-known methods for purification include ammonium sulfate or ethanol precipitation, acid extraction, anion or cation exchange chromatography, phosphocellulose chromatography, hydrophobic interaction chromatography, high performance liquid chromatography, hydroxylapatite chromatography and lectin chromatography. Most preferably, affinity chromatography is employed for purification. For example, the FGF-23 or a fragment thereof such as FGF23CTP may be purified using a standard anti-FGF-23 C-terminal polypeptide antibody column. Ultrafiltration and dialysis techniques, in conjunction with protein concentration, are also useful (see, for example, Scopes, R., Protein Purification, Springer-Verlag, New York, N.Y., 1982). Well-known techniques for refolding proteins may be employed to regenerate active conformation when the polypeptide is denatured during isolation and or purification

*Labeling of Expressed Polypeptide.* The nucleic acids, proteins and antibodies of the invention may be labeled. By labeled herein is meant that a compound has at least one element, isotope or chemical compound attached to enable the detection of the compound. In general, labels fall into three classes: a) isotopic labels, which may be radioactive or heavy isotopes; b) immune labels, which may be antibodies or antigens; and c) colored or fluorescent dyes. The labels may be incorporated into the compound at any position that does not interfere with the biological activity or characteristic of the compound which is being detected.

*Chemical Manufacture of FGF-23 and FGF-23 fragments.* Polypeptides or fragments thereof may be produced not only by recombinant methods, but also by using chemical methods well known in the art. Solid phase peptide synthesis may be carried out in a batchwise or continuous flow process which sequentially adds alpha-amino and side chain-protected amino acid residues to an insoluble polymeric support via a linker group. A linker group such as methylamine-derivatized polyethylene glycol is attached to poly(styrene-co-divinylbenzene) to form the support resin. The amino acid residues are Nalpha-protected by acid labile Boc (t-butyloxycarbonyl) or base-labile Fmoc (9-fluorenylmethoxycarbonyl). The carboxyl group of the protected amino acid is coupled to the amine of the linker group to anchor the residue to the solid phase support resin. Trifluoroacetic acid or piperidine are used to remove the protecting group in the case of Boc or Fmoc, respectively. Each additional amino acid is added to the anchored residue using a coupling agent or pre-activated amino acid derivative, and the resin is washed. The full length peptide is synthesized by sequential deprotection, coupling of derivatized amino acids, and washing with dichloromethane and/or N, N-dimethylformamide. The peptide is cleaved between the peptide carboxy terminus and the linker group to yield a peptide acid or amide. (Novabiochem 1997/98 Catalog and Peptide Synthesis Handbook, San Diego Calif. pp. S1-S20). Automated synthesis may also be carried out on machines such as the ABI 431 A peptide synthesizer (Applied Biosystems). A polypeptide or a fragment thereof may be purified by preparative high performance liquid chromatography and its composition confirmed by amino acid analysis or by sequencing (Creighton T.E. (1984) Proteins, Structures and Molecular Properties, W H Freeman, New York N.Y.).

*Variants.* Variants of the natural polypeptide may be desirable in a variety of circumstances. For example, undesirable side effects might be reduced by certain variants, particularly if the side effect activity is associated with a different part of the polypeptide from that of the desired activity. In some expression systems, the native polypeptide may be susceptible to degradation by proteases. In such cases, selected substitutions and/or deletions of amino acids which change the susceptible sequences can significantly enhance yields. Variants may also increase yields in purification procedures and/or increase shelf lives of proteins by eliminating amino acids susceptible to oxidation, acylation, alkylation, or other chemical modifications. Preferably, such variants include alterations that are conformationally neutral, i.e. they are designed to produce minimal changes in the tertiary structure of the variant polypeptides as compared to the native polypeptide, and (ii) antigenically neutral i.e. they are designed to produce minimal changes in the antigenic determinants of the variant polypeptides as compared to the native polypeptide.

*Manufacture of a medicamen for use in the treatment of a disease.* The aforementioned polypeptides may according to the invention be used for the manufacture of a medicament for use in the treatment of a disease associated with deregulated angiogenesis or a cell proliferative disorder.

The term "treatment" refers to both therapeutic treatment and prophylactic or preventative measures. Those in need of treatment include those already with the disorder as well as those in which the disorder is to be prevented.

A "disorder" or a "disease" is any condition that would benefit from treatment with FGF-23 or a fragment of FGF-23 as defined above and further below. This includes both chronic and acute disorders, as well as those pathological conditions which predispose to the disorder or disease in question. Non-limiting examples of disorders or diseases to be treated herein include any condition which results from deregulated angiogenesis or from deregulated cell proliferation. Examples of diseases associated with deregulated angiogenesis include: ocular neovascularisation, such as retinopathies (including diabetic retinopathy), age-related macular degeneration, psoriasis, haemangioblastoma, haemangioma, arteriosclerosis, inflammatory diseases, such as rheumatoid or rheumatic inflammatory diseases, especially arthritis, such as rheumatoid arthritis, or other chronic inflammatory disorders, such as chronic asthma, arterial or post-transplantational atherosclerosis, endometriosis, and especially neoplastic diseases, for example so-called solid tumors and liquid tumors (such as leukemias).

A preferred example of a diseases associated with deregulated angiogenesis is selected from the group of retinopathies, age-related macular degeneration, haemangioblastoma, haemangioma, and tumors. A particularly preferred example of a diseases associated with deregulated angiogenesis is retinopathy.

Examples of cell proliferative disorders include: chronic or acute renal diseases, e.g. diabetic nephropathy, malignant nephrosclerosis, thrombic microangiopathy syndromes or transplant rejection, or especially inflammatory renal disease, such as glomemloncephritis, especially mesangioproliferative glomerulonephritis, haemolyticuraemic syndrome, diabetic nephropathy, hypertensive nephrosclerosis, atheroma, arterial restinosis, actinic keratosis, arteriosclerosis, atherosclerosis, bursitis, hepatitis, mixed connective tissue disease (MCTD), myelofibrosis, paroxysmal nocturnal hemoglobinuria, polycythemia vera, psoriasis, primary thrombocythemia, and autoimmune diseases, acute inflammation, fibric disorders (e.g. hepatic cirrhosis), diabetes, endometriosis, chronic asthma, neurodegenerative disorders and especially neoplastic diseases such as adenocarcinoma, gliomas, leukemia, lymphoma, melanoma, myeloma, sarcoma, Kaposi's sarcoma, teratocarcinoma, and, in particular, cancers of the adrenal gland, bladder, bone, bone marrow, brain, breast, cervix, colon, gall bladder, ganglia, gastrointestinal tract, heart, kidney, liver, lung (especially small-cell lung cancer), muscle, ovary, pancreas, parathyroid, penis, prostate, salivary glands, skin, spleen, testis, thymus, thyroid, and uterus.

A preferred example of a cell proliferative disorder is selected from the group of chronic or acute renal diseases, arteriosclerosis, atherosclerosis, psoriasis, endometriosis, diabetes, chronic asthma and cancer. A particularly preferred example of a cell proliferative disorder is cancer.

Another aspect of the invention relates to a method for the treatment of a disease associated with deregulated angiogenesis which comprises administering an effective amount of a polypeptide to a mammal including a human suffering from the disease, wherein the polypeptide is selected from the groups consisting of (a) FGF-23 (SEQ. ID No: 1) or a fragment of FGF-23; (b) a bioactive polypeptide having a percentage of identity of at least 50% with the amino acid sequence of any one of the polypeptides of (a); or (c) a bioactive variant of any one of the polypeptides of (a) or (b). Accordingly, a polypeptide as described above may be administered.

Another aspect of the invention relates to a method for the treatment of a cell proliferative disorder comprising administering an effective amount of FGF-23 or an FGF-23 fragment such as FGF23CTP as described above to a mammal including a human suffering from the disorder.

"Mammal" for purposes of treatment refers to any animal classified as a mammal, including humans, domestic and from animals, and zoo, sports, or pet animals, such as dogs, horses, cats, sheep, pigs, cattle, etc. Preferably, the mammal is human.

*Pharmaceutical composition.* Another aspect of the invention provides a pharmaceutical composition for use in a disease associated with deregulated angiogenesis or a proliferative disorder comprising FGF-23 or a fragment thereof according to the invention as described above and a pharmaceutically-acceptable carrier. The composition of the invention is administered in effective amounts.

The pharmaceutical composition may be used in the foregoing methods of treatment. Such compositions are preferably sterile and contain an effective amount of FGF-23 or an FGF-23 fragment such as FGF23CTP or a nucleic acid encoding the polypeptide for inducing the desired response in a unit of weight or volume suitable for administration to a patient.

An "effective amount" of FGF-23 or fragment thereof, compound, or pharmaceutical composition is an amount sufficient to effect beneficial or desired results including clinical results such as inhibiting premature or diabetic retinopathy, inhibiting angiogenesis, shrinking the size of the tumor, retardation of cancerous cell growth, decreasing one or more symptoms resulting from the disease or disorder, increasing the quality of life of those suffering from the disease or disorder, decreasing the dose of other medications required to treat the disease or disorder, enhancing effect of another medication, delaying the progression of the disease or disorder, and/or prolonging survival of patients, either directly or indirectly.

Such amounts will also depend on the particular condition being treated, the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation.

An effective amount can be administered in one or more administrations and may or may not be achieved in conjunction with another drug, compound, or pharmaceutical composition. Thus, an "effective amount" may be considered in the context of administering one or more therapeutic agents, and a single agent may be considered to be given in an effective amount if, in conjunction with one or more other agents, a desirable result may be or is achieved.

An effective amount of FGF-23 or an FGF-23 fragment or the pharmaceutical composition comprising the polypeptide of the invention, alone or in conjunction with another drug, compound, or pharmaceutical composition can be administered by any conventional route, including injection or by gradual infusion over time. The administration may, for example, be oral, intravenous, intraperitoneal, intramuscular, intracavity, subcutaneous, topical or transdermal.

When administered, the pharmaceutical composition of the present invention is administered in pharmaceutically acceptable preparations. The term "pharmaceutically-acceptable carrier" as used herein means one or more compatible solid or liquid fillers, diluents or encapsulating substances which are suitable for administration into mammals including humans. The term "carrier" denotes an organic or inorganic ingredient, natural or synthetic, with which the active ingredient is combined to facilitate the application.

The term "pharmaceutically acceptable" means a non-toxic material that does not interfere with the effectiveness of the biological activity of the active ingredients. Such preparations may routinely contain pharmaceutically acceptable concentrations of salts, buffering agents, preservatives, compatible carriers, supplementary immune potentiating agents such as adjuvants and cytokines and optionally other therapeutic agents, such as chemotherapeutic agents.

When used in medicine, the salts should be pharmaceutically acceptable, but non-pharmaceutically acceptable salts may conveniently be used to prepare pharmaceutically-acceptable salts thereof and are not excluded from the scope of the invention.

The pharmaceutical compositions may contain suitable buffering agents, including: acetic acid in a salt; citric acid in a salt; boric acid in a salt; and phosphoric acid in a salt.

The pharmaceutical compositions also may contain, optionally, suitable preservatives, such as: benzalkonium chloride; chlorobutanol; parabens and thimerosal.

The doses of polypeptide or nucleic acid encoding said polypeptide administered to a subject can be chosen in accordance with different parameters, in particular in accordance with the mode of administration used and the state of the subject Other factors include the desired period of treatment. In the event that a response subject is insufficient at the initial doses applied, higher doses (or effectively higher doses by a different, more localized delivery route) may be employed to the extent that patient tolerance permits.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous liquids such as a syrup, elixir or an emulsion.

Compositions suitable for parenteral administration conveniently comprise a sterile aqueous or non-aqueous preparation of a polypeptide or nucleic acid encoding the polypeptide, which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid may be used in the preparation of injectables.

Carrier formulation suitable for oral, subcutaneous, intravenous, intramuscular, etc. administrations can be found in Remington's Pharmaceutical Sciences, Mack Publishing Co., Easton, PA.

The following EXAMPLES are presented in order to more fully illustrate the preferred embodiments of the invention. These EXAMPLES should in no way be construed as limiting the scope of the invention, as defined by the appended claims.

### EXAMPLE I

### INTEGRATED INVESTIGATIVE PHARMACOLOGY THROUGH IN VIVO GENE EXPRESSION PROFILING IN MICE

In this EXAMPLE, 100 unknown chemically synthesized peptides are functionalized using the discovery method of the invention. Most of these peptides are present in human plasma.

As a control, twenty reference drugs are concurrently investigated with the discovery method of the invention. For this screening, one control and four treated groups of six males are treated for two weeks by daily administration by the subcutaneous route of the proteins. The reference drugs can be active for treating conditions in the areas of glaucoma, neuroprotection, neovascularisation, antiangiogenesis, acne, asthma and allergy, cardiovascular diseases, neurological disorder, pain, diabetes, hypercholesterolemia, osteoporosis and oncology.

The expectations from those selected active peptides are that (a) several potential therapeutic drugs could be identified; (b) target peptides for therapeutic antibodies could be identified; (c) new targets for research, deduced from the reconstructed biochemical pathways, could be identified; and (d) biomarkers, to be used to develop diagnostic tests could be identified. Bioinformatic investigations of gene expression in mice suggest therapeutic indications and insight through the analysis of molecular pathways and functions, which allow prioritizing for the verification of selected proteins/peptides in monkeys. The protein/peptide selection rate is approximately 20%. The selection/prioritization criteria are the type of activity, the therapeutic interest and the suspected toxicity.

In an initial analysis, following administration of a peptide (FGF23CTP, *see below,* EXAMPLE III) to mice, transcript level changes were observed in several organs which indicate that the compound is active and impacts on pathways involved in cell differentiation.

### EXAMPLE II

### A 7-DAY PHARMACOLOGY AND TOXICITY STUDY BY SUBCUTANEOUS ROUTE IN MICE; MICROARRAY GENE EXPRESSION ANALYSIS

*Introduction and summary.* Five peptides with unidentified function were tested in mice to obtain biochemical and pharmacogenomic data that would allow a specification of their activity. Outbred CD-1 mice were treated with peptides GPA018, GPA019, GPA020, GPA022, and GPA023 for seven days, observed for clinical signs of treatment effects (mortality, clinical signs, body weight, food consumption, haematology, clinical biochemistry) and, after sacrifice, a selected set of tissues were used for gene expression profiling. A snap freezing sampling of the tissues was performed at necropsy at the end of the treatment period. These tissues were used for mRNA expression profiling and for histopathological analysis (formalin fixation). In addition, parameters investigated in a standard exploratory study were recorded. None of the peptides had any influence on clinical or pharmacogenomic parameters. Gene expression profiling revealed no significant changes between control and treated animals. It was concluded that the peptides were inactive and decided that no further investigations on these peptides would follow.

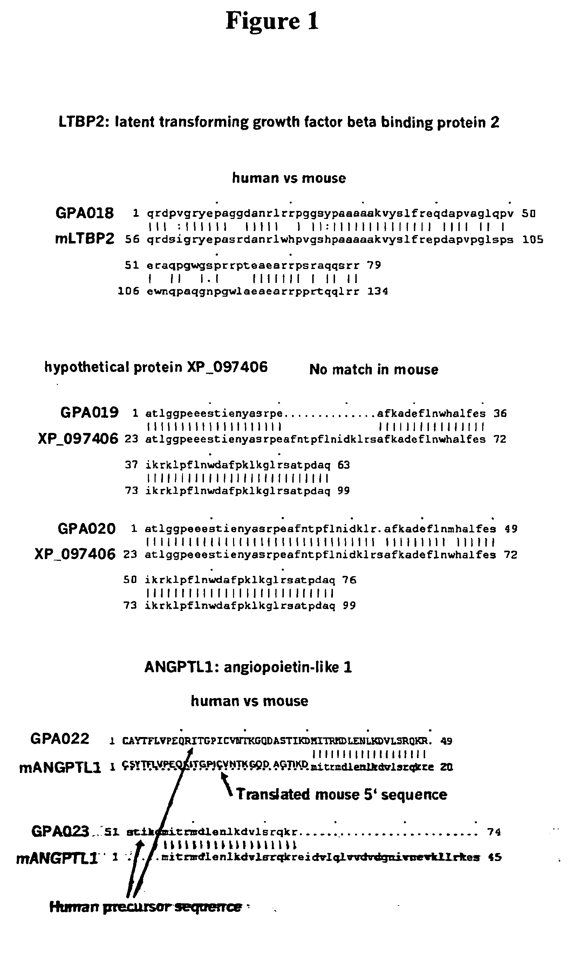

*Treatment.* Peptides GPA018, GPA019, GPA020, GPA022, and GPA023 (GeneProt, Geneva, Switzerland; see, FIG. 1) were administered subcutaneously to CD-1 mice for seven days at a dose of 300 mcg/day. The choice of CD-1 mice (an outbred strain from Charles River Laboratories, l'Arberesle, France) was made to increase the mouse organ weight and, hence, yield of RNA for microarray analysis. Four animals per treatment arm and gender were used. At the beginning of the treatment period, the animals were 12 to 14 weeks old. Body weight averaged 42.2g (38 to 45.6 g). Animals were kept under standard conditions for animal welfare.

Dosage forms were prepared once before the beginning of the treatment period. Each test item was dissolved in the vehicle (PBS) in order to achieve the required concentration. The dosage forms obtained were divided into aliquots and stored at -20°C pending use. Two aliquots were prepared for each group and day. The aliquots for treatment were delivered twice on each day of treatment to the animal room.

**TABLE 1**

| Animal Allocation and Test Item Dosages | | | |
|---|---|---|---|
| Treatment | Number of animals | Dose-level (µg/kg/day) | Concentration (µg/mL) |
| Control | 4 male and 4 female | 0 | 0 |
| GPA018 | 4 male and 4 female | 150 x 2 | 15 |
| GPA019 | 4 male and 4 female | 150 x 2 | 15 |
| GPA020 | 4 male and 4 female | 150 x 2 | 15 |
| GPA022 | 4 male and 4 female | 150 x 2 | 15 |
| GPA023 | 4 male and 4 female | 150 x 2 | 15 |

*In vivo examinations.* Animals were examined at least twice daily for mortality, food consumption and clinical observations. Body weight was recorded once per week.

Blood samples were collected from each animal. The serum samples were deep frozen (approximately -80°C) until analyses for hormone determination.

For tissue sampling, animals were asphyxiated by carbon dioxide approximately 12 to 16 hours after the last injection. A gross macroscopic post-mortem examination was performed in order to specify the possible reduced size of main organs (with particular attention to lymphoid tissues). No treatment-related morphological changes were noticed.

Snap freezing of many organs was performed at necropsy at the end of the treatment period. Within 15 to 20 after the sacrifice, all sampling for snap freezing was performed.

Twenty-eight tissues were sampled, including brain, duodenum (caecum), liver, kidney, muscle and spleen (blood). For this EXAMPLE, male and female animals were used in the kidney and in the liver assays. All other tissue samples were derived from males only.

Samples for histopathology were fixed in phosphate-buffered 10% formalin. Bone demineralization was performed with 10% formic acid.

Samples for gene expression profiling were quick-frozen in liquid nitrogen immediately after excision, stored on dry ice and subsequently in a deep-freezer at approximately -80°C until further use.

*RNA extraction and purification.* Briefly, total RNA was obtained by acid guanidinium thiocyanate phenol chloroform extraction (Trizol®, Invitrogen Life Technologies, Carlsbad, Calif. USA) from each frozen tissue section and the total RNA was then purified on an affinity resin (Rneasy®, Qiagen) according to the manufacturer's instructions, and quantified. Total RNA was quantified by the absorbance at λ = 260 nm (A₂₆₀ₙₘ) and the purity was estimated by the ratio A₂₆₀ₙₘ/A₂₈₀ₙₘ. Integrity of the RNA molecules was confirmed by non-denaturing agarose gel electrophoresis. RNA was stored at approximately -80°C until analysis. One part of each individual RNA sample was kept for the analysis of critical genes by means of Real-Time PCR.

*GeneChip*® *assays.* All GeneChip® assays were conducted as recommended by the manufacturer of the GeneChip system (Affymetrix, *Expression Analysis Technical Manual,* (Affymetrix, Santa Clara, Calif. USA, 2003). Genome MG-U74Av2 expression probe array set (Affymetrix, Inc., San Diego, Calif. USA) were used.

Double stranded cDNA was synthesized with a starting amount of approximately 5 µg full-length total RNA using the Superscript Choice System (Invitrogen Life Technologies) in the presence of a T7-(dT)24 DNA oligonucleotide primer. Following synthesis, the cDNA was purified by phenovchloroformlisoamylalcohol extraction and ethanol precipitation. The purified cDNA was then transcribed in vitro using the BioArray® High Yield RNA Transcript Labeling Kit (ENZO, Farmingdale, New York, USA) in the presence of biotinylated ribonucleotides form biotin labelled cRNA. The labelled cRNA was then purified on an affinity resin (Rneasy®, Qiagen), quantified and fragmented. An amount of approximately 10 µg labelled cRNA was hybridized for approximately 16 hours at 45°C to an expression probe array. The array was then washed and stained twice with streptavidin-phycoerythrin (Molecular Probes, Eugene, Oregon, USA) using the GeneChip® Fluidics Workstation 400 (Affymetrix).

The array was then scanned twice using a confocal laser scanner (GeneArray® Scanner, Agilent, Palo Alto, Calif. USA) resulting in one scanned image. This resulting ".dat-file" was processed using the MAS4 program (Affymetrix) into a "cel-life". The ".cel file" was captured and loaded into the Affymetrix GeneChip Laboratory Information Management System (LIMS). The LIMS database is connected to a UNIX Sun Solaris server through a network filing system that allows for the average intensities for all probes cells (CEL file) to be downloaded into an Oracle database. Raw data was converted to expression levels using a "target intensity" of 150. The data were checked for quality and loaded into the GeneSpring® software 5.0.3 (Silicon Genetics, Redwood City, Calif. USA) for analysis.

As a quality control, hybridizations were performed using GAPDH or β-action probes.

*Data analysis.* RNA samples were studied by using the human Affymetrix MG-U74Av2 GeneChip®. On such chip platform, probe-sets for individual genes contain 20 oligonucleotide pairs, each composed of a "perfect match" 25-mer and a "mismatch" 25-mer differing from the "perfect" match oligonucleotide at a single base. After probe labelling, hybridization, and laser scanning, the expression level is estimated by averaging the differences in signal intensity measured by oligonucleotide pairs of a given probe (AvgDiff value). The image acquisition and numerical translation software used for this study was the Affymetrix Microarray Suite version 4 (MAS5). The numerical values were stored and transferred for the analysis into the Silicon Genetics GeneSpring® 5.0.3 software toolkit.

Various filtering and clustering tools in these programs were used to explore the datasets and identify transcript level changes that inform on altered cellular and tissue functions and that can be used to establish working hypotheses on the modes of action of the compound. All datasets were normalized to the median. To identify pleiotropic or unique effects, scripts provided by Silicon Genetics, were applied. Pleiotropic effects were also investigated manually by analyzing genes with an expression level of more than 60 in at least three of eight conditions (i.e. organs) and hereafter applying statistical analysis (parametric, assume variances not equal, p<0.1) to identify genes with significant changes in their expression between control and treated animals.

To identify significant changes in gene expression in individual organs, an expression restriction filter was applied (ADV >60 in 50% of the samples), and a statistical filter as described above was added.

In some instances, a fold change filter (provided by Silicon Genetics) was used after the expression restriction to find genes with a change in gene expression above a certain level, and then a statistical filter applied.

The decision to considers a specific gene relevant is based on a conjunction of numerical changes identified by exploratory filtering and statistical algorithms as described above and the relationship to other modulated genes that point to a common biological theme. The weight of that relationship is assessed by the analyst through a review of the relevant scientific literature.

*Gene expression profiling.* Despite a multitude of statistical approaches, no significant changes in the gene expression patterns of the treatment groups were observed. In every analysis the number of changed genes was always smaller than the number of genes that would have been found by chance. The range of fold change was usually very small, seldom exceeding 2-fold.

Interestingly, the strongest changes were gender-specific. Comparison of control males and control females, as well as treated males versus treated females, showed about 3 times as many genes changed than did control males or females versus treated males or females. In addition, the genelists of treatment specific effects in females and males showed little overlap.

In the GPA018 treated kidneys of males and females combined (eight samples per group), some possible influence on genes affected by or affecting TGFβ signalling were observed (TABLE 2A and TABLE2B). GPA018 shows some sequence similarity to the N-terminal region of mLTBP-2 (murine latent transforming growth factor binding protein-2). LTBP proteins aid the LAP (latency associated protein)-TGFβ complex to become secreted and binds it to the ECM (extracellular matrix)-structural protein fibrillin (Annes JP et al., J. Cell Sci. 116(Pt2):217-24 (2003); Chen S et al., Nucl. Acids Res. 31(4):1302-10 (2003); Vehivilainen P et al., J. Biol. Chem. 278(27):24705-13 (2003)). However, the extent of the changes after treatment was very small, usually below 1.5-fold, and similar patterns were not observed when female or male groups were investigated separately.

**TABLE 2A**

| GPA018 in Kidney | |
|---|---|
| Expressed Gene | Comments and Literature |
| all-spectrin | Cleaved during TGF induced apoptosis (Brown TL et al., J. Biol. Chem. 274(33):23256-23262 (1999)) |
| β-spectrin | Disruption of β-spectrin ELF leads to disruption of TGFβ signalling in mice (Tang Y et al., Science 299(5606):574-7 (2003)) |
| β5-Integrin | TGFβ induces integrin (Ludbrook SB et al., Biochem. J. 369(Pt2):311-8 (2003)) |
| Integrin α(v)β3 | Gene is a receptor for the LAP of TGFβ 1 and 3 (Ludbrook SB et al., Biochem. J. 369(Pt2):311-8 (2003)) |
| TGFβ type I-receptor (ALK-5) | Overexpression downregulates integrin β5 and LTBP1 expression (Ota T et al., J. Cell Physiol. 193(3):299-318 (2002)) |
| LEF | TGFβ induces expression of LEF1 target genes; crosstalk between wnt and TGFβ pathway (Hu MC et al., Development 130(12):2753-2766 (2002); Letamendia A et al., J. Bone Joint Surg. Am. 83-A Suppl 1(Pt1):S31-9 (2001)) |
| Fas Death Domain-Associated Protein (DAXX) | TGFβ-induced apoptosis is mediated by DAXX that facilitates JNK activation (Perlman R et al., Nature Cell Biology 3(8):708-14 (2001)) |
| Forkheadbox D2 | Identification of FOXC 1 as a TGFβ1 responsive gene and its involvement in negative regulation of cell growth (Zhou Y et al., Genomics 80(5):465-72 (2002)) |
| Biglycan | Smad4-dependent regulation of biglycan gene expression by TGFbeta in pancreatic tumour cells (Chen WB et al., J. Biol. Chem. 277(39):36118-28 (2002)) |
| Amyloid β precursor protein | TGFβ1 potentates amyloid-β generation in astrocytes and in transgenic mice; induces overexpression of APP (Lesne S et al., J. Biol. Chem. 278(20):18408-18 (2003)) |
| Transgelin (SM22α) | TGFβ type I-receptor (ALK-5) overexpression leads to upregulation of SM22α (Chen S et al., Nucl. Acids Res. 31(4):1302-10 (2003); Ota T et al., J. Cell Physiol. 193(3):299-318 (2002)) |
| Vitamin D receptor | Vitamin D3 induces expression of TGFβ2; Smad3 binds and activates VDR (Yang L et al., J. Cell Physiol. 188(3):383-93 (2001); Aschenbrenner JK et al., T. Surg. Res. 100(2):171-5 (2001)) |
| Ephrin B2 | Ephrin A1 expression is decreased by ALK-5 overexpression (Ota T et al., J. Cell Physiol. 193(3):299-318 (2002)) |

Table 2B GPA018 in Kidney: Differentially Expressed Genes with Relation to TGFβ Signaling

| Description | p-value | FC | Treatment: GPA018 | Treatment: control | Probe set ID |
|---|---|---|---|---|---|
| vitamin D receptor | 0.019 | 2.30 | 226.194 (128.4 to 298.52) | 120.65 (32.02 to 278.91) | 99965_at |
| bone morphogenic protein receptor, type II | 0.036 | 2.00 | 87.798 (42.42 to 168.21) | 47.956 (14.39 to 106.76) | 99865 at |
| | | | | | |
| cadherin 5 | 0.005 | -1.32 | 78.321 (59.21 to 109.76) | 102.412 (86.02 to 120.66) | 104083_at |
| lymphoid enhancer binding factor 1 | 0.010 | 1.56 | 145.94 (108.53 to 171.06) | 97.804 (57.08 to 155.06) | 103628_at |
| catenin src | 0.049 | 1.30 | 124.611 (109.53 to 147.6) | 98.962 (48.99 to 128.24) | 98151_s_at |
| calcium/calmodulin-dependentserine protein kinase | 0.049 | -1.31 | 88.542 (50.23 to 119.39) | 113.935 (90.22 to 167.09) | 102248_f_at |
| | | | | | |
| MAP kinase-interacting serine/threonine kinase 2 | 0.048 | 1.29 | 205.875 (181.61 to | 249.07) 164.699 (99.8 to 208.85) | 92473_at |
| mitogen-activated protein kinase 12 | 0.039 | -1.19 | 72.744 (52.6 to 87.22) | 86.312 (69.76 to 108.47) | 92323_at |
| mesoderm development candiate 2 | 0.041 | 1.22 | 238.53 (178.42 to 283.5) | 196.015 (143.39 to 269.01) | 95405_at |
| forkhead box D2 | 0.037 | 1.20 | 118.66 (96.56 to 152.03) | 98.895 (75.88 to 129.5) | 94621_at |
| Fas death domain-associated protein | 0.050 | -1.21 | 77.96 (56.46 to 100.72) | 94.31 (73.12 to 115.51) | 96125_at |
| | | | | | |
| ephrin β2 | 0.021 | -1.28 | 75.761 (54.43 to 102.77) | 95.971 (69.63 to 113.05) | 160857_at |
| tyroslne kinase receptor 1 | 0.009 | -1.32 | 74.769 (58.19 to 98.31) | 99.618 (74.5 to 131.81) | 161184_at |
| biglycan | 0.015 | -1.36 | 152.224 (112.82 to 207.61) | 208-264 (155.91 to 280.86) | 162346f_at |
| Tnf receptor-associated factor 5 | 0.008 | -1.39 | 93.96 (68.53 to 144.09) | 129.381 (94.61 to 154.04) | 103255_at |
| | 0.005 | 1.13 | 5345.84 (4783.18 to | 6115) 4793,84 (4472.95 to 4995.46) | 93063_at |
| actin, beta, cytoplasmic | 0.002 | 1.57 | 861.716 (470.94 to 1102.93) | 539.534 (368 to 641.11) | 95705s_at |
| integrin beta 5 | 0.023 | -1.14 | 192.941 (167.13 to 215.24) | 219.201 (191.49 to 252.53) | 161786_f_at |
| integrin beta 5 | 0.048 | -1.20 | 348.915 (309.8 to 427.48) | 422.901 (311.02 to 527.04) | 100601_a_t |
| procollagen, type IV, alpha 4 | 0.045 | 1.42 | 149.262 (124.51 to 175.51) | 111.732 (54.42 to 160.81) | 101774_at |
| spectrin beta 2 | 0.004 | 1.12 | 829.471 (759.59 to 928.53) | 741.779 (642.34 to 791.98) | 93571_at |
| talin | 0.032 | 1.12 | 542.186 (490.64 to 593.16) | 486.978 (420.91 to 561.39) | 99448_at |
| myosin VIIa | 0.042 | -1.13 | 132.021 (116 to 166.57) | 148.674 (126.49 to 166.78) | 94713_at |
| transgelin | 0.045 | -1.26 | 158.629 (138.26 to 177.61) | 205.163 (132.68 to 308.45) | 93541_at |

### EXAMPLE III

### MULTI-ORGAN GENE EXPRESSION PROFILING OF CYNOMOLGUS MONKEY TREATED WITH FGF23CTP (100 µg/DAY SC)

*Introduction and summary.* The aim of this EXAMPLE was to identify for the peptide FGF23CTP modes of action with possible therapeutic applications by multi-organ microarray profiling in monkey. The peptide FGF23CTP (GPA006, GeneProt, Geneva, Switzerland) is derived from a unique COOH-terminal domain of the FGF-23. It is a unique 75-mer COOH-terminal peptide of FGF-23 with no homology to regions of other FGF family members. See, PCT patent application WO 02/088,358, the contents of which are incorporated by reference. In the brain FGF-23 transcripts are preferentially expressed in the thalamus (Yamashita T et al., Biochem. Biophys. Res. Commu., 277: 494-8 (2000)). Mutations in this region of the FGF-23 molecule were proposed as causative events in a renal phosphate wasting syndrome responsible for a form of autosomal dominant rickets (Saito H et al., Am. J. Pathol. 156: 697-707 (2002), White KE et al., Kidney Int. 60: 2079-86 (2001)). A similar paraneoplastic form of this syndrome was accompanied by ectopic expression of FGF-23 in the tumour tissue (Shimada T et al., Proc. Natl. Acad. Sci. USA 98: 6500-5 (2001)). FGF-23 is expressed in the ventrolateral thalamic nucleus of the brain. FGF23CTP has been derived *in silico* as a possible processing product of the fibroblast growth factor FGF-23 known to be involved in renal phosphate wasting syndromes. It was hypothesized that the 75-mer peptide could affect phosphate homeostasis.

FGF23CTP is given two weeks subcutaneously to cynomolgus monkeys and is found to affect critical pathways of cell differentiation in a multi-organ gene expression profiling analysis with high density human microarray assays. Comparison of the expression changes in sixteen different organs indicated common transcript level changes for genes involved in cell to cell signalling of growth and lineage determination, in cell cycling and in amino acid and ion transport. Although the FGF23CTP domain is unique and not found in other FGF family members, transcript levels for components of the FGF signalling pathway are affected in several organs. Serum protein levels of circulating IGF2-binding protein are.decreased in the treated animals.

Genes involved in angiogenesis/vasculogenesis are found in to be impacted in several organs. The effect of FGF23CTP on angiogenesis is confirmed in a hypoxic vascular retinopathy mouse model (see EXAMPLE VIII).

*Methods.* FGF23CTP is administered subcutaneously to cynomolgus monkeys for two weeks at a dose of 100 µg/day. At the end of the treatment period samples from all organs are subjected to snap freezing at necropsy and are analyzed with GeneChip® expression profiling.

Total RNA is extracted from these frozen tissues using TRIzol® reagent (Life Technologies) according to the manufacturer's instructions. Total RNA is quantified by the absorbance at λ = 260 nm (A₂₆₀ₙₘ) and the purity is estimated by the ratio A₂₆₀ₙₘ/A₂₈₀ₙₘ. Integrity is checked by denaturing gel electrophoresis. RNA is stored at -80°C until analysis.

Good quality total RNA is used to synthesize double-stranded cDNA using the Superscript® Choice System (Technologies, Gaithersburg, Maryland USA). The cDNA is then *in vitro* transcribed (MEGAscript™ T7 Kit, Ambion) to form biotin labelled cRNA. Next, 12 to 15 µg of labelled cRNA is hybridized to the Affymetrix Human U95A Version 2 expression probe arrays for 16 hours at 45°C. Arrays are then washed according to the EukGE-WS2 protocol (Affymetrix), and stained with 10 µg/ml of streptavidin-phycoerythrin conjugate (Molecular Probes). The signal is antibody-amplified with 2 mg/ml acetylated BSA (Life Technologies, Gaithersburg, Maryland USA), 100 mM MES, 1 M [Na+], 0.05 % Tween 20, 0.005 % Antiofoam (Sigma), 0.1 mg/ml goat IgG and 0.5 mg/ml biotinylated antibody and re-stained with the streptavidin solution. After washing, the arrays are scanned twice with the Gene Array® scanner (Affymetrix).

The expression level is estimated by averaging the differences in signal intensity measured by oligonucleotide pairs of a given probe (AvgDiff value). The image acquisition and numerical translation software used was the Affymetrix Microaray Suite version 4 (MAS4).

To identify genes that are impacted by treatment, the dataset is initially filtered to exclude in a first wave of analysis genes whose values are systematically in the lower expression ranges where the experimental noise is high (at least an AvgDiff value of 80 in a number of assays corresponding to the smallest number of replicas of any assay point). In a second round of selection a threshold t-test p-value (0.05) identifies genes with different values between treated and non-treated based on a two component error model (Global Error Model) and, where possible, with a stepdown correction for multi-hypothesis testing (Benjamini and Hochberg false discovery rate).

The selected genelists are then compared with established genelists for pathways and cellular components using Fisher's exact test. Venn diagrams are used to identify the gene changes that are in common between the different organs. Expression profiles of highly relevant genes are used to find genes with correlated changes at individual assay points, using several distance metrics (standard, Pearson).

The decision to consider a specific gene relevant is based on a conjunction of numerical changes identified by exploratory filtering and statistical algorithms as described above and the relationship to other modulated genes that point to a common biological theme.

A filter is applied on the FGF23CTP rostral hypothalamus expression raw data from a raw value of at least 80.0 in at least four out of eight conditions.

A selection is made on treatment conditions for FGF23CTP rostral hypothalamus based on statistically significant differences with a p-value cutoff 0.05 and a multiple testing correction based on the Benjamini and Hochberg False Discovery Rate. This restriction tested 5134 genes. About 5.0% of the identified genes would be expected to pass the restriction by chance.

*Results.* At the RNA level, it is clear that the compound affects key genes controlling cellular differentiation and proliferation, especially growth factors and growth factor receptors. Genes critically involved in angiogenesis are found in several organs. There is also a multi-organ effect on transcripts for components of the retinoblastoma cycling control checkpoint. The rostral hypothalamus shows the most pronounced changes especially for transport proteins and cytoarchitecture.

A cross comparison of organs analysed reveals that FGF23CTP affects the same or closely related pathways and provokes similar cellular effects. No routine clinical or biochemical changes are observed in the treated animals. Surprisingly, no effect on phosphate metabolism is observed.

TABLES 3 to 6 show that at the RNA level FGF23CTP affects key genes controlling cellular differentiation and proliferation, especially growth factors and growth factor receptors (TABLE 3). Genes critically involved in angiogenesis are found to be altered in several organs upon treatment with FGF23CTP (TABLE 4). There is also a multi-organ effect on transcripts for components of the retinoblastoma cycling control checkpoint (TABLE 5). The rostral hypothalamus shows the most pronounced changes especially on transcripts corresponding to proteins involved in transport and cytoarchitecture (TABLE 6).

In particular, FGF23CTP affects several molecules that have been described to play a role in the pathogenesis of malignant proliferation of glial cells and precursors: Epidermal growth factor (EGF) (Hoi Sang U et al., J. Neurosurg. 82: 841-846 (1995); Wu CJ et al., Oncogene 19: 3999-4010 (2000)), Bax (Streffer JR et al., J. Neurooncol. 56: 43-49 (2002); Martin S et al., J. Neurooncol. 52: 129-139 (2001)), connexin 43 (Huang R et al., Cancer Res. 62: 2806-2812 (2002); Soroceanu L et al., Glia 33: 107-117 (2001)), PKR (Shir A & Levitzki A, Nature Biotechnology 20: 895-900 (2002)), neurofibromin (NF1) (Cichowski K & Jacks T, Cell 104: 593-604 (2001); Gutmann, DH et al., Hum. Mol. Genet. 10:3009-3016 (2001)).

*Growth factors and growth factor receptors.* Several families of growth factors and related growth factor receptors including fibroblast growth factor (FGF)-receptors and other important extracellular signalling molecules for cell differentiation and maintenance, like members of the bone morphogenetic proteins (BMP), transforming growth factor (TGF), insulin-like growth factor (IGF), tumour necrosis factor (TNF) families are found to be impacted in more than one organ by treatment with FGF23CTP (TABLE 3).

**TABLE 3**

| Genes for Growth Factors and Receptors | | | |
|---|---|---|---|
| | | Treatment group of 2 males and 2 females | |
| | | Control | FGF23CTP |
| Gene name | Organ | Gene expression level after 2 weeks (mean and range) | |
| Activin A receptor type II | ROHY | 24.8 (20 to 32.6) | 54.9 (37.2 to 78.7) |
| | Adrenal | 20.6 (7.8 to 34.1) | 58.5 (34.3 to 72.8) |
| | Retina | 57.4 (48.2 to 70.6) | 34.1 (27.7 to 38) |
| Bone morphogenetic protein 10 | Kidney Liver | 74.6 (68.9 to 79.7) 96.4 (92.3 to 103.3) | 138.6 (114.9 to 172.3) 134.1 (108.8 to 152.6) |
| Bone morphogenetic protein 1 | Adrenal | 131.1 (81.5 to 150.6) | 210.6 (147.5 to 361.8) |
| Growth differentiation factor 1 | ROHY | 866 (753.5 to 911.2) | 1,410.2 (1,333.2 to 1,542.9) |
| Bone morphogenetic protein 2A | Duodenum | 121.3 (100.4 to 146.9) | 55.3 (37.9 to 80.1) |
| Bone morphogenetic protein | Adrenal | 64.6 (57.9 to 69.8) | 101.25 (71.2 to 156.5) |
| receptor, type II | | | |
| Colony-stimulating factor-1 | ROHY | 535.7 (470 to 598.2) | 802.5 (667.6 to 884.5) |
| | Kidney | 424.3 (316.1 to 576.8) | 659.75 (574.5 to 870.1) |
| | B marrow | 1,269.2 (934.5 to 1,493.8) | 981.3 (782.2 to 1,091.5) |
| Epidermal growth factor receptor | ROHY | 156.5 (138.4 to 167) | 103.1 (88.4 to 112.1) |
| EGF-response factor 1 | AV node | 1,141.3 (1,044.4 to 1,317.9) | 1,819.7(1,641.5 to 2,170.2) |
| EPHB2v protein-tyrosine kinase | ROHY | 173.5 (119.8 to 204.5) | 119.1 (113.2 to 121.8) |
| | Liver | 52.85 (35.2 to 64.7) | 82.6 (61.6 to 95.6) |
| FGF receptor-1 | ROHY | 669.9 (637 to 699.9) | 932.4 (782.6 to 1,125.6) |
| | Thymus | 224.5 (209.1 to 248.5) | 153.2 (133.2 to 169.6) |
| | Adrenal | 521.3(482.8 to 599) | 624.2 (559.4 to 721.9) |
| Farnesol receptor HRR-1 | Liver | 267.7 (259.6 to 276.4) | 383.3 (342.5 to 460.8) |
| | Duodenum | 28.3 (21.3 to 36.4) | 53.1 (44.6 to 67.1) |
| Insulin-like growth factor | AV node | 1,371.2 (1,134 to 1,523.8) | 880.75 (707.4 to 1,226.9) |
| binding protein 2 | Liver | 5'840.5 (4'269.2 to 6'849.6) | 3,858.6 (3,160.1 to 5'301.8) |
| | Retina | 1,396.8 (723.4 to 2,068.7) | 807.9 (506.4 to 1,143.3) |
| Insulin-like growth factor I | Muscle | 157.4 (115.9 to 179.9) | 107.65 (83.5 to 140.1) |
| | Liver | 649.675 (429.7 to 879.7) | 1,055.4 (838.1 to 1,331.6) |
| Insulin-like growth factor | Liver | 3,238.1 (2,346.3 to 3,807.7) | 1,517 (105.7 to 2,449.4) |
| binding protein 1 | | | |
| Insulin-like growth factor II | AV node | 401.275 (359.7 to 462.2) | 565275 (524.7 to 619.9) |
| | Liver | 426.5 (401.1 to 482.9) | 606.9 (554 to 687.6) |
| | B Marrow | 295.4 (251.2 to 339.8) | 209.5 (183.5 to 236.8) |
| Neuregulin 1 | Kidney | 66.3 (42.7 to 105.8) | 150.725 (119.4 to 193.9) |
| | Hippocampus | 125.4 (105.1 to 133.3) | 86.075 (70.7 to 97.8) |
| Retinoid X receptor-beta | ROHY | 136.7 (120.7 to 154.4) | 106.925 (84.4 to 139.5) |
| Retinoid X receptor-gamma | Muscle | 253.925 (193.1 to 285.1) | 580.9 (391.3 to 704-5) |
| Transforming growth factor β2 | Muscle | 20.85 (20 to 23.4) | 58.3 (39.3 to 66.9) |
| Transforming growth factor β 3 | ROHY | 37.9 (20 to 65.6) | 88.4 (77 to 104.1) |
| Tumine necrosis factor receptor-2 | ROHY | 70.3 (43.2 to 92.2) | 126.4 (117.8 to 136.4) |
| TNFR-related death receptor-6 | Re atrium | 100.6 (93.7 to 109.3) | 144.65 (123.9 to 175.5) |
| | ROHY | 155.1 (128.2 to 181.7) | 98.8 (69.1 to 137.3) |
| TNFR-related death receptor-3 | Muscle | 119.5 (92.9 to 133.6) | 82.4 (43.2 to 104.5) |

| | | | |
|---|---|---|---|
| ROHY: rostral hypothalamus; B Marrow: bone marrow; AV node: heart atrioventricular (AV) node | | | |

*Angiogenesis*/*vasculogenesis.* Transcript level changes for genes specifically involved in angiogenesis/vasculogenesis are found in several organs of animals treated with FGF23CTP (TABLE 4).

**TABLE 4**

| Genes for Angiogenesis | | | |
|---|---|---|---|
| | | Treatment group of 2 males and 2 females | |
| | | Control | FGF23CTP |
| Gene name | Organ | Gene expression level after 2 weeks(mean and range) | |
| Vascular endothelial growth | ROHY | 491 (431.1 to 570.1) | 326.175 (309.6 to 366.3) |
| factor-A (VEGFA) | AV node | 170.3 (106.6 to 234.9) | 363.7 (302.7 to 506.7) |
| VEGFB | ROHY | 484 (421.6 to 535.3) | 626.2 (527.4 to 776.7) |
| Placental growth factor | Muscle | 45.6 (20 to 78.4) | 91.4 (60.8 to 112.6) |
| FLT receptor tyrosine kinase 1 | Duodenum | 30.1 (20 to 60.5) | 70.7 (52.3 to 80.6) |
| | AV node | 56.5 (22.7 to 76.1) | 120.1 (77 to 156.4) |
| FLK1 receptor tyrosine kinase | Adrenal | 57.1 (49 to 62.5) | 42.7 (32.1 to 51) |
| Endothelial differentiation | Muscle | 258.6 (215.2 to 328) | 181.9 (167.9 to 199.7) |
| receptor 6 | AV node | 161.1 (111.6 to 188) | 306.9 (251.2 to 365.4) |
| Endothelial differentiation | Bone Marrow | 261.3 (214.2 to 356.9) | 494.8 (355.9 to 700.9) |
| receptor 4 | Liver | 279 (227.2 to 361) | 391.7 (347.7 to 453) |
| Brain-specific angiogenesis | Retina | 27.1 (20 to 38.2) | 153.2 (82.2 to 201) |
| inhibitor 2 | | | |
| Brain-specific angiogenesis | ROHY | 97.8 (82.8 to 118.8) | 225.1 (169.2 to 283.8) |
| inhibitor 1 (MASS) | | | |
| VE-cadherin | Adrenal | 63.8 (46.7 to 86.3) | 110.2 (108 to 113.8) |
| Angiopoietin-1 | Bone Marrow | 198.6 (175.4 to 221.8) | 123.2 (106.3 to 151.1) |
| | ROHY | 235.7 (204.7 to 306.9) | 162.5 (154.4 to 179.6) |
| TEK tyrosine kinase, | Bone Marrow | 91.8 (67.1 to 106.3) | 52.7 (33.3 to 63.7) |
| angiopoietin receptor | | | |
| ROHY: rostral hypothalamus; AV node: heart atrioventricular (AV) node. | | | |

*Cycling: Retinoblastoma checkpoint*. Transcript levels of genes involved in cell cycle control, especially those genes involved in the transition from the G1 to S-phase, are affected in several organs by treatment with FGF23CTP (TABLE 5). In particular, expression of those genes upstream or downstream of the retinoblastoma gene product (Rb) phosphorylation step, a major downstream control step for growth factor-induced proliferation, is altered. FGF23CTP also affects transcript levels for cyclin-dependent kinase 4 and cyclins D2, D3 and E2 involved in Rb phosphorylation. A second control level is also mobilized with cyclin kinase inhibitors like p19INK4D, p21CIP1 and p27Kip1. Also affected is the inhibitor of p53 and Rb, Mdm2. The targets of the retinoblastoma protein are also involved: E2F1, E2F2, E2F5 and their binding partner Dp-2.

**TABLE 5**

| Cell Cycling | | | |
|---|---|---|---|
| | | Treatment group of 2 males and 2 females | |
| | | Control | FGF23CTP |
| Gene name | Organ | Gene expression level after 2 weeks (mean and range) | |
| Cyclin-dependent kinase 4 | ROHY | 266 (240.1 to 283.7) | 197.6 (167.6 to 231.7) |
| Cdk-inhibitor p57KIP2 (KIP2) | Duodenum Thymus | 233.7 (212.1 to 251.9) 312.7 (292 to 341.1) | 138.6 (114.9 to 172.3) 247.9 (227.3 to 284.2) |
| Cyclin A/CDK2-associated p19 | ROHY | 1349.8 (1,306.6 to 1,420.1) | 1069.6 (902.3 to 1,278) |
| (Skp1) | | | |
| Cyclin D2 | Retina | 210.2 (172.2 to 233.2) | 132 (96.2 to 171.5) |
| | ROHY | 231.8 (201.1 to 248.6) | 197.1 (182.7 to 203.9) |
| Cyclin D3 | Muscle | 182.4 (157.2 to 198.2) | 149.2 (135 to 162) |
| Cyclin-dependent kinase 5 | Hippocampus | 213.9 (172.3 to 254.7) | 118.7 (96.6 to 162.3) |
| Cyclin E2 | Bone Marrow | 407 (367.4 to 491.4) | 257 (175.2 to 294.8) |
| Cyclin I | Duodenum | 1109.9 (1035.5 to 1157.2) | 900.6 (758.7 to 1054.7) |
| E2F-1 | Kidney | 94 (51.9 to 150.9) | 198.5 (135.8 to 263) |
| E2F-4 | Muscle | 888 (765.7 to 981.2) | 643.8 (471.5 to 765.7) |
| | Hippocampus | 208.7 (185.7 to 221.5) | 153.4 (116.7 to 228.6) |
| | ROHY | 812 (748.4 to 859.5) | 245.9 (63.2 to 581.9) |
| | Kidney | 505.6 (432.1 to 574.8) | 741.6 (652.5 to 802.8) |
| E2F-5 | Adrenal | 53.9 (49.2 to 57.6) | 39.6 (33.5 to 45) |
| E2F dimerization partner 2 (DP2) | Bone Marrow | 2091 (1617.9 to 2735.1) | 1222 (1021.1 to 1387.7) |
| Mdm2 | Bone Marrow | 40.925 (33.4 to 44.3) | 20 |
| Cdk-inhibitor p 19 | ROHY | 163.6 (148.6 to 194.9) | 267.8 (243.2 to 314.2) |
| Cdk-inhibitor 1A p21 (Cip1) | Adrenal | 106.9 (85 to 125.1) | 51.75 (20 to 96.8) |
| p53 binding protein | Muscle | 269.35 (173 to 374.7) | 138.7 (97.7 to 172) |
| | ROHY | 217.5 (210.1 to 226.3) | 154.7 (143.7 to 159.9) |
| Retinoblastoma 1 | ROHY | 255.9 (248.3 to 266.1) | 467.9 (361.1 to 570) |
| Retinoblastoma binding protein 4 | ROHY | 361.1 (283.4 to 398.7) | 164 (131.5 to 225.4) |
| S-phase response | ROHY | 231.5 (199.8 to 270.3) | 176 (159.8 to 205.6) |
| (cyclin-related) | Re atrium | 126.75 (100.7 to 146.9) | 159.1 (153.4 to 168.9) |
| RalGDS/AF-6 | ROHY | 414.9 (390.1 to 439.7) | 300.5 (284 to 313.9) |
| (inhibitor of Cyclin D1) | | 438.1 (392.6 to 520.8) | 354.5 (335.8 to 369.9) |
| ROHY: rostral hypothalamus | | | |

*Rostral hypothalamus.* The rostral hypothalamus is the organ with the most pronounced changes in transcript levels in animals treated with FGF23CTP. TABLE6 reflects genes of defined pathways and cellular actions with the most significant changes in this organ. In particular, effects on cytoarchitecture genes are especially pronounced in brain tissues.

**TABLE 6**

| Rostral Hypothalamus Genes | | |
|---|---|---|
| | Treatment group of 2 males and 2 females | |
| | Control | FGF23CTP |
| Gene name | Gene expression level after 2 weeks (mean and range) | |
| Receptors | | |
| Retinoic acid receptor | 31.2 (26.8 to 41) | 110 (100.9 to 122.8) |
| Cholecystokinin A receptor | 87.1 (66.6 to 119.4) | 196 (172.3 to 218.3) |
| Jagged 1 | 417.2 (402.1 to 437.7) | 70.3 (20 to 161.9) |
| Protein kinases | | |
| c-ab1 | 214.4 (187.3 to 252.5) | 119.4 (103.4 to 127) |
| c-yes | 116.65 (107.9 to 127.8) | 64.7 (50.8 to 77.4) |
| c-raf | 404.7 (370.8 to 458.7) | 327.5 (281.6 to 356.9) |
| Pim1 | 487.7 (432.9 to 557.6) | 327.5 (281.6 to 356.9) |
| FYN | 1,086.1 (1,018.4 to 1,172.4) | 958.1 (891.3 to 1,007.6) |
| PKR Interferon-inducible | 179.475 (162 to 192.7) | 128.25 (112.1 to 140.9) |
| TYRO3 | 90.45 (78.7 to 106.8) | 163.1 (134.7 to 179.5) |
| Axl | 122.8 (112.4 to 140) | 60.3 (34.7 to 89.4) |
| TrkB | 108.6 (87.5 to 127.9) | 40.8 (23.6 to 59.4) |
| Erk3 | 588.5 (551.5 to 679.4) | 409.5 (370.5 to 482.7) |
| Phosphoinositide 3-kinase | 90.3 (78.2 to 111.9) | 241.5 (215.7 to 289.2) |
| Glycogen synthase kinase 3 | 70.4 (66.3 to 75.3) | 155.5 (127.9 to 180.1) |
| JNK2 | 137.225 (122.2 to 159.8) | 79.9 (64 to 95.2) |
| Ribosomal protein kinase B (RSK-B) | 49.8 (35.4 to 74.5) | 120 (90.4 to 141.8) |
| Janus kinase 1 | 106.7 (88.9 to 136.3) | 43.3 (20 to 64.7) |
| SFRS protein kinase 2 | 396.1 (369.5 to 420.3) | 205.8 (180.3 to 249.6) |
| Mnb (Minibrain) | 567.4 (561.4 to 571.6) | 446 (410.2 to 477.2) |
| Other signalling molecules | | |
| Phosphoinositide-3-kinase, catalytic, | 90.3 (78.2 to 111.9) | 241.5 (215.7 to 289.2) |
| delta polypeptide | | |
| RAS p21 protein activator | 243.9 (211.5 to 270) | 163 (139.4 to 198.5) |
| STAT1 | 182 (171.2 to 188.5) | 119.3 (110.6 to 134.5) |
| Calcineurin A1 | 410.5 (367.6 to 486.4) | 173.975 (114.5 to 239.7) |
| 14-3-3 protein tau | 2,110 (1,964.8 to 2,211.2) | 1,748.5 (1,649.5 to 1,852) |
| Neurofibromin | 78.6 (74.5 to 88.1) | 144 (126.4 to 155.8) |
| Phosphodiesterase 4B | 123 (110.1 to 133.3) | 60.6 (46.7 to 72.3) |
| Phospholipase C | 125.9 (107.4 to 136.2) | 72.1 (56.8 to 81) |
| Transcription factors | | |
| Hypoxia-inducible factor 1 alpha | 611.5 (515.7 to 745.1) | 286.5 (223 to 339.5).1) |
| CREB binding protein | 165.4 (160 to 176.9) | 93 (82.4 to 100) |
| Jun D | 4'882.5 (4'592.6 to 5'361.6) | 6'499.1 (6'353.9 to 6'745.1) |
| Inhibitor of DNA binding 4 | 140.3 (113.3 to 157.9) | 67.6 (55.9 to 72.7) |
| Bmi-1 homeobox gene repressor | 553.7 (513.6 to 599.8) | 374.7 (352.6 to 410.4) |
| Solute transport | | |
| Ca2+-ATPase | 325.1 (274.6 to 401) | 174.4 (130.8 to 247.5) |
| Sodium/hydrogen exchanger | 1,062.4 (1,039.5 to 1,095.9) | 785.5 (743.4 to 851.2) |
| Anion exchanger | 627.1 (555.6 to 676.3) | 844.5 (760.8 to 916.2) |
| Na,K-ATPase | 2,032.2 (1,743.5 to 2,239.6) | 1,453.2 (1,246.3 to 1,736.8) |
| CMP-sialic acid transporter | 265.8 (241 to 329.6) | 135.075 (112.8 to 159.1) |
| Vesicle transport | | |
| RAB1 | 293.5 (246.9 to 320.3) | 143.9 (93.8 to 214.9) |
| RAB5 | 1,266.2 (1,146.6 to 1,349) | 809.8 (745.3 to 894) |
| RAB11A | 554.8 (544.8 to 565.4) | 344.9 (284.1 to 395.4) |
| RAB11B | 182.3 (153.2 to 207.4) | 71.2 (41.8 to 110.9) |
| RNP24 | 867.4 (809.5 to 903.7) | 558.2 (486.3 to 642.9) |
| SNARE | 140 (127.2 to 158.5) | 55.4 (41.2 to 69.7) |
| GDP dissociation inhibitor beta | 166.6 (156.5 to 173.4) | 125.8 (104.1 to 142.6) |
| NIPSNAP2 | | |
| (glioblastoma co-amplified) | 212.9 (188.1 to 226.2) | 118.7 to (88.9 172) |
| Apoptosis | | |
| Bcl-2-binding protein Nip3 | 1,099 (1,056.1 to 1,135.1) | 794.8 (709.9 to 841.2) |
| BAX | 30.95 (20 to 57.2) | 134.4 (75.2 to 171) |
| Cytoarchitecture | | |
| Tropomyosin 2 (beta) | 20 | 131.6 (112.4 to 150.2) |
| Myosin regulatory light chain | 328.5 (298.3 to 374) | 177.2 (153.5 to 214) |
| Tropomyosin 4 | 72 (54.7 to 86.6) | 140.6 (126.5 to 166.8) |
| Pinin, | 181.7 (164 to 203.2) | 119.7 (108 to 131.5) |
| Desmosome associated protein | | |
| Connexin 43 | 410.4 (321.4 to 446.4) | 184.5 (132.2 to 265.8) |
| Delta 2 catenin | 239.6 (178.9 to 281.2) | 118 (103.9 to 146.9) |
| Reticulon 4 | 2,343,7 (2,019.1 to 2,516.7) | 1,309.2 (1,126.2 to 1,645.4) |
| Neural cell adhesion molecule 1 | 428 (297.1 to 524.2) | 988.6 (841.6 to 1,196) |
| Amyloid beta precursor protein | 400 (351.1 to 435) | 191.3 (161.2 to 218.8) |
| (cytoplasmic tail)-binding protein 2 | | |
| Myelinization | | |
| Oligodendrocyte myelin glycoprotein | | |
| OMGP | 1,144 (1,037.9 to 1,260.2) | 773.5 (619.7 to 986) |
| oligodendrocyte glycoprotein | | |
| Myelin NPD | 176.8 (139.9 to 209.4) | 62.7 (25.9 to 80.7) |
| Sphingomyelinase | 585.5 (544.2 to 637.7) | 259.5 (229.5 to 281.4) |

As can be seen in the TABLE 7 (genelists per organ), there are genes representing these pathways with less pronounced changes in other organs. Effects on genes coding for transport molecules and intracellular signalling molecules are found in most organs. The effects on cytoarchitecture genes are especially pronounced in brain tissues. Shown in TABLE 7 are the Affymetrix chip probeset ID, p-value, and gene description of the 602 selected genes.

**TABLE 7**

| Gene Expression Changes at 4 Weeks in the Rostral Hypothalamus | | |
|---|---|---|
| 1 37316_r_at | 0.001189 3' | Source: oy31e07.x1 Soares_parathyroid_tumour_NbHPA *Homo sapiens* cDNA clone IMAGE:1667460 similar to WP:F48E8.2 CE01954; mRNA sequence. |
| 2 32841_at | 0.001189 | Source: Human nucleic acid binding protein gene, complete cds. |
| 3 32290_at | 0.005998 | Source: *Homo sapiens* mRNA; cDNA DKFZp564L1916 (from clone DKFZp564L1916). |
| 4 38035_at | 0.005998 | hMTMR6 |
| 5 38568_at | 0.005998 | Source: *Homo sapiens* p53 binding protein mRNA, complete cds. |
| 6 33683_at | 0.005998 | Source: Human mRNA for TI-227H. |
| 7 37995_s_at | 0.005998 | Source: Human Fragile X mental retardation 1 FMR-1 gene, 3' end, clones BC72 and BC22. |
| 8 821_s_at | 0.005998 | Source: Human folate receptor alpha (hFR) mRNA, partial cds. |
| 9 41562_at | 0.005998 | putative |
| 10 AFFX- | 0.005998 | Source: *Homo sapiens* transcription factor ISGF-3 mRNA, complete cds. |
| HUMISGF3 | | |
| A/M97935_3_at | | |
| 11 1132_s_at | 0.005998 | Source: *Homo sapiens* retinoic acid receptor (gamma-7) mRNA. |
| 12 38363_at | 0.005998 | Source: zd27g05.s1 Soares_fetal_heart_NbHH19W *Homo sapiens* cDNA clone IMAGE:341912 3' similar to PIR:S47066 S47066 zinc finger protein - human ;, mRNA sequence. |
| 13 32037_r_at | 0.005998 | Source: *Homo sapiens* ribonuclease P protein subunit p14 (Rpp14) mRNA, complete cds. |
| 14 31879_at | 0.005998 | Source: Human FUSE binding protein 3 (FBP3) mRNA, partial cds. |
| 15 38102_at | 0.005998 | Source: 51f12 Human retina cDNA randomly primed sublibrary *Homo sapiens* cDNA, mRNA sequence. |
| 16 193_at | 0.005998 | Source: Human transcriptional activation factor TAF1132 mRNA, complete cds. |
| 17 40246_at | 0.005998 | sequences encoding: d1g homology repeats (DHR): DHR1 855..1118, DHR2 1143..1403, DHR3 1563..1826; SH3 domain 1935..2132; guanylate kinase-related domain 2331..2858 |
| 18 40453_s_at | 0.005998 | member of the family of SR protein pre-mRNA splicing factors |
| 19 35247_at | 0.005998 | Source: PT2,1_13_A09.r tumour2 *Homo sapiens* cDNA 3', mRNA sequence. |
| 20 39280_at | 0.005998 | polyleucine rich |
| 21 36971_at | 0.005998 | Similar to a C. elegans protein encoded in cosmid C27F2 (U40419) |
| 22 39628_at | 0.005998 | Source: wb33e07.x1 NCl_CGAP_GC6 *Homo sapiens* cDNA clone IMAGE:2307492 3' similar to SW:RAB9_HUMAN P51151 RAS-RELATED PROTEIN RAB-9. ;, mRNA sequence. |
| 23 41437_at | 0.005998 | Source: *Homo sapiens* mRNA; cDNA DKFZp564F1123 (from clone DKFZp564F1123); partial cds. |
| 24 39167_r_at | 0.005998 | Source: Human mRNA for collagen binding protein 2, complete cds. |
| 25 40467_at | 0.005998 | cytochrome b small subumit of complex II (succinate-ubiquinone oxidoneductase) |
| 26 33831_at | 0.005998 | transcriptional adaptor |
| 27 38694_at | 0.005998 | Source: *Homo sapiens* mRNA for KIAA0738 protein, complete cds. |
| 28 34644_at | 0.005998 | Source: *Homo sapiens* mRNA for beta 2-microglobulin, complete cds. |
| 29 34763_at | 0.005998 | SMC family |
| 30 40128_at | 0.005998 | similar to hypothetical protein L8167.6 of *Saccharomyces cerevisiae*. |
| 31 33109_f_at | 0.00617 | putative |
| 32 36972_at | 0.007286 | microsomal fraction |
| 33 36576_at | 0.007579 | Source: *Homo sapiens* histors macroH2A1.2 mRNA, complete cds. |
| 34 36868_at | 0.007579 | Source: *Homo sapiens* clone 23741 mRNA sequence. |
| 35 33359_at | 0.007579 | Source: *Homo sapiens* mRNA for K1AA0768 protein, partial cds. |
| 36 35094_f_at | 0.007579 | Source: *Homo sapiens* leukocyte immumoglobulin-like receptor-4 (LIR-4) mRNA, complete cds. |
| 37 40354_at | 0.007579 | member of 110-kDa heat shock protein (hsp110) family |
| 38 38318_at | 0.00765 | Source: *Homo sapiens* mRNA; cDNA DKFZp586G051 (from clone DKFZp586G051). |
| 39 36864_at | 0.007879 | Source: *Homo sapiens* mRNA for Pex3 protein. |
| 4032171_at | 0.008176 | HUMAN EUKARYOTIC TRANSLATION INITIATION FACTOR 5 |
| 41 34393_r_at | 0.008176 | Rab1, splice variant |
| 42 1117_at | 0.008176 | Source: *Homo sapiens* cytidine deaminase (CDA) mRNA, complete cds. |
| 43 1191_s_at | 0.008176 | Source: *Homo sapiens* mRNA for 26S proteasome subunit p44.5, complete cds. |
| 44 33705_at | 0.008176 | putative |
| 45 37409_at | 0.008458 | similar to human serine kinase SRPK1, encoded by GenBank Accession Number U09564; specific for serine/arginine-rich splicing factors |
| 46 949_s_at | 0.008458 | proteasome (prosome, macropain) 26S subunit, ATPase, 6 |
| 47 35642_at | 0.008458 | mitochondrial outer membrane protein; binds to metaxin 1, a related protein that participates in protein import into mitochondria |
| 48 35784_at | 0.008458 | SNARE protein |
| 49 34383_at | 0.008458 | Source: *Homo sapiens* hUBP mRNA for ubiquitin specific protease, complete cds. |
| 50 36691_at | 0.008458 | Source: *H. sapiens* mRNA for glutamine transaminase K. |
| 51 36542_at | 0.008458 | Source: *Homo sapiens* sodium-hydrogen exchanger 6 (NHE-6) mRNA, nuclear gene encoding mitochondrial protein, complete cds. |
| 52 40631_at | 0.008458 | Source: Human mRNA for Tob, complete cds. |
| 53 40605_at | 0.008458 | sorting nexin 4 |
| 54 37350_at | 0.008704 | match: proteins Q99795 Q91665 Q91664 009052 P78310 P97792 Q91667 060939 P54900 Q61148 Q62861 000426 P06907 P25189 Q92677 P20938 P27573 P10522 P37301 match: patented sequence I80040 supported by GENSCAN and FGENES |
| 55 38687_at | 0.009005 | Source: *Homo sapiens* mRNA; cDNA DKFZp566D193 (from clone DKFZp566D193); partial cds. |
| 56 36546_r_at | 0.009584 | Start codon is not identified. hg04325 cDNA clone for KIAA0542 has a 933-bp insertion between 3296-3297, and a 187-bp insertion between 3368-3369 of the sequence of KIAA0542 |
| 57 39767_at | 0.009584 | Source: Human mRNA for KIAA0002 gene, complete cds. |
| 58 36110_at | 0.009584 | Source: *Homo sapiens* GTP-binding protein (RAB5) mRNA, complete cds. |
| 59 38093_at | 0.009584 | Source: Human clone 23722 mRNA sequence. |
| 60 37826_at | 0.009584 | Source: *Homo sapiens* unknown mRNA, sequence. |
| 61 32232_at | 0.009584 | Source: *Homo sapiens* NADH-ubiquinone oxidoreductase subunit CI-SGDH mRNA, complete cds. |
| 62 39544_at | 0.009584 | Source: Human mRNA for KIAA0353 gene, partial cds. |
| 63 33399_at | 0.009602 | Source: zl43c04.s1 Soares_pregnant_uterus_NbHPU *Homo sapiens* cDNA clone IMAGE:504678 3', mRNA sequence. |
| 64 1085_s_at | 0.009679 | phospholipase C |
| 65 34931_at | 0.009679 | Source: *Homo sapiens* mRNA for KIAA0940 protein, complete cds. |
| 66 39099_at | 0.009679 | COPII component; isoform A |
| 67 AFFX- | 0.009806 | ***ALU WARNING: Human Alu-Sq subfamily consensus sequence. |
| hum_alu_at | | |
| 68 35734_at | 0.009806 | Source: wo97g09.x1 NCl_CGAP_Kid11 *Homo sapiens* cDNA clone IMAGE:2463328 3', mRNA sequence. |
| 69 995_g_at | 0.009839 | Source: *H. sapiens* hR-PTPu gene for protein tyrosine phosphatase. |
| 7038040_at | 0.01011 | SF |
| 71_37705_at | 0.01011 | Source: Human (ard-1) mRNA, complete cds. |
| 72 40893_at | 0.010187 | Source: *Homo sapiens* ATP-specific succinyl-CoA synthetase beta subumit (SCS) mRNA, partial cds. |
| 73 36980_at | 0.010256 | Source: Human B4-2 protein mRNA, complete cds. |
| 74 1674_art | 0.010419 | cellular yes-I protein |
| 75 39989_at | 0.010453 | Source: *H. sapiens* mRNA for ragB protein. |
| 76 34905_at | 0.010453 | Source: oq24f02.s1 NCl_CGAP_GC4 *Homo sapiens* cDNA clone IMAGE:1587291 3' similar to TR:Q62643 Q62643 GLUTAMATE RECEPTOR KA2 SUBUNIT. [1];, mRNA sequence. |
| 77 31503_at | 0.010453 | Source: 50h7 Human retina cDNA randomly primed sublibrary *Homo sapiens* cDNA, mRNA sequence. |
| 78 38808_at | 0.010453 | Source: Human mRNA for Mr 110,000 antigen, complete cds. |
| 79 39923_at | 0.010453 | Source: wo84c08.x1 NCI_CGAP_Kid11 *Homo sapiens* cDNA clone IMAGE:2462030 3', mRNA sequence. |
| 80 32695_at | 0.010685 | isoform 2 match: protein Q99991 |
| 81 36224_g_at | 0.011036 | Source: wf12b02.x1 Soares_NFL_T_GBC_S1 *Homo sapiens* cDNA clone IMAGE:2350347 3', mRNA sequence. |
| 82 36171_at | 0.011036 | Source: th60h07.x1 NCl_CGAP_Ov23 *Homo sapiens* cDNA clone IMAGE:2122717 3' similar to SW:P15_HUMAN P53999 ACTIVATED RNA POLYMERASE II TRANSCRIPTIONAL COACTIVATOR P15 ;, mRNA sequence. |
| 83 39598_at | 0.011036 | gap junction protein (aa 1-283) |
| 84 33543_s_at | 0.011036 | desmosome associated protein; phosphoprotein |
| 85 429_f_at | 0.011036 | Source: Human beta-tubulin gene (5-beta) with ten Alu family members. |
| 86 36326_at | 0.011036 | Source: Human NSCL-2 gene sequence. |
| 87 35767_at | 0.011436 | Source: tn20b01.x1 NCI_CGAP_Brn25 *Homo sapiens* cDNA clone IMAGE:2168137 3' similar to TR:O08765 008765 GEF-2. ;, mRNA sequence. |
| 88 34819_at | 0.011497 | Source: Human mRNA for MGC-24, complete cds. |
| 89 40861_at | 0.011497 | Source: Human mRNA for KIAA0026 gene, complete cds. |
| 90 34302_at | 0.011497 | Source: Human translation initiation factor eIF3 p44 subunit mRNA, complete cds. |
| 91 34677_f_at | 0.011497 | Source: *Homo sapiens* mRNA for TL132. |
| 92 32744_at | 0.011881 | Source: DU3.2-7.G08.r DU-145 *Homo sapiens* cDNA 5', mRNA sequence. |
| 93 38010_at | 0.012279 | pro-apoptotic mitochondrial protein |
| 94 41651_at | 0.012367 | Source: *Homo sapiens* mRNA for KIAA1033 protein, partial cds. |
| 95 36991_at | 0.012367 | SR protein family member; SR domain: (bp. 583..1529); RNA binding domains: RNP-2 (bp. 57..80) and RNP-1 (bp. 150..173) |
| 96 33107_at | 0.012863 | Source: *Homo sapiens* mRNA for KIAA0898 protein, partial cds. |
| 97 35297_at | 0.012932 | Transcriptional coactivator P15 like |
| 98 35999_r_at | 0.012932 | Source: *Homo sapiens* mRNA for KIAA0781 protein, partial cds. |
| 99 40864_at | 0.013006 | Source: *Homo sapiens* mRNA, clone:P02ST9. |
| 100 41178_at | 0.013006 | Source: *H. sapiens* mRNA for ribosomal protein L11. |
| 101 1030_s_at | 0.013006 | found in the camptothecin resistant clone CEM/C2 |
| 102 40854_at | 0.013006 | core protein II precursor |
| 103 39382_at | 0.013006 | Source: *Homo sapiens* mRNA for KIAA0517 protein, partial cds. |
| 104 32823_at | 0.013006 | Source: 51a1 Human retina cDNA randomly primed sublibrary *Homo sapiens* cDNA, mRNA sequence. |
| 105 39748_at | 0.013006 | Source: *Homo sapiens* mRNA; cDNA DKFZp564D016 (from clone DKFZp564D016). |
| 106 35005_at | 0.013006 | Source: *Homo sapiens* type 6 nucleoside diphosphate kinase NM23-H6 (NM23-H6) mRNA, complete cds. |
| 107 32781_f_at | 0.013006 | Source: zk65d06.r1 Soares_pregnant_uterus_NhHPU *Homo sapiens* cDNA clone IMAGE:487691 5', mRNA sequence. |
| 108 38705_at | 0.013006 | Source: qo77c11.x1 NCI_CGAP_Kid5 *Homo sapiens* cDNA clone IMAGE:1914548 3' similar to ENDOTHELIN B RECEPTOR PRECURSOR (HUMAN);, mRNA sequence |
| 109 35838_at | 0.013006 | gb:S57283 similar to human zinc finger protein encoded by Gen Bank Accession Number M91592 |
| 110 185_at | 0.013006 | paraneoplastic Ri antigen |
| 111 38738_at | 0.013006 | ubiquitin-like protein |
| 112 36588_at | 0.13006 | hk05647 cDNA clone for KIAA0810 has a142-bp insertion at position 1029 of the sequence of KIAA0810. |
| 113 36596_r_at | 0.013006 | This sequence comes from Fig. 4 |
| 114 1537_at | 0.013006 | Source: Human mRNA for precursor of epidermal growth factor receptor. |
| 115 31881_at | 0.013154 | Source: *Homo sapiens* mRNA for Hmob33 protein, 3' untranslated region. |
| 116 33440_at | 0.013327 | Source: Human two-handed zinc finger protein ZEB mRMA, partial cds. |
| 117 37359_at | 0.013357 | Source: Human mRNA for KIAA0102 gene, complete cds. |
| 118 35681_r_at | 0.013357 | Source: *Homo sapiens* mRNA for KIAA0569 protein, complete cds. |
| 119 34381_at | 0.013357 | Source: as86g01.x1 Barstead colon HPLRB7 *Homo sapiens* cDNA clone IMAGE:2335632 3' similar to gb:X16S60 CYTOCHROME C OXIDASE POLYPEPTIDE VIIC PRECURSOR (HUMAN);, mRNA sequence. |
| 120 192_at | 0.013357 | Source: Human TFIID subunit TAFII55 (TAFII55) mRNA, complete cds. |
| 121 41547_at | 0.013381 | amino terminus similar to protein encoded by GenBank Accession Number AF047473; structurally similar to *Homo sapiens* Rael protein encoded by GenBank Accession Number U84720; similar to Mus musculus protein encoded by GenBank Accession Number U67327; contains WD40 repeat motif, also termed trp-asp, G-beta repeat; binds C-terminal domain of BUB1 I kinase in vitro |
| 122 31850_at | 0.013494 | Source: Human gamma-glutamylcysteine synthetase (GCS) mRNA, complete cds. |
| 123 36660_at | 0.013702 | GTPase |
| 124 40588_r_at | 0.013889 | Source: *Homo sapiens* p18 protein mRNA, complete cds. |
| 125 36585_at | 0.014379 | Source: Human ADP-ribosylation factor 4 (ARF4) mRNA, complete cds. |
| 126 39388_at | 0.014587 | Source: ok71f11.s1 NCI_CGAP_GC4 *Homo sapiens* cDNA clone IMAGE:1519437 3', mRNA sequence. |
| 127 38971_r_at | 0.014994 | HIV-1, Nef-associated factor 1 beta |
| 128 32335_r_at | 0.015192 | Source: *Homo sapiens* mRNA for polyubiquitin UbC, complete cds. |
| 129 725_i_at | 0.015192 | |
| 130 40122_at | 0.015227 | NS1-associatedprotein 1 |
| 131 38908_s_at | 0.015746 | DNA polymerase zeta unspliced 3' region |
| 132 35709_at | 0.015746 | Source: *Homo sapiens* clone 23923 mRNA sequence. |
| 133 31936_s_at | 0.015746 | Start codon is not identified. hh01734 cDNA clone for KIAA0430 has a 61-bp deletion at the region from 3167 to 3227 of the sequence of KIAA0430 |
| 134 40146_at | 0.016164 | Human mRNA for Rap1B protein |
| 135 34231_at | 0.016164 | binds the large subunit (ORC I L) of the origin recognition complex (ORC) |
| 136 38375_at | 0.016327 | Source: *Homo sapiens* esterase D mRNA, complete cds. |
| 137 35727_at | 0.016387 | Source: qj64d06.x1 NCI_CGAP_Kid3 *Homo sapiens* cDNA clone IMAGE:1864235 3' similar to WP:F19B6.1 CE05666 URIDINE KINASE ;, mRNA sequence. |
| 138 34532_at | 0.016387 | Source: *Homo sapiens* clone 23705 mRNA sequence. |
| 139 35969_at | 0.016529 | Source: yx67h12.s1 Soares melanocyte 2NbHM *Homo sapiens* cDNA clone IMAGE:266855 3', mRNA sequence. |
| 140 41635_at | 0.017145 | Source: Human mRNA for KIAA0105 gene, complete cds. |
| 141 35221_at | 0.017213 | Source: *H. sapiens* mRNA for pur alpha extended 3'untranslated region. |
| 142 33456_at | 0.017213 | Source: *Homo sapiens* EB1 mRNA, complete cds. |
| 143 41866_s_at | 0.017213 | member of the dystrophin gene family |
| 144 39733_at | 0.017293 | similar to *Homo sapiens* KIA0025 product encoded by GenBank Accession Number D14695 |
| 145 35826_at | 0.017369 | Source: *Homo sapiens* transcription factor Tat-CT1 mRNA, complete cds. |
| 14633178_at | 0.017509 | similar to R. norvegicus Jagged1 protein |
| 147 35329_at | 0.017509 | similar to *Saccharomyces cerevisiae* ORF YIL043C/CBR1/CBR5/CBR |
| 148 35271_at | 0.017509 | one of seven subunits of the Arp2/3 protein complex; actin-related protein |
| 14939722_at | 0.011509 | Source: *Homo sapiens* nuclear receptor co-repressor N-Cor mRNA, complete cds. |
| 150 39740_g_at | 0.017509 | Source: *Homo sapiens* alpha NAC mRNA, complete cds. |
| 151 38086_at | 0.017509 | Source: *Homo sapiens* mRNA for KIAA0466 protein, partial cds. |
| 152 940_g_at | 0.017509 | neurofibromic 1 (neurofibromatosis, von Recklinghansen disease, Watson disease) |
| 153 37928_at | 0.017509 | Source: af53a04.s1 Soares_total_fetus_Nh2HF8_9w *Homo sapiens* cDNA clone IMAGE:1035342 3' similar to gb:X59710 CCAAT-BINDING TRANSCRIPTION FACTOR SUBUNIT A (HUMAN);, mRNA sequence. |
| 154 40852_at | 0.017509 | similar to Drosophila tudor |
| 155 35786_at | 0.017509 | Source: *Homo sapiens* mRNA for KIAA0476 protein, complete cds. |
| 156 1039_s_at | 0.017509 | basic helix-loop-helix transcription factor |
| 157 37010_at | 0.017509 | Source: qf76b12.x1 Soares_fetal_lung_NbHL19W *Homo sapiens* cDNA clone IMAGE:1755935 3' similar to SW:T2AG_HUMAN P52657 TRANSCRIPTION INITIATION FACTOR IIA GAMMA CHAIN ;, mRNA sequence. |
| 158 1772_s_at | 0.017509 | Source: Human farnesyl-protein transferase alpha-subunit mRNA, complete cds. |
| 159 38485_at | 0.017509 | Source: nz14h07.s1 NCl_CGAP_GCB1 *Homo sapiens* cDNA clone IMAGE:1287805 3', mRNA sequence. |
| 160 32752_at | 0.017509 | Source: zd65e10.s1 Soares_fetal_heart_NbHH19W *Homo sapiens* cDNA clone IMAGE:345546 3' similar to SW:N4AM_BOVIN Q05752 NADH-UBIQUINONE OXIDOREDUCTASE SUBUNIT B14.5A ;, mRNA sequence. |
| 161 37900_at | 0.017509 | membrane protein |
| 162 38648_at | 0.017682 | contains leucine repeat |
| 163 33438_at | 0.0178 | Source: *Homo sapiens* mRNA; cDNA DKFZp564D012 (from clone DKFZp564D012). |
| 164 38277_at | 0.0178 | calcineurin A1 |
| 165 39601_at | 0.0178 | RDA32; similar to norel |
| 166 36641_at | 0.0178 | similar to chicken alpha2 isoform |
| 167 33873_at | 0.017874 | Nuclear protein with DNA-binding ability |
| 168 37740_r_at | 0.018039 | ADP/ATP carrier protein |
| 169 32210_at | 0.018039 | Source: Human phosphoglucomutase 1 (PGM1) mRNA, complete cds. |
| 170 32998_at | 0.019081 | Source: Human cholecystokinin A receptor mRNA, complete cds. |
| 171 38470_i_at | 0.019104 | Source: Human mRNA for KIAA0228 gene, partial cds. |
| 172 36627_at | 0.019304 | Source: *H. sapiens* mRNA for hevin like protein. |
| 173 32183_at | 0.019304 | Gene product is 54 kDa but migrates aberrantly on SDS gels as a 70 kDa protein.; In mammalian cells, the protein colocalizes with many components of the pre-mRNA splicing machinery in the nucleus. |
| 174 32597_at | 0.019304 | Source: *H. sapiens* p130 mRNA for 130K protein. |
| 175 33912_at | 0.019346 | Source: *Homo sapiens* mRNA for famesylated-proteins converting enzyme 1. |
| 176 36112_r_at | 0.019346 | Source: *H. sapiens* PR264 gene. |
| 177 40125_at | 0.019346 | Source: *Homo sapiens* integral membrane protein, calnexin, (IP90) mRNA, complete cds. |
| 178 1446_at | 0.019389 | proteasome subunit C3 |
| 179 38702_at | 0.019389 | Source: *Homo sapiens* clone 24781 mRNA sequence. |
| 180 39762_at | 0.019389 | Source: *Homo sapiens* KIAA0425 mRNA, complete cds. |
| 181 36860_at | 0.01939 | Source: *Homo sapiens* mRNA for KIAA1064 protein, partial cds. |
| 182 409_at | 0.01939 | Source: Human mRNA for 14.3.3 protein, a protein kinase regulator. |
| 183 731_f_at | 0.019479 | |
| 184 31526_f_at | 0.019479 | Source: *H*. *sapiens* mRNA for tre oncogene (clone 213). |
| 185 36975 _at | 0.019479 | Source: 34d2 Human retina cDNA randomly primed sublibrary *Homo sapiens* cDNA, mRNA sequence. |
| 186 41418_at | 0.019479 | Source: *Homo sapiens* mRNA for KIAA0821 protein, complete cds. |
| 187 38542_at | 0.019479 | Source: *Homo sapiens* nucleophosmin phosphoprotein (NPM) gene, 3' flanking sequence. |
| 188 37994_at | 0.019479 | unnamed protein product |
| 189 32574_at | 0.019479 | Source *H*. *sapiens* mRNA for sphingomyelinase. |
| 190 33094_s_at | 0.019479 | Source:wq32a03x1 NCI_GCAP_GC6 *Homo sapiens* IMAGE:2472940 3' similar to TR:Q1515 QI3515 BEADED FILAMENT PROTEIN CP49. mRNA sequence. |
| 191 36408_at | 0.019479 | Source: *Homo sapiens* ORCTL4 mRNA for organic cation transporter like 4, complete cds. |
| 192 33740_at | 0.019673 | Source: *Homo sapiens* clk2 kinase (CLK2), propin1, cote1, glucocerebrosidase (GBA), and metaxin genes, complete cds; metaxin pseudogene and glucocerebrosidase pseudogene; and thrombospondin3 (THBS3) gene, partial cds. |
| 193 40074_at | 0.020159 | precursor polypeptide (AA -29 to 315) |
| 194 34829_at | 0.020183 | nucleolar protein; similar to yeast Cbf5p |
| 195 41474_at | 0.020183 | Source: *H. sapiens* mRNA for kinesin-2. |
| 196 35355_at | 0.020183 | Source: *Homo sapiens* mRNA for KIAA0890 protein, complete cds. |
| 197 39677_at | 0.020183 | Source: Human mRNA for KIAA0186 gene, complete cds. |
| 198 39346_at | 0.020223 | Source: Human p62 mRNA, complete cds. |
| 199 1448_at | 0.020223 | proteasome subunit C8 |
| 200 32710_at | 0.020223 | Source: *H. sapiens* mRNA for voltage gated potassium channels, beta subunit. |
| 201 887_at | 0.020223 | ORF |
| 202 33847_s_at | 0.020223 | Source: qo19f03.x1 NCI_CGAP_Lu5 *Homo sapiens* cDNA clone IMAGE:1908989 3', mRNA sequence. |
| 203 38431_at | 0.020243 | Source: Human protein kinase (JNK2) mRNA, complete cds. |
| 204 37785_at | 0.020243 | Source: U69563 Soares infant brain INIB *Homo sapiens* cDNA clone 25050, mRNA sequence. |
| 205 39856_at | 0.020243 | Source: at02f03.x1 Barstead aorta HPLRB6 *Homo sapiens* cDNA clone IMAGE:2353949 3' similar to gb:M15661 60S RIBOSOMAL PROTEIN LA4 (HUMAN);, mRNA sequence. |
| 206 766_at | 0.020243 | Source: *Homo sapiens* mRNA for galectin-9 isoform, complete cds. |
| 207 37569_at | 0.020243 | Source: *Homo sapiens* calcium binding protein (ALG-2) mRNA, complete cds. |
| 208 34312_at | 0.020243 | Source: oy33a12.x1 Soares_parathyroid_tumour_NbHPA *Homo sapiens* cDNA clone IMAGE:1667614 3',mRNA sequence. |
| 209 40083_at | 0.020243 | Source: *Homo sapiens* mRNA for KIAA0625 protein, partial cds. |
| 210 40832_s_at | 0.020283 | strong similarity to rat lamina associated polypeptide 1C |
| 211 1696_at | 0.020317 | Source: Human mRNA for DNA polymerase beta, complete cds. |
| 212 39136_at | 0.020317 | Source: *Homo sapiens* mRNA for oxidative-stress responsive 1, complete cds. |
| 213 34808_at | 0.020317 | Source: *Homo sapiens* mRNA for KIAA0999 protein, partial cds. |
| 214 33433_at | 0.020449 | Source: *Homo sapiens* mRNA; cDNA DKFZp564F0522 (from clone DKFZp564F0522). |
| 215 1663_at | 0.020494 | |
| 216 32803_at | 0.020494 | expression elevated in dividing 786-0 cells compared with confluent 786-0 cells |
| 217 34454_r_at | 0.020507 | apolipoprotein |
| 218 33247_at | 0.020554 | human homolog of fission yeast pad1 |
| 219 41606_at | 0.020554 | developmentally regulated |
| 220 34478_at | 0.020641 | Source: *H. sapiens* YPT3 mRNA. |
| 221 34306_at | 0.020641 | Source: *Homo sapiens* KIAA0428 mRNA, complete cds. |
| 222 36295_at | 0.020641 | Source: Human zinc finger protein ZNF134 mRNA, complete cds. |
| 223 35656_at | 0.020641 | Source: *Homo sapiens* mRNA for RING-H2 protein RNF6, alternative exon 1a. |
| 224 36935_at | 0.020832 | ras p21 GTP-ase-activating protein (GAP) |
| 225 37620_at | 0.020832 | TAF1120; contains homology to histone H2B; TFIID subunit |
| 226 1848_at | 0.020832 | Source: Human ras-related protein (Krev-1) mRNA, complete cds. |
| 227 35818_at | 0.021007 | Source: *Homo sapiens* mRNA for cytochrome c, partial cds. |
| 228 39180_at | 0.021107 | translocated in liposarcoma |
| 229 39792_at | 0.021292 | hnRNP R |
| 230 34063_at | 0.021292 | Source: *Homo sapiens* RecQ5 mRNA for DNA helicase, complete cds. |
| 231 1998_i_at | 0.021292 | this sequence is a new splice variant called BAX delta in which exon 3 is deleted and exons 2 and 4 are spliced to each other |
| 232 33614_at | 0.021292 | unnamed protein product |
| 233 36456_at | 0.021292 | similarity to A. thaliana PRTI |
| 234 36926_at | 0.021292 | Source: *H. sapiens* ERK3 mRNA. |
| 235 33849_at | 0.021292 | Source: Human pre-B cell enhancing factor(PBEF) mRNA, complete cds. |
| 236 32624_at | 0.021292 | strong similarity to rat tulip 1/2 |
| 237 38974_at | 0.021292 | Source: *Homo sapiens* RNA-binding protein regulatory subunit mRNA, complete cds. |
| 238 36754_at | 0.021292 | Source: *H. sapiens* gene PACAP for pituitary adenylate cyclase activating polypeptide. |
| 239 40637_at | 0.021292 | Source: Human hsc70 gene for 71 kd heat shock cognate protein. |
| 240 32565_at | 0.021292 | Unlike BAF60a and BAF60b this gene is highly expressed in muscle cells; similar to human BAF60a, BAF60b, BAF60c and yeast SWP73 and SWP73b |
| 241 36822_at | 0.021292 | putative RNA binding protein; similar to human FUS/TLS and EWS |
| 242 38099_r_at | 0.021356 | Source: *Homo sapiens* acyl-CoA synthetase 4 (ACS4) mRNA, complete cds. |
| 243 31536_at | 0.021356 | Source: *Homo sapiens* mRNA for KIAA0886 protein, complete cds. |
| 244 36196_at | 0.021356 | Source: Human phosphofructokinase (PFKM) mRNA, complete cds. |
| 245 36459_at | 0.021356 | Source: *Homo sapiens* mRNA for KIAA0879 protein, complete cds. |
| 246 40423_at | 0.021356 | Source: *Homo sapiens* mRNA for KIAA0903 protein, partial cds. |
| 247 32879_at | 0.021356 | Source: *Homo sapiens* mRNA; cDNA DKFZp586L111 (from clone DKFZp586L111). |
| 248 39389_at | 0.021356 | Source: Human CD9 antigen mRNA, complete cds. |
| 249 33368_at | 0.021356 | Source: *H. sapiens* mRNA for Lon protease-like protein. |
| 250 41127_at | 0.021356 | 2 potential N-linked glycosylation sites at residues 201 and 206; in-frame stop codon 24 bp upstream of initiator methionine |
| 251 38443_at | 0.021356 | Source: Human clone 23721 mRNA sequence. |
| 252 297_g_at | 0.021356 | |
| 253 37392_at | 0.021356 | beta subunit |
| 254 32205_at | 0.021356 | PKR interacting protein |
| 255 32665_at | 0.02145 | Source: *Homo sapiens* mRNA for protein phosphatase 2C (beta). |
| 256 39018_at | 0.02145 | GSH-transferase; GSH-peroxidase |
| 257 35839_at | 0.021826 | Source: *Homo sapiens* mRNA for squalene epoxidase, complete cds. |
| 258 32215_i_at | 0.021876 | Source: *Homo sapiens* mRNA for KIAA0878 protein, complete cds. |
| 259 329_s_at | 0.022103 | |
| 260 33027_at | 0.022103 | Source: 39d11 Human retina cDNA randomly primed sublibrary *Homo sapiens* cDNA, mRNA sequence. |
| 261 381_s_at | 0.022103 | Source: *H. sapiens* mRNA for phosphoinositide 3-kinase. |
| 262 38972_at | 0.022103 | Source: *Homo sapiens* clone 24775 mRNA sequence. |
| 263 37668_at | 0.022175 | Source: Human pre-mRNA splicing factor SF2p32, complete sequence. |
| 264 37868_s_at | 0.022178 | MOG-25.6kD |
| 265 38809_s_at | 0.022233 | Source: *Homo sapiens* mRNA for KIAA0519 protein, complete cds. |
| 266 810_at | 0.022455 | similar to Dbl exchange factor and to pleckstrin |
| 267 1612_s_at | 0.022455 | Source: Human junD mRNA. |
| 268 36981_at | 0.022455 | Source: *Homo sapiens* clone 24452 mRNA sequence. |
| 269 40570_at | 0.022455 | Source: *Homo sapiens* forkhead protein (FKHR) mRNA, complete cds. |
| 270 36536_at | 0.022475 | Source: *Homo sapiens* clone 24732 unknown mRNA, partial cds. |
| 271 38801_at | 0.022475 | Source: wg46h09.x1 Soares_NSF_F8_9W_OT_PA_P_SI *Homo sapiens* cDNA clone IMAGE:2368193 3' similar to TR:O75453 075453 VAMP-ASSOCIATED PROTEIN OF 33 KDA. mRNA sequence |
| 272 35777_at | 0.027475 | Source: *Homo sapiens* mRNA for zinc finger protein, complete cds, clone: RES4-26 |
| 273 38254_at | 0.022475 | Source: *Homo sapiens* mRNA for KIAA0882 protein, partial cds. |
| 274 38075_at | 0.022475 | Source: *H. sapiens* tr-SpI mRNA. |
| 275 34192_at | 0.022475 | Source: *Homo sapiens* mRNA for KIAA0532 protein, partial cds. |
| 276 41212_r_at | 0.022485 | Source: Human mRNA for KIAA0038 gene, partial cds. |
| 277 31510_s_at | 0.023142 | Source: *H. sapiens* hH3.3B gene for histone H3.3. |
| 278 32667_at | 0.023142 | Source: Human alpha-5 collagen type IV (COL4A5) mRNA, 3' end. |
| 279 41536_at | 0.023264 | match: proteins P47928 P41139 Q13005 |
| 280 34387_at | 0.023264 | similar to putative product coded in C. elegans cosmid C01C10. |
| 281 36042_at | 0.023264 | Source: *H. sapiens* trkB mRNA for protein-tyrosine kinase. |
| 282 38357_at | 0.023264 | Source: *Homo sapiens* mRNA; cDNA DKFZp564D156 (from clone DKFZp564D156). |
| 283 36118_at | 0.023264 | Source: *Homo sapiens* mRNA for steroid receptor coactivator 1e. |
| 284 34680_s_at | 0.023336 | Source: Human mRNA for KIAA0107 gene, complete cds. |
| 285 31962_at | 0.023449 | Source: *Homo sapiens* ribosomal protein L37a (RPL37A) mRNA, complete cds. |
| 286 33158_at | 0.023449 | Source: *Homo sapiens* Kallmann syndrome (KAL) mRNA, complete cds. |
| 287 35184_at | 0.023449 | Source: *Homo sapiens* mRNA for KIAA0546 protein, partial cds. |
| 288 35083_at | 0.023449 | similar to SW:GOLI_DROME Q06003 GOLIATH PROTEIN |
| 289 32531_at | 0.023449 | gap junction protein (AA 1-382) |
| 290 37038_at | 0.023449 | Source: *H. sapiens* PXMP1 gene, exon I (and joined CDS). |
| 291 35750_at | 0.023449 | Source: *Homo sapiens* mRNA; cDNA DKFZp564K0222 (from clone DKFZp564K0222). |
| 292 41823_at | 0.023581 | Source: *Homo sapiens* mRNA for staufen protein, partial. |
| 293 1970_s_at | 0.023697 | Source: *H. sapiens* FGFR2 mRNA. |
| 294 36488_at | 0.023982 | Source: *Homo sapiens* mRNA for MEGF9, partial cds. |
| 295 33666_at | 0.024001 | C protein |
| 296 39150_at | 0.024001 | Source: U69559 Soares infant brain 1NIB *Homo sapiens* cDNA clone 26077, mRNA sequence. |
| 297 471_f_at | 0.024001 | class III isotype; beta-3 |
| 298 33118_at | 0.024016 | match to U03056 (PID:g532974); H_LUCA14.3 |
| 299 36988_at | 0.024016 | Source: Human B12 protein mRNA, complete cds. |
| 300 32039_at | 0.024096 | similar to cerebellar degeneration antigen beta-NAP and to the beta-1-adaptin and beta-2-adaptin subunits of clathrin-associated complexes |
| 301 37040_at | 0.024109 | The ha1225 gene product is related to human alpha-glucosidase. |
| 302 36845_at | 0.024145 | The KIAA0136 gene product is novel. |
| 303 1636_g_at | 0.024172 | ABL is the cellular homolog proto-oncogene of Abelson's murine leukemia virus and is associated with the t9:22 chromosomal translocation with the BCR gene in chronic myelogenous and acute lymphoblastic leukemia; alternative splicing using exon 1a |
| 304 38710_at | 0.024734 | Source: *Homo sapiens* mRNA; cDNA DKFZp564E242 (from clone DKFZp564E242). |
| 305 37914_at | 0.024734 | Source: Human mRNA for KIAA0305 gene, complete cds. |
| 306 34355_at | 0.024895 | Source: *Homo sapiens* mRNA for methyl-CpG-binding protein 2. |
| 307 1311_art | 0.024965 | Source: Human mRNA for proteasome subunit HsN3, complete cds. |
| 308 35294_at | 0.024965 | ribonucleoprotein autoantigen 60 kd subunit |
| 309 32313_at | 0.025167 | fibroblast tropomyosin |
| 310 33082_at | 0.025167 | Source: *Homo sapiens* integrin subunit alpha 10 precursor, mRNA, complete cds. |
| 311 33821_at | 0.025167 | match: proteins: Tr:Q9Y3M0 Tr:Q9WU14 Sw:P39540 Tr.Q9Y396 |
| 312 35232_f_at | 0.025167 | Source: oz26h05.x1 Soares_total_fetus_Nb2HF8_9w *Homo sapiens* cDNA clone IMAGE:1676505 3' similar to SW:CATR_GIALA Q24956 CALTRACTIN; mRNA sequence. |
| 313 37663_at | 0.025167 | Source: *Homo sapiens* DDX1 gene, complete CDS. |
| 314 612_s_at | 0.025167 | 2',3'-cyclic-nucleotide 3'-phosphodiesterase (EC 3.1.4.37) |
| 315 36164 _at | 0.025167 | similar to the Ratins norvegiens dihydroliponamide acetyltransferase component (E2) of pyruvate dehydrogenase complex: Swiss Prot Accession Number P08461 |
| 316 38805_at | 0.025167 | Source: *H. sapiens* mRNA for TGIF protein. |
| 317 07710_f_at | 0.025182 | heterogeneous nuclear-ribonucleoprotein D-like |
| 318 34304_s_at | 0.025556 | Source: *Homo sapiens* mRNA; cDNA DKFZp586G1923 (from clone DKFZp586G1923). |
| 319 34783_s_at | 0.025678 | amino terminus similar to protein encoded by GenBank Accession Number AF047472; structurally similar to *Homo sapiens* Rael protein encoded by GenBank Accession Number U84720; similar to Mus musculus protein encoded by GenBank Accession Number U67327; contains WD40 repeat motif, also termed trp-asp, G-beta repeat; binds BUB1 kinase |
| 320 37575_at | 0.025678 | Source: *Homo sapiens* mRNA; cDNA DKFZp586C1723 (from clone DKFZp586C1723). |
| 321 39755_at | 0.025678 | match: proteins: Sw:P17861 Tr:O35426 |
| 322 37307_at | 0.025678 | G protein alpha-subunit (AA 1-355) |
| 323 36946_at | 0.025684 | human homolog of Drosophila mnb (minibrain) gene |
| 324 34425_at | 0.025684 | does not contain alpha 3 domain |
| 325 40347_at | 0.025788 | Source: o139a08.s1 Soares_NFL_T_GBC_S1 *Homo sapiens* cDNA clone IMAGE:1525814 3' similar to contains element L1 repetitive element ;, mRNA sequence. |
| 326 32402_s_at | 0.025992 | Source: *H. sapiens* mRNA for symplekin. |
| 327 32235_at | 0.026262 | Source: *Homo sapiens* mRNA for KIAA0544 protein, partial cds. |
| 328 41063_g_at | 0.02649 | Source: zc52c04.r1 Soares_senescent_fibroblasts_NbHSF *Homo sapiens* cDNA clone IMAGE:325926 5' similar to SW:BMI1_MOUSE P25916 DNA-BINDING PROTEIN BMI-1. [1] ;, mRNA sequence. |
| 329 33373_at | 0.026577 | Source: *Homo sapiens* mRNA; cDNA DKFZp564O0122 (from clone DKFZp56400122). |
| 330 34647_at | 0.026577 | p68 protein (AA 1-614) |
| 331 32240_at | 0.026673 | Source: Human mRNA for KIAA0072 gene, partial cds. |
| 332 37324_at | 0.026673 | put. transferrin receptor (aa 1-760) |
| 333 36337_at | 0.02682 | Source: wi67f11.x1 NCI_CGAP_Kid12 *Homo sapiens* cDNA clone IMAGE:2398413 3' similar to TR:O75257 075257 R31180_1. ;contains TAR1 .t1 TAR1 repetitive element;, mRNA sequence. |
| 334 33447_at | 0.02682 | myosin regulatory light chain |
| 335 39373_at | 0.026839 | Source: *Homo sapiens* clone 23716 mRNA sequence. |
| 336 32844_at | 0.026839 | elF4GI |
| 337 31509_at | 0.026839 | Source: *H. sapiens* BBCI mRNA. |
| 338 35246_at | 0.026839 | Source: Human receptor tyrosine kinase (DTK) mRNA, complete cds. |
| 339 31853_at | 0.026839 | polycomb-group protein; homolog of Drosophila esc (extra sex combs); EED |
| 340 37619 _at | 0.026839 | KIAA0094 gene product is related to *S.cerevisiae* methionine aminopeptidase. |
| 341 35790_at | 0.026839 | Source: *Homo sapiens* H beta 58 homolog mRNA, complete cds. |
| 342 32730_at | 0.027032 | Source: *Homo sapiens* mRNA; cDNA DKFZp564H142 (from clone DKFZp564H142). |
| 343 226_at | 0.027032 | Source: Human cAMP-dependent protein kinase type I-alpha subunit (PRKAR1A) mRNA, complete cds. |
| 344 37424_at | 0.027032 | a-helix coiled-coil rod homologue |
| 345 38982_at | 0.027032 | Source: 53g9 Human retina cDNA randomly primed sublibrary *Homo sapiens* cDNA, mRNA sequence. |
| 346 32504_at | 0.027032 | Source:wu69c05.x1 NCI_CGAP_Kid3 *Homo sapiens* cDNA clone IMAGE:2525288 3', mRNA sequence. |
| 347 41463_at | 0.027032 | Source: DKFZp434B0222_s1 434 (synonym: htes3) *Homo sapiens* cDNA clone DKFZp434B0222 3', mRNA sequence. |
| 348 35759_at | 0.027088 | similar to mouse chaperonin-containing TCP-1 beta subunit |
| 349 168_at | 0.027139 | Source: Human adenosine kinase mRNA, complete cds. |
| 350:35140_at | 0.027139 | Source: yh11b03.s1 Soares infant brain 1NIB *Homo sapiens* cDNA clone IMAGE:42880 3', mRNA sequence. |
| 351 36580_at | 0.027139 | Source: *Homo sapiens* mRNA; cDNA DKFZp586M141 (from clone DKFZp586M141). |
| 352 40387_at | 0.027139 | similar to mouse vzg-1 |
| 353 38657_s_at | 0.027139 | clathrin light chain a |
| 354 33110_at | 0.027139 | Source: qy22a10.x1 NCI_CGAP_Brn23 *Homo sapiens* cDNA clone IMAGE:201 2730 3' similar to gb:X71136 SOX-10 PROTEIN (HUMAN);, mRNA sequence. |
| 355 36948_at | 0.027261 | Source: *Homo sapiens* mRNA full length insert cDNA clone EUROIMAGE 45620. |
| 356 34354_at | 0.027592 | putative |
| 357 40779_at | 0.027592 | Smg GDS-associated protein having arm repeats and phosphorylated by Src tyrosine kinase |
| 358 39072_at | 0.027634 | Source: Human MXI1 mRNA, complete cds. |
| 359 1179_at | 0.027634 | |
| 360 35768_at | 0.027634 | Source: *Homo sapiens* mRNA for KIAA0661 protein, complete cds. |
| 361 37373_at | 0.027634 | similar to uridine diphosphoglucose pyrophosphorylase in human liver, Swiss-Prot Accession Number Q07131; the 5'UTR and 3'UTR of this clone are completely different from those of the liver form |
| 362 41338_at | 0.027634 | Source: wx39f10.x1 NCI_CGAP_Pit1 *Homo sapiens* cDNA clone IMAGE:2546059 3', mRNA sequence. |
| 363 35837_at | 0.027634 | Source: *Homo sapiens* mRNA for scrapie responsive protein 1. |
| 364 1675_at | 0.027634 | ras p21 GTP-ase-activating protein (GAP) |
| 365 37395_at | 0.027634 | Source: *Homo sapiens* mRNA for vacuolar ATPase, complete cds. |
| 366 41759_at | 0.027634 | Source: *H. sapiens* mRNA for RNA polymerase II elongation factor-like protein. |
| 367 36046_at | 0.027634 | similarity to SPAC2F3.16 |
| 368 35468_at | 0.027854 | Source: *Homo sapiens* mRNA; cDNA DKFZp586B2023 (from clone DKFZp586B2023). |
| 369 35403_at | 0.028018 | Source: *Homo sapiens* mRNA for KIAA1094 protein, complete cds. |
| 370 38676_at | 0.028018 | Source: z196e07.r1 Stratagene corneal stroma (#937222) *Homo sapiens* cDNA clone IMAGE:512484 5', mRNA sequence. |
| 371 1318_at | 0.028018 | Source: *H. sapiens* RbAp48 mRNA encoding retinoblastoma binding protein. |
| 372 33170_at | 0.028018 | Source: *Homo sapiens* mRNA for KIAA0962 protein, partial cds. |
| 373 38190_r_at | 0.028018 | Source: *Homo sapiens* mRNA for KIAA0645 protein, complete cds. |
| 374 35218_at | 0.028018 | apoptosis-related protein |
| 375 41528_at | 0.02862 | Source: zd62h08.s1 Soares_fetal_heart_NbHH19W *Homo sapiens* cDNA clone IMAGE:345279 3', mRNA sequence. |
| 376 37666_at | 0.02862 | Source: Human mRNA for proteasome subunit X, complete cds. |
| 377 37229_at | 0.02862 | similar to FRAP, Mec1p, Tor1p, Tor2p, and ATM |
| 378 38943_at | 0.028882 | putative |
| 379 1074_at | 0.028882 | Source: *Homo sapiens* GTP-binding protein (RAB1) mRNA, complete cds. |
| 380 1942_s_at | 0.028882 | Cyclin-dependent kinase 4 |
| 381 411_i_at | 0.029002 | Source: Human 1-8D gene from interferon-inducible gene family. |
| 382 32669_at | 0.029424 | Source: *Homo sapiens* mRNA for KIAA0671 protein, complete cds. |
| 383 37543_art | 0.029842 | this sequence overlaps D13631, it covers 954..4359 of this sequence. |
| 384 34845_at | 0.029842 | remainder of gene in clone 549K18 (AL023654) |
| 385 38817 _at | 0.029842 | Source: *Homo sapiens* sperm acrosomal protein mRNA, complete cds. |
| 386 41825_at | 0.029966 | Source: 34c6 Human retina cDNA randomly primed sublibrary *Homo sapiens* cDNA, mRNA sequence. |
| 387 41850_s_at | 0.030352 | isolated in a two hybrid screen to identity cellular proteins that interact with hepatitis delta antigen; similar to hepatitis delta antigen, and has two regions predicted to form coiled-coil protein interaction domains |
| 388 40480_s_at | 0.0309 | c-syn protooncogene; belongs to the protein-tyrosine kinase family of retroviral oncogenes |
| 389 1463_at | 0.0309 | Source: Human protein tyrosine phosphatase (PTP-PEST) mRNA, complete cds. |
| 390 AFFX-HUMISGF3 A/M97905_MB_at | 0.0309 | Source: *Homo sapiens* transcription factor ISGF-3 mRNA, complete cds. |
| 39L 37774_at | 0.0309 | Source: wj88e02.x1 NCI_CGAP_Lym12 *Homo sapiens* cDNA clone IMAGE:2409914 3' similar to SW:GBB5_HUMANG014775GUANINE NUCLEOTIDE-BINDING PROTEIN BETA SUBUNIT 5 mRNA sequence. |
| 392 38832_r_at | 0.0309 | M11319; *Homo sapiens* chromosome 7q22, sequence, complete sequence. |
| 393 33722_at | 0,031224 | Source: *Homo sapiens* mRNA for KlAA0548 protein, partial cds. |
| 394 36107_at | 0.031626 | Source: ak04e09.s1 Soares_parathyroid_tumour_NbHPA *Homo sapiens* cDNA clone IMAGE:1405000 3' similar to gb:M37104 ATP SYNTHASE COUPLING FACTOR 6, MITOCHONDRIAL PRECURSOR (HUMAN);, mRNA sequence. |
| 395 39736_at | 0.031702 | GTP-binding protein G25K |
| 396 38069_at | 0.032016 | Source: *H. sapiens* mRNA for CLC-7 chloride channel protein. |
| 397 556_s_at | 0.032345 | Source: Human glutathione transferase class mu number 4 (GSTM4) gene, complete cds. |
| 398 38336_at | 0.032745 | hj06791 cDNA clone for KIAA1013 has a 4-bp deletion at position between 1855 and 1860 of the sequence of KIAA1013. |
| 399 33913_at | 0.032745 | HLA-B-associated transcript 2 (BAT2) |
| 400 33358_at | 0.03283 | Source: 56b8 Human retina cDNA randomly primed sublibrary *Homo sapiens* cDNA, mRNA sequence. |
| 401 767_at | 0.032913 | Source: Human Chromosome 16 BAC clone CIT987SK-A-815A9, complete sequence. |
| 402 35163_at | 0.033005 | Source: *Homo sapiens* mRNA for KIAA1041 protein, complete cds. |
| 403 1278_at | 0.033089 | |
| 404 1695_at | 0.033089 | Source: *Homo sapiens* mRNA for ubiquitin-like protein, complete cds. |
| 405 34648_at | 0.033089 | Source: *H. sapiens* mRNA for SSR alpha subunit. |
| 406 40211_at | 0.033171 | Source: Human gene for heterogeneous nuclear ribonucleoprotein (hnRNP) core protein A1. |
| 407 34085_at | 0.033329 | Source: *H. sapiens* gene for ribosomal protein L38. |
| 408 1635_at | 0.033329 | ABL is the cellular homolog proto-oncogene of Abelson's murine leukemia virus and is associated with the t9:22 chromosomal translocation with the BCR gene in chronic myelogenous and acute lymphoblastic leukemia; alternative splicing using exon 1a |
| 409 37675_at | 0.033565 | Source: *H. sapiens* mRNA for mitochondrial phosphate carrier protein. |
| 410 38110_at | 0.033587 | mda-9; pbp-1 |
| 411 40771_at | 0.033587 | match: proteins: Sw:P26038 Tr:O35763 Sw:P26041 Sw:P26042 Sw:P26044 Sw:P35241 Sw:P26043 Sw:P15311 Sw:P31976 Sw:P26040 Tr:Q26520 Tr:Q24788 Tr:Q24796 Tr:Q94815 |
| 412 869_at | 0.033639 | general transcription factor IIA, 2 (12kD subunit) |
| 41341203_at | 0.033639 | putative |
| 414 40426_at | 0.033639 | Source: *H. sapiens* mRNA for BCL7B protein. |
| 415 36032_at | 0.033639 | partially supported by FGENES and GENSCAN |
| 416 39376_at | 0.033662 | Source: *Homo sapiens* mRNA for KIAA0630 protein, partial cds. |
| 417 39380_at | 0.033775 | Source: *Homo sapiens* mRNA for KIAA0697 protein, partial cds. |
| 418 40091_at | 0.033775 | 6 C2H2 zinc finger zinger repeats from 520 to 691 in protein sequence |
| 419 33866_at | 0.03399 | tropomyosin (AA 1-248) |
| 420 34809_at | 0.03399 | Source: yq87g03.r1 Soares fetal liver spleen INFLS *Homo sapiens* cDNA clone IMAGE:202804 5', mRNA sequence. |
| 421 39778_at | 0.03402 | Source: Human N-acetylglucosaminyltransferase I (GlcNAc-TI) mRNA, complete cds. |
| 422 36121_at | 0.034483 | Source: *Homo sapiens* mRNA for KIAA1065 protein, complete cds. |
| 423 41116_art | 0.034861 | Source: wc43d09.x1 NCI_CGAP_Pr28 *Homo sapiens* cDNA clone IMAGE:2321393 3' similar to SW:YD84 SCHPO Q10409 HYPOTHETICAL 32.6 KD PROTEIN C1F3.04C IN CHROMOSOME 1. mRNA sequence. |
| 424 34781_at | 0.034861 | Source: Human WS-3 mRNA, complete cds. |
| 425 34812_at | 0.034907 | Source: 22f11 Human retina cDNA randomly primed sublibrary *Homo sapiens* cDNA, mRNA sequence |
| 426 32276_at | 0.035041 | rpI.32 (aa I-135) |
| 427 36636_at | 0.035115 | Source: Human ornithine aminotransferase mRNA, complete cds. |
| 428 37674_at | 0.035221 | 5-aminolevulinate synthase precursor |
| 429 41814_at | 0.033359 | alpha-L-fucosidase precursor (EC 3215) |
| 430 37907_at | 0.035531 | CpG island protein |
| 431 35195_at | 0.03601 | Source: *H. sapiens* mRNA for phosphate cyclase. |
| 432 35720_at | 0.036032 | Source: *Homo sapiens* mRNA for KIAA0893 protein, complete cds. |
| 433 34600_s_at | 0.036032 | Source: Human tub homolog mRNA, complete cds. |
| 434 32526_at | 0.036255 | hypothetical protein FLJ14529 |
| 435 33295_at | 0.036255 | Source: *H. sapiens* DARC gene. |
| 436 41404_at | 0.036255 | Source: *Homo sapiens* mRNA for Ribosomal protein kinase B (RSK-B). |
| 437 41170_at | 0.036255 | Source: *Homo sapiens* mRNA for KIAA0663 protein, complete cds. |
| 438 34774_at | 0.036255 | Source: Human palmitoyl protein thioesterase mRNA, complete cds. |
| 439 35670_at | 0.036255 | Source: Human Na+,K+ -ATPase catalytic subunit alpha-III isoform gene, exon 23, clone lambda-NK-alpha-R3-2. |
| 440 33835_at | 0.036255 | Source: *Homo sapiens* mRNA for KIAA0721 protein, partial cds. |
| 441 35756_at | 0.036255 | Source: *Homo sapiens* RGS-GAIP interacting protein GIPC mRNA, complete cds. |
| 442 38679_g_at | 0.036255 | Source: zg79b05.s1 Soares_fetal_heart_NbHH19W Homo *sapiens* cDNA clone IMAGE:399537 3' similar to gb:M65125_ma1 U1 AND U2 SMALL NUCLEAR RIBONUCLEOPROTEIN E (HUMAN);, mRNA sequence. |
| 443 1081_at | 0.036261 | ornithine decarboxylase |
| 444 41559_at | 0.036261 | Source: zw24f03.r1 Soares ovary tumour NbHOT *Homo sapiens* cDNA clone IMAGE:770237 5', mRNA sequence. |
| 445 40587_s_at | 0.03647 | Source: *Homo sapiens* p18 protein mRNA, complete cds. |
| 446 1884_s_at | 0.03647 | cyclin |
| 447 37348_s_at | 0.036478 | Source: ak01go01.s1 Soares_parathyroid_tumour_NbHPA *Homo sapiens* cDNA clone IMAGE:1404720 3' similar to SW:TRI7_HUMAN Q15651 THYROID RECEPTOR INTERACTING PROTEIN 7 ;, mRNA sequence. |
| 448 37759_at | 0.036478 | Source: Human lysosomal-associated multitransmembrane protein (LAPTm5) mRNA, complete cds. |
| 449 40182_s_at | 0.036776 | Source: *Homo sapiens* clone 24658 mRNA sequence. |
| 450 32543_at | 0.036921 | Source: Human autoantigen calreticulin mRNA, complete cds. |
| 451 32750_r_at | 0.036934 | actin-binding protein |
| 452 117_at | 0.036934 | heat-shock protein HSP70B |
| 453 32548_at | 0.036934 | Source: Human (p23) mRNA, complete cds. |
| 454 41594_at | 0.036934 | Source: Human protein-tyrosine kinase (JAK1) mRNA, complete cds. |
| 455 37732_at | 0.036934 | Source: *Homo sapiens* mRNA; cDNA DKFZp564E1922 (from clone DKFZp564E1922). |
| 456 41128_at | 0.03697 | Source: *Homo sapiens* clone 24606 mRNA sequence. |
| 457 35903_at | 0.037598 | Source: Human oligodendrocyte-myelin glycoprotein (OMGP) mRNA, complete cds. |
| 458 33809_at | 0.037604 | GTP-binding regulatory protein Gi alpha-1 chain |
| 459 33466_at | 0.037604 | Source: *Homo sapiens* clone 23860 mRNA sequence. |
| 460 39897_at | 0.037937 | Source: yy39g07.s1 Soares melanocyte 2NbHM *Homo sapiens cDNA clone* IMAGE:273660 3'; mRNA sequence. |
| 461 34950_at | 0.037937 | Source: *Homo sapiens* mRNA for KIAA0760 protein, partial cds. |
| 462 41530_at | 0.037937 | Source: Human mRNA for mitochondrial 3-oxoacyl-CoA thiolase, complete cds. |
| 463 34753_at | 0.037937 | Source: *H. sapiens* mRNA for novel gene in Xq28 region. |
| 464 36160_s_at | 0.037937 | Source: Human protein tyrosine phosphatase receptor pi (PTPRP) mRNA, complete cds. |
| 465 34592_g_at | 0.037937 | ribosomal protein S 17 |
| 466 34752_at | 0.038065 | Source: *Homo sapiens*; mRNA; cDNA DKFZp586G2222 (from clone DKFZp586G2222). |
| 467 34213_at | 0-038065 | Source *Homo sapiens* mRNA for KIAA0869 protein, partial cds. |
| 468 33893_r_at | 0.038065 | Source: *Homo sapiens* mRNA for KIAA0470 protein; complete cds. |
| 469 34392_s_at | 0.038065 | Rab1, splice variant |
| 470 38045_at | 0.038065 | a novel member of the Armadillo family interacting with presenilin 1 (PS1); presenilin associated protein |
| 471 38062_at | 0.038065 | Similar to a C.elegans guanine nucleotide releasing factor homolog (S4 2368) |
| 472 38573_at | 0.038065 | zinc finger protein |
| 473 32119_at | 0.038065 | Source: *Homo sapiens* mRNA; cDNA DKFZp586B211 (from clone DKFZp586B211). |
| 474 31955_at | 0.038065 | Source: *H. sapiens* fau mRNA. |
| 475 31899_at | 0.038065 | Source: Human mRNA for KIAA0103 gene, complete cds. |
| 476 38991_at | 0.038065 | Source: HSU55980 Human brain ARSanders *Homo sapiens* cDNA clone 25453 3', mRNA sequence. |
| 477 39809_at | 0.038065 | HBP1 |
| 478 41375_at | 0.038065 | Source: *Homo sapiens* mRNA for G7b protein (G7b gene, located in the class III region of the major histocompatibility complex. |
| 479 1356_at | 0.038065 | Source: Human ionizing radiation resistance conferring protein mRNA, complete cds. |
| 480 39009_at | 0.038065 | Source: yy66d08.r1 Soares_multiple_sclerosis_2NbHMSP *Homo sapiens* cDNA clone IMAGE:278511 5', mRNA sequence. |
| 481 39327_at | 0.038065 | similar to D.melanogaster peroxidasin(U11052) |
| 482 41449_at | 0.038065 | Source: *Homo sapiens* mRNA for epsilon-sarcoglycan. |
| 483 40775_at | 0.038065 | 5' end of gene beyond this clone; match: protein Q61500 |
| 484 36511_at | 0.038065 | Source: *Homo sapiens* mRNA for KIAA0851 protein, complete cds. |
| 485 35833_at | 0.038065 | Source: *Homo sapiens* mRNA; cDNA DKFZp4340071 (from clone DKFZp434O071). |
| 486 35845_at | 0.038065 | Source: *Homo sapiens* mRNA for Sec24 protein (Sec24B isoform). |
| 487 36271_at | 0.038065 | Source: *Homo sapiens* mRNA for KIAA1024 protein, partial cds. |
| 488 41715_at | 0.038065 | Source: *H. sapiens* mRNA for phosphoinositide 3-kinase. |
| 489 41665_at | 0.038065 | Source: *Homo sapiens* mRNA for KIAA0824 protein, partial cds. |
| 490 35342_at | 0.038065 | Source: *Homo sapiens* clone 24416 mRNA sequence. |
| 491 41799_at | 0.038065 | Source: 48h8 Human retina cDNA randomly primed sublibrary *Homo sapiens* cDNA, mRNA sequence. |
| 492 36603_at | 0.038065 | similar to Yeast translation activator GCN1 (P1:A48126) |
| 493 39766_r_at | 0.038229 | Source: tr08h04.x1 NCI_CGAP_Ov23 *Homo sapiens* cDNA clone IMAGE:2217751 3' similar to SW:RPCX_HUMAN P53803 DNA-DIRECTED RNA POLYMERASES I, II, AND III 7.0 KD POLYPEPTIDE ;, mRNA sequence. |
| 494 38100_at | 0.038407 | Source: *Homo sapiens* mRNA for translocation protein-1, complete cds. |
| 495 39441_at | 0.038506 | Source: *Homo sapiens* mRNA for lanthionine synthetase C-like protein 1 (LANCL1 gene). |
| 496 38398_at | 0.038994 | Source: Human mRNA for KIAA0358 gene, complete cds. |
| 497 33108_i_at | 0.039132 | putative |
| 498 35102_at | 0.039332 | Source: *Homo sapiens* zinc finger protein mRNA, 3' end. |
| 499 2065_s_at | 0.039432 | Source: Human Bax alpha mRNA, complete cds. |
| 500 34814_at | 0.039631 | Source: DKFZp434D0717_s1 434 (synonym: htes3) *Homo sapiens* cDNA clone DKFZp434D0717 3', mRNA sequence. |
| 501 41436_at | 0.039781 | Source: *Homo sapiens* mRNA for ZNF198 protein. |
| 502 33451_s_at | 0.039781 | Source: DU3.2-7.G09 DU-145 *Homo sapiens* cDNA 3', mRNA sequence. |
| 503 37489_s_at | 0,039781 | Source: Human anion exchanger 3 brain isoform (bAE3) mRNA, complete cds. |
| 504 36650_at | 0.040102 | Source: Human mRNA for KIAK0002 gene, complete cds. |
| 505 34849_at | 0.040102 | Source: *H. sapiens* mRNA for seryl-tRNA synthase. |
| 506 743_at | 0.040122 | putative |
| 507 1072_g_at | 0.040122 | source: Human transcription factor GATA-2 (GATA-2) mRNA, complete cds. |
| 508 1660_at | 0.040122 | Source: Human epidermoid carcinum mRNA for ubiquitin-conjugating enzyme EZ similar to Drosophila bendless gene product, complete cds. |
| 509 428_s_at | 0.040122 | Source: Human messenger RNA fragment for the beta-2 microglobulin. |
| 510 837_s_at | 0.040122 | Source: Human breast cancer cytosolic NADP(+)-dependent malic enzyme mRNA, partial cds. |
| *511* 38652_at | 0.040388 | Source: *Homo sapiens* clone 24742 mRNA sequence. |
| 512 33301_g_at | 0.040401 | |
| 513 35307_at | 0.040403 | Source: *Homo sapiens* mRNA for GDP dissociation inhibitor beta. |
| 514 40537_at | 0.040659 | Source: *Homo sapiens* mRNA for KIAA0741 protein, complete cds. |
| 515 40018_at | 0.040659 | Source: *Homo sapiens* KIAA0410 mRNA, complete cds. |
| 516 38993_r_at | 0.040683 | Source: 32a12 Human retina cDNA randomly primed sublibrary *Homo sapiens* cDNA, mRNA sequence. |
| 517 33369_at | 0.040683 | Source: P9-C4.T3.P9.D4 conorm *Homo sapiens* cDNA 3', mRNA sequence. |
| 518 38065_at | 0.040683 | Source: *H. sapiens* HMG-2 mRNA. |
| 519 630_at | 0.040702 | Source: *Homo sapiens* deoxycytidylate deaminase gene, complete cds. |
| 520 39026_r_at | 0.040775 | Source: *Homo sapiens* clone 23887 mRNA sequence. |
| 521 37298_at | 0.040775 | Source: *Homo sapiens* MM46 mRNA, complete cds. |
| 522 566_at | 0.040775 | 145 kda neural cell adhesion molecule; This sequence comes from Fig. 4 |
| 523 1226_at | 0.041055 | transmembrane metalloproteinase/disintegrin; adamalysin; TACEA |
| 524 36165_at | 0.041055 | Source: zc48b04.r1 Soares_senescent_fibroblasts_NbHSF *Homo sapiens* cDNA clone IMAGE:325519 5' similar to gb:X13238 CYTOCHROME C OXIDASE POLYPEPTIDE VIC PRECURSOR (HUMAN);, mRNA sequence. |
| 525 32510_at | 0.041215 | 2-carboxybenzaldehyde reductase; member of aldo-keto reductase AKR7 family |
| 526 39791_at | 0.041257 | Source: *Homo sapiens* calcium-ATPase (HK1) mRNA, complete cds. |
| 527 31809 _at | 0.041257 | Source: *H. sapiens* contactin mRNA,. |
| 528 36100_at | 0.041257 | Source: *Homo sapiens* vascular endothetial growth factor mRNA, complete cds. |
| 529 33924_at | 0.041338 | Source: *Homo sapiens* mRNA for KIAA1091 protein, partial cds. |
| 530 40846_g_at | 0.041338 | Source: Human nuclear factor NF90 mRNA, complete cds. |
| 531 38664 at | 0.041338 | Source: *Homo sapiens* BCNT mRNA, complete cds. |
| 532 41283_at | 0.041494 | Source: *Homo sapiens* clone 23930 mRNA sequence. |
| 533 38042_at | 0.041843 | G6PD(AA 1-515) |
| 534 1627_at | 0.041843 | |
| 535 35152_at | 0.041927 | Source: *Homo sapiens* mRNA encoding RAMP3. |
| 536 33864_at | 0.041927 | binds directly to adenovirus type 5 E1A protein |
| 537 32254_at | 0.041979 | Source: *Homo sapiens* mRNA; cDNA DKFZp586L1323 (from clone DKFZp586L1323). |
| 538 40435_at | 0.041983 | ADP.ATP translocase |
| 539 37616_at | 0.041993 | Source: *H. sapiens* AUH mRNA. |
| 540 1213_at | 0.042177 | similar to human serine kinase SRPK1, encoded by GenBank Accession Number U09564; specific for serine/arginine-rich splicing factors |
| 541 818_s_at | 0.042207 | XH2; XNP; alternatively spliced product 1; contains all exons; translation starts in exon 9; ATRX gene deposited in GenBank Accession Numbers U72900-U72935 |
| 542 37181_at | 0.042334 | Source: *H. sapiens* Mpv17 mRNA. |
| 543 35776_at | 0.042686 | Source: *Homo sapiens* intersectin short form mRNA, complete cds. |
| 544 31820_at | 0.042778 | haematopoietic lineage cell protein (AA 1-486) |
| 545 41344_s_at | 0.042778 | Source: *H. sapiens* Pur(pur-alpha) mRNA, complete cds. |
| 546 1468_at | 0.042778 | TNF type I receptor associated protein |
| 547 38306_at | 0.043327 | Source: zu44b03.r1 Soares ovary tumour NbHOT *Homo sapiens* cDNA clone IMAGE:740813 5', mRNA ' sequence |
| 548 34326_at | 0.043502 | Source: *H.* sapiens mRNA for beta-COP. |
| 549 39442_at | 0.043534 | une-50 related protein homolog |
| 550 35619_at | 0.043649 | Source: *Homo sapiens* mRNA for KlAA0634 protein, partial cds. |
| 551 1953_at | 0.043649 | Vascular endothelial growth factor |
| 552 37580_at | 0.043649 | predicted; contains SH3 domain; member of the EEN gene family |
| 553 33351_at | 0.04411 | similar to SUII |
| 554 33752_at | 0.044234 | Source: *Homo sapiens* mRNA for K1AA0850 protein, complete cds. |
| 555 41542_at | 0.044234 | Source: *Homo sapiens* zinc finger protein 216 splice variant 1(ZNF216) mRNA, complete cds. |
| 556 32853_at | 0.044234 | Source: *Homo sapiens* mRNA for KIAA0719 protein, complete cds. |
| 557 34876_at | 0.044268 | duck gp180 homolog |
| 558 35722_at | 0.044288 | similarity to *S.cerevisiae* Nmd2p |
| 559 40636_at | 0.044411 | Source: wf49b01.xl Soares_NFL_T_GBC_S1 *Homo sapiens* cDNA clone IMAGE:2358889 3' similar to TR:O75955 O75955 FLOTILLIN-1. ;, mRNA sequence. |
| 560 33443_at | 0.044411 | match: proteins P38533 Q03933 P38530 P41154 Q00613 P38529 P38531 Q63717 P38532 Q99472 |
| 561 555_at | 0.044411 | small GTP binding protein, homologous to *Saccharomyces cerevisiae* SEC4, Swiss-Prot Accession Number P07560 |
| 562 38120_at | 0.044659 | autosomal dominant polycystic kidney disease type II |
| 563 40263_at | 0.045291 | Source: *Homo sapiens* clone lambda MENI region unknown protein mRNA, complete cds. |
| 564 31797 _at | 0.046069 | Source: Human DNA sequence from clone 73H22 on chromosome 6q23, complete sequence. |
| 56541194_at | 0.046069 | Source: PT1.3_04_C04.r tumour1 *Homo sapiens* cDNA 5', mRNA sequence. |
| 566 37532_at | 0.0468 | Source: Human medium-chain acyl-CoA dehydrogenase (MCAD) gene, exon 12. |
| 567 883_s_at | 0.0468 | Source: Human h-pim-1 protein (h-pim-1) mRNA, complete cds. |
| 568 AFFX-HUMRGE/M 10098_5_at | 0.0468 | Source: Human 18S rRNA gene, complete. |
| 569 39033_at | 0.047068 | Source: HSZ78368 Human fetal brain S. Meier-Ewert *Homo sapiens* cDNA clone 3.142 (CEPH), mRNA sequence. |
| 570 33249_at | 0.047068 | mineralocorticoid receptor |
| 571 36480_at | 0.047068 | alpha subunit |
| 572 36563_at | 0.047068 | Source: *Homo sapiens* clone 23582 mRNA sequence. |
| 573 35746_r_at | 0.047095 | Source: *H. sapiens* hnRNP-E2 mRNA. |
| 574 38743_f_at | 0.047214 | Source: Human mRNA fragment for activated c-raf-1 (exons 8-17). |
| 575 41110_at | 0.047214 | Source: *H. sapiens* mRNA for vasopressin activated calcium mobilizing receptor-like protein. |
| 576 35187_at | 0.048079 | Source: *Homo sapiens* mRNA; cDNA DKFZp586K1123 (from clone DKFZp586K1123). |
| 577 41296_s_at | 0.048079 | Source: 37c5 Human retina cDNA randomly primed sublibrary *Homo sapiens* cDNA, mRNA sequence. |
| 578 781_at | 0.048079 | subunit of modification enzyme; beta subunit |
| 579 32228_at | 0.048079 | Source: *Homo sapiens* mRNA for KIAA0899 protein, partial cds. |
| 580 35090_g_at | 0.048079 | Source: *Homo sapiens* mRNA for NTAK, complete cds. |
| 581 457_s_at | 0.048079 | conjugated post-transiationally to RanGAP1; ubiquitin-related protein; similar to UBL1 encoded by GenBank Accession Number U38784, PIC1 encoded by GenBank Accession Number U61397 and GMP1 encoded by GenBank Accession Number U72722 |
| 582 37333_at | 0.048079 | Source: *H. sapiens* mRNA for DNA (cytosin-5)-methyltransferase. |
| 583 35683_at | 0.048079 | Source: *Homo sapiens* mRNA for KIAA0852 protein, complete cds. |
| 584 1253_at | 0.048079 | Source: Human protein kinase mRNA, complete cds. |
| 585 31807_at | 6.04809 | Source: U69190 Soares infant brain 1N1B *Homo sapiens* cDNA 27655, mRNA sequence |
| 586 430_at | 0.048334 | PNP |
| 587 1280_i_at | 0.048396 | |
| 588 37274_at | 0.048396 | biotin-amide amidohydrolase |
| 589 34940_at | 0.048884 | Source: we38g03.x1 NCI_CGAP_Lu24 *Homo sapiens* cDNA clone IMAGE:2343412 3', mRNA sequence. |
| 590 40308_at | 0.049324 | Source: wh51h03.x1 NCl_CGAP_Kid11 *Homo sapiens* cDNA clone IMAGE:2384309 3', mRNA sequence. |
| 591 41573_at | 0.049324 | Source: *H. sapiens* SPR-2 mRNA for GT box binding protein. |
| 592 34739_at | 0.049324 | Source: 18c3 Human retina cDNA randomly primed sublibrary *Homo sapiens* cDNA, mRNA sequence. |
| 593 40517_at | 0.049343 | Source: Human mRNA for KIAA0372 gene, complete cds. |
| 594 35983_at | 0.049343 | Hypothetical human protein (partial CDS); CDS constructed from combination of BLASTX, EST matches and Xgrail predictions. N-terminus of protein likely encoded in flanking cosmid R29942. Predicted protein exhibits weak similarity to hypothetical protein PID\|e1226191 (AL021106) from *Drosophila melanogaster* |
| 595 37895_at | 0.049343 | Source: *Homo sapiens* mRNA for CMP-sialic acid transporter, complete cds. |
| 596 33877_s_at | 0.049343 | Source: *Homo sapiens* mRNA for KIAA1067 protein, partial cds. |
| 597 34368_at | 0.049343 | similar to yeast RPD3, encoded by GenBank Accession Number X78454 |
| 598 41266_at | 0.049343 | Source: Human mRNA for integrin alpha 6. |
| 599 32589_at | 0.049516 | Source: Human chromatin assembly factor-I p150 subunit mRNA, complete cds. |
| 600 36907_at | 0.049516 | Source: *Homo sapiens* mevalonate kinase mRNA, complete cds. |
| 601 35703_at | 0.049564 | PDGF-A (AA 1 - 196) |
| 602 31932_f_at | 0.04966 | Source: Human basic transcription factor 3a (BTF3a) gene, complete cds. |

Serum levels were determined for IGFB2 and found to be decreased, in agreement with the decreased transcript levels in several organs, including the liver.

**TABLE 8**

| Serum Determination of Insulin-Like Growth Factor Binding Protein 2 | | |
|---|---|---|
| Treatment | conc (ng/mL) | mean (ng/mL) |
| | 1467 | |
| | 1742 | |
| control | 922 | 1583 |
| | 2201 | |
| | 1239.8 | |
| FGF23CTP100 µg/day | 987.6 | 893 |
| | 596.7 | |
| | 749.5 | |

*Analysis.* Angiogenesis is further shown to be inhibited by FGF23CTP in a hyperoxia-induced angioproliferative retinopathy model (Aiello FP et al., Proc. Natl. Acad. Sci. USA, 92: 10457-61(1995), Ozaki H et al., Am. J. Pathol. 156: 697-707 (2000)) in C57/B6 mice (see EXAMPLE VIII).The proliferative components of the retinal angiopathy induced through a relative ischemia by transition of postnatal mice from hyperoxic to normoxic conditions is significantly inhibited (p=0.018) by the intravitreal injection of the FGF23CTP (GPA006) peptide.

In the rostral hypothalamus, besides its effects on angiogenesis and cycling genes, FGF23CTP affects several molecules that have been described to play a role in the pathogenesis of malignant proliferation of glial cells and precursors (malignant brain tumours): epithelial growth factor (EGF; Hoi Sang U et al., J. Neurosurg. 82: 841-6 (1995), Wu CJ et al., Oncogene 19: 3999-4010 (2000)), Bax (Streffer JR et al., J. Neurooncol. 56: 43-9 (2002); Martin *et al.,* (2001)), connexin 43 (Huang R et al., Cancer Res. 62: 2806-12 (2002), Soroceanu L et al., Glia 33: 107-17 (2001)), PKR (Shir A & Levitzki A, Nature Biotechnology 20: 895-900 (2002)), NF1 (Cichowski K & Jacks T, Cell 104: 593-604 (2001), Gutmann DH et al., Hum. Mol. Genet. 10: 3009-16 (2001)).

### EXAMPLE IV

### INTEGRATED INVESTIGATIVE PHARMACOLOGY THROUGH IN VIVO GENE EXPRESSION PROFILING IN NON-HUMAN PRIMATES

The discovery method of the invention has been validated through a "blind" monkey trial using three peptides of well known pharmacological activity: (1) the somatostatin analogue SOM230, (2) gonadotrophin releasing hormone (GnRH), (3) and leukemia inhibitory factor (LIF). In each case, a "blind" test with 3 "unknown" polypeptides was performed. The results demonstrated the capacity of the gene expression analyst teams to identify, within four months, the pharmacological activities, most of the therapeutic indications and side effects, and even the identity of the proteins. These first results demonstrate that the discovery method of the invention usefully provide the art with advantage in the understanding of drug pharmacological mechanisms and the potential side effects, the selection of biomarkers and potential new indications.

For the verification of the selected human proteins or peptides in cynomolgus monkeys, one control and four treated groups (*i.e.*, each of three peptides and placebo) of two males and two females are treated for two weeks by daily administration of the proteins dissolved in autologous serum (*e.g.*, in each animal's own serum) through the subcutaneous route. The administration of the peptides was blinded. The amount of peptide administrated was 100 µg/animal /day (5-6 mg of peptide total).

Drug profiling in these non-human primates was analyzed using gene expression profiling of more than 100 organs on Affymetrix U95 chips (each containing 1/3 of the human genome). In addition, extensive biochemistry and clinical chemistry screening (> 60 parameters) and histopathology (ca. 60 organs) were performed.

Data mining procedures were then used. The data were probed to answer four questions: (1) Is the polypeptide worth further investigations? (2) What are the physiological pathways impacted? (3)What are the potential indications? (4) What is the likelihood of the identity of the polypeptide?
*Peptide 1.* Peptide 1 was SOM230. SOM230 (pasireotide) has a chemical structure cyclo[4-(NH₂-C₂H₄-NH-CO-O)Pro-Phg-DTrp-Lys-Tyr(4-Bzl)-Phe] as follows:

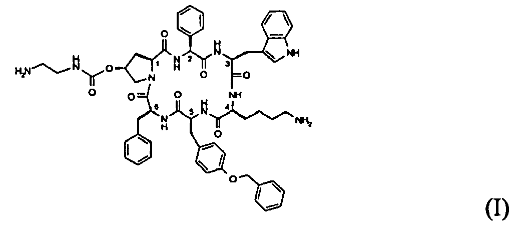

Here, Phg means -HN-CH(C₆H₅)-CO- and Bzl means benzyl. See, PCT patent application WO 02/10192. SOM230 is a somatostatin analogue with binding affinities for the five somatostatin receptors except somatostatin receptor 4 (SSTR4). S O M 2 3 0 has been developed for several indications, including those disclosed above for other somatostatin analogues. See, Lewis I et al., J. Med. Chem. 46(12):2334-44 (June 5, 2003); Weckbecker G et al., Endocrinology 143(10): 4123-4130 (2002); Kneissel M et al., Bone 28:237-250 (2001); and Thomsen JS et al., Bone 25:561-569 (1999), the contents of which are incorporated herein by reference.

SOM230 was developed for the approved Sandostatin® indications, but as a more potent somatostatin analogue with a longer plasma half-life *in vivo.* Lewis I et al., J Med Chem 46(12): 2334-44 (June 5, 2003); Weckbecker G et al., Endocrinology 143(10): 4123-30 (October 2002). In contrast with other analogues, SOM230 binds to all somatostatin receptors except SSTR4. The binding affinity for the different somatostatin receptors was a basis for defining the scope of possible new clinical indications for SOM230. Bruns C et al., Eur J Endocrinol 143 (Suppl 1) : S3 7 (2000); Bruns C et al., Eur J Endocrinol. 146(5):707-16 (May 2002). In addition, other possible new indications were suggested due to the improved activity of SOM230 for growth hormone and IGF-1 regulation and its different inhibitory effects on insulin and glucagon secretions.

Following the blinded administration of peptide 1 (SOM230), the following results were obtained:

**TABLE 9**

| Findings by the Data Mining Team | |
|---|---|
| Analysis | Results |
| Biochemistry/ | Thymus and spleen atrophy, aldosterone-, glucagon-, vitamin D-, PTH+, |
| Histopathology | RBC hemoglobin-, urinary calcium-, NAG+, TSH+ in male, |
| Pituitary | *IGFR1-,* GH1/2-, CSH(L)-, TSH-, CHRH2-, Prostaglandin D2 synthase++, Immunoglobulin-- |
| Adrenals | IGF2-, somatostatin-, insulin receptor+, urocortin+ |
| Pancreas | *Metallothionein 1A,1E*++, trypsin, chymotrypsin, carboxypeptidases, phospholipaseA2/1B ++, *18S--,* |
| Stomach | *Gastrin*+, hypocretin+, glutamate receptor+, GABAB-, IGFBP1+, |
| Thymus | MHC classII-, Ig-, FKBP12-, CD4-, defensin alphal+, |
| Bone marrow | VIP-, peptide YY-, cyclin-, GAPDH- |
| Spleen | *IGF2-*, IGBP6-, insulin receptor-, urocortin+, Ig-, ribosomal protein --, Nk cells+ |
| Thyroid | NPY-, cyclins--, ATPase calcium channel+, somatostatin receptor 4-, |
| Liver | *IGF2-,* NMDA receptor+, glutamate dehydrogenase+, VIP, insulin-induced protein1, *STR5* |
| Kidney | Solute carrier protein 9++, urocortin+, IGF2-, arrestin beta 2-, THRα+, |

The underlined results in the TABLE above were identified for further investigation.

**TABLE 10**

| Peptide 1: Conclusions of the *in vivo* Profiling by the Data Mining Team | |
|---|---|
| Function/Pathway | Potential Indications |
| Anti-growth activity | Cytostolic; cancer |
| GI tract/pancreatic function | Obesity, food intake regulation, diabetes, pancreatic function regulation |
| Immunosuppression | Suppression of specific immunity, maintenance of innate immunity |

Based upon these results, the Data Mining Team made predictions as to the identity of the administered Peptide 1.

**TABLE 11**

| Predicted Identity of Peptide 1 (Somatostatin Analogue SOM230) | |
|---|---|
| Data miner | Prediction |
| 1 | Somatostatin, NPY, |
| 2 | IGF-1, Somatostatin, IGF-2 |
| 3 | Somatostatin, IGF-1, IGF-2 |
| 4 | Somatostatin, IGF-1, IGF-2 |
| 5 | Secretin, IGF-1, Renin |
| Summary Prediction | Somatostatin, IGF-1, IGF-2 |

Based upon these results, SOM230 was selected for further development as a somatostatin analogue to treat the somatostatin-approved neuroendocrine indications (*e.g.*, acromegaly, gastroenteropancreatic tumours) and also has potential for further indications with a pancreatic endocrine eetiology such as diabetic angiopathy and morbid obesity associated with hyperinsulinemia. IGF-1 serum level was proposed as surrogate marker. Other potential indications for SOM230 are therefore inflammation (*e.g.*, psoriasis), pain and immunosuppression (*e.g.*, chronic rejection).

*Peptide 2.* Following the blinded administration of peptide 2 (gonadotrophin releasing hormone), the following results were obtained:

**TABLE 12**

| Findings by the Data Mining Team | |
|---|---|
| Analysis | Results |
| Biochemistry | Slight increase in aldosterone |
| Kidney | Thiol protease inhibitor (aldosterone + in biochemistry), metabolism: +GAPDH, +STAT, +JAK, +TEGT |
| Pituitary Gland | *GH-, GNRH receptor-,* Somatostatin+, *FSH+, LH-,* TSH-, CSH(L)-, IGF/IGFBP-/+, tubulin+, |
| Ovary | *Nuclear receptors-, aromatase and estrogen synthase+,* activinA-, inhibin beta B+, |
| Testis | *CREM+* |
| Thyroid | Ocludin+, thyroid hormone binding protein+, THR alpha- |
| Adrenals | Follistatin+, IGF2+, catecholamines synthesis+, activin A-/inhibin+ |
| Hypothalamus | Transthyretin+, oxytocin |
| Bone marrow | Macrophages+, eosinophils+, activin A-, inhibin betaB+, |
| Thymus | Integrin alpha6+, VAP1+, lipid metabolism, selenium binding protein+, |
| Mammary | Inconclusive; Sampling heterogenous |
| gland | |
| Liver | Activin A-, inhibin betaB+, lipid metabolism+, glycolysis+, follistatin+, IDO1+, VEGF- |

The underlined results in the TABLE above were identified for further investigation.

**TABLE 13**

| Peptide 2: Conclusions of the *in vivo* Profiling by the Data Mining Team | |
|---|---|
| Function/Pathway | Potential Indications |
| Ovarian and testicular | Fertility, stimulation of reproductive function, |
| functions | stimulation of steroidogenesis |

Based upon these results, the Data Mining Team made predictions as to the identity of the administered Peptide 2.

**TABLE 14**

| Predicted Identity of Peptide 2 (GnRH) | |
|---|---|
| Data miner | Prediction |
| 1 | GnRH, FSH, HCG |
| 2 | FSH, IGF1, GnRH |
| 3 | FSH, Somatotropin, LH |
| 4 | GnRH, FSH, IGF1 |
| 5 | FSH, LHRH, IGF-1 |
| Summary Prediction | FSH, GnRH, LH-(RH) |

Based upon these results, the predicted indications for the administered Peptide 2 (gonadotropin releasing hormone) were be for gonadotrophin releasing hormone-related indications or for lutenizing hormone releasing hormone-related indications, such as antiproliferative disorders (cancer), ovarian and testicular functions (hypothalamic gonadotropc hypogonadism, fertility control and delayed or arrested puberty/ precocious puberty), and growth hormone deficiencies.

*Peptide 3.* Following the blinded administration of peptide 3 (leukemia inhibitory factor), the following results were obtained:

**TABLE 15**

| Findings by the Data Mining Team | |
|---|---|
| Analysis | Results |
| Biochemistry/ | *Calcitonin*++++, osteocalcin+, PTH-, bone alkaline phosphatase-, |
| Haematology | IC terminal polypeptide-, T4+, TSH+, alpha globin1+, platelet count +, WBC- |
| Liver | C/EBP delta+, JunB+, NFIL3+, CREM, quiescin Q6+, BTG2+, TCR-, BCL6+, antileukoproteinase+, selectinP+, VCAM+, ephrin A1+, *Ostepontin+, calcitonin+,* TIMP-1,, plasminogen inhibitor+, IL1R1, cholesterol hydrolase+, LDL receptor, phospholipase AII type II + |
| Kidney | C/EBP delta+, JunB+, NFIL3+, CREM, immunoglobulin-, MCP1+, mannose receptor+, BCR+, VCAM+, *ephrinA2*+, osteopontin+, stanniocalcin+, *TIMP-1*+, +, urokinase+, ILR1+, LDL receptor, phospholipase AII type II + |
| Thyroid | C/EBP delta+, JunB+, NFIL3+, CREM, STAT3+, CXCR4+, myeloid leukemia sequence+, *Ig*-, ephrinA1+, osteopontin+, TIMP-1+, haptoglobin, fibronectin+, phospholipase A2 typeIIA+, PDGF+ |
| Skeletal muscle | JunB, haptoglobin, phospholipase A2 typeIIA+ |
| Spleen | JunB+, STAT inhibitor 3, calgranulinB+, CD5+, BCR+, ephrin A1 exostoses+, TIMP-1 +, fibronectin+, ILR1+, LDL receptor+ |
| Adrenals | C/EBP delta+, JunB+, NFIL3+, CREM, BCR+, TIMP-1+, urokinase+, IL1R1+, LDL receptor |
| Pituitary | C/EBP delta+, JunB+, NFIL3+, TCR-, BCL6+" antileukoproeinase+, ephrin A1+, osteopontin+, chitinase 3-like1+, TIMP-1, plasminogen activator inhibitor, metallothionein+, haptoglobin+, IL1R1+ |

The underlined results were identified for further investigation.

**TABLE 16**

| Peptide 3: Conclusions of the *in vivo* Profiling by the Data Mining Team | |
|---|---|
| Function/Pathway | Potential Indications |
| Ca⁺⁺ bone metabolism | Osteoarthritis, Osteoporosis |
| Inflammation | |
| Immunosuppression | Transplantation |
| Vascular differentiation | Angiogenesis |
| growth factors | |

Based upon these results, the Data Mining Team made predictions as to the identity of the administered Peptide 2

**TABLE 17**

| Predicted Identity of Peptide 3 (LIF) | |
|---|---|
| Data miner | Prediction |
| 1 | TGFβ2, IL-6, IFN gamma |
| 2 | TGFβ2, IL-6, Calcitonin |
| 3 | VIP |
| 4 | PDGF, GMCSF, Sertonin |
| 5 | LIF, IL3, TGFβ2 |
| 6 | Oncostatin M, TGFβ2, IL3 |
| Summary Prediction | IL-6, IL-3, TGFβ2, LIF/Onccostatin |

Based upon these results, the predicted actions for the administered Peptide 3 would be to increase platelets, myeloid cells and megakaryocytes; to increase acute phase proteins (such as C-Reactive Protein (CRP)and haptoglobin); to decrease lipogenicity (with therapeutic indications for treating obesity and cardiovascular risk); to decrease transaminase, albumin and lactate dehydrogenase (LDH); and to decrease alkaline phosphatase, bone sialoprotein (BSP) and osteocalcin (with therapeutic indications for treating osteoarthritis and osteoporosis).

In summary, the three questions that were posed to the data miners could be answered almost fully by the data miners. All three polypeptides were identified as being worth further investigation. All of the physiological pathways known to be impacted by these three administered polypeptides were discovered (*e.g.* bone fractures for LIF, gastrointestinal tract for SOM230). Eighty % of their known potential indications were discovered and some new ones were identified.

### EXAMPLE V

### SOM230-INDUCED GENE EXPRESSION PROFILING IN MONKEYS

*Introduction and summary.* Microarray gene expression assays were performed using tissues of monkeys treated with SOM230 at sub-therapeutic dose for 14 days. The assays were analyzed to identify the modes of actions of SOM230 with possible relationships to therapeutic applications. For a description of SOM230, see the EXAMPLE above.

All monkey tissues examined (thyroid, brown fat, pituitary, pancreas, liver, kidney, spleen) demonstrated changes in the genes regulated by the binding of the natural somatostatin 14 (SST-14) and somatostatin 28 (SST-28) to somatostatin receptors (SSTRs). The transcript profiles reflected the known somatostatin actions on the growth hormone/insulin-like growth factor 1 (GH/IGF-1), glucagon/insulin axes and on cell proliferation. However, the compound affected significantly the transcript levels of other related genes like insulin-like growth factor 2 (IGF-2) in the pituitary and kidneys. This could be a candidate biological marker (biomarker) of drug efficacy provided that the change in protein biosynthesis would be reflected in an easily accessible tissue like the blood. Other known effects of somatostatin and agonists on growth factors, cells of the immune system and the cardio-vascular and renal functions were also reflected by the changes in the profiles of these classes of genes after SOM230.

*Origin of tissue and processing.* Male and female cynomolgus monkeys received subcutaneously SOM230 (100 µg/animal/day) or the vehicle for 14 days. On day 15, all animals were sacrificed and tissues for RNA extraction were immediately snap frozen and kept at -80° C until processing.

**TABLE 18**

| Origin of Tissues Used for Analysis | | | | | |
|---|---|---|---|---|---|
| Tissue Sample | Animal or sample no. | Sex | Tissue/ organ | Compound | Dose(µg/animal/day) |
| x547e | W62405 | Male | Brown fat | SOM230 | 100 |
| x548e | W62406 | Male | Brown fat | SOM230 | 100 |
| x549e | W62425 | Female | Brown fat | SOM230 | 100 |
| x550e | W62426 | Female | Brown fat | SOM230 | 100 |
| x673e | W62401 | Male | Brown fat | Control | 0 |
| x675e | W62421 | Female | Brown fat | Control | 0 |
| x676e | W62422 | Female | Brown fat | Control | 0 |
| x857e | W62501* | Male | Brown fat | Control | 0 |
| x858e | W62502* | Male | Brown fat | Control | 0 |
| x859e | W62551* | Female | Brown fat | Control | 0 |
| x860e | W62552* | Female | Brown fat | Control | 0 |
| d32e | W62551 | Female | Kidney | Control | 0 |
| d35e | W62502 | Male | Kidney | Control | 0 |
| d37e | W62552 | Female | Kidney | Control | 0 |
| d45e | W62501 | Male | Kidney | Control | 0 |
| x407e | W62401 | Male | Kidney | Control | 0 |
| x408e | W62402 | Male | Kidney | Control | 0 |
| x409e | W62421 | Female | Kidney | Control | 0 |
| x410e | W62422 | Female | Kidney | Control | 0 |
| x521e | W62405 | Male | Kidney | SOM230 | 100 |
| x522e | W62406 | Male | Kidney | SOM230 | 100 |
| x523e | W62425 | Female | Kidney | SOM230 | 100 |
| x524e | W62426 | Female | Kidney | SOM230 | 100 |
| x401e | W62401 | Male | Liver left lateral lobe | Control | 0 |
| x402e | W62402 | Male | Liver left lateral lobe | Control | 0 |
| x403e | W62421 | Female | Liver left lateral lobe | Control | 0 |
| x404e | W62422 | Female | Liver left lateral lobe | Control | 0 |
| x517e | W62405 | Male | Liver left lateral lobe | SOM230 | 100 |
| x518e | W62406 | Male | Liver left lateral lobe | SOM230 | 100 |
| x519e | W62425 | Female | Liver left lateral lobe | SOM230 | 100 |
| x520e | W62426 | Female | Liver left lateral lobe | SOM230 | 100 |
| x529e | W62405 | Male | Pancreas | SOM230 | 100 |
| x530e | W62406 | Male | Pancreas | SOM230 | 100 |
| x531e | W62425 | Female | Pancreas | SOM230 | 100 |
| x532-2e | W62426 | Female | Pancreas | SOM230 | 100 |
| x641e | W62401 | Male | Pancreas | Control | 0 |
| x642e | W62402 | Male | Pancreas | Control | 0 |
| x645e | W62421 | Female | Pancreas | Control | 0 |
| x646e | W62422 | Female | Pancreas | Control | 0 |
| x413e | W62401 | Male | Pituitary gland | Control | 0 |
| x414e | W62402 | Male | Pituitary gland | Control | 0 |
| x415e | W62421 | Female | Pituitary gland | Control | 0 |
| x513-2e | W62405 | Male | Pituitary gland | SOM230 | 100 |
| x514e | W62406 | Male | Pituitary gland | SOM230 | 100 |
| x515e | W62425 | Female | Pituitary gland | SOM230 | 100 |
| x516e | W62426 | Female | Pituitary gland | SOM230 | 100 |
| x425e | W62401 | Male | Spleen | Control | 0 |
| x426e | W62402 | Male | Spleen | Control | 0 |
| x427e | W62421 | Female | Spleen | Control | 0 |
| x428e | W62422 | Female | Spleen | Control | 0 |
| x525e | W62405 | Male | Spleen | SOM230 | 100 |
| x526e | W62406 | Male | Spleen | SOM230 | 100 |
| x527e | W62425 | Female | Spleen | SOM230 | 100 |
| x528e | W62426 | Female | Spleen | SOM230 | 100 |
| d33e | W62501 | Male | Thyroid | Control | 0 |
| d40e | W62551 | Female | Thyroid | Control | 0 |
| d43e | W62502 | Male | Thyroid | Control | 0 |
| d48e | W62552 | Female | Thyroid | Control | 0 |
| x443e | W62401 | Male | Thyroid | Control | 0 |
| x445e | W62421 | Female | Thyroid | Control | 0 |
| x446e | W62422 | Female | Thyroid | Control | 0 |
| x505e | W62425 | Female | Thyroid | SOM230 | 100 |
| x506e | W62426 | Female | Thyroid | SOM230 | 100 |
| x507e | W62405 | Male | Thyroid | SOM230 | 100 |
| x508e | W62406 | Male | Thyroid | SOM230 | 100 |

RNA expression profiling was conducted by means of the HG-U95A gene expression probe array (Affymetrix; Santa Clara, Calif., USA), containing more than 12,600 probe sets interrogating primarily full-length human genes and also some control probe sets. The assays were conducted according to the recommendations of the manufacturer. Briefly, total RNA was obtained by acid guanidinium thiocyanate-phenol-chloroform extraction (TRIzol®, Invitrogen Life Technologies, San Diego, Calif., USA) from each frozen tissue section. The total RNA was then purified on an affinity resin (Rneasy®, Qiagen) and quantified. Double stranded cDNA was synthesized with a starting amount of appproximately 5 µg full-length total RNa using the Superscript® Choice System (Invitrogen Life Technologies, Carlsbad, Calif. USA) in the presence of a T7-(dT)24 DNA oligonucleotide primer. Following synthesis, the cDNA was purified by phenol/chloroform/isoamylalcohol extraction and ethanol precipitation. The purified cDNA was then transcribed in vitro using the BioArray® High Yield RNA Transcript Labelling Kit (ENZO, Farmingdale, New York USA) in the presence of biotinylated ribonucleotides form biotin labelled cRNA. The labelled cRNA was then purified on an affinity resin (Rneasy®, Qiagen), quantified and fragmented. An amount of approximately 10 µg labelled cRNA was hybridized for 16 hours at 45°C to an expression probe array. The array was then washed and stained twice with streptavidin-phycoerythrin (Molecular Probes, ) using the GeneChip® Fluidics Workstation 400 (Affymetrix, Santa Clara, Calif. USA). The array was then scanned twice using a confocal laser scanner (GeneArray® Scanner, Agilent, Palo Alto, Calif. USA) resulting in one scanned image. This resulting ".dat-file" was processed using the MAS4 program (Affymetrix) into a ".cel-file". The ".cel file" was captured and loaded into the Affymetrix GeneChip® Laboratory Information Management System (LIMS). The LIMS database is connected to a UNIX Sun Solaris server through a network filing system that allows for the average intensities for all probes cells (CEL file) to be downloaded into an Oracle database (NPGN). Raw data was converted to expression levels using a "target intensity" of 150. The data were evaluated for quality control and loaded in the GeneSpring® software 4.2.4 (Silicon Genetics, Calif. USA) for analysis.

On the human Affymetrix HGU95Av2 chip, probe sets for individual genes contain 20 oligonucleotide pairs, each composed of a "perfect match" 25-mer and a "mismatch" 25-mer differing from the "perfect" match oligonucleotide at a single base. After probe labelling, hybridization, and laser scanning, the expression level was estimated by averaging the differences in signal intensity measured by oligonucleotide pairs of a given probe (AvgDiff value). The fold changes and directions were calculated for selected genes, from the differences of the AvgDiff values between control and treated.

To identify genes that were impacted by SOM230, the dataset was initially filtered to exclude in a first wave of analysis, genes whose values were systematically in the lower expression ranges where the experimental noise is high (at least 80 in a number of assays corresponding to the smallest number of replicas of any assay point). In a second round of selection a threshold p-value of 0.05 (based on a t-test) identified differences between treated and control based on a two component error model (Global Error Model) and, whenever possible, with a stepdown correction for multi-hypothesis testing (Benjamini and Hochberg false discovery rate). The decision to keep or reject a specific gene was based on the conjunction of numerical changes identified by comparative and statistical algorithms and the relationship to other modulated genes that point to a common biological theme. The weight of this relationship was assessed by the analyst through a review of the relevant scientific literature.

For the assay analysis described herein: (1) The increase and decrease in expression referred to the RNA expression level unless specifically stated. (2) If there were multiple probe sets representing the same gene, the probe set designed for sense target was favored. (3) The changes in gene expression indicated that a pathway, a cellular activity or component represented by an individual gene might be impacted. Understanding the functional implication is dependent on the information available on the biological context of the transcript level change (gene function, physiological variation, other gene changes, tissue, compound). RT-PCR is used to identify the extent of absolute change in mRNA levels, but this method in general does not add more information on the relevance of the transcript level changes.

Among the 12,600 genes per chip, about 100 genes were found to reflect the compound signature in a particular tissue. For clarity, they were divided in different classes and subdivided, with many overlaps, into functional categories in the following TABLE.

**TABLE 19**

| SOM230 Gene Expression Profiling | | | |
|---|---|---|---|
| CLASS | PITUITARY | BROWN FAT | PANCREAS |
| SIGNAL TRANSDUCTION | | | |
| *1) Phophatidyl inositol and related pathways*/*PKC, phospho- lipases* | •IP-4-phosphatase, type | • PI-3-kinase regulatory | •IP-1-phosphatase |
| | 1, isoform b ↓x2 | subunit, polypeptide 2 | ↑x2.5 |
| | • PI-3-kinase,catalytic, α | (p85 β) ↓x3.5 | • PI-4-kinase, |
| | polypeptide ↓x3 | • PI glycan, class F ↓x2 | catalytic, |
| | •PI-3-kinase, catalytic, δ | •PLCβ4 ↑ x2 | αpolypeptide ↑x1.5 |
| | polypeptide | • PI glycan, class L ↑x3.5 | •PL A2, group IVC |
| | ↓x2 | | (cytosolic, calcium- |
| | • 1-PI-4-phospahate 5- | | independent) ↑ x1.5 |
| | kinase isoform C ↓x1.5 | | |
| | -PI transfer protein, β | | |
| | ↓ x 2.5 | | |
| | • PLCγ1 ↓1.5 | | |
| | • PKC inhibitor ↑x2 | | |
| | •IP3 receptor, type 1 | | |
| | ↑ x1.5 | | |
| *2) Other calcium*/ *calcineurin*/ *calmodulin dependent pathways and associated proteins* | • Calcium/calmodulin- dependent protein kinase I ↑x2.5 | • Calcium/calmodulin- dependent protein kinase I ↓ x3.5 | |
| | • Receptor (calcitonin) activity modifying protein 2 precursor ↑x3.5 | | |
| | | | |
| *3) Ras*/*MAPK kinase*/*ERK kinase related pathways and adaptor proteins* | • Rab | • Ras homolog gene | • SH3 domain |
| | geranylgeranyltransferase, | family, member G (rho G) | binding glutamic |
| | α subunit ↓x1.5 | • MAPKAPK 3 | acid-rich protein |
| | • Rab3 GTPase-activating | • MAPKK1 | • MAPKAPK 3 |
| | protein, non catalytic | • MAPK 8 | • Ras like GTPase |
| | subunit ↓x2 | • RAB6, member RAS | • RaP2 interacting |
| | • SHB adaptor protein (a | oncogene family | protein 8 |
| | Src homology 2 protein) | • Adaptor-related protein | |
| | ↓ x2.5 | complex 3, σ1 subunit | |
| | • MAPKKK5 ↑x2 | • Ras-related nuclear | |
| | • Rab geranyltransferase, | protein | |
| | βsubunit ↑ x2 | • Rab acceptor 1 | |
| | • RAB 5C, member RAS | (prenylated) | |
| | oncogene family ↑ x3 | • RAB 2, member RAS | |
| | | oncogene family | |
| | | • IQ motif containing | |
| | | GTPase activating protein | |
| | | 2 | |
| *4) JAK*/*STAT pathway and related kinases* | • STAT 1, 91kδ ↓ x2 | •JAK 3 | • STAT 5B |
| | • JAK 1 ↑x2 | | • STAT 1, 91kδ |
| *5) Protein tyrosine phosphatases*/*other phosphatases* | • Dual specificity | • PP 2, regulatory subunit | • STAT 2 PTP δ |
| | phosphatase 8 ↓ x3 x3 | B (B56), γ isoform ↑ x1.5 | • PP 1, regulatory |
| | • Phosphatase and tensin | • PP 5, catalytic subunit | (inhibitor) subunit 8 |
| | homolog (mutated in | ↑ x2.5 | • PP 2A, catalytic |
| | multiple advanced cancers | • Dual specificity PP | subunit B' |
| | 1) ↓x3.5 | MAP-5 ↑ x2.5 | • PP 1A (formerly |
| | • PTP, receptor type, T | | 2C), magnesium- |
| | ↑ x2 | | dependent, α isoform |
| | • PP 1, regulatory | | • PP 2A, regulatory |
| | (inhibitor) subunit 5 | | subunit B' |
| | ↑x3.5 | | • Dual specificity |
| | | | phosphatase 8 |
| *6) Other protein kinases and associated binding proteins* | • Arg PTK-binding | • PTK 9-like (A6-related | • Protein kinase |
| | protein ↓ x2.5 | protein) | (c AMP-dependent, |
| | | •PTK A kinase (PRKA) | catalytic), inhibitor γ |
| | | anchor protein 1 | • Serine/threonine |
| | | • cAMP-dependent | protein kinase |
| | | protein kinase R1-β | • Receptor PTK |
| | | regulatory subunit | • Serine/threonine |
| | | • Ribosomal protein S6 | kinase 11 (Peutz- |
| | | kinase, 90kD, polypeptide | Jeghers syndrome) |
| | | | • Tyrosine kinase |
| | | | • Ribosomal protein |
| | | | S6 kinase, 90kδ, |
| | | | polypeptide 3 |
| | | | |
| *7) Adenylate*/*guanylate cyclases and related pathways* | • Soluble adenylyl cyclase | | |
| | ↓ x2 | | |
| | | | |
| CELL SURFACE | | | |
| RECEPTORS | | | |
| 1*) G-protein coupled receptors and related binding proteins*/ *G proteins* | • GTP-binding protein (G | • G α inhibiting activity | • G protein-coupled |
| | protein), q polypeptide | polypeptide 3 interacting | receptor 39 |
| | ↓x1.5 | protein ↓x5 | • G protein-coupled |
| | • GTP-binding protein | • G protein-coupled | receptor 49 |
| | like-1 ↓x3 | receptor 1↓x2.5 | • G protein-coupled |
| | • G-protein coupled | • Guanine nucleotide | receptor 3 |
| | receptor 49 ↓ x2 | binding protein (G | • Regulator of G- |
| | • G protein-coupled | protein), β polypeptide 3 | protein signalling 10 |
| | receptor, family C, group | ↑x2.5 | • GTP-binding |
| | 5, member B ↑ x2 | • SSTR3↑x6.5 | protein |
| | • GTP-binding protein 11 | • Endothelial | • SSTR2 ↓x1.5 |
| | ↑ x2.5 | differentiation, | |
| | • Receptor tyrosine | sphingolipid G-protein- | |
| | kinase-like orphan | coupled receptor, 5 ↑x2.5 | |
| | receptor 2 ↑x2 | | |
| | • ATP(GTP)-binding | | |
| | protein ↑x1.5 | | |
| | • G-protein coupled | | |
| | receptor 9 ↑x2.5 | | |
| | • Regulator of G-protein | | |
| | signalling 9 | | |
| | ↑x2 | | |
| | -SSTR3 ↑x3 | | |
| *2) Growth factors, their receptors and related binding proteins* | •FGFR 2 ↓ x2 | • Fms-related tyrosine | •Smad 3 ↓ x1.5 |
| | • EGFRBP 2 ↓ x1.5 | kinase 1 (VEGF/ vascular | • G-CSF protein ↓ x2 |
| | • Fms-related tyrosine | permeability factor | • PDGFR α ↑ x2.5 |
| | kinase 1 (VEGF/vascular | receptor) ↓ x4.5 | • PDGFR, |
| | permeability factor | • EGF receptor pathway | α polypeptide ↑ x1.5 |
| | receptor) ↓ x1.5 | substrate 15 ↓ x2 | |
| | • Catenin (cadherin- | • CSF 1 (macrophage) | |
| | associated protein), | ↓ x2 | |
| | α 1 (102kD) ↓ x1.5 | • Cadherin 13, H-cadherin | |
| | • PDGFβ ↓ x2 | (heart) ↓ x2 | |
| | • GFR bound protein 10 | • Cadherin F1B ↓ x4 | |
| | ↑ x 1.5 | • Endothelial cell GF 1 | |
| | • Butyrate response factor | (platelet derived) ↓ x2 | |
| | 2 (EGF-response factor 2) | • TGF β -activated kinase- | |
| | ↑ x3 | binding protein 1 ↑ x2.5 | |
| | • VEGF B ↑ x1.5 | • CSF 3 receptor | |
| | | (granulocyte) ↑ x3 | |
| | | • TGF β 3 ↑ x2.5 | |
| | | • Cadherin 5, VE-cadherin | |
| | | (vascular epithelium) ↑ x3 | |
| | | • VGF nerve growth | |
| | | factor inducible ↑ x2 | |
| | | • IL 3 (CSF, multiple) | |
| | | ↑ x2 | |
| | | • IL 7R precursor ↑ x2 | |
| *3) Glutamate receptor and related binding proteins* | • GLUR 2, precursor ↑ x1.5 | • GLUR, metabotropic 1 ↑ x2 | • GLUR precursor, flip isoform ↑ x3 |
| ATP-DEPENDENT | | | |
| TRANSPORT | | | |
| PROTEINS | | | |
| *Ion channels and related pathways* | • K+ channel, subfamily | • Ca++ channel, voltage | • K+ voltage-gated |
| | K, member 3 (TASK) | dependent, α 1H subunit | channel, KQT-like |
| | ↓ x4 | ↑ x3 | subfamily, member3 |
| | • K + voltage-gated | • G protein-activated | ↓ x2.5 |
| | channel, Shab-related | inwardly rectifying K+ | • Ca++ channel, |
| | subfamily, member 1 ↓ x2 | channel ↑ x3.5 | voltage dependent, α |
| | • ATPase, H+/K+ | | 1F subunit |
| | exchanging, α polypeptide | | ↓ x4.5 |
| | ↓ x5 | | • Na+ channel, |
| | • ATPase, Na+/K+ | | voltage-gated, type I, |
| | transporting, α 2 (+) | | β polypeptide ↑ x2 |
| | polypeptide ↑ x2.5 | | |
| | • ATPase, Na+/K+ | | |
| | transporting, β 3 | | |
| | polypeptide ↑ x2.5 | | |
| | • ATPase, Ca++ | | |
| | transporting cardiac | | |
| | muscle, slow twitch 2 | | |
| | ↑ x1.5 | | |
| | • Putative Ca++ | | |
| | transporting ATPase ↑ x2 | | |
| CELL BIOLOGY/ | | | |
| SPECIALIZED | | | |
| FUNCTIONS | | | |
| *1) Neuromediators*/ *neuromodulators and related pathways* | • Cholinergic | • Dopamine receptor D3 ↑ | |
| | receptor,nicotinic, | x2.5 | |
| | β polypeptide 4 ↓ x2 | • Adrenergic, β-3-, | |
| | • Cholinergic receptor, | receptor ↑ x2.5 | |
| | muscarinic 3 ↓ x3 | | |
| | • Brain cannabinoid | | |
| | receptor 1 ↑ x2 | | |
| | • GABA-B R 1, isoform a | | |
| | precursor | | |
| | ↑ x1.5 | | |
| *2)Pancreatic*/ *gastro- intestinal secretions and related pathways* | • Cholecystokinin | | Chymotrypsin-like |
| | receptor ↓ x4.5 | | ↑ x3.5 |
| | • Gastrin receptor ↓ x2 | | • Gastrin-releasing peptide receptor ↓ x5 |
| *3) Hormones and related pathways* | • IGF-2 ↓ x1.5 | • THR interactor 10 ↓ x3 | • CRHR 1 ↓ x2 |
| | • Thyroid transcription | • THR interactor 12 | • THR binding |
| | factor 1 ↑ x2.5 | ↓ x1.5 | protein |
| | • Glucagon receptor ↑ x7 | • IGF-1 ↓ x1.5 | ↓ x2 |
| | • IGFBP, acid labile | • IGF-binding protein 4 | • THR interactor 10 |
| | subunit ↑ x3.5 | ↓ x2 | ↑ x2.5 |
| | • Adrenomedullin ↑ x2.5 | • IRS (insulin receptor | • IGF-1 ↑ x4.5 |
| | • ANP (atrial natriuretic | substrate) 2 ↑ x2.5 | • Prostacyclin |
| | peptide precursor B) ↑ x2 | • T3 receptor ↑ x2 | synthase |
| | • SSTR3 ↑ x3 | • SSTR3 ↑ x6.5 | ↑ x2 |
| | | • Oxytocin, prepro- | • SSTR2 ↓ x1.5 |
| | | (neurophysin I) ↑ x2.5 | |
| | | • FSHR ↑ x2.5 | |
| *4) Cytoskeletton and associated proteins* | • Thrombospondin-p50 | • Capping protein (actin | • Integrin α 2b |
| | ↓ x2 | filament), gelsolin-like | precursor ↑ x1.5 |
| | • CD36 antigen ↓ x2 | ↓ x2.5 | |
| | | • Actin related protein 2/3 | |
| | | complex, subunit 1A | |
| | | (41kD) ↓ x2.5 | |
| *5) Enzymes* | | • Coagulation factor | • Thrombospondin 2 |
| | | XIIIAI subunit precursor | ↑ x3.5 |
| | | ↓ x5 | |
| IMMUNITY | • TNFR-associated factor | • IFN γ-inducible protein | • IL 1 receptor |
| | 2 ↓ x4 | 30 (IP30) ↓ x3.5 | antagonist ↓ x2 |
| | • TNFR subfamily, | • Pentaxin-related gene, | • LT b4 receptor |
| | member 14; herpesvirus | rapidly induced by IL-1 | (chemokine receptor |
| | entry mediator ↓ x2.5 | ↓ x7.5 | like-1) ↓ x1.5 |
| | • IFNR2 (α, β and ω) | • IFN induced | • Phosphotyrosine |
| | ↓ x2.5 | transmembrane protein 1 | independent ligand |
| | • CC chemokines STCP-1 | ↑ x2.5 | p62B for the Lck SH2 |
| | ↑ x1.5 | • TNF type 1 receptor | domain B-cell |
| | • IFN stimulated gene | associated protein ↓ x2 | isoform ↓ x2 |
| | ↑ x3.5 | • IL 5R, α↑ x2.5 | • IFN regulatory |
| | • IFNγ-inducible protein | • CD2 antigen | factor 3 ↑ x5 |
| | 30 (IP30) ↑ x1.5 | (cytoplasmic tail)-binding | |
| | | protein 2 ↑ x2.5 | |
| CELL CYCLE | • Forkhead box O3A | • G1 to S phase transition | • Cyclin T2 ↓x2 |
| | ↓ x1.5 | ↓ x1.5 | • Cyclin D1 ↑ x2 |
| | • Cyclin F ↓ x2 | • Extra spindle poles, | • Cdki 1C ↑ x2 |
| | • Core-binding factor, runt | *S. cerevisiae,* homolog of | |
| | domain, α subunit 2; | ↓ x2.5 | |
| | translocated to, 1; cyclin | • PCNA ↓ x2.5 | |
| | D-related ↑ x3 | • Follistatin-like 3 | |
| | • S-phase response | glycoprotein ↑ x3 | |
| | (cyclin-related) ↑ x2 | • Cyclin T2 ↑ x2.5 | |
| | • Cell division cycle 25B | | |
| | ↑ x5 | | |
| | • Cyclin D3 ↑ x2.5 | | |
| | • Cdki2C (p18, inhibits | | |
| | CDK4) ↑ x2 | | |
| | • Cdki2D (p19, inhibits | | |
| | CDK4) ↑ x1.5 | | |
| | • Forkhead box H1 ↑ x2 | | |
| APOPTOSIS | | | |
| | • BCL2-associated | • Neuroblastoma ↓ x2.5 | • BCL2/adenovirus |
| | athanogene ↑ x2 | • Neuroblastoma | E1B 19kD-intracting |
| | • BCL2-antagonist of cell | apoptosis-related RNA | protein 1, isoform |
| | death ↑ x2 | bindingprotein ↓ x3 | BNIP1-a ↓ x1.5 |
| | • Bax γ ↑ x1.5 | • Apotosis-associated | • Neuroblastoma |
| | • BCL2/adenovirus E1B | tyrosine kinase ↑ x3.5 | apoptosis-related |
| | 19kD-interacting protein 3 | | RNA binding protein |
| | ↑ x1.5 | | ↓ x3 |
| | • Programmed cell death 6 | | |
| | ↑ x1.5 | | |
| | • Neuroblastoma- | | |
| | amplified protein | | |
| | ↑ x1.5 | | |

**TABLE 20**

| SOM230 Gene Expression Profiling (Continued) | | | | |
|---|---|---|---|---|
| CLASS | KIDNEY | LIVER | SPLEEN | THYROID |
| SIGNAL | | | | |
| TRANSDUCTION | | | | |
| *1) Phophatidyl inositol and related pathways*/*PKC, phospholipases* | • PI-3- kinase, | • PI transfer protein, | • PI-3-kinase, | • IP3 receptor |
| | catalytic, α | β ↓ x1.5 | catalytic, α | type 3 ↓ x1.5 |
| | polypeptide ↓ x3 | • 1- PI-4-phosphate | polypeptide ↓ x2.5 | • PLC, γ 1 |
| | | 5-kinase isoform C | • PI-3- kinase, class | (formerly |
| | | ↓ x2 | 3 ↑ x2 | subtype 148) |
| | | • PLA2 ↑ | x2 | ↓ x2 |
| | | Glycosylphosphatid | • PKC, binding | • PIP 5- |
| | | ylinositol specific | α T x2 | phosphatase |
| | | phospholipase D1 | protein • IP-4-phosphatase, | type IV ↓ x3 |
| | | ↓ x1.5 | type 1, isoform b | • DAG 1 |
| | | • PKC, τ ↓ x1.5 | ↑ x2 | kinase, α |
| | | • PLC, γ 1 (formerly | • PI-3-kinase, class | (80kD) ↓ x4 |
| | | subtype 148) ↑ x2 | 2, β polypeptide | |
| | | • PKC substrate | ↑ x1.5 | |
| | | 80K-H ↑ x1.5 | • Phosphatidyl | |
| | | • PLA2, group IIA | inositol glycan, class | |
| | | (platelets, synovial | B ↑ x1.5 | |
| | | fluid) ↑ x5.5 | | |
| | | • PI transfer protein | | |
| | | ↑ x3.5 | | |
| | | • Nck, Ash and PLC | | |
| | | γ binding protein | | |
| | | NAP4 ↑ x3.5 | | |
| | | • DAG kinase, α | | |
| | | (80kD) ↑ x3 | | |
| | | • DAG kinase, δ | | |
| | | (130kD) ↑ x1.5 | | |
| | | • IP 5-phosphatase | | |
| | | ↑ x2 | | |
| *2) Other calcium*/ *calcineurin*/ *calmodulin dependent pathways and associated proteins* | • PP 3 (formerly | • Calcium/ | • Nuclear factor of | • FKBP- |
| | 2B), catalytic | calmodulin- | activated T-cells, | associated |
| | subunit, β isoform | dependent protein | cytoplasmic, | protein ↓ x2.5 |
| | (calcineurin A β) | kinase kinase 2, β | calcineurin- | • Calmodulin- |
| | ↓ x2 | ↑ x3 | dependent 1 ↓ x5 | dependent PK |
| | • Calmodulin 3 | • Calmodulin 2 | • Calmodulin I | IV (CaM-kinase |
| | (phosphorylase | (phosphorylase | (phosphorylase | IV) ↓ x7.5 |
| | kinase, δ) ↓ x1.5 | kinase, δ) ↑ x2 | kinase, δ) ↑ x2 | • Calcium/ |
| | • Calcium/ | • Receptor | • Calmodulin 2 | calmod-ulin- |
| | calmodulin- | (calcitonin) activity | (phosphorylase | dependent PK |
| | dependent protein | modifying protein 1 | kinase, δ) ↑ x1.5 | IV ↓ x7.5 |
| | kinase kinase 2 β | precursor ↑ x1.5 | • Calcium/ | • c-AMP |
| | ↑ x1.5 | | calmodulin- | responsive |
| | | | dependent protein | element binding |
| | | | kinase (CaM kinase) | protein 1 ↓ x2 |
| | | | II β ↑ x2 | • Calcium/ |
| | | | | calmodulin |
| | | | | dependent |
| | | | | protein kinase 1 |
| | | | | ↓ x2 |
| *3) Ras*/*MAPK kinase*/*ERK kinase related pathways and adaptor proteins* | • Ras suppressor | • Ras association | • Rho/rac guanine | • Ras homolog |
| | protein I | (RaIGDS/AF-6) | nucleotide exchange | gene family, |
| | • Rho GTPase | domain family I | factor (GEF) 2 | member B |
| | activating protein 4 | • Rho GDP | • Rab | • Ras-GTPase |
| | • MAPKK 5 | dissociation | geranylgeranyl- | activating protein |
| | • Rho GTPase | inhibitor (GDI) γ | transferase | • SH3 domain- |
| | activating protein 5 | • Rab | • Human rho GDP- | binding protein 2 |
| | • RAB4, member | geranylgeranyl- | dissociation | • Rho-associated, |
| | RAS oncogene | transferase, α | inhibitor 2 (IEF | coiled-coil |
| | family | subunit | 8120) | containing |
| | • RAB interacting | • MAPK 10 | • SHP2 interacting | protein kinase 1 |
| | factor | • RAB13, member | transmembrane | • RAD54 |
| | • Realted RAS viral | RAS oncogene | adaptor | *(S.cerevisiae)-* |
| | | family | • RAS p21 protein | like |
| | (r-ras) oncogene homolog | • Ras homolog gene | activator (GTPase | • RAB6, member |
| | • RAP2A, member | family, member G | activating protein) 1 | RAS oncogene |
| | of RAS oncogene | (rho G) | • SH3 domain | family |
| | family | • RAB2, member | binding glutamic | • RAB5A, |
| | • Human rho GDP- | RAS oncogene | acid-rich protein like | member RAS |
| | dissociation | family | • Neuronal shc | oncogene family |
| | inhibitor | • MAPK 1 | • MAPKK 1 | • MAPKK 4 |
| | • MAPKK 1 | | • MAKKK 5 | • SH3 domain |
| | | • C-src tyrosine kinase | • RAB11B, member | binding glutamic |
| | | • MAPK 14 | of RAS oncogene | acid-rich protein |
| | | • Rho GTPase- | family | • MAPK 8 |
| | | activating protein 1 | • GTPase | • Adaptor protein |
| | | • MAPKK 1 | • RAB5B, member | with pleckstrin |
| | | • ATP(GTP)- | RAS oncogene | homology and src |
| | | binding protein | family | homology 2 |
| | | • RAB interacting | • MAPKAPK 2 | domains |
| | | factor | • MAPK 6 | • SHP2 |
| | | • MAPK 6 | • Ras-related C3 | interacting |
| | | • KAPKK 5 | botulinum toxin | transmembrane |
| | | • RAB 30, member | substrate I isoform | adapter |
| | | RAS | Rac 1b | • Ras homolog |
| | | oncogene family | • RAB1, member | gene family, |
| | | • RAB 4, member | ras oncogene family | member H |
| | | RAS oncogene | • RAP1A, member | • RaP2 |
| | | | of ras | interacting |
| | | family | oncogene family | protein 8 |
| | | • MAPKK 13 | • MAPKKKK | • RAB5C, |
| | | • MAP/ERK kinase | • RAS guanyl | member RAS |
| | | kinase 4, isoform a | relasing protein 2 | oncogene family |
| | | | (calcium and DAG- • Grb2- | |
| | | | regulated) | associated binder |
| | | | • Grb2-associated | 2 |
| | | | binder 2 | |
| *4) JAK*/*STAT pathway and related kinases* | • STAT 2, 113kD | • STAT 1, 91kδ | • JAK 3 | • JAK 1 |
| | • STAT 5B | | | • STAT 6, IL-4 |
| | | | | induced |
| | | | | • Protein |
| | | | | inhibitor of |
| | | | | STATX |
| | | | | • STAT 1, 91kδ |
| | | | | • STAT 3 |
| | | | | (acute-phase |
| | | | | response factor) |
| *5) Protein tyrosine phosphatases*/*other phosphatases* | • PP 1, regulatory | • PTP | • Dual specificity | • PTP σ |
| | subunit 7 | • PP 2 (formerly | phosphatase 8 | • Phosphatase |
| | • PP 1A (formerly | 2A), regulatory | • Myosin | and tensin |
| | 2C), Mg-dependent, | subunit A (PR 65), | phosphatase target | homolog 2 |
| | α isoform | α Isoform | subunit 1 | • PP 5, catalytic |
| | • PTP | •PP2A subunit- α | • Dual specificity | subunit |
| | • PP 2A, regulatory | • PTP, non receptor | phosphatase 9 | • PTP, non- |
| | subunit B' (PR 53) | type 1 | • PTP, non- receptor | receptor type 6 |
| | • PP | •PTP, | type 1 | • PP 1A |
| | 2A, regulatory subunit -β | non receptor type | • PP 1, regulatory | (formerly 2C), |
| | • PTP, non- receptor | substrate 1 | (inhibitor) subunit 8 | Mg- dependent, |
| | type 1 | • PP 6, catalytic | • PTP, receptor | α isoform |
| | • PTP type IVA, | subunit | type, N | • PTP, receptor |
| | member 3 | • PTP, receptor | • PTP type IVA, | type, C |
| | • PP5, catalytic | type, C | member 3 | • PTP, receptor |
| | subunit | • PP 1, regulatory | • PP 5, catalytic | type, N |
| | | (inhibitor) subunit 5 | subunit | • Phosphatidid |
| | | • | • PTP σ | acid |
| | | PTP, receptor type, f polypeptide | | phosphatase |
| | | (PTPRF), interacting | | type 2A |
| | | protein (liprin), α 1 | | • PTP, receptor |
| | | | | type, A |
| *6) Other protein kinases and associated binding proteins* | • Receptor tyrosine | • Protein kinase, | • Serine/threonine | • Serine kinase |
| | kinase | cAMP-dependent, | kinase 14 α | • Serine/ |
| | • Protein kinase | catalytic, inhibitor α | • Serine/threonine | threonine |
| | • Serine/threonine | • Tyrosine kinase 2 | kinase | kinase 25 |
| | kinase 3 | • Protein kinase | • Protein kinase, | • Serine/ |
| | • SNF1-like protein | •PTK2 protein | AMP-activated, γ 1 | threonine |
| | kinase | tyrosine kinase 2 | non-catalytic | protein kinase |
| | • Serine/threonine | • Ribosomal protein | subunit | • Ste-20 related |
| | kinase 9 | S6 kinase, 90kδ, | • Protein kinase, | kinase |
| | • Membrane- | polypeptide 3 | cAMP-dependent, | • Ribosomal |
| | associated kinase | • Protein kinase, | catalytic inhibitor α | protein S6 |
| | • Ser-Thr protein | cAMP-dependent, | • Dual-specificity | kinase, 90kδ, |
| | kinase related to the | catalytic, γ | tyrosine-(Y)- | polypeptide 3 |
| | myotonic dystrophy | • Serine threonine | phosphorylation | • Serine/ |
| | protein kinase | protein kinase | regulated kinase 1A | threonine kinase |
| | • cAMP-dependent | | • Dual-specificity | 13 (aurora/ |
| | protein kinase | | tyrosine-(Y)- | IPL1-like) |
| | • RI-β regulatory | | phosphorylation | • Protein- |
| | subunit | | regulated kinase 2 | tyrosine kinase |
| | • Ribosomal protein | | isoform I | • Membrane- |
| | S6 kinase, 90kδ, | | • Serine/threonine | associated |
| | polypeptide 4 | | kinase 19 | kinase |
| | • Serine/threonine | | | • Dual- |
| | kinase 25 | | | specificity |
| | • Fms-related | | | tyrosine-(Y)- |
| | tyrosine kinase 3 | | | phosphorylation regulated kinase 2 isoform 1 |
| *7) Adenylate*/*guany- late cyclases and related pathways* | • Natriuretic peptide | • Natriuretic peptide | | • Adenylate |
| | receptor | receptor A/ | | cyclase |
| | A/guanylate cyclase | guanylate cyclase A | | activating |
| | A (atrionatriuretic | (atrionatriuretic | | polypeptide |
| | peptide receptor A) | peptide receptor A) | | precursor ↓ |
| | ↑ x2 | ↑ x6 | | x1.5 |
| | | • Adenylyl cyclase- | | |
| | | associated protein | | |
| | | ↑ x1.5 | | |
| CELL SURFACE | | | | |
| RECEPTORS | | | | |
| *1) G-protein coupled receptors and related binding proteins*/*G proteins* | • Guanine | • G protein- | • Guanine | • Guanine |
| | nucleotide | coupled | nucleotide | nucleotide binding |
| | binding protein | receptor 12 | binding | protein (G protein), |
| | (G protein), | • G protein- | protein 11 | α 15 (Gq class) |
| | β polypeptide 1 | coupled receptor | • Guanine | • G α inhibiting |
| | • G protein- | kinase | nucleotide | activity |
| | coupled | • G protein- | binding protein | polypeptide 3 |
| | receptor 20 | receptor | (G protein), | interacting protein |
| | • G protein- | coupled 35 | β polypeptide 3 | • Regulator of G |
| | coupled | • G protein- | • G protein- | protein signalling |
| | receptor 9 | coupled | coupled | • Guanine |
| | • G protein- | receptor 3 | receptor 56 | nucleotide binding |
| | coupled | • G protein- | • Regulator of | protein 11 |
| | receptor 39 | receptor | G-protein | • G protein - |
| | • G protein- | coupled 39 | signalling 9 | coupled receptor 3 |
| | coupled receptor | • Regulator of | • Guanine | • G protein - |
| | kinase | G-protein | nucleotide | coupled receptor |
| | • G protein- | signalling 6 | binding protein | kinase 1 |
| | coupled | • Coagulation | (G protein), α | • Ca++-sensing |
| | receptor 15 | factor II | 11 (Gq class) | receptor |
| | • Guanine | (thrombin) | • G protein- | (hypocalciuric |
| | nucleotide | receptor-like 1 | coupled | hypercalcemia 1, |
| | binding protein | precursor | receptor 35 | severe neonatal |
| | (G protein), β | | • Angiotensin | hyperparathyroid |
| | polypeptide 2 | | receptor- | ism) |
| | • G protein- | | like 1 ↑ x1.5 | • G protein- |
| | coupled | | • SSTR2 ↑ x2 | coupled receptor, |
| | receptor 35 | | • GDP | family C, group 5, |
| | • SSTR 3↑ x3 | | dissociation | member B |
| | • SSTR 2↑ x2 | | inhibitor | • Guanine |
| | | | | nucleotide binding |
| | | | | protein 11 |
| | | | | • 5-hydroxy- |
| | | | | tryptamine 7 |
| | | | | receptor isoform b |
| | | | | • Develop- |
| | | | | mentally regulated |
| | | | | • GTP-binding |
| | | | | protein 2 |
| | | | | • Endothelial |
| | | | | differentiation |
| | | | | related factor I ↑ |
| *2) Growth factors, their receptors and related binding proteins* | • GFR- bound | • Fms-related | • EGF (β- | • COL1A1 and |
| | protein 7 ↑ x2 | tyrosine kinase | urogastrone) ↓ x2 | PDGFB fusion |
| | • GFR-bound | 1 (VEGF /vascular | • TGF induced | transcript ↓x6 |
| | protein 14 ↑ x2 | permeability factor | protein ↑ x1.5 | • Egf-like |
| | • CSF-1 receptor, | receptor) ↓ x1.5 | • VEGF ↑ x1.5 | module |
| | formerly Mc | • GFR- bound | • PDGF α | containing, |
| | Donough feline | protein 2 ↓ x2.5 | polypeptide ↑ x2.5 | mucin-like, |
| | sarcoma viral (v- | • Bone-derived GF | • PDGFR, α | hormone |
| | fms) oncogene | ↑ x3 | polypeptide ↑ x1.5 | receptor-like |
| | homolog T x2.5 | • Growth | • TGFβ receptor III | sequence 1 ↓ x5 |
| | • IL-7 R precursor | differentiation factor | (betaglycan, 300kD) • | PDGFR α |
| | ↑ x2 | 1 ↑ x3 | ↓ x1.5 | ↓ x3 |
| | • PDGFF, α | • TGF, β1 ↓ x1.5 | HGF activator | • Fms-related |
| | polypeptide ↑ x1.5 | • TGFβR III | • inhibitor precursor ↑ | tyrosine kinase |
| | • TGF, β 1 ↑ x1.5 | (betaglycan, 300kδ) | x1.5 | 1 (VEGF/ |
| | | ↓ x2 | | vascular |
| | | • EGF (β- | | permeability factor |
| | | urogastrone) ↓ x2.5 | | receptor) ↓ x2 |
| | | • EGFR (avian | | |
| | | erythroblastic | | • PDGF- |
| | | leukemia viral (v- | | associated T |
| | | erb-b) oncogene | | Protein x2 |
| | | homolog) ↑ x2 | | • PDGFR β |
| | | • Butyrate response | | ↑x2 |
| | | factor 2 (EGF- | | • Cadherin 13, |
| | | response factor 2) ↑ | | H-cadherin |
| | | x2 | | (heart) |
| | | • FGFR 2 (bacteria- | | ↑ x2 |
| | | expressed kinase, | | |
| | | keratinocyte growth | | |
| | | factor receptor, | | |
| | | craniofacial | | |
| | | dysplasia) ↑ x2 | | |
| | | • TGFβ activated | | |
| | | kinase-binding | | |
| | | protein 1 ↑ x2.5 | | |
| | | • GCSF ↑ x3.5 | | |
| | | • EGF-like repeats | | |
| | | and discoidin I-like | | |
| | | domains 3 ↓ x15 | | |
| | | • PDGFR, α | | |
| | | polypeptide ↑ x2 | | |
| | | • PDGF, α | | |
| | | polypeptide ↑ x1.5 | | |
| *3) Glutamate receptor and related binding proteins* | • Glutamate | • Glutamate | | • Glutamate |
| | receptor | receptor, | | receptor, |
| | metabotropic 2 | metabotropic 4 | | metabotropic 2 |
| | precursor ↓ x3 | ↑ x1.5 | | precursor ↓3.5 |
| ATP-DEPENDENT | | | | |
| TRANSPORT | | | | |
| PROTEINS | | | | |
| *Ion channels and related pathways* | • Solute carrier | • Ca++ channel, | • K + voltage-gated | • K+ voltage- |
| | family 6 | voltage-dependent, | channel, shaker- | gated channel, |
| | (neurotransmitter | P/Q type, alpha 1A | related subfamily, | shaker-related |
| | transporter, creatin), | subunit ↓ x2.5 | member 3 ↓ x3 | subfamily, |
| | member 8 ↓ x3 | • ATPase, H+/K+ | • Solute carrier | member 3 |
| | • Na+ channel, | exchanging, beta | family 9 (Na+/H+ | ↓ x4.5 |
| | nonvoltage-gated 1, | polypeptide ↓ x2 | exchanger) isoform | • ATPase, |
| | β (Liddle syndrome) | • Solute carrier | 3 regulatory factor 1 | Ca++ |
| | ↓x2 | family 9 (sodium/ | ↑ x10.5 | transporting, |
| | • Ca++ channel, | hydrogen | • ATPase, Na+/K+ | cardiac muscle, |
| | voltage-dependent, | exchanger), isoform | transporting, β1 | fast twitch 1 |
| | α IH subunit ↑ x2 | 3 regulatory factor 1 | polypeptide ↑ x2.5 | ↓ x4.5 |
| | • K+ voltage-gated | ↑ x11.5 | • K+ large | |
| | channel, Shaw- | • Solute carrier | conductance | |
| | related subfamily, | family 11 | Ca++-activated | |
| | member 3 ↑ x2.5 | (Na+/phosphate | channel, subfamily | |
| | • Solute carrier | symporters), | M, β member 1 | |
| | family 9 (Na+/H+ | member 1 ↑ x2.5 | ↑ x2.5 | |
| | exchanger) isoform | | • Ca++ channel, | |
| | 3 regulatory factor 1 | | voltage-dependent, | |
| | ↑ x2 | | α 2/δ subunit 2 | |
| | | | ↑ x1.5 | |
| | | | • Ca++ channel, | |
| | | | dependent, | |
| | | | voltage-α 2/δ subunit 1 ↑ x2 | |
| CELL BIOLOGY/ | | | | |
| SPECIALIZED | | | | |
| FUNCTIONS | | | | |
| *1) Neuromediatorsl neuromodulators and related pathways* | • δ sleep inducing | • GABA (A) | • Dopamine | • GABA (A) |
| | peptide, | receptor, γ2 | receptor D4 ↓ x2 | receptor, |
| | immunoreactor ↑ | precursor ↑ x4 | • Adrenergic α-2C- | γ 2 precursor |
| | x2.5 | • Dopamine | receptor | ↓ x1.5 |
| | • Opioid receptor, | receptor D2 ↑ x3.5 | ↓ x2 | • Brain |
| | δ1 ↑ x2.5 | • GABA(A) | • β adrenergic | cannabinoid |
| | • GABA (A) | receptor-associated | receptor | receptor 1 |
| | receptor, γ2 | protein ↑ x1.5 | kinase 1 ↑ x3 | ↓ x2 |
| | precursor ↑ x2 | • Dopamine | | • GABA (B) |
| | • Acetylserotonin | receptor D3 ↑ x2 | | receptor 1, |
| | O-methyl | • δ sleep inducing | | isoform a |
| | transferase-like ↓ x3 | peptide, | | precursor |
| | • LIF (cholinergic | immunoreactor | | ↑x2.5 |
| | differentiation | ↑ x2.5 | | • Cannabinoid |
| | factor) ↑ x2 | •5-hydroxytrypt | | receptor 2 |
| | • Dopamine | amine (serotonine) | | (macrophage) |
| | receptor D2 ↑ x4.5 | receptor 6 ↑ x2.5 | | ↑ x3 |
| | | | | • Phosphatidyl |
| | | | | ethanolamine |
| | | | | N-methy- |
| | | | | ltransferase |
| | | | | ↑ x2 |
| | | | | • Adrenergic, α |
| | | | | -2C-, receptor |
| | | | | ↓ x2.5 |
| *2) Pancreatic*/ *gastro-intestinal secretions and related pathways* | | | • Gastric inhibitory | •Chole- |
| | | | polypeptide receptor | cystokinin B |
| | | | ↑x2 | receptor ↓ x3 |
| | | | | • Gastric |
| | | | | inhibitory |
| | | | | polypeptide 1 |
| | | | | receptor ↓ x2 |
| *3) Hormones and related pathways* | • Angiotensin | • Insulin promoter | • PTHR 1 ↓ x2.5 | • TSHR ↓ x2 |
| | receptor | factor 1, | • Arginine | • IGF-1 ↓ x 1.5 |
| | 1B ↓ x1.5 | homeodomain | vasopressine | • Solute carrier |
| | • Glucocorticoid | transcription factor | receptor 1B ↑ x2 | family 21 (PG |
| | receptor DNA | ↓ x1.5 | • IGFBP6 ↑ x2.5 | transporter), |
| | binding factor I | • IGF-2 ↓ x2.5 | • IGF-1 ↑ x1.5 | member 2 ↑ x2 |
| | ↓ x4.5 | • Corticosteroid | | |
| | • Insulin receptor | binding globulin | | |
| | ↓ x3 | precursor ↑ x2 | | |
| | • THR, a (avian | • THR interacting | | |
| | erythroblastic | protein 15 ↑ x1.5 | | |
| | leukemia viral (v- | - IGFBP2 ↑ x2 | | |
| | erb-a) oncogene | • Arginine | | |
| | homolog) ↑x1.5 | vasopressine | | |
| | • Arginine | receptor 2 ↑ x2.5 | | |
| | vasopressin | • THR sulfo | | |
| | (neurophysin II, | transferase ↑ x2.5 | | |
| | antidiuretic | • Glucacon receptor | | |
| | hormone, diabete | ↑ x5 | | |
| | insipidus, | | | |
| | neurohypophyseal) | | | |
| | ↑ x2 | | | |
| | • Vasopressin- | | | |
| | activated calcium- | | | |
| | mobilizing receptor- | | | |
| | 1 ↓ x2 | | | |
| | • Corticotropin | | | |
| | releasing hormone | | | |
| | receptor type 2 beta | | | |
| | isoform ↑ x1.5 | | | |
| | • IGF-2 ↓ x2 | | | |
| | • IGF-1 ↑ x2.5 | | | |
| | • IGFBP2 ↑ x1.5 | | | |
| | • THR-associated | | | |
| | protein, 240kδ | | | |
| | subunit ↓ x1.5 | | | |
| | • THR binding | | | |
| | protein ↑x1.5 | | | |
| | • PG-endoperoxide | | | |
| | synthase 1 | | | |
| | (prostaglandin G/H | | | |
| | synthase and | | | |
| | cyclooxygenase) ↓ | | | |
| | x3.5 | | | |
| | • Adrenomedullin ↑ | | | |
| | x1.5 | | | |
| | • SSTR 3↑ x3 | | | |
| | • SSTR 2↑ x2 | | | |
| *4) Cytoskeletton and associated proteins* | • VWF precursor ↑ x2 | | • Vasodilatator- stimulated phosphoprotein ↑ x2 | • VWF precursor ↑ x2 |
| *5) Enzymes* | | | | • Thrombo- spondin 2 ↑ x2 |
| | | | | • Pro-platelet basic protein (includes platelet basic protein, β- thromboglobuli n, connective tissue-activating peptide III, neu) 2 ↑ x3.5 |
| | | | | |
| IMMUNITY | | | | |
| | • TNF, α-induced | • TNF-α converting | • IFN-inducible | • LTB4 |
| | protein 3 | enzyme ↓ x2 | RNA-dependent | receptor |
| | ↓ x1.5 | • IFN-stimulated | protein kinase | (chemokine |
| | • IRF5 ↑ x2 | protein, 15kDa ↓ x2 | ↑ x2.5 | receptor-like 1) |
| | • Putative | • IFN-related | • TNF (cachectin) | ↓ x1.5 |
| | chemokine receptor; | developmental | ↑ x2 | • IL2-inducible |
| | GTP-binding protein | regulator 2 ↓ x1.5 | (ligand) • TNF (ligand) | T-cell kinase |
| | ↑ x2 | • IFN-inducible | superfamily, | ↓ x12 |
| | • TNFR | RNA-dependent | member 13 ↑ x2.5 | • P56lck ↓ x18 |
| | superfamily, | protein kinase ↑ x4 | • IL 1 receptor- like | • RAG1 ↓ x18 |
| | member 12 ↑ x2 | • IL2 R, γ chain, | 1 ↑ x2 | • IFNγ |
| | | precursor ↑ x2.5 | • IFNγ responsive | responsive |
| | | • IFN regulatory | transcript | transcript |
| | | factor 5 ↑ x2.5 | ↑ x2 | ↓ x1.5 |
| | | • Bruton | • LTb4 (chemokine | • SH2 domain |
| | | agammaglobulinemi | rceptor-like 1) ↑ x3 | protein 1A, |
| | | a tyrosine kinase | • Putative | Duncan's |
| | | ↑ x1.5 | chemokine receptor; | disease |
| | | • B lymphoid | GTP-binding protein | (lymphoprolifer ative syndrome) |
| | | tyrosine kinase | ↑ x2.5 | ↓ x7.5 |
| | | ↑ x3.5 | • IFNγ receptor 2 ↑ receptor 2 ↑ | • CD2 |
| | | • IFN γ responsive | x1.5 | antigen • CD2 antigen |
| | | transcript ↑ x1.5 | (IFNγ transducer 1) | (p50), sheep red |
| | | • INF (ligand) | • TNFR | blood cell |
| | | superfamily, | superfamily, superfamily, | receptor |
| | | member 10↑ x1.5 | member 12 ↑ x.15 | ↓ x7.5 -TCR ζ chain |
| | | • IFN-induced | • IL-8 receptor type - TCR ζ chain | precursor |
| | | leucine zipper | B ↑ x1.5 | ↓ |
| | | protein ↑x15 | • IFN regulatory factor 2 ↑ x3.5 | x5.5 • RAG2 ↓ x5 |
| IMMUNITY | | | | • Signalling lymphocytic |
| (Continued) | | | | activation molecule |
| | | | | ↓ x4.5 |
| | | | | • Flt3 ligand ↓ x4.5 |
| | | | | • Lymphocyte specific |
| | | | | protein tyrosine kinase |
| | | | | ↓ x4 |
| | | | | • Chemokine (C-X-C |
| | | | | motif), receptor 4 (fusin) |
| | | | | ↓x3 |
| | | | | • Transcription factor 7 |
| | | | | (T-cell specific, HMG- |
| | | | | box) ↓ x 16.5 |
| | | | | • IL9 receptor ↓ x2 |
| | | | | • RANTES ↑ x10.5 |
| | | | | • CD2 antigen |
| | | | | (cytoplasmic tail)- |
| | | | | binding protein 2 |
| | | | | ↑ x3.5 |
| | | | | • IFNγ - inducible |
| | | | | protein 30 ↑ x3.5 |
| | | | | • IFNα-inducible protein |
| | | | | 27 ↑ x3 |
| | | | | • TNF (ligand) |
| | | | | superfamily member 10 |
| | | | | ↑ x2 |
| CELL CYCLE | | | | |
| | • Cdk (CDC2- | • Cdki 2D (p19, | • Cyclin I ↓ x1.5 | • Cdc-like 5 |
| | like) ↓ x2 | inhibits CDK4) | • S-phase kinase- | (cholinesterase-related |
| | • Growth arrest- | ↓ x2.5 | associated protein | cell division controller) ↓ |
| | specific 6 ↑ x1.5 | • Cdk like-2 ↓ | 1A (p19A) ↑ x2 | x2 |
| | • Cdk 5, | x2 | • Cdk 6 ↑ x3 | • Cdk (CDC2-like) ↓ x9 |
| | regulatory subunit | • Cdk5, | • Cdk5, | • Cdk 5, regulatory |
| | 2 (p39) | regulatory | regulatory subunit | subunit I (p35) ↓ x5 |
| | ↑ x1.5 | subunit 1 ↑ x2.5 | 1 (p35) ↑ x2 | • Growth arrest-specific |
| | • CDC37 (cell | • CdkilA | • Cyclin D2 ↑ | 1 ↓ x2.5 |
| | division cycle 37, | (p21/Cip1) ↑ x6 | x1.5 | • Cyclin B2 ↓ x2.5 |
| | *S. cerevisiae,* | • Cdk like-2 ↓ | • S-phase kinase- | |
| | homolog) ↑ x1.5 | x2 | associated protein | |
| | • Cdki 1A (p21, = | | 1A ↑ x2 | |
| | Cip 1) ↑ x5 | | • Cdk 2 ↑ x1.5 | |
| | | | • Follistatin-like | |
| | | | 1 | |
| | | | ↑ x1.5 | |
| APOPTOSIS | | | | |
| | • Death effector | • BCL2-like 1 | • Rb binding protein | • Bcl-2 binding |
| | domain-containing | ↓ x3.5 | ↑ x2 | component 3 |
| | ↓ x3.5 | • Rb I (including | Caspase 8, | |
| | • Fas/Apo-1/CD95 | osteosarcoma) | apoptosis-related | |
| | ↑ x1.5 | ↓ x2 | cysteine protease | |
| | | • Death-associated ↑ | x2.5 | |
| | | protein 6 | • TNF (cachectin) | |
| | | ↓ x2.5 | ↑ x2 | |
| | | • Rb binding protein | • TNF (ligand) | |
| | | ↑ x2.5 | superfamily, | |
| | | • Rb-like 2 (p130) ↑ | member 13 ↑ x2.5 | |
| | | x3 | • Bcl-2 binding | |
| | | • Fas-activated | component 3 | |
| | | serine/threonine | ↑ x2.5 | |
| | | kinase ↑ x1.5 | • Death-associated | |
| | | | protein ↑ x1.5 | |
| | | | • Tumour protein | |
| | | | p53-binding protein | |
| | | | ↑ x1.5 | |
| | | | • Rb-binding protein | |
| | | | 8 ↑ x1.5 | |
| | | | • Programmed cell | |
| | | | death 10 ↑ x1.5 | |

These results show that several signal transduction pathways were affected. They included the phosphatidyl inositol/PKC/phospholipases/calcium-calcineurin-calmodulin pathway, the Ras/MAPK kinase/ERK kinase dependent pathway, the JAK/STAT pathway, and adenylate/guanylate cyclases with their dependent pathways. The changes for the cell surfaces receptors included numerous G-protein coupled receptors, receptors for growth factors and glutamate receptors. The changes in ATP-dependent transport proteins involved ion channels and associated proteins. The compound also affected neuromediators/neuromodulators, pancreatic and gastrointestinal secretions, hormones, cytoskeletal proteins and enzymes/catalysts.

Examples of genes reflecting several SSTR signalling pathways in the pituitary are shown in TABLE 21. Selected genes from the primary gene lists were produced by a succession of filtering and statistical algorithms (t-test: p value: 0.05). The numerical values correspond to the AvgDiff (see above) of the relevant probe set for each assay with the range of observed values between brackets. Of particular interest in this analysis were the transcript level changes for molecules known to be closely associated with the binding of the natural peptides, SST-14 and SST-28, to the SSTRs.

**TABLE 21**

| Examples of Genes Reflecting Several SSTR Signalling Pathways in the Pituitary | | |
|---|---|---|
| GENES | CONTROL | SOM230 (0.1 mg/animal/14 day) |
| SIGNAL TRANSDUCTION | | |
| 1) Phophatidyl inositol and related pathways/PKC, | | |
| phospholipases | | |
| • IP-4-phosphatase, type 1, isoform b | 296 (241 to 342) | 177 (107 to 232) |
| • PI-3-kinase, catalytic, δ polypeptide | 91 (45 to 146) | 34 (20 to 67) |
| • PI-3-kinase,catalytic, α polypeptide | 72 (26 to 135) | 21 (20 to 24) |
| • PI transfer protein, β | 125 (93 to 187) | 42 (34 to 50) |
| • PKC inhibitor | 2,351 (2,135 to 2,755) | 3,333 (2,339 to 3,878) |
| • PLC, γ 1 (formerly subtype 148) | 111 (100 to 131) | 40 (20 to 63) |
| • PKC inhibitor | 2,351 (2,1345 to 2,755) | 3,332 (2,339 to 3,878) |
| 2) Ras/MAPK kinase/ERK kinase related pathways | | |
| and adaptor proteins | | |
| • MAPKKK5 | 171 (148 to 207) | 278 (221 to 351) |
| • Rab geranylgeranyltransferase, α subunit | 164 (152 to 173) | 104 (70 to 172) |
| • Rab geranygeranyltransferase, βsubunit | 230 (187 to 250) | 284 (246 to 374) |
| • SHB adaptor protein (a Src homology 2 protein) | 112 (43 to 190) | 38 (20 to 55) |
| • RAB 5C, member RAS oncogene family | 72 (20 to 138) | 162 (109 to 212) |
| 3) Protein tyrosine phosphatases/other phosphatases | | |
| • Dual specificity phosphatase 8 | 493 (344 to 625) | 170 (67 to 238) |
| • Phosphatase and tensin homolog (mutated in | 129 (58 to 228) | 36 (20 to 63) |
| multiple advanced cancers 1) | | |
| • PTP, receptor type, T | 58 (41 to 78) | 101 (48 to 129) |
| • PP 1, regulatory (inhibitor) subunit 5 | 20 | 75 (60 to 90) |
| 4) Adenylate/guanylate cyclases and related pathways | | |
| • Soluble adenylyl cyclase | 54 (51 to 57) | 22 (20 to 27) |
| CELL SURFACE RECEPTORS | | |
| 1) G-protein coupled receptors | | |
| • SSTR3 | 22 (20 to 24) 22 (20 to 24) | 57 (20 to 90) 57 (20 |
| 2) Glutamate receptor and related binding proteins | | |
| • GLUR 2 precursor | 42 (20 to 86) | 59 (20 to 177) |
| ATP-DEPENDENT TRANSPORT PROTEINS | | |
| Ion channels and related pathways | | |
| ATPase, Na+/K+ transporting, β 3 polypeptide | 292 (246 to 353) | 610 (335 to 949) |
| ATPase, Na+/K+ transporting, α 2 (+) polypeptide | 86 (52 to 130) | 184 (69 to 325) |
| ATPase, H+/K+ exchanging, α polypeptide | 128 (50 to 245) | 20 |
| K+ channel, subfamily K, member 3 (TASK) | 132 (69 to 188) | 26 (20 to 43) |
| K+ voltage-gated channel, Shab-related subfamily, | 66 (20 to 112) | 22 (20 to 31) |
| member 1 | | |
| Putative Ca++ transporting ATPase | 61 (38 to 98) | 101 (84 to 112) |
| CELL CYCLE | | |
| Core-binding factor, runt domain, α subunit 2; | 225 (113 to 343) | 491 (251 to 677) |
| translocated to, 1; cyclin D-related | | |
| Forkhead box O3A | 497 (447 to 553) | 257 (186 to 324) |
| Forkhead box H1 | 225 (113 to 343) | 117 (251 to 677) |
| Cyclin F | 198 (171 to 229) | 74 (48 to 132) |
| Cyclin D3 | 187 (173 to 201) | 338 (202 to 446) |
| S-phase response (cyclin-related) | 91 (88 to 97) | 129 (111 to 148) |
| Cell division cycle 25B | 40 (20 to 67) | 162 (134 to 187) |
| Cdk inhibitor 2C (p18, inhibits CDK4) | 81 (58 to 99) | 184 (140 to 229) |
| Cdk inhibitor 2D (p19, inhibits CDK4) | 198 (171 to 229) | 99 (83 to 118) |
| APOPTOSIS | | |
| BCL2-associated athanogene | 216 (207 to 231) | 318 (235 to 409) |
| BCL2-antagonist of cell death | 44 (33 to 47) | 69 (42 to 89) |
| Bax gamma | 258 (207 to 297) | 326 (221 to 448) |
| BCL2/adenovirus E1B 19kD-interacting protein 3 | 342 (288 to 401) | 458 (388 to 526) |
| Programmed cell death 6 | 504 (443 to 547) | 635 (513 to 747) |
| Neuroblastoma-amplified protein | 178 (149 to 210) | 237 (201 to 258) |

The effects on the growth hormone/ insulin-like growth factor-1 (GH/IGF-1) and glucagon/insulin axes (Macaulay VM, Br J Cancer 65: 311-20 (1992); Pollak MN & Schally AV, Proc Soc Exp Biol Med 217: 143-52 (1998)were reflected in transcript level changes in several organs. The results are shown in TABLE 22. Beside the expected change in IGF-1 transcript level, there was an effect on insulin-like growth factor-2 (IGF-2) as well (in the pituitary and kidneys) that might be useful as a biological marker of SOM230 activity if reflected in the blood. The genes were selected as above in TABLE 21.

**TABLE 22**

| Genes Reflecting the Effects of SOM230 on the GH/IGF and Glucagon/Insulin Axes in Different Tissues | | |
|---|---|---|
| ORGANS/GENES | CONTROL | SOM230 (0.1 mg/animal/14day) |
| PITUITARY | | |
| IGF-2 | 126 (40 to 179) | 70 (20 to 150) |
| GR | 20 | 109 (51 to 215) |
| IGFBP, acid labile subunit | 30 (20 to 49) | 83 (20 to 110) |
| SSTR3 | 22 (20 to 24) | 57 (20 to 90) |
| BROWN FAT | | |
| IGF-1 | 548 (279 to 810) | 389 (315 to 449) |
| IGFBP 4 | 1410 (916 to 2173) | 763 (429 to 1058) |
| IRS 2 | 48 (20 to 84) | 146 (80 to 222) |
| SSTR3 | 25 (20 to 52) | 194 (87 to 248) |
| PANCREAS | | |
| IGF-1 | 20 | 89 (20 to 298) |
| SSTR2 | 258 (205 to 366) | 156 (120 to 210) |
| KIDNEY | | |
| IR | 654 (187 to 1,187) | 196 (163 to 265) |
| IGF-2 | 117 (47 to 176) | 49 (20 to 39) |
| IGF-1 | 65 (24 to 103) | 25 (20 to 39) |
| IGFBP2 | 375 (211 to 625) | 563 (457 to 655) |
| SSTR 3 | 31 (20 to 69) | 82 (33 to 120) |
| SSTR 2 | 74 (20 to 153) | 126 (93 to 158) |
| LIVER | | |
| Insulin promoter factor 1, homeodomain | 89 (58 to 160) | 42 (23 to 52) |
| transcription factor | | |
| IGF-2 | 701 (403 to 961) | 269 (224 to 291) |
| IGFBP2 | 2,722 (1,321 to 3,363) | 4,476 (3,191 to 5,422) |
| GR | 44 (20 to 82) | 80 (70 to 360) |
| SPLEEN | | |
| IGFBP6 | 495 (130 to 982) | 1,043 (853 to 1,155) |
| IGF-1 | 72 (42 to 103) | 85 (52 to 125) |
| SSTR2 | 56 (20 to 83) | 93 (87 to 95) |
| THYROID | | |
| IGF-1 | 91 (20 to 179) | 58 (20 to 114) |

Other genes of interest affected by SOM230 were the transcript levels of growth factors (PDGF, FGF, EGF, TGFβ), their receptors and factors of angiogenesis (PDGF, VEGF, thrombospondin) involved in tumour growth and spreading (Woltering EA et al., New Drugs 1.5:77-86 (1997)). Also reported for somatostatin and and analogures genes involved in immunity were changed, *i.e.* cytokines (IL-1, TNF, IFN), regulators of T and B cell genesis and function (CD2 antigen, IL-2 receptor, B-lymphoid tyrosine kinase, IL-2 inducible T cell kinase, p561ck, RAG1, TCRζ chain precursor, RAG2, FLT 3 ligand) (van Hagen PM et al. Eur J Clin Invest 24: 91-9 (1994)), as well as genes involved in blood pressure control and diuresis, *i.e.* atrial natriuretic peptide and its receptor guanylyl cyclase A, arginine vasopressin and its receptor (Aguilera G et al., Nature 292: 262-3 (1981); Aguilera G et al., Endocrinology 111: 1376-84 (1982); Ray C et al., Clin Sci (Lond) 84: 455-60 (1993); Cheng H et al., Biochem J 364: 33-9 (2002)). A specific gene involved in the control of fat storage is the adrenergic β3 receptor in brown fat (Bachman E et al., Science 297: 843- 45 (2002)).

Protein products of the above genes are useful as surrogate markers of the biological activity of SOM230, especially the findings for IGF-2 in the pituitary and kidneys.

To conclude, the gene profiling of monkey tissues treated with SOM230 at sub-therapeutic is a sensitive approach to identify signalling and effector pathways known for somatostatin. The finding of clear transcriptional signatures for this agonist argues for a comparison with gene expression changes induced by Sandostatin®.

### EXAMPLE VI

### SALMON CALCITONIN AND PTS893, PHARMACOGENOMICS EXPLORATORY STUDY IN MONKEYS; MICROARRAY GENE EXPRESSION ANALYSIS

Calcitonins are endogenous regulator of calcium homeostasis and can be used as anti resorptive agents for the treatment of hypercalcaemia-associated disorders. Various calcitonins, including e.g. salmon and eel calcitonin, are commercially available and are commonly employed in the treatment of e.g. Paget's disease and osteoporosis. *See,* U.S. Pat. Nos. 5,733,569 and 5,759,565, the contents of which are incorporated by reference. See also, U.S. Pat. Nos. 5,719,122, 5,175,146, and 5,698,6721, and U.S. Pat. Appln. 003015815. A version of calcitonin (Miacalcin®) is available as a nasal spray. Information regarding the administration of Miacalcin® (calcitonin-salmon) nasal spray is available in the Miacalcin® Prescribing Information (Novartis, November 2002).

Parathyroid hormone (PTH) is a polypeptide of 84 amino acids. Parathyroid hormone regulates borne remodeling and Ca²⁺ homeostasis. Parathyroid hormone is also a known paracrine activator of ostaoclast differentiation and activity. PTS893 is an analogue of the endogenous parathyroid hormone, in which certain sites of chemical instability are eliminated within N-terminal parathyroid hormone fragments by making appropriate amino acid substitutions at particular residues which results in stable and biologically active human parathyroid hormone fragments. PTS893 [SDZ PTS 893; Leu8, Asp10, Lys11, Ala16, Gln18, Thr33, Ala34 human PTH 1-34 [hPTH(1-34)]] is a 34 amino acid parathyroid analogue that enhances bone mass and biomechanical properties. Kneissel M et al., Bone 28: 237-50 (March 2001); Stewart AF et al., J. Bone. Miner. Res. 15(8): 1517-25 (August 2000); Thomsen JS et al., Bone 25(5):561-9 (November 1999). N-terminal fragments of human parathyroid hormones include hPTH(1-34)OH muteins and hPTH(1-38)OH muteins. PTS893 comprises at least the first 27 N-terminal amino acid units of parathyroid hormone. Preferred parathyroid hormone derivatives are those comprising at least one amino acid unit replaced in one or more of the following positions of the parathyroid hormone sequence: 8-11, 13, 16-19, 21, 22, 29 to 34, particularly 8-11, 16-19, 33 and/or 34. These compounds exhibit desirable bone-forming properties both *in vivo* and *in vitro* which are equal to or above the level of natural PTH and its N-terminal fragments. See, European patent EP 0 672 057; published PCT patent application WO 94/02510; Kneissel M et al., Bone 28: 237-50 (March 2001); Stewart AF et al., J Bone Miner Res 15(8): 1517-25 (August 2000); Thomsen JS et al., Bone 25(5):561-9 (November 1999).

*Introduction and summary.* The purpose of this EXAMPLE was to evaluate the gene expression changes in cynomolgus monkeys following a two-week subcutaneous treatment with salmon calcitonin (sCT) at 50 µg/animal/day and PTS893 at 5 µg/animal/day to elucidate the mechanisms of action mediating their effects as well as the identification of biomarkers of therapeutic indications. This EXAMPLE is believed to be the first analysis that globally describes the molecular mechanisms of action of salmon calcitonin and a parathyroid hormone analogue by multiorgan-gene-profiling analysis in primates. This is also believed to be the first gene profiling analysis which describes the molecular mechanisms of action of hormonal-mediated bone remodeling by salmon calcitonin and PTS893.

In this EXAMPLE, salmon calcitonin and PTS993 were both found to have modulating effects on genes affecting the direct, antocrine, paracrine and endocrine regulation of the mesenchymal cell functions such as transforming growth factor betas (TGF-βs), insulin-like growth factors (IGFs), bone morphogenetic proteins (BMPs) and vascular endothelial growth factor (VEGF). Both compounds also regulate the synthesis and degradation of extracellular matrix components. Salmon calcitonin also regulates estrogen receptor and steroidogenic factor, whereas PTS893 produced a strong up-regulation on nuclear receptors of the steroid/thyroid receptor family. These data therefore support the role of calcitonin as an anabolic agent.

In addition, salmon calcitonin and PTS893 also influenced some aspects of the mineralization of the extracellular matrix, since changes in amelogenin, dentin and ectonucleotide pyrophosphatases were observed.

In addition, PTS893 showed an effect on mediating the paracrine activation of osteoclast differentiation and activity, through cytokine and RANK ligand.

No significant differences in gene expression profiling were attributable to the fact of administering salmon calcitonin and PTS893 in combination, with respect to the single therapy.

Thus, gene profiling analysis in this EXAMPLE allowed the reconstruction of the pathways involved in calcitonin and parathyroid hormone signal transduction, triggered by protein-G-linked-receptor stimulation and their influence on cell cycle, as indicated by the changes observed in cyclins.

*Animals.* A two-week subcutaneous treatment was carried out with salmon calcitonin (sCT), PTS893 or a combination of the two, each of which were dissolved in phosphate buffered saline (PBS) containing 9% autologous serum. Solvent was used as vehicle for the control group.

The animals used in this analysis were cynomolgus monkeys *(Macaca fascicularis),* supplied by Centre de Recherches Primatologiques, Port Louis, Mauritius. Two animals were used per group and sex. At the beginning of the treatment period, the animals were at least 24 months old, with a body weight of approximately 3 kg. Animals were kept under standard conditions for animal welfare. Animals were examined daily for mortality, food consumption and clinical observations. Body weight was recorded once per week. The dosages were 0 µg/animal/day (as the control), 50 µg/animal/day of salmon calcitonin and 5 µg/animal/day of PTS893.

*In vivo examinations.* No significant histopathological changes were observed. No relevant changes were observed other than a body weight decrease ranging from 8 to 12% in the salmon calcitonin group. A decrease in food consumption was also observed, although not always consistent with the decrease in body weight.

**TABLE 23**

| Food Consumption - Males | | | | | | | | | | | |
|---|---|---|---|---|---|---|---|---|---|---|---|
| Control | | | | | | | | | | | |
| Day | -6 | -5 | -4 | -3 | -2 | -1 | 1 | 2 | 3 | 4 | 5 |
| Animal no. W62S01 | 50 | 100 | 100 | 100 | 100 | 100 | 100 | 100 | 100 | 100 | 100 |
| Animal no. W62S02 | 50 | 100 | 100 | 100 | 100 | 100 | 100 | 100 | 100 | 100 | 25 |
| Day | 6 | 7 | 8 | 9 | 10 | 11 | 12 | 13 | 14 | | Avg. |
| Animal no. W62S01 | 75 | 100 | 100 | 100 | 100 | 100 | 100 | 25 | | | 91.7 |
| Animal no. W62S02 | 100 | 75 | 75 | 100 | 100 | 100 | 75 | 100 | 50 | | 91.7 |
| Both animals | | | | | | | | | | | 91.7 |

| Salmon Calcitonin | | | | | | | | | | | |
|---|---|---|---|---|---|---|---|---|---|---|---|
| Day | -6 | -5 | -4 | -3 | -2 | -1 | 1 | 2 | 3 | 4 | 5 |
| Animal no. W62503 | 50 | 75 | 50 | 75 | 100 | 75 | 75 | 25 | 50 | 100 | 100 |
| Animal no. W62504 | 50 | 75 | 75 | 75 | 100 | 75 | 50 | 25 | 100 | 75 | 100 |
| Day | 6 | 7 | 8 | 9 | 10 | 11 | 12 | 13 | 14 | | Avg. |
| Animal no.W62503 | 75 | 100 | 100 | 100 | 100 | 75 | 75 | 25 | | | 70.8 |
| Animal no.W62504 | 75 | 75 | 75 | 100 | 100 | 75 | 75 | 25 | 75 | | 75.0 |
| Both animals | | | | | | | | | | | 72.9 |

| PTS893 | | | | | | | | | | | |
|---|---|---|---|---|---|---|---|---|---|---|---|
| Day | -6 | -5 | -4 | -3 | -2 | -1 | 1 | 2 | 3 | 4 | 5 |
| Animal no. W62505 | 50 | 100 | 100 | 100 | 75 | 100 | 100 | 100 | 100 | 100 | 100 |
| Animal no. W62S06 | 50 | 100 | 100 | 100 | 100 | 100 | 100 | 100 | 100 | 100 | 100 |
| Day | 6 | 7 | 8 | 9 | 10 | 11 | 12 | 13 | 14 | | Avg. |
| Animal no. W62505 | 100 | 100 | 100 | 100 | 100 | 100 | 100 | 50 | 75 | | 875 |
| Animal no. W62506 | 100 | 100 | 100 | 100 | 100 | 100 | 100 | 100 | 100 | | 91.7 |
| Both animals | | | | | | | | | | | 89.6 |

The animals to whom salmon calcitonin was administered presented with a decrease in body weight ranging between 8 to 12%, which can be attributed to a decrease in food consumption. An anorectic effect had previously been described for salmon calcitonin acting through amylin receptors Eiden S et al., J. Physiol. 541(pt3): 1041-1048 (2002); Lutz TA et al., Peptides 21 (2): 233-8 (2000). However, no signs of toxicity were observed here. Hormonal and lipid changes observed in this EXAMPLE are most probably related to a consequent metabolic adaptation.

No relevant changes in electrocardiograms (ECG) or blood pressure were observed.

**TABLE 24**

| Blood Pressure | | | | | |
|---|---|---|---|---|---|
| Animal | Sex | Compound | Week -1 | Week 2 | Difference |
| number | | administered | (mm Hg) | (mm Hg) | (mm Hg) |
| W62501 | Male | Control | 121 | 98 | -23 |
| W62501 | Male | Control | 90 | 29 | -61 |
| W62502 | Male | Control | 86 | 107 | 21 |
| W62502 | Male | Control | 26 | 34 | 8 |
| W62503 | Male | Salmon Calcitonin | 135 | 99 | -36 |
| W62503 | Male | Salmon Calcitonin | 61 | 40 | -21 |
| W62504 | Male | Salmon Calcitonin | 102 | 79 | -23 |
| W62504 | Male | Salmon Calcitonin | 56 | 35 | -21 |
| W62505 | Male | PTS893 | 76 | 87 | 11 |
| W62505 | Male | PTS893 | 18 | 22 | 4 |
| W62506 | Male | PTS893 | 106 | 101 | -5 |
| W62506 | Male | PTS893 | 53 | 33 | -20 |
| W62551 | Female | Control | 96 | 76 | -20 |
| W62551 | Female | Control | 27 | 26 | -1 |
| W62552 | Female | Control | 102 | 93 | -9 |
| W62552 | Female | Control | 26 | 36 | 10 |
| W62553 | Female | Salmon Calcitonin | 98 | 82 | -16 |
| W62553 | Female | Salmon Calcitonin | 50 | 25 | -25 |
| W62554 | Female | Salmon Calcitonin | 92 | 44 | -48 |
| W62554 | Female | Salmon Calcitonin | 26 | 30 | 4 |
| W62555 | Female | PTS893 | 92 | 70 | -22 |
| W62555 | Female | PTS893 | 43 | 42 | -1 |
| W62556 | Female | PTS893 | 78 | 87 | 9 |
| W62556 | Female | PTS893 | 24 | 28 | 4 |

*Blood sampling.* Animals were fasted overnight before blood collection but had free access to water. Blood samples were taken from a peripheral vein. Standard haematology and clinical chemistry analysis were performed once during pretest and at the end of the treatment period. Blood samples were collected from each animal at the same intervals as described for the clinical chemistry investigations. The serum samples were deep-frozen (approximately -80°C) until analyses for hormone determination.

*Clinical chemistry and hormone determinations.* A slight anaemia was observed in all animals of the study, including the controls. This was attributed to the repeated blood sampling and not considered to be relevant.

**TABLE 25**

| Haematology - Males | | | | | | | |
|---|---|---|---|---|---|---|---|
| Control | | | | | | | |
| Animal no. | | | W62501 | | | W62502 | |
| Test | Units | d-6 | d7 | d13 | d-6 | d7 | d13 |
| WBC | G/l | 10.0 | 11.1 | 12.9 | 6.1 | 11.2 | 6.3 |
| RBC | T/l | 7.3 | 6.5 | 6.4 | 6.8 | 6.5 | 6.2 |
| HB | g/dl | 12.9 | 11.9 | 11.7 | 13.1 | 12.3 | 11.9 |
| PCV | l/l | 0.44 | 0.40 | 0.44 | 0.42 | 0.41 | 0.41 |
| MCV | fl | 60 | 61 | 68 | 61 | 63 | 66 |
| MCH | pg | 17.8 | 18.2 | 18.1 | 19.3 | 19.0 | 19.0 |
| MCHC | g/dl | 29.8 | 29.6 | 26.8 | 31.5 | 30.1 | 28.9 |
| PLAT | G/l | 316 | 371 | 266 | 458 | 500 | 547 |
| N | G/l | 6.46 | 4.93 | 3.65 | 2.09 | 6.77 | 1.24 |
| E | G/l | 0.01 | 0.14 | 0.20 | 0.10 | 0.10 | 0.10 |
| B | G/l | 0.02 | 0.03 | 0.06 | 0.02 | 0.02 | 0.00 |
| L | G/l | 3.05 | 5.45 | 8.44 | 3.60 | 3.65 | 4.51 |
| M | G/l | 0.46 | 0.51 | 0.54 | 0.33 | 0.64 | 0.46 |

| Salmon Calcitonin | | | | | | | |
|---|---|---|---|---|---|---|---|
| Animal no. | | | W62503 | | | W62504 | |
| Test | Units | d-6 | d7 | d13 | d-6 | d7 | d13 |
| WBC | G/l | 7.7 | 11.8 | 8.0 | 11.5 | 9.5 | 8.8 |
| RBC | T/l | 6.3 | 5.9 | 5.6 | 6.9 | 6.0 | 5.4 |
| HB | g/dl | 12.6 | 11.7 | 11.2 | 13.6 | 11.5 | 10.3 |
| PCV | l/l | 0.40 | 0.39 | 0.39 | 0.43 | 0.37 | 0.36 |
| MCV | fl | 64 | 66 | 70 | 62 | 62 | 67 |
| MCH | pg | 20.2 | 19.9 | 20.2 | 19.7 | 19.2 | 19.2 |
| MCHC | g/dl | 31.4 | 30.3 | 29.0 | 32.0 | 31.3 | 28.7 |
| PLAT | G/l | 351 | 396 | 302 | 247 | 330 | 389 |
| N | G/l | 3.36 | 4.11 | 1.90 | 3.93 | 3.31 | 3.04 |
| E | G/l | 0.02 | 0.10 | 0.13 | 0.16 | 0.09 | 0.01 |
| B | G/l | 0.02 | 0.04 | 0.03 | 0.08 | 0.04 | 0.03 |
| L | G/l | 4.00 | 6.79 | 5.38 | 6.55 | 5.57 | 4.92 |
| M | G/l | 0.30 | 0.73 | 0.57 | 0.76 | 0.45 | 0.76 |

| Haematology - Males | | | | | | | |
|---|---|---|---|---|---|---|---|
| PTS893 | | | | | | | |
| Animal no. | | W62505 | | | W62506 | | |
| Test | Units | d-6 | d7 | d13 | d-6 | d7 | d13 |
| WBC | G/l | 10.4 | 8.4 | 8.8 | 9.1 | 15.0 | 11.9 |
| RBC | T/l | 7.6 | 6.4 | 6.8 | 6.5 | 5.9 | 5.8 |
| HB | g/dl | 13.6 | 11.3 | 11.7 | 13.2 | 11.9 | 11.8 |
| PCV | 1/l | 0.43 | 0.38 | 0.43 | 0.40 | 0.40 | 0.41 |
| MCV | fl | 57 | 60 | 63 | 62 | 67 | 70 |
| MCH | pg | 18.0 | 17.7 | 17.3 | 20.4 | 20.2 | 20.3 |
| MCHC | g/dl | 31.5 | 29.3 | 27.5 | 33.1 | 30.2 | 29.2 |
| PLAT | G/l | 325 | 456 | 330 | 459 | 589 | 452 |
| N | G/l | 4.45 | 1.77 | 2.88 | 4.80 | 8.73 | 6.51 |
| E | G/l | 0.21 | 0.30 | 0.19 | 0.03 | 0.08 | 0.07 |
| B | G/l | 0.00 | 0.02 | 0.04 | 0.02 | 0.03 | 0.03 |
| L | G/l | 5.07 | 5.91 | 5.37 | 3.99 | 5.30 | 4.86 |
| M | G/l | 0.62 | 0.39 | 0.27 | 0.27 | 0.83 | 0.46 |

| | | | | | | | |
|---|---|---|---|---|---|---|---|
| d-6, d7 and d13 indicate day -6, day 7 and day 13 relative to the starting day of dosing d-6, d7 and d13 indicate day -6, day 7 and day 13 relative to the starting day of dosing | | | | | | | |

**TABLE 26**

| Haematology - Females | | | | | | | |
|---|---|---|---|---|---|---|---|
| Control | | | | | | | |
| Animal no. | | | W62551 | | | W62552 | |
| Test | Units | d-8 | d7 | d13 | d-8 | d7 | d13 |
| WBC | pg/ml | 8.2 | 13.7 | 10.0 | 10.1 | 9.1 | 10.4 |
| RBC | nmol/l | 6.5 | 6.2 | 5.8 | 6.7 | 6.2 | 5.8 |
| HB | pg/ml | 12.8 | 11.8 | 11.3 | 13.1 | 11.7 | 11.4 |
| PCV | mU/l | 0.42 | 0.43 | 0.41 | 0.42 | 0.42 | 0.41 |
| MCV | pg/ml | 64 | 69 | 71 | 63 | 68 | 70 |
| MCH | ng/ml | 19.7 | 19.1 | 19.4 | 19.5 | 18.9 | 19.5 |
| MCHC | pg/ml | 30.6 | 27.7 | 27.4 | 30.9 | 27.7 | 27.9 |
| PLAT | nmol/l | 463 | 445 | 468 | 286 | 292 | 275 |
| N | nmol/l | 4.45 | 5.86 | 3.53 | 6.69 | 3.13 | 4.23 |
| E | mUI/l | 0.03 | 0.13 | 0.12 | 0.01 | 0.15 | 0.19 |
| B | pg/ml | 0.03 | 0.07 | 0.04 | 0.02 | 0.03 | 0.03 |
| L | pg/ml | 3.40 | 7.09 | 5.91 | 3.14 | 5.39 | 5.34 |
| M | nmol/l | 0.27 | 0.51 | 0.39 | 0.25 | 0.39 | 0.59 |

| | | | | | | | |
|---|---|---|---|---|---|---|---|
| d-8, d7 and d13 indicate day -8, day 7 and day 13 relative to the starting day of dosing | | | | | | | |

**TABLE 27**

| Haematology - Females | | | | | | | |
|---|---|---|---|---|---|---|---|
| Salmon Calcitonin | | | | | | | |
| Animal no. | | W62553 | | | W62554 | | |
| Test | Units | d-8 | d7 | d13 | d-8 | d7 | d13 |
| WBC | pg/ml | 7.0 | 9.5 | 12.0 | 8.3 | 17.0 | 13.3 |
| RBC | nmol/l | 6.5 | 6.2 | 5.2 | 7.0 | 6.6 | 5.7 |
| HB | pg/ml | 12.3 | 11.5 | 10.1 | 13.8 | 12.7 | 11.0 |
| PCV | mU/l | 0.40 | 0.40 | 0.33 | 0.45 | 0.44 | 0.37 |
| MCV | pg/ml | 61 | 64 | 64 | 65 | 68 | 65 |
| MCH | ng/ml | 19.1 | 18.6 | 19.5 | 19.8 | 19.4 | 19.5 |
| MCHC | pg/ml | 31.2 | 29.0 | 30.3 | 30.6 | 28.7 | 29.9 |
| PLAT | nmol/l | 549 | 594 | 451 | 304 | 356 | 229 |
| N | nmol/l | 3.45 | 3.83 | 5.41 | 3.13 | 9.82 | 6.16 |
| E | mUl/l | 0.03 | 0.36 | 0.73 | 0.03 | 0.04 | 0.06 |
| B | pg/ml | 0.02 | 0.03 | 0.03 | 0.01 | 0.07 | 0.05 |
| L | pg/ml | 3.26 | 4.61 | 5.18 | 4.79 | 6.21 | 6.58 |
| M | nmol/l | 0.25 | 0.63 | 0.69 | 0.30 | 0.82 | 0.39 |

| PTS893 | | | | | | | |
|---|---|---|---|---|---|---|---|
| Animal no. | | W62555 | | | W62556 | | |
| Test | Units | d-8 | d7 | d13 | d-8 | d7 | d13 |
| WBC | pg/ml | 10.1 | 18.4 | 13.2 | 14.3 | 12.3 | 10.1 |
| RBC | nmol/l | 6.9 | 6.2 | 5.9 | 6.7 | 6.4 | 5.9 |
| HB | pg/ml | 13.4 | 11.7 | 11.3 | 12.9 | 12.1 | 11.3 |
| PCV | mU/l | 0.44 | 0.41 | 0.40 | 0.43 | 0.43 | 0.39 |
| MCV | pg/ml | 63 | 67 | 67 | 64 | 68 | 66 |
| MCH | ng/ml | 19.3 | 18.9 | 19.3 | 19.3 | 19.0 | 19.2 |
| MCHC | pg/ml | 30.6 | 28.2 | 28.6 | 30.2 | 28.1 | 29.2 |
| PLAT | nmol/l | 501 | 525 | 496 | 213 | 382 | 309 |
| N | nmol/l | 5.34 | 10.8 | 6.36 | 9.05 | 5.49 | 4.18 |
| E | mUI/l | 0.00 | 0.12 | 0.21 | 0.26 | 0.49 | 0.29 |
| B | pg/ml | 0.00 | 0.06 | 0.03 | 0.03 | 0.04 | 0.04 |
| L | pg/ml | 3.92 | 6.29 | 5.81 | 4.40 | 5.87 | 5.21 |
| M | umol/l | 0.80 | 1.12 | 0.82 | 0.54 | 0.44 | 0.37 |

| | | | | | | | |
|---|---|---|---|---|---|---|---|
| d-8, d7 and d1 3 indicate day -8, day 7 and day 13 relative to the starting day of dosing | | | | | | | |

Among the standard clinical chemistry tests performed, slight to moderate decreases in phosphorus and/or magnesium and a moderate to marked decrease in triglycerides were seen in the groups administered salmon calcitonin and PTS893.

**TABLE 28**

| Clinical Chemistry - Males | | | | | | | |
|---|---|---|---|---|---|---|---|
| Control | | | | | | | |
| Animal no. | | W62501 | | | W62502 | | |
| Test | Units | d-6 | d7 | d13 | d-6 | d7 | d13 |
| Na+ | mmol/l | 154 | 151 | 153 | 152 | 153 | 148 |
| K+ | mmol/l | 4.05 | 5.31 | 4.26 | 4.09 | 4.05 | 4.51 |
| Cl- | mmol/l | 109 | 113 | 108 | 107 | 110 | 111 |
| Ca++ | nunol/l | 2.57 | 2.47 | 2.69 | 2.72 | 2.52 | 2.75 |
| LPHOS | mmol/l | 2.21 | 1.93 | 2.76 | 1.88 | 1.69 | 1.99 |
| Mg++ | mmol/l | 1.09 | 0.91 | 0.95 | 0.88 | 0.79 | 1.14 |
| GLUC | mmol/l | 3.85 | 4.51 | 4.68 | 3.44 | 5.30 | 6.13 |
| UREA | mmol/l | 9.7 | 4.9 | 5.0 | 7.6 | 6.1 | 5.2 |
| CREAT | µmol/l | 85 | 60 | 75 | 65 | 55 | 57 |
| TOT.BIL. | µmol/l | 6.0 | 2.0 | 2.0 | 7.0 | 3.0 | 4.0 |
| PROT | g/l | 89 | 80 | 88 | 90 | 83 | 85 |
| A/G | | 1.89 | 1.57 | 1.45 | 1.62 | 1.53 | 1.50 |
| CHOL | mmol/l | 3.30 | 3.20 | 3.50 | 3.30 | 3.40 | 3.10 |
| HDL-CHOL | mmol/l | 1.49 | 1.45 | 1.70 | 1.54 | 1.45 | 1.49 |
| LDL-CHOL | mmol/l | 1.63 | 1.62 | 1.84 | 1.56 | 1.93 | 1.49 |
| TRIG | mmol/l | 0.94 | 0.36 | 0.43 | 0.65 | 0.36 | 0.45 |
| ALP | IU/l | 1559 | 1241 | 1313 | 1463 | 1423 | 1493 |
| BAP-E | IU/l | 543 | 439 | 457 | 452 | 476 | 464 |
| ASAT | IU/l | 22 | 22 | 25 | 30 | 26 | 26 |
| ALAT | IU/l | 22 | 32 | 30 | 29 | 41 | 37 |
| CK | IU/l | 150 | 45 | 127 | 74 | 67 | 102 |
| LDH | IU/l | 392 | 585 | 549 | 421 | 518 | 592 |
| GGT | IU/l | 128 | 92 | 111 | 89 | 71 | 75 |
| ALB | % | 65 | 61 | 59 | 62 | 61 | 60 |
| A1-GLOB | % | 1.90 | 2.70 | 2.50 | 1.90 | 2.10 | 2.30 |
| A2-GLOB | % | 7.60 | 8.30 | 7.90 | 8.20 | 8.90 | 8.50 |
| B-GLOB | % | 16 | 18 | 19 | 18 | 19 | 19 |
| G-GLOB | % | 9.2 | 9.9 | 10.9 | 9.6 | 9.3 | 10.2 |
| ALB | g/l | 58 | 49 | 52 | 56 | 50 | 51 |
| A1-GLOB | g/l | 1.70 | 2.20 | 2.20 | 1.70 | 1.70 | 2.00 |
| A2-GLOB | g/l | 6.80 | 6.60 | 7.00 | 7.40 | 7.40 | 7.20 |
| B-GLOB | g/l | 14 | 14 | 17 | 17 | 16 | 16 |
| G-GLOB | g/l | 8.2 | 7.9 | 9.6 | 8.6 | 7.7 | 8.7 |

| Salmon Calcitonin | | | | | | | |
|---|---|---|---|---|---|---|---|
| Animal no. | | W62503 | | | W62504 | | |
| Test | Units | d-6 | d7 | d13 | d-6 | d7 | d13 |
| Na+ | mmol/l | 151 | 145 | 148 | 154 | 142 | 144 |
| K+ | mmol/l | 4.24 | 4.90 | 4.34 | 4.85 | 5.15 | 4.48 |
| Cl⁻ | mmol/l | 107 | 104 | 104 | 113 | 106 | 101 |
| Ca++ | mmol/l | 2.66 | 2.68 | 2.91 | 2.71 | 2.54 | 2.73 |
| I.PHOS | mmol/l | 2.05 | 1.67 | 2.06 | 2.10 | 1.73 | 1.94 |
| Mg++ | mmol/l | 0.97 | 0.68 | 0.73 | 0.99 | 0.71 | 0.72 |
| GLUC | mmol/l | 3.57 | 3.58 | 4.29 | 3.70 | 4.98 | 6.19 |
| UREA | mmol/l | 7.9 | 1.3 | 2.9 | 6.6 | 3.3 | 2.9 |
| CREAT | µmol/l | 78 | 57 | 62 | 64 | 50 | 56 |
| TOT.BIL. | µmol/l | 5.0 | 2.0 | 1.0 | 3.0 | 2.0 | 2.0 |
| PROT | g/l | 87 | 82 | 87 | 91 | 83 | 89 |
| A/G | | 1.76 | 1.68 | 1.42 | 1.42 | 1.26 | 1.05 |
| CHOL | mmol/l | 3.30 | 3.60 | 3.70 | 3.80 | 3.90 | 3.40 |
| HDL-CHOL | mmol/l | 1.49 | 2.09 | 2.44 | 1.46 | 1.48 | 1.39 |
| LDL-CHOL | mmol/l | 1.21 | 1.28 | 1.26 | 1.87 | 2.51 | 1.83 |
| TRIG | mmol/l | 0.96 | 0.24 | 0.27 | 0.92 | 0.22 | 0.68 |
| ALP | IU/l | 1488 | 1023 | 1226 | 857 | 587 | 626 |
| BAP-E | IU/l | 508 | 363 | 302 | 311 | 188 | 180 |
| ASAT | IU/l | 28 | 31 | 28 | 24 | 17 | 24 |
| ALAT | IU/l | 38 | 39 | 43 | 48 | 24 | 31 |
| CK | IU/l | 124 | 56 | 119 | 75 | 45 | 173 |
| LDH | IU/l | 439 | 400 | 427 | 356 | 384 | 519 |
| GGT | IU/l | 105 | 80 | 75 | 121 | 75 | 69 |
| ALB | % | 64 | 63 | 59 | 59 | 56 | 51 |
| A1-GLOB | % | 1.60 | 2.00 | 2.40 | 1.90 | 2.80 | 3.60 |
| A2-GLOB | % | 8.00 | 8.80 | 8.80 | 8.70 | 8.70 | 7.80 |
| B-GLOB | % | 18 | 18 | 20 | 19 | 21 | 24 |
| G-GLOB | % | 8.3 | 8.5 | 9.7 | 12.0 | 12.1 | 13.6 |
| ALB | g/l | 56 | 51 | 51 | 54 | 46 | 46 |
| A1-GLOB | g/l | 1.40 | 1.60 | 2.10 | 1.70 | 2.30 | 3.20 |
| A2-GLOB | g/l | 7.00 | 7.20 | 7.70 | 7.90 | 7.20 | 6.90 |
| B-GLOB | g/l | 16 | 15 | 18 | 17 | 17 | 21 |
| G-GLOB | g/l | 7.2 | 7.0 | 8.4 | 10.9 | 10.0 | 12.1 |

| PTS893 | | | | | | | |
|---|---|---|---|---|---|---|---|
| Animal no. | | W62505 | | | W62506 | | |
| Test | Units | d-6 | d7 | d13 | d-6 | d7 | d13 |
| Na+ | mmol/l | 151 | 151 | 152 | 151 | 149 | 149 |
| K+ | mmol/l | 5.13 | 4.00 | 4.27 | 4.72 | 4.76 | 4.12 |
| Cl- | mmol/l | 110 | 107 | 110 | 112 | 106 | 106 |
| Ca++ | mmol/l | 2.81 | 2.39 | 2.59 | 2.64 | 2.45 | 2.51 |
| I.PHOS | mmol/l | 2.59 | 1.68 | 2.22 | 2.12 | 1.12 | 1.77 |
| Mg++ | mmol/l | 1.04 | 0.71 | 0.77 | 0.97 | 0.70 | 0.76 |
| GLUC | mmol/l | 5.09 | 4.76 | 5.42 | 3.88 | 5.26 | 4.96 |
| UREA | mmol/l | 11.6 | 3.7 | 6.4 | 15.0 | 4.9 | 5.8 |
| CREAT | µmol/l | 86 | 66 | 79 | 77 | 63 | 70 |
| TOT.BIL. | µmol/l | 5.0 | 2.0 | 1.0 | 7.0 | 2.0 | 1.0 |
| PROT | g/l | 81 | 74 | 81 | 88 | 86 | 89 |
| A/G | | 1.89 | 1.70 | 1.76 | 1.58 | 1.28 | 1.40 |
| CHOL | mmol/l | 3.20 | 3.30 | 3.10 | 2.50 | 2.50 | 2.60 |
| HDL-CHOL | mmol/l | 1.49 | 1.49 | 1.61 | 1.24 | 1.25 | 1.38 |
| LDL-CHOL | mmol/l | 1.39 | 1.73 | 1.51 | 1.27 | 1.22 | 1.38 |
| TRIG | mmol/l | 0.96 | 0.30 | 0.63 | 0.49 | 0.39 | 0.35 |
| ALP | IU/l | 1703 | 1494 | 1768 | 1414 | 1363 | 1486 |
| BAP-E | IU/l | 523 | 532 | 564 | 445 | 423 | 497 |
| ASAT | IU/l | 24 | 18 | 24 | 25 | 27 | 29 |
| ALAT | IU/l | 32 | 30 | 27 | 23 | 19 | 20 |
| CK | IU/l | 111 | 82 | 148 | 86 | 73 | 125 |
| LDH | IU/l | 367 | 400 | 528 | 354 | 432 | 464 |
| GGT | IU/l | 133 | 99 | 105 | 112 | 85 | 91 |
| ALB | % | 66 | 63 | 64 | 61 | 56 | 59 |
| A1-GLOB | % | 2.20 | 2.80 | 2.60 | 2.40 | 3.60 | 2.80 |
| A2-GLOB | % | 8.80 | 8.90 | 8.70 | 7.30 | 8.30 | 7.50 |
| B-GLOB | % | 17 | 18 | 19 | 19 | 22 | 20 |
| G-GLOB | % | 6.9 | 6.9 | 6.3 | 9.8 | 10.5 | 10.9 |
| ALB | g/l | 53 | 47 | 52 | 54 | 48 | 52 |
| A1-GLOB | g/l | 1.80 | 2.10 | 2.10 | 2.10 | 3.10 | 2.50 |
| A2-GLOB | g/l | 7.10 | 6.60 | 7.10 | 6.40 | 7.10 | 6.70 |
| B-GLOB | g/l | 14 | 14 | 15 | 17 | 19 | 18 |
| G-GLOB | g/l | 5.6 | 5.1 | 5.1 | 8.6 | 9.0 | 9.7 |

| | | | | | | | |
|---|---|---|---|---|---|---|---|
| d-6, d7 and d13 indicate day -6, day 7 and day 13 relative to the starting day dosing. d-6, d7 and d13 indicate day -6, day 7 and day 13 relative to the starting day of dosing d-6, d7 and d13 indicate day -6, day 7 and day 13 relative to the starting day of dosing | | | | | | | |

**TABLE 29**

| Clinical Chemistry - Females | | | | | | | |
|---|---|---|---|---|---|---|---|
| Control | | | | | | | |
| Animal no. | | W62551 | | | W62552 | | |
| Test | Units | d-8 | d7 | d13 | d-8 | d7 | d13 |
| Na+ | mmol/l | 152 | 148 | 155 | 148 | 150 | 148 |
| K+ | mmol/l | 4.16 | 4.23 | 4.92 | 3.82 | 4.11 | 5.27 |
| Cl- | mmol/l | 110 | 105 | 111 | 109 | 106 | 108 |
| Ca++ | mmol/l | 2.64 | 2.61 | 2.61 | 2.48 | 2.44 | 1.80 |
| I.PHOS | mmol/l | 1.98 | 2.61 | 2.28 | 1.84 | 1.98 | 1.84 |
| Mg++ | mmol/l | 1.00 | 0.97 | 1.03 | 0.88 | 0.84 | 0.31 |
| GLUC | mmol/l | 3.65 | 8.39 | 3.86 | 2.79 | 3.86 | 3.60 |
| UREA | mmol/l | 11.0 | 8.3 | 8.2 | 11.3 | 6.9 | 6.3 |
| CREAT | µmol/l | 73 | 77 | 62 | 67 | 60 | 50 |
| TOT.BIL. | µmol/l | 4.00 | 2.00 | 3.00 | 5.00 | 1.00 | 2.00 |
| PROT | g/l | 85 | 80 | 80 | 83 | 83 | 77 |
| A/G | | 1.77 | 1.67 | 1.55 | 1.68 | 1.39 | 1.27 |
| CHOL | mmol/l | 3.20 | 2.80 | 3.00 | 3.70 | 3.40 | 3.50 |
| HDL-CHOL | mmol/l | 1.63 | 1.44 | 1.49 | 1.75 | 1.82 | 1.80 |
| LDL-CHOL | mmol/l | 1.55 | 1.25 | 1.90 | 1.57 | 1.28 | 1.66 |
| TRIG | mmol/l | 0.64 | 0.54 | 0.57 | 0.83 | 0.48 | 0.50 |
| ALP | IU/l | 1037 | 1088 | 1187 | 1332 | 1298 | 1182 |
| BAP-E | IU/l | 310 | 369 | 346 | 432 | 419 | 379 |
| ASAT | IU/l | 27 | 33 | 31 | 21 | 22 | 23 |
| ALAT | IU/l | 44 | 52 | 46 | 16 | 19 | 20 |
| CK | IU/l | 69 | 169 | 81 | 83 | 68 | 87 |
| LDH | IU/l | 420 | 520 | 481 | 474 | 471 | 516 |
| GGT | IU/l | 104 | 95 | 102 | 84 | 67 | 66 |
| ALB | % | 64 | 63 | 61 | 63 | 58 | 56 |
| A1-GLOB | % | 1.90 | 2.60 | 3.40 | 2.00 | 2.60 | 3.50 |
| A2-GLOB | % | 8.00 | 7.60 | 7.70 | 7.00 | 8.10 | 7.70 |
| B-GLOB | % | 17 | 18 | 18 | 15 | 18 | 18 |
| G-GLOB | % | 9.4 | 9.2 | 9.9 | 12.9 | 13.2 | 14.8 |
| ALB | g/l | 54 | 50 | 49 | 52 | 48 | 43 |
| A1-GLOB | g/l | 1.60 | 2.10 | 2.70 | 1.70 | 2.20 | 2.70 |
| A2-GLOB | g/l | 6.80 | 6.10 | 6.20 | 5.80 | 6.70 | 5.90 |
| B-GLOB | g/l | 14 | 14 | 15 | 13 | 15 | 14 |
| G-GLOB | g/l | 8.0 | 7.4 | 7.9 | 10.7 | 11.0 | 11.4 |

| Salmon Calcitonin | | | | | | | |
|---|---|---|---|---|---|---|---|
| Animal no. | | W62553 | | | W62554 | | |
| Test | Units | d-8 | d7 | d13 | d-8 | d7 | d13 |
| Na+ | mmol/l | 145 | 147 | 147 | 145 | 143 | 147 |
| K+ | mmol/l | 3.51 | 3.73 | 4.62 | 3.89 | 4.07 | 4.95 |
| Cl- | mmol/l | 106 | 104 | 107 | 100 | 96 | 107 |
| Ca++ | mmol/l | 2.62 | 2.77 | 2.57 | 2.73 | 2.91 | 2.68 |
| I.PHOS | mmol/l | 1.62 | 1.48 | 1.81 | 1.97 | 1.75 | 1.83 |
| Mg++ | mmol/l | 0.87 | 0.63 | 0.76 | 0.91 | 0.77 | 0.80 |
| GLUC | mmol/l | 3.84 | 4.88 | 4.98 | 4.11 | 5.31 | 4.04 |
| UREA | mmol/l | 10.3 | 6.6 | 5.0 | 10.0 | 6.3 | 5.9 |
| CREAT | µmol/l | 81 | 71 | 61 | 88 | 77 | 65 |
| TOT.BIL. | µmol/l | 3.00 | 2.00 | 2.00 | 6.00 | 5.00 | 2.00 |
| PROT | g/l | 88 | 90 | 80 | 91 | 95 | 83 |
| A/G | | 1.46 | 1.45 | 1.30 | 1.48 | 1.42 | 1.26 |
| CHOL | mmol/l | 2.70 | 2.80 | 2.20 | 3.30 | 4.00 | 3.00 |
| HDL-CHOL | mmol/l | 1.04 | 1.11 | 0.96 | 1.46 | 1.99 | 1.66 |
| LDL-CHOL | mmol/l | 1.61 | 1.51 | 1.46 | 1.13 | 1.93 | 1.42 |
| TRIG | mmol/l | 0.79 | 0.25 | 0.39 | 0.88 | 0.30 | 0.38 |
| ALP | IU/l | 1197 | 965 | 842 | 1132 | 877 | 890 |
| BAP-E | IU/l | 416 | 326 | 304 | 344 | 325 | 294 |
| ASAT | IU/l | 24 | 21 | 25 | 20 | 18 | 20 |
| ALAT | IU/l | 21 | 24 | 19 | 19 | 14 | 19 |
| CK | IU/l | 99 | 72 | 107 | 76 | 64 | 77 |
| LDH | IU/l | 286 | 423 | 429 | 319 | 372 | 363 |
| GGT | IU/l | 88 | 63 | 54 | 82 | 72 | 62 |
| ALB | % | 59 | 59 | 57 | 60 | 59 | 56 |
| A1-GLOB | % | 2.70 | 2.70 | 3.10 | 2.20 | 2.20 | 3.10 |
| A2-GLOB | % | 6.50 | 6.10 | 6.80 | 8.00 | 7.70 | 7.80 |
| B-GLOB | % | 21 | 23 | 21 | 15 | 17 | 17 |
| G-GLOB | % | 10.8 | 8.6 | 12.4 | 14.9 | 14.6 | 16.3 |
| ALB | g/l | 52 | 54 | 45 | 54 | 56 | 46 |
| A1-GLOB | g/l | 2.40 | 2.40 | 2.50 | 2.00 | 2.10 | 2.60 |
| A2-GLOB | g/l | 5.70 | 5.50 | 5.40 | 7.30 | 7.30 | 6.50 |
| B-GLOB | g/l | 18 | 21 | 17 | 14 | 16 | 14 |
| G-GLOB | g/l | 9.5 | 7.7 | 9.9 | 13.6 | 13.9 | 13.5 |
| d-8, d7 and d13 indicate day -8, day 7 and day 13 relative to the starting day of dosing | | | | | | | |

| PTS893 | | | | | | | |
|---|---|---|---|---|---|---|---|
| Animal no. | | | W62555 | | | W62556 | |
| Test | Units | d-8 | d7 | d13 | d-8 | d7 | d13 |
| Na+ | mmol/l | 153 | 151 | 152 | 150 | 148 | 149 |
| K+ | mmol/l | 4.82 | 4.54 | 4.63 | 3.85 | 3.81 | 4.31 |
| Cl- | mmol/l | 107 | 109 | 111 | 108 | 107 | 114 |
| Ca++ | mmol/l | 2.77 | 2.61 | 2.20 | 2.64 | 2.62 | 2.35 |
| I.PHOS | mmol/l | 2.11 | 1.31 | 1.51 | 2.10 | 1.60 | 1.50 |
| Mg++ | mmol/l | 0.96 | 0.65 | 0.59 | 0.90 | 0.74 | 0.66 |
| GLUC | mmol/l | 3.57 | 4.18 | 3.59 | 3.22 | 4.45 | 3.52 |
| UREA | mmol/l | 8.2 | 8.7 | 6.3 | 8.4 | 6.6 | 6.8 |
| CREAT | µmol/l | 77 | 62 | 58 | 68 | 63 | 58 |
| TOT.BIL. | µmol/l | 5.00 | 1.00 | 2.00 | 5.00 | 2.00 | 2.00 |
| PROT | g/l | 89 | 87 | 78 | 84 | 83 | 76 |
| A/G | | 1.64 | 1.62 | 1.65 | 1.84 | 1.78 | 1.50 |
| CHOL | mmol/l | 2.90 | 2.70 | 2.80 | 2.70 | 2.40 | 2.70 |
| HDL-CHOL | mmol/l | 1.31 | 1.48 | 1.51 | 1.12 | 0.99 | 1.25 |
| LDL-CHOL | mmol/l | 1.69 | 1.12 | 1.71 | 1.62 | 1.28 | 1.58 |
| TRIG | mmol/l | 0.59 | 0.27 | 0.25 | 0.67 | 0.34 | 0.47 |
| ALP | IU/l | 1535 | 1223 | 1332 | 1638 | 1307 | 1313 |
| BAP-E | IU/l | 457 | 350 | 426 | 456 | 390 | 400 |
| ASAT | IU/l | 23 | 18 | 25 | 24 | 20 | 25 |
| ALAT | IU/l | 35 | 25 | 32 | 33 | 19 | 21 |
| CK | IU/l | 84 | 65 | 175 | 63 | 144 | 172 |
| LDH | IU/l | 468 | 465 | 557 | 309 | 313 | 358 |
| GGT | IU/l | 85 | 71 | 70 | 103 | 85 | 83 |
| ALB | % | 62 | 62 | 62 | 65 | 64 | 60 |
| A1-GLOB | % | 2.30 | 2.50 | 2.50 | 1.90 | 2.10 | 2.70 |
| A2-GLOB | % | 7.50 | 8.00 | 8.30 | 7.50 | 7.50 | 8.10 |
| B-GLOB | % | 18 | 19 | 18 | 17 | 17 | 20 |
| G-GLOB | % | 9.7 | 8.4 | 8.7 | 8.8 | 9.1 | 8.7 |
| ALB | g/l | 55 | 54 | 49 | 55 | 53 | 46 |
| A1-GLOB | g/l | 2.10 | 2.20 | 2.00 | 1.60 | 1.70 | 2.10 |
| A2-GLOB | g/l | 6.70 | 7.00 | 6.50 | 6.30 | 6.20 | 6.20 |
| B-GLOB | g/l | 16 | 17 | 14 | 14 | 14 | 16 |
| G-GLOB | g/l | 8.6 | 7.3 | 6.8 | 7.4 | 7.6 | 6.6 |
| d-8, d7 and d13 indicate day -8, day 7 and day 13 relative to the starting day of dosing | | | | | | | |

| | | | | | | | |
|---|---|---|---|---|---|---|---|
| d-8, d7 and d13 indicate day -8, day 7 and day 13 relative to the starting day of dosing | | | | | | | |

No relevant changes were observed in the standard urinalysis tests performed.

**TABLE 30**

| Urinary Analysis - Males | | | | | | | |
|---|---|---|---|---|---|---|---|
| Control | | | | | | | |
| Animal no. | | W62501 | | | W62502 | | |
| Test | Units | -6 | -5 | 13 | -6 | -5 | 13 |
| VOLUME | ml | 15 | 10 | 77 | 22 | 130 | 30 |
| CREAT | µmol/l | 18000 | 17000 | 5460 | 7920 | 2480 | 5160 |
| NTx | nM BCE | - | 9954 | 3425 | - | 11979 | 3167 |
| CTx | µg/l | - | 21592 | 6810 | - | 27169 | 5323 |
| D-PYR | nmol/l | - | 2345 | 1110 | - | 2904 | 1461 |
| LDH | IU/L | 6.0 | | nd | 8.0 | | 8.0 |
| NAG | IU/l | 3.5 | | 1.5 | 3.2 | | 1.6 |
| Na+ | mmol/l | 163 | | 43 | 87 | | 77 |
| K+ | mmol/l | 258 | | 67 | 125 | | 75 |
| Cl- | mmol/l | 132 | | 43 | 52 | | 59 |
| Ca2+ | mmol/l | 5.15 | | 16.80 | 15.95 | | 15.50 |
| I.PHOS | mmol/l | 11.10 | | 1.05 | 11.30 | | 8.90 |
| Mg2+ | mmol/l | 2.75 | | 7.50 | 7.85 | | 6.25 |
| Na/Crea | mM/mM | 9.10 | | 7.90 | 11.00 | | 14.90 |
| K/Crea | mM/mM | 14.30 | | 12.20 | 15.80 | | 14.50 |
| Cl/Crea | mM/mM | 7.40 | | 7.90 | 6.50 | | 11.40 |
| Ca/Crea | mM/mM | 0.29 | | 3.08 | 2.01 | | 3.00 |
| Pho/Crea | mM/mM | 0.62 | | 0.19 | 1.43 | | 1.73 |
| Mg/Crea | mM/mM | 0.20 | | 1.40 | 1.00 | | 1.20 |
| LDH/crea | IU/mM | 0.33 | | nd | 1.01 | | 1.55 |
| NAG/crea | IU/mM | 0.19 | | 0.28 | 0.40 | | 0.31 |
| NTx/Crea | nME/mM | | 586 | 627 | | 4830 | 614 |
| CTx/Crea | µg/µm | | 1270 | 1247 | | 10955 | 1032 |
| Pyr/Crea | nM/mM | | 138 | 203 | | 1171 | 283 |
| d-6, d-5 and d13 indicate day -6, day -5 and day 13 relative to the starting day of dosing | | | | | | | |

| Salmon Calcitonin | | | | | | | |
|---|---|---|---|---|---|---|---|
| Animal no. | | | W62503 | | | W62504 | |
| Test | Units | -6 | -5 | 13 | -6 | -5 | 13 |
| VOLUME | ml | 62 | 38 | 68 | 37 | 10 | 54 |
| CREAT | µmol/l | 4300 | 7840 | 4620 | 13600 | 17360 | 4400 |
| NTx | nM BCE | - | 6023 | 5186 | - | 16067 | 3790 |
| CTx | µg/l | - | 11618 | 10088 | - | 26370 | 6130 |
| D-PYR | nmol/l | - | 1733 | 1083 | - | 5113 | 1476 |
| LDH | IU/L | 9.0 | | 7.0 | 13.0 | | 17.0 |
| NAG | IU/l | 2.7 | | 1.4 | 4.2 | | 7.2 |
| Na+ | mmol/l | 22 | | 14 | 119 | | 15 |
| K+ | mmol/l | 65 | | 78 | 134 | | 76 |
| Cl- | mmol/l | 10 | | 55 | 64 | | 68 |
| Ca2+ | mmol/l | 0.90 | | 18.25 | 3.70 | | 23.40 |
| I.PHOS | mmol/l | 4.35 | | 2.50 | 5.33 | | 3.00 |
| Mg2+ | mmol/l | 1.40 | | 7.05 | 7.55 | | 9.80 |
| Na/Crea | mM/mM | 5.20 | | 3.10 | 8.70 | | 3.40 |
| K/Crea | mM/mM | 15.10 | | 16.90 | 9.90 | | 17.20 |
| Cl/Crea | mM/mM | 2.20 | | 11.80 | 4.70 | | 15.30 |
| Ca/Crea | mM/mM | 0.21 | | 3.95 | 0.27 | | 5.32 |
| Pho/Crea | mM/mM | 1.01 | | 0.54 | 0.39 | | 0.68 |
| Mg/Crea | mM/mM | 0.30 | | 1.50 | 0.60 | | 2.20 |
| LDH/crea | IU/mM | 2.09 | | 1.52 | 0.96 | | 3.86 |
| NAG/crea | IU/mM | 0.63 | | 0.30 | 0.31 | | 1.64 |
| NTx/Crea | nME/mM | | 768 | 1123 | | 926 | 861 |
| CTx/Crea | µg/µm. | | 1482 | 2184 | | 1519 | 1393 |
| Pyr/Crea | nM/mM | | 221 | 234 | | 295 | 336 |

| PTS893 | | | | | | | |
|---|---|---|---|---|---|---|---|
| Animal no. | | W62505 | | | W62506 | | |
| Test | Units | -6 | -5 | 13 | -6 | -5 | 13 |
| VOLUME | ml | 14 | 14 | 48 | 58 | 34 | 130 |
| CREAT | µmol/l | 16160 | 16160 | 7840 | 9940 | 16120 | 3840 |
| NTx | nM BCE | - | 5403 | 4871 | - | 8757 | 2102 |
| CTx | µg/l | - | 11865 | 9365 | - | 20108 | 3705 |
| D-PYR | nmol/l | - | 1660 | 1676 | - | 2278 | 782 |
| LDH | IU/L | 7.0 | | 14.0 | 9.0 | | 19.0 |
| NAG | IU/l | 23.4 | | 2.9 | 7.1 | | 2.6 |
| Na+ | mmol/l | 174 | | 111 | 59 | | 35 |
| K+ | mmol/l | 86 | | 107 | 125 | | 69 |
| Cl- | mmol/l | 22 | | 117 | 50 | | 48 |
| Ca2+ | mmol/l | 5.10 | | 7.55 | 3.50 | | 13.10 |
| I.PHOS | mmol/l | 74.40 | | 0.10 | 3.86 | | 0.17 |
| Mg2+ | mmol/l | 11.25 | | 8.70 | 2.95 | | 5.25 |
| Na/Crea | mM/mM | 10.80 | | 14.10 | 6.00 | | 9.10 |
| K/Crea | mM/mM | 5.30 | | 13.60 | 12.60 | | 17.90 |
| Cl/Crea | mM/mM | 1.40 | | 15.00 | 5.00 | | 12.60 |
| Ca/Crea | mM/mM | 0.32 | | 0.96 | 0.35 | | 3.41 |
| Pho/Crea | mM/mM | 4.60 | | 0.01 | 0.39 | | 0.04 |
| Mg/Crea | mM/mM | 0.70 | | 1.10 | 0.30 | | 1.40 |
| LDH/crea | IU/mM | 0.43 | | 1.79 | 0.91 | | 4.95 |
| NAG/crea | IU/mM | 1.45 | | 0.37 | 0.71 | | 0.68 |
| NTx/Crea | nME/mM | | 334 | 621 | | 543 | 547 |
| CTx/Crea | µg/µm. | | 734 | 1195 | | 1247 | 965 |
| Pyr/Crea | nM/mM | | 103 | 214 | | 141 | 204 |

| | | | | | | | |
|---|---|---|---|---|---|---|---|
| d-6, d-5 and d13 indicate day -6, day -5 and day 13 relative to the starting day of dosing d-6, d-5 and d13 indicate day -6, day -5 and day 13 relative to the starting day of dosing | | | | | | | |

**TABLE 31**

| Urinary Analysis - Females | | | | | | | |
|---|---|---|---|---|---|---|---|
| Control | | | | | | | |
| Animal no. | | W62551 | | | W62552 | | |
| Test | Units | -8 | -7 | 13 | -8 | -7 | 13 |
| VOLUME | ml | 21 | 21 | 43 | 18 | 53 | 53 |
| CREAT | µmol/l | 16420 | 16420 | 9560 | 14300 | 6700 | 5380 |
| NTx | nM BCE | - | 9248 | 7824 | - | 5053 | 4695 |
| CTx | µg/l | - | 19280 | 17916 | - | 12014 | 10557 |
| D-PYR | nmol/l | - | 2500 | 2748 | - | 1397 | 2159 |
| LDH | IU/L | 10.0 | | 15.0 | 9.0 | | 25.0 |
| NAG | IU/l | 19.2 | | 4.2 | 10.3 | | 3.5 |
| Na+ | mmol/l | 110 | | 44 | 140 | | 64 |
| K+ | mmol/l | 82 | | 122 | 124 | | 87 |
| Cl- | mmol/l | 24 | | 73 | 72 | | 56 |
| Ca2+ | mmol/l | 2.90 | | 16.10 | 11.90 | | 19.50 |
| I.PHOS | mmol/l | 88.2 | | 7.7 | 20.3 | | 3.5 |
| Mg2+ | mmol/l | 2.35 | | 7.20 | 9.00 | | 5.45 |
| Na/Crea | mM/mM | 6.70 | | 4.60 | 9.80 | | 11.90 |
| K/Crea | mM/mM | 5.00 | | 12.80 | 8.70 | | 16.20 |
| Cl/Crea | mM/mM | 1.50 | | 7.60 | 5.10 | | 10.50 |
| Ca/Crea | mM/mM | 0.18 | | 1.68 | 0.83 | | 3.63 |
| Pho/Crea | mM/mM | 5.37 | | 0.81 | 1.42 | | 0.64 |
| Mg/Crea | mM/mM | 0.10 | | 0.80 | 0.60 | | 1.00 |
| LDH/crea | IU/mM | 0.61 | | 1.57 | 0.63 | | 4.65 |
| NAG/crea | IU/mM | 1.17 | | 0.44 | 0.72 | | 0.65 |
| NTx/Crea | nME/mM | | 563 | 818 | | 754 | 873 |
| CTx/Crea | µg/µm. | | 1174 | 1874 | | 1793 | 1962 |
| Pyr/Crea | nM/mM | | 152 | 288 | | 209 | 401 |

| Salmon Calcitonin | | | | | | | |
|---|---|---|---|---|---|---|---|
| Animal no. | | W62553 | | | W62554 | | |
| Test | Units | -8 | -7 | 13 | -8 | -7 | 13 |
| VOLUME | ml | 11 | | 58 67 | 32 | -14 | 49 |
| CREAT | µmol/l | 10780 | 6920 | 4800 | 11260 | 13380 | 4200 |
| NTx | nM BCE | - | 4624 | 3465 | - | 7393 | 2812 |
| CTx | µg/l | - | 6983 | 5392 | - | 13411 | 5631 |
| D-PYR | nmol/l | - | 2762 | 1644 | - | 2016 | 1110 |
| LDH | IU/L | 14.0 | | 6.0 | 6.0 | | 36.0 |
| NAG | IU/l | 10.2 | | 2.8 | 1.2 | | 2.7 |
| Na+ | mmol/l | 98 | | 40 | 156 | | 32 |
| K+ | mmol/l | 104 | | 53 | 172 | | 57 |
| Cl- | mmol/l | 31 | | 63 | 156 | | 65 |
| Ca2+ | mmol/l | 3.00 | | 17.55 | 3.50 | | 12.70 |
| I.PHOS | mmol/l | 25.4 | | 5.1 | 10.8 | | 5.8 |
| Mg2+ | mmol/l | 3.35 | | 5.40 | 3.80 | | 4.85 |
| Na/Crea | mM/mM | 9.10 | | 8.30 | 13.90 | | 7.60 |
| K/Crea | mM/mM | 9.60 | | 11.10 | 15.20 | | 13.50 |
| Cl/Crea | mM/mM | 2.90 | | 13.20 | 13.80 | | 15.40 |
| Ca/Crea | mM/mM | 0.28 | | 3.66 | 0.31 | | 3.02 |
| Pho/Crea | mM/mM | 2.35 | | 1.05 | 0.96 | | 1.38 |
| Mg/Crea | mM/mM | 0.30 | | 1.10 | 0.30 | | 1.20 |
| LDH/crea | IU/mM | 1.30 | | 1.25 | 0.53 | | 8.57 |
| NAG/crea | IU/mM | 0.95 | | 0.58 | 0.11 | | 0.64 |
| NTx/Crea | nME/mM | | 668 | 722 | | 553 | 670 |
| CTx/Crea | µg/µm. | | 1009 | 1123 | | 1002 | 1341 |
| Pyr/Crea | nM/mM | | 399 | 343 | | 151 | 264 |

| PTS893 | | | | | | | |
|---|---|---|---|---|---|---|---|
| Animal no. | | W62555 | | | W62556 | | |
| Test | Units | -8 | -7 | 13 | -8 | -7 | 1x3 |
| VOLUME | ml | 14 | | 15 52 | 39 | 69 | 42 |
| CREAT | µmol/l | 19160 | 18240 | 5620 | 14060 | 7600 | 8060 |
| NTx | nM BCE | - | 10499 | 2514 | - | 4818 | 5679 |
| CTx | µg/l | - | 21919 | 3813 | - | 8877 | 11236 |
| D-PYR | nmol/l | - | 2963 | 1356 | - | 1377 | 2036 |
| LDH | IU/L | 11.0 | | 10.0 | 18.0 | | 9.0 |
| NAG | IU/l | 0.5 | | 1.2 | 5.9 | | 5.1 |
| Na+ | mmol/l | 145 | | 71 | 118 | | 146 |
| K+ | mmol/l | 302 | | 150 | 164 | | 70 |
| Cl- | mmol/l | 119 | | 101 | 53 | | 133 |
| Ca2+ | mmol/l | 11.50 | | 20.05 | 6.60 | | 12.35 |
| I.PHOS | mmol/l | 0.2 | | 0.1 | 7.6 | | 2.9 |
| Mg2+ | mmol/l | 7.35 | | 6.90 | 4.00 | | 5.90 |
| Na/Crea | mM/mM | 7.60 | | 12.60 | 8.40 | | 18.10 |
| K/Crea | mM/mM | 15.80 | | 26.80 | 11.70 | | 8.60 |
| Cl/Crea | mM/mM | 6.20 | | 18.00 | 3.70 | | 16.50 |
| Ca/Crea | mM/mM | 0.60 | | 3.57 | 0.47 | | 1.53 |
| Pho/Crea | mM/mM | 0.01 | | 0.02 | 0.54 | | 0.36 |
| Mg/Crea | mM/mM | 0.40 | | 1.20 | 0.30 | | 0.70 |
| LDH/crea | IU/mM | 0.57 | | 1.78 | 1.28 | | 1.12 |
| NAG/crea | IU/mM | 0.03 | | 0.21 | 0.42 | | 0.63 |
| NTx/Crea | nME/mM | | 576 | 447 | | 634 | 705 |
| CTx/Crea | µg/µm. | | 1202 | 679 | | 1168 | 1394 |
| Pyr/Crea | nM/mM | | 163 | 241 | | 181 | 253 |

| | | | | | | | |
|---|---|---|---|---|---|---|---|
| d-8, d-7 and d13 indicate day -8, day -7 and day 13 relative to the starting day of dosing d-8, d-7 and d13 indicate day -8, day -7 and day 13 relative to the starting day of dosing d-8, d-7 and d13 indicate day -8, day -7 and day 13 relative to the starting day of dosing | | | | | | | |

The salmon calcitonin group presented with moderate decreases in serum somatomedin (S.MED, see TABLES 32 and 42).

**TABLE 32**

| Hormones - Males | | | | | | | |
|---|---|---|---|---|---|---|---|
| Control | | | | | | | |
| Animal no. | | W62501 | | | W62502 | | |
| Test | Units | d-6 | d7 | d13 | d-6 | d7 | d13 |
| ACTH | pg/ml | 91 | 63 | 87 | 117 | 136 | 150 |
| CORTISOL | nmol/l | 2183 | 1415 | 1328 | 1378 | 1020 | 1348 |
| ALDOST | pg/ml | 316 | 433 | 484 | 501 | 644 | 622 |
| INSULIN | mU/l | 26.0 | 33.0 | 37.0 | 12.0 | 30.0 | 9.0 |
| GLUCAG | pg/ml | 791 | 486 | 704 | 577 | 353 | 585 |
| C-PEPTI | ng/ml | n/a | 5.20 | 5.50 | n/a | 3.60 | 1.60 |
| GASTRIN | pg/ml | n/a | 105 | 93 | n/a | 147 | 148 |
| T3 | nmol/l | 1.34 | 2.61 | 2.94 | 2.19 | 2.73 | 2.50 |
| T4 | mnol/l | 56 | 61 | 44 | 57 | 68 | 48 |
| TSH | mUI/l | 0.17 | 0.18 | 0.42 | 0.00 | 0.05 | 0.04 |
| IPH | pg/ml | 103 | 75 | 108 | 174 | 173 | 155 |
| CT | pg/ml | 5.9 | 4.6 | 4.8 | 16.4 | 15.0 | 13.1 |
| VD25-H | nmol/l | 49 | 47 | 54 | 76 | 71 | 58 |
| VD1-25dh | pmol/l | n/a | - | - | n/a | - | - |
| OSTEO | ng/ml | n/a | 26 | 34 | n/a | 41 | 40 |
| CTx | nmol/l | 10 | 15 | 20 | 17 | 19 | 20 |
| ICTP | ng/ml | 18 | 13 | 19 | 26 | 16 | 15 |
| PICP | ng/ml | n/a | 311 | 395 | n/a | 610 | 495 |
| G.H. | ng/ml | 13.8 | 7.0 | 16.2 | 15.2 | 3.6 | 17.2 |
| S.STA | pg/ml | n/a | - | - | n/a | - | - |
| S.MED | ng/ml | n/a | 888 | 1185 | n/a | 793 | 689 |
| PROLACT | ng/ml | 0.0 | 3.3 | 3.6 | 21.6 | 22.5 | 22.5 |
| TESTO | nmol/l | 10.5 | 8.4 | n. s. | 7.9 | 4.7 | n. s. |
| ESTR | nmol/l | n/a | n/a | n/a | n/a | n/a | n/a |
| PROG | pmol/l | n/a | n/a | n/a | n/a | n/a | n/a |
| d-6, d7 and d13 indicate day -6, day 7 and day 13 relative to the starting day of dosing | | | | | | | |

| Salmon Calcitonin | | | | | | | |
|---|---|---|---|---|---|---|---|
| Animal no. | | W62503 | | | W62504 | | |
| Test | Units | d-6 | d7 | d13 | d-6 | d7 | d13 |
| ACTH | pg/ml | 98 | 87 | 87 | 115 | 78 | 73 |
| CORTISOL | nmol/l | 2316 | 979 | 1611 | 1578 | 1523 | 1709 |
| ALDOST | pg/ml | 983 | 1058 | 819 | 465 | 987 | 977 |
| INSULIN | mU/l | 13.0 | 14.0 | 17.0 | 4.0 | 10.0 | 22.0 |
| GLUCAG | pg/ml | 905 | 247 | 428 | 869 | 218 | 503 |
| C-PEPTI | ng/ml | n/a | 1.70 | 1.80 | n/a | 1.20 | 2.30 |
| GASTRIN | pg/ml | n/a | 83 | 88 | n/a | 128 | 136 |
| T3 | nmol/l | 1.06 | 2.35 | 2.51 | 1.48 | 1.65 | 1.90 |
| T4 | nmol/l | 53 | 64 | 47 | 62 | 79 | 65 |
| TSH | mUI/l | 0.99 | 1.12 | 1.03 | 0.14 | 0.41 | 0.40 |
| IPH | pg/ml | 213 | 75 | 78 | 99 | 62 | 71 |
| CT | pg/ml | 6.7 | 4.0 | 2.4 | 5.1 | 2.5 | 4.9 |
| VD25-H | nmol/l | 63 | 50 | 49 | 62 | 44 | 45. |
| VD1-25dh | pmol/l | n/a | - | - | n/a | - | - |
| OSTEO | ng/ml | n/a | 33 | 41 | n/a | 27 | 30 |
| CTx | nmol/l | 12 | 26 | 38 | 18 | 22 | 24 |
| ICTP | ng/ml | 21 | 15 | 15 | 22 | 21 | 20 |
| PICP | ng/ml | n/a | 284 | 363 | n/a | 361 | 439 |
| G.H. | ng/ml | 11.5 | 1.7 | 16.2 | 14.6 | 13.6 | 15.7 |
| S.STA | pg/ml | n/a | - | - | n/a | - | - |
| S.MED | ng/ml | n/a | 268 | 332 | n/a | 307 | 384 |
| PROLACT | ng/ml | 8.1 | 8.6 | 4.6 | 0.0 | 0.0 | 6.6 |
| TESTO | nmol/l | 8.5 | 3.6 | n. s. | 9.5 | 7.3 | n. s. |
| ESTR | nmol/l | n/a | n/a | n/a | n/a | n/a | n/a |
| PROG | pmol/l | n/a | n/a | n/a | n/a | n/a | n/a |
| d-6, d7 and d13 indicate day -6, day 7 and day 13 relative to the starting day of dosing | | | | | | | |

| PTS893 | | | | | | | |
|---|---|---|---|---|---|---|---|
| Animal no. | | W62505 | | | W62506 | | |
| Test | Units | d-6 | d7 | d13 | d-6 | d7 | d13 |
| ACTH | pg/ml | 96 | 101 | 83 | 115 | 88 | 91 |
| CORTISOL | nmol/l | 1662 | 1156 | 1299 | 1506 | 1432 | 1212 |
| ALDOST | pg/ml | 265 | 380 | 592 | 141 | 471 | 651 |
| INSULIN | mU/l | 16.0 | 22.0 | 14.0 | 12.0 | 38.0 | 10.0 |
| GLUCAG | pg/ml | 858 | 656 | 786 | 694 | 497 | 739 |
| C-PEPTI | ng/ml | n/a | 2.90 | 2.10 | n/a | 4.40 | 2.40 |
| GASTRIN | pg/ml | n/a | 84 | 78 | n/a | 98 | 94 |
| T3 | nmol/l | 2.48 | 3.47 | 3.55 | 1.38 | 2.76 | 2.43 |
| T4 | nmol/l | 84 | 90 | 68 | 59 | 80 | 56 |
| TSH | mUI/l | 0.22 | 0.40 | 0.15 | 0.00 | 0.07 | 0.03 |
| IPH | pg/ml | 123 | 96 | 78 | 71 | 62 | 55 |
| CT | pg/ml | 6.1 | 4.0 | 4.6 | 10.4 | 7.8 | 7.6 |
| VD25-H | nmol/l | 77 | 62 | 50 | 88 | 62 | 50 |
| VD1-25dh | pmol/l | n/a | - | - | n/a | - | - |
| OSTEO | ng/ml | n/a | 43 | 55 | n/a | 32 | 42 |
| CTx | nmol/l | 19 | 20 | 31 | 12 | 12 | 16 |
| ICTP | ng/ml | 28 | 23 | 22 | 18 | 16 | 18 |
| PICP | ng/ml | n/a | 420 | 500 | n/a | 774 | 706 |
| G.H. | ng/ml | 13.4 | 15.8 | 12.1 | 8.5 | 11.6 | 14.0 |
| S.STA | pg/ml | n/a | - | - | n/a | - | - |
| S.MED | ng/ml | n/a | 749 | 914 | n/a | 828 | 867 |
| PROLACT | ng/ml | 7.1 | 15.7 | 7.5 | 7.5 | 5.5 | 2.2 |
| TESTO | nmol/l | 11.8 | 10.5 | n. s. | 5.3 | 3.7 | n. s. |
| ESTR | nmol/l | n/a | n/a | n/a | n/a | n/a | n/a |
| PROG | pmol/l | n/a | n/a | n/a | n/a | n/a | n/a |
| d-6, d7 and d13 indicate day -6, day 7 and day 13 relative to the starting day of dosing | | | | | | | |

**TABLE 33**

| Hormones - Females | | | | | | | |
|---|---|---|---|---|---|---|---|
| Control | | | | | | | |
| Animal no. | | W62551 | | | W62552 | | |
| Test | Units | d-8 | d7 | d13 | d-8 | d7 | d13 |
| ACTH | pg/ml | 146 | 276 | 121 | 58 | 60 | 101 |
| CORTISOL | nmol/l | 1983 | 1546 | 827 | 1894 | 837 | 818 |
| ALDOST | pg/ml | 244 | 953 | 312 | 149 | 90 | 199 |
| INSULIN | mU/l | 8.0 | 12.0 | 7.0 | 2.0 | 29.0 | 21.0 |
| GLUCAG | pg/ml | 729 | 779 | 583 | 818 | 507 | 514 |
| C-PEPTI | ng/ml | n/a | 2.40 | 1.40 | n/a | 3.30 | 2.30 |
| GASTRIN | pg/ml | n/a | 84 | 102 | n/a | 90 | 92 |
| T3 | nmol/l | 2.22 | 2.95 | 3.40 | 2.04 | 3.09 | 3.23 |
| T4 | nmol/l | 78 | 67 | 59 | 51 | 50 | 49 |
| TSH | mUI/l | 0.14 | 0.27 | 0.49 | 0.15 | 0.54 | 0.50 |
| IPH | pg/ml | 155 | 149 | 129 | 145 | 129 | 112 |
| CT | pg/ml | 4.70 | 3.90 | 4.10 | 11.50 | 11.60 | 11.20 |
| VD25-H | nmol/l | 64 | 59 | 51 | 80 | 78 | 70 |
| VD1-25dh | pmol/l | n/a | - | - | n/a | - | - |
| OSTEO | ng/ml | n/a | 37 | 39 | n/a | 34 | 39 |
| CTx | nmol/l | 11 | 26 | 28 | 12 | 16 | 20 |
| ICTP | ng/ml | 21 | 23 | 22 | 19 | 16 | 15 |
| PICP | ng/ml | n/a | 864 | 503 | n/a | 339 | 298 |
| G.H. | ng/ml | 8.5 | 13.4 | 1.7 | 7.0 | 12.0 | 4.5 |
| S.STA | pg/ml | n/a | - | - | n/a | - | - |
| S.MED | ng/ml | n/a | 696 | 839 | n/a | 1173 | 1527 |
| PROLACT | ng/ml | 4.30 | 8.30 | 5.90 | 2.90 | 0.00 | 0.00 |
| TESTO | nmol/l | | | | | | |
| ESTR | nmol/l | 58 | 64 | 61 | 48 | 45 | 60 |
| PROG | pmol/l | 3.40 | 3.50 | 1.70 | 2.70 | 1.10 | 1.40 |
| d-8, d7 and d13 indicate day -8, day 7 and day 13 relative to the starting day of dosing | | | | | | | |

| Salmon Calcitonin | | | | | | | |
|---|---|---|---|---|---|---|---|
| Animal no. | | W62553 | | | W62554 | | |
| Test | Units | d-8 | d7 | d13 | d-8 | d7 | d13 |
| ACTH | pg/ml | 72 | 129 | 97 | 157 | 233 | 141 |
| CORTISOL | nmol/l | 1536 | 1220 | 1202 | 1222 | 1705 | 1128 |
| ALDOST | pg/ml | 185 | 948 | 523 | 155 | 1073 | 457 |
| INSULIN | mU/l | 12.0 | 8.0 | 9.0 | 20.0 | 18.0 | 24.0 |
| GLUCAG | pg/ml | 585 | 295 | 258 | 619 | 594 | 303 |
| C-PEPTI | ng/ml | n/a | 1.60 | 1.00 | n/a | 1.50 | 2.20 |
| GASTRIN | pg/ml | n/a | 83 | 84 | n/a | 91 | 84 |
| T3 | nmol/l | 1.17 | 1.68 | 1.51 | 1.43 | 1.51 | 2.00 |
| T4 | nmol/l | 58 | 76 | 60 | 61 | 87 | 60 |
| TSH | mUI/l | 0.81 | 1.31 | 1.16 | 0.08 | 0.34 | 0.41 |
| IPH | pg/ml | 59 | 47 | 58 | 145 | 82 | 53 |
| CT | pg/ml | 3.10 | 6.40 | 4.90 | 7.00 | 3.60 | 2.30 |
| VD25-H | nmol/l | 61 | 43 | 40 | 72 | 56 | 60 |
| VD1-25dh | pmol/l | n/a | - | - | n/a | - | - |
| OSTEO | ng/ml | n/a | 21 | 25 | n/a | 35 | 35 |
| CTx | nmol/l | 12 | 21 | 25 | 17 | 34 | 28 |
| ICTP | ng/ml | 28 | 28 | 24 | 29 | 30 | 24 |
| PICP | ng/ml | n/a | 115 | 142 | n/a | 240 | 287 |
| G.H. | ng/ml | 6.3 | 15.2 | 8.6 | 5.1 | 17.9 | 13.1 |
| S.STA | pg/ml | n/a | - | - | n/a | - | - |
| S.MED | ng/ml | n/a | 374 | 297 | n/a | 204 | 488 |
| PROLACT | ng/ml | 0.00 | 2.30 | 4.30 | 19.30 | 20.20 | 24.40 |
| TESTO | nmol/l | | | | | | |
| ESTR | nmol/l | 47 | 63 | 59 | 141 | 82 | 170 |
| PROG | pmol/l | 1.80 | 1.90 | 1.50 | 2.60 | 4.00 | 1.60 |
| d-8, d7 and d13 indicate day -8, day 7 and day 13 relative to the starting day of dosing | | | | | | | |

| PTS893 | | | | | | | |
|---|---|---|---|---|---|---|---|
| Animal no. | | W62555 | | | W62556 | | |
| Test | Units | d-8 | d7 | d13 | d-8 | d7 | d13 |
| ACTH | pg/ml | 109 | 104 | 110 | 95 | 132 | 126 |
| CORTISOL | nmol/l | 1482 | 1331 | 917 | 1532 | 1253 | 1375 |
| ALDOST | pg/ml | 314 | 217 | 330 | 210 | 228 | 226 |
| INSULIN | mU/l | 1.0 | 22.0 | 19.0 | 15.0 | 30.0 | 22.0 |
| GLUCAG | pg/ml | 711 | 591 | 657 | 696 | 437 | 380 |
| C-PEPTI | ng/ml | n/a | 3.00 | 2.40 | n/a | 3.80 | 3.50 |
| GASTRIN | pg/ml | n/a | 83 | 82 | n/a | 96 | 91 |
| T3 | nmol/l | 2.08 | 2.74 | 2.63 | 1.98 | 2.69 | 2.05 |
| T4 | nmol/l | 72 | 56 | 55 | 59 | 61 | 45 |
| TSH | mUI/l | 0.34 | 0.14 | 0.25 | 0.88 | 0.89 | 0.69 |
| IPH | pg/ml | 95 | 45 | 64 | 111 | 67 | 58 |
| CT | pg/ml | 2.50 | 1.90 | 2.70 | 1.80 | 2.90 | 2.80 |
| VD25-H | nmol/l | 72 | 53 | 47 | 55 | 44 | 43 |
| VD1-25dh | pmol/l | n/a | - | - | n/a | - | - |
| OSTEO | ng/ml | n/a | 38 | 43 | n/a | 32 | 36 |
| CTx | nmol/l | 13 | 11 | 15 | 17 | 14 | 14 |
| ICTP | ng/ml | 22 | 16 | 16 | 20 | 15 | 15 |
| PICP | ng/ml | n/a | 612 | 436 | n/a | 478 | 393 |
| G.H. | ng/ml | 3.5 | 1.5 | 0.0 | 1.1 | 8.2 | 11.8 |
| S.STA | pg/ml | n/a | - | - | n/a | - | - |
| S.MED | ng/ml | n/a | 533 | 502 | n/a | 432 | 589 |
| PROLACT | ng/ml | 0.00 | 0.20 | 3.20 | 9.90 | 5.70 | 3.60 |
| TESTO | nmol/l | | | | | | |
| ESTR | nmol/l | 67 | 68 | 60 | 59 | 66 | 57 |
| PROG | pmol/l | 2.80 | 1.70 | 1.50 | 2.40 | 2.20 | 2.40 |
| d-8, d7 and d13 indicate day -8, day 7 and day 13 relative to the starting day of dosing | | | | | | | |

*Tissue sampling.* Animals were killed by deep anaesthesia induced by intravenous injection of Pentothal®, followed by exsanguinations. All relevant tissues were sampled for histopathology and gene expression profiling. The following tissue samples were processed for analysis: liver, kidney, pituitary, muscle, bone, duodenum, spleen and trachea. Samples for histopathology were fixed in phosphate-buffered 10% formalin. Bone demineralization was performed with 10% formic acid. Tissue samples were embedded in Paraplast® and sectioned at 4 microns, for staining with haematoxylin and eosin. Samples for gene expression profiling were quickly frozen in liquid nitrogen immediately after excision, stored on dry ice and subsequently in a deep-freezer at approximately -80°C until. further use. All selected tissues for gene expression profiling were examined histopathologically.

*Histopathology.* Histopathological examination of the tissues selected for gene profiling analysis exhibited a normal spectrum of incidental lesions which were in terms of severity and distribution of lesions not different to the controls in all groups of treatment.

A slightly higher incidence of inflammatory and regenerative changes in the kidneys of females administered salmon calcitonin was observed. These changes were not considered to be relevant, since no records of kidney toxicity exist after 40 years of calcitonin therapeutic use.

Bone sections were stained for osteonectin, osteopontin and osteocalcin and were evaluated histopathologically. Histomorphometry of the bone tissue was performed regarding parameters for bone resorption and synthesis (osteoid formation).

The osteonectin, osteopontin, and osteocalcin staining of the tibia showed no difference between the groups one (control) and two (salmon calcitonin). Osteonectin exhibited a major enlargement and deterioration of the epiphysial growth plate of animal no 2553 due to a severe non-treatment related pathological status (severe, subacute epiphysiolysis).

Histomorphometry of bone tissue was performed to determine parameters related to bone resorption and bone synthesis (osteoid formation).

The results (see, TABLES 34 and 35) showed that salmon calcitonin increased trabecular volume and thickness in about a 17% in tibia, but not in vertebra. PTS893 reduced the cortical thickness (18%) and increased the cortical porosity (54%) in tibia (T), but not in vertebra (V). In contrast, PTS893 induced an increase in osteoid volume (37%T, 213%V) and surface (49%T, 37%V), as well as an increase in the osteoblast surface (40%T, 24%V), in both tibia and vertebra, respectively.

**TABLE 34**

| Histomorphometry Tibia (Average Males and Females) | | | | | | | | | | |
|---|---|---|---|---|---|---|---|---|---|---|
| | BT/TV | Tb Th | Tb N | 10 Sp | Ct Por | Ct Th | OS/BS | OV/BV | ES/BS | Obs/BS |
| | % | µm | mm⁻¹ | µm | % | µm | % | % | % | % |
| Control | 20.70 | 106.32 | 1.95 | 407.20 | 2.53 | 1583.13 | 40.00 | 8.76 | 5.73 | 17.53 |
| | 17.72 | 97.99 | 1.81 | 454.90 | 2.59 | 976.66 | 33.37 | 8.51 | 4.70 | 12.77 |
| | 28.74 | 109.18 | 2.63 | 270.69 | 1.21 | 1036.24 | 29.45 | 5.79 | 10.19 | 11.70 |
| | 20.15 | 103.59 | 1.94 | 410.62 | 1.19 | 1031.89 | 29.19 | 5.29 | 5.71 | 15.80 |
| mean | 21.83 | 104.27 | 2.08 | 385.85 | 1.88 | 1156.98 | 33.00 | 7.09 | 6.58 | 14.45 |
| SD | 4.79 | 4.77 | 0.37 | 79.79 | 0.78 | 285.39 | 5.04 | 1.80 | 2.45 | 2.69 |
| sCT | 32.28 | 140.64 | 2.30 | 295.01 | 2.10 | 895.98 | 42.71 | 11.72 | 5.02 | 18.32 |
| | 25.00 | 122.19 | 2.05 | 366.51 | 1.98 | 1022.55 | 31.58 | 5.86 | 2.31 | 6.37 |
| | 29.96 | 129.05 | 2.32 | 301.75 | 1.61 | 939.32 | 35.21 | 5.03 | 6.89 | 18.58 |
| | 16.08 | 115.65 | 1.39 | 603.45 | 2.40 | 1178.70 | 30.37 | 4.01 | 5.61 | 19.36 |
| mean | 25.83 | 126.88 | 2.01 | 391.68 | 2.02 | 1009.14 | 34.97 | 6.65 | 4.95 | 15.66 |
| SD | 7.17 | 10.68 | 0.43 | 144.81 | 0.33 | 124.65 | 5.56 | 3.46 | 1.93 | 6.21 |
| PTS893 | 19.69 | 129.22 | 1.52 | 526.99 | 2.76 | 1022.62 | 54.84 | 11.24 | 4.62 | 16.16 |
| | 16.65 | 93.20 | 1.79 | 466.69 | 2.94 | 893.43 | 43.57 | 9.61 | 4.76 | 21.25 |
| | 25.74 | 120.52 | 2.13 | 347.63 | 2.94 | 950.33 | 43.63 | 8.14 | 4.21 | 18.46 |
| | 24.78 | 126.07 | 1.97 | 382.61 | 2.95 | 939.53 | 54.97 | 9.95 | 2.85 | 25.25 |
| mean | 21.72 | 117.25 | 1.85 | 430.98 | 2.90 | 951.48 | 49.25 | 9.74 | 4.11 | 20.28 |
| SD | 4.30 | 16.43 | 0.26 | 81.20 | 0.09 | 53.46 | 6.53 | 1.28 | 0.87 | 3.91 |

| | | | | | | | | | | |
|---|---|---|---|---|---|---|---|---|---|---|
| sCT: salmon Calcitonin; SD: Standard deviation BV/TV trabecular bone volume; Tb. Th. Trabecular thickness; Tb. N. Trabecular number; Tb. Sp. Trabecular Separation; Ct. Por. Cortical porosity; Ct, Th. Cortical thickness; OS/BS osteoid surface; OV/BV osteoid volume; ES/BS eroded surface; Obs/BS osteoblast surface. | | | | | | | | | | |

**TABLE 35**

| Histomorphometry Vertebra (Average Males and Females) | | | | | | | | | | |
|---|---|---|---|---|---|---|---|---|---|---|
| | BT/TV | Tb Th | Tb N | Tb Sp | Ct Por | Ct Th | OS/BS | OV/BV | ES/BS | Obs/BS |
| | % | µm | mm⁻¹ | µm | % | µm | % | % | % | % |
| Control | 2-1.67 | 179.80 | 1.21 | 649.79 | 0.88 | 887.91 | 23.61 | 1.22 | 8.94 | 16.14 |
| | 15.85 | 144.89 | 1.09 | 769.35 | 0.26 | 639.93 | 20.77 | 2.02 | 8.81 | 5.96 |
| | 19.54 | 122.91 | 1.59 | 506.23 | 0.87 | 416.48 | 17.91 | 1.58 | 5.85 | 4.07 |
| | 21.95 | 131.30 | 1.67 | 466.91 | 0.85 | 604.45 | 11.58 | 0.97 | 1.82 | 4.79 |
| mean | 19.75 | 144.72 | 1.39 | 598.07 | 0.71 | 637.20 | 18.47 | 1.45 | 6.36 | 7.74 |
| SD | 2.82 | 25.07 | 0.28 | 138.62 | 0.30 | 193.78 | 5.15 | 0.45 | 3.34 | 5.65 |
| sCT | 17.32 | 113.29 | 1.53 | 540.84 | 1.70 | 705.10 | 3.95 | 0.46 | 11.60 | 3.21 |
| | 19.33 | 144.31 | 1.34 | 602.15 | 1.18 | 810.09 | 5.82 | 0.86 | 2.55 | 3.97 |
| | 20.11 | 118.49 | 1.70 | 470.71 | 1.18 | 576.42 | 11.48 | 1.43 | 4.93 | 6.81 |
| | 19.46 | 123.71 | 1.57 | 511.96 | 0.12 | 907.16 | 4.91 | 0.32 | 3.47 | 1.23 |
| mean | 19.06 | 124.95 | 1.53 | 531.42 | 1.05 | 749.69 | 6.54 | 0.77 | 5.64 | 3.80 |
| SD | 1.21 | 13.59 | 0.15 | 55.24 | 0.66 | 141.96 | 3.38 | 0.50 | 4.09 | 2.31 |
| PTS893 | 15.15 | 105.46 | 1.44 | 590.67 | 1.49 | 707.43 | 18.84 | 3.24 | 9.31 | 10.36 |
| | 20.23 | 118.79 | 1.70 | 468.39 | 1.45 | 629.35 | 41.28 | 8.42 | 2.30 | 9.07 |
| | 23.56 | 134.66 | 1.75 | 436.79 | 0.41 | 740.87 | 23.65 | 3.49 | 2.55 | 10.47 |
| | 24.86 | 134.82 | 1.84 | 407.56 | 0.92 | 624.35 | 17.66 | 2.66 | 3.96 | 8.33 |
| mean | 20.95 | 123.43 | 1.68 | 475.85 | 1.07 | 675.50 | 25.36 | 4.45 | 4.53 | 9.56 |
| SD | 4.33 | 14.15 | 0.17 | 80.47 | 0.51 | 57.85 | 10.93 | 2.67 | 3.27 | 1.04 |

| | | | | | | | | | | |
|---|---|---|---|---|---|---|---|---|---|---|
| sCT: salmon Calcitonin; SD: Standard deviation BV/TV trabecular bone volume; Tb. Th. Trabecular thickness; Tb. N. Trabecular number; Tb. Sp. Trabecular Separation; Ct. Por. Cortical porosity; Ct, Th. Cortical thickness; OS/BS osteoid surface; OV/BV osteoid volume; ES/BS eroded surface; Obs/BS osteoblast surface. | | | | | | | | | | |

Histomorphometry showed inconsistent results between tibial and vertebral bone, except for an increase in osteoid synthesis induced by PTS893. This effect is well documented for parathyroid hormone, when administered in a discontinuous way.

*RNA extraction and purification.* A set of tissues was selected for gene expression profiling. These set included samples from kidney, bone, muscle, duodenum, pituitary and liver. Briefly, total RNA was obtained by acid guanidinium thiocyanate-phenol-chloroform extraction (Trizol®, Invitrogen Life Technologies, Carlsbad, Calif. USA) from each frozen tissue section and the total RNA was then purified on an affinity resin (RNeasy®, Qiagen) according to the manufacturer's instructions. Total RNA was quantified by the absorbance at λ = 260 nm (A260nm), and the purity was estimated by the ratio A₂₆₀ₙₘ/A₂₈₀ₙₘ. Integrity of the RNA molecules was confirmed by non-denaturing agarose gel electrophoresis RNA was stored at approximately -80°C until analysis. One part of each individual RNA sample was kept for the analysis of critical genes by means of Real-time PCR.

*Hybridization assay.* Transcript profiling by means of GeneChip® expression probe arrays was done as recommended by the manufacturer of the GeneChip® system *(GeneChip Expression Analysis Technical Manual,* Affymetrix Inc., Santa Clara, Calif. USA). HG-U95Av2 GeneChip® expression probe arrays (Affymetrix, Santa Clara Calif. USA) were used. Double stranded cDNA was synthesized with a starting amount of approximately 5 µg full-length total RNA using the Superscript Choice System (Invitrogen Life Technologies) in the presence of a T7-(dT) 24 DNA oligonucleotide primer. Following synthesis, the cDNA was purified by phenol/chlorofom/isoamylalcohol extraction and ethanol precipitation. The purified cDNA was then transcribed in vitro using the BioArray® High Yield RNA Transcript Labeling Kit (ENZO) in the presence of biotinylated ribonucleotides form biotin labeled cRNA. The labeled cRNA was then purified on an affinity resin (Rneasy®, Qiagen), quantified and fragmented. An amount of approximately 10 µg labeled cRNA was hybridized for approximately 16 hours at 45°C to an expression probe array. The array was then washed and stained twice with streptavidin-phycoerythrin (Molecular Probes) using the GeneChip Fluidics. Workstation 400 (Affymetrix). The array was then scanned twice using a confocal laser scanner (GeneArray® Scanner, Agilent) resulting in one scanned image.

This resulting ".data-file" was processed using the Micro Array Analysis Suite version 4 (MAS4) program (Affymetrix) into a ".cel-file". The ".cel file" was captured and loaded into the Affymetrix GeneChip Laboratory Information Management System (LIMS). The LIMS database is connected to a UNIX Sun Solaris server through a network filing system that allows for the average intensities for all probes cells (CEL file) to be downloaded into an Oracle database. Raw data was converted to expression levels using a "target intensity" of 150. The numerical values displayed are weighted averages of the signal intensities of the probe-pairs comprised in a probe-set for a given transcript sequence (AvgDiff value). The data were checked for quality and loaded into the GeneSpring® software versions 4.Z.4 and 5 (Silicon Genetics, Calif. USA) for analysis.

- *Data analysis*. Data analysis was performed with the Silicon Genetics software package GeneSpring version 4.2.1 and 5. Average difference values below 20 were set to 20. Various filtering and clustering tools in these programs were used to explore the data sets and identify transcript level changes that informer or altered cellular and tissue functions and that can be used to establish working hypotheses on the modes of action of the compound.

The threshold range for considering as up or down regulation was determined within the context of the biological interpretation of the study.

The information content of these data sets is a conjunction of numerical changes and biological information. The decision to consider a specific gene relevant was based on a conjunction of numerical changes identified by comparative and statistical algorithms and the relationship to other modulated genes that point to a common biological theme. The weight of that relationship was assessed by the analyst through a review of the relevant scientific literature.

Increase and decrease reported here refer to transcript abundance, unless specifically stated.

*Gene expression profiling.* Multiorgan comparative gene profiling analysis was performed in the group administered salmon calcitonin at 50 µg/animal/day. The organs chosen for analysis were liver, kidney, pituitary, skeletal muscle, bone, duodenum, spleen and trachea.

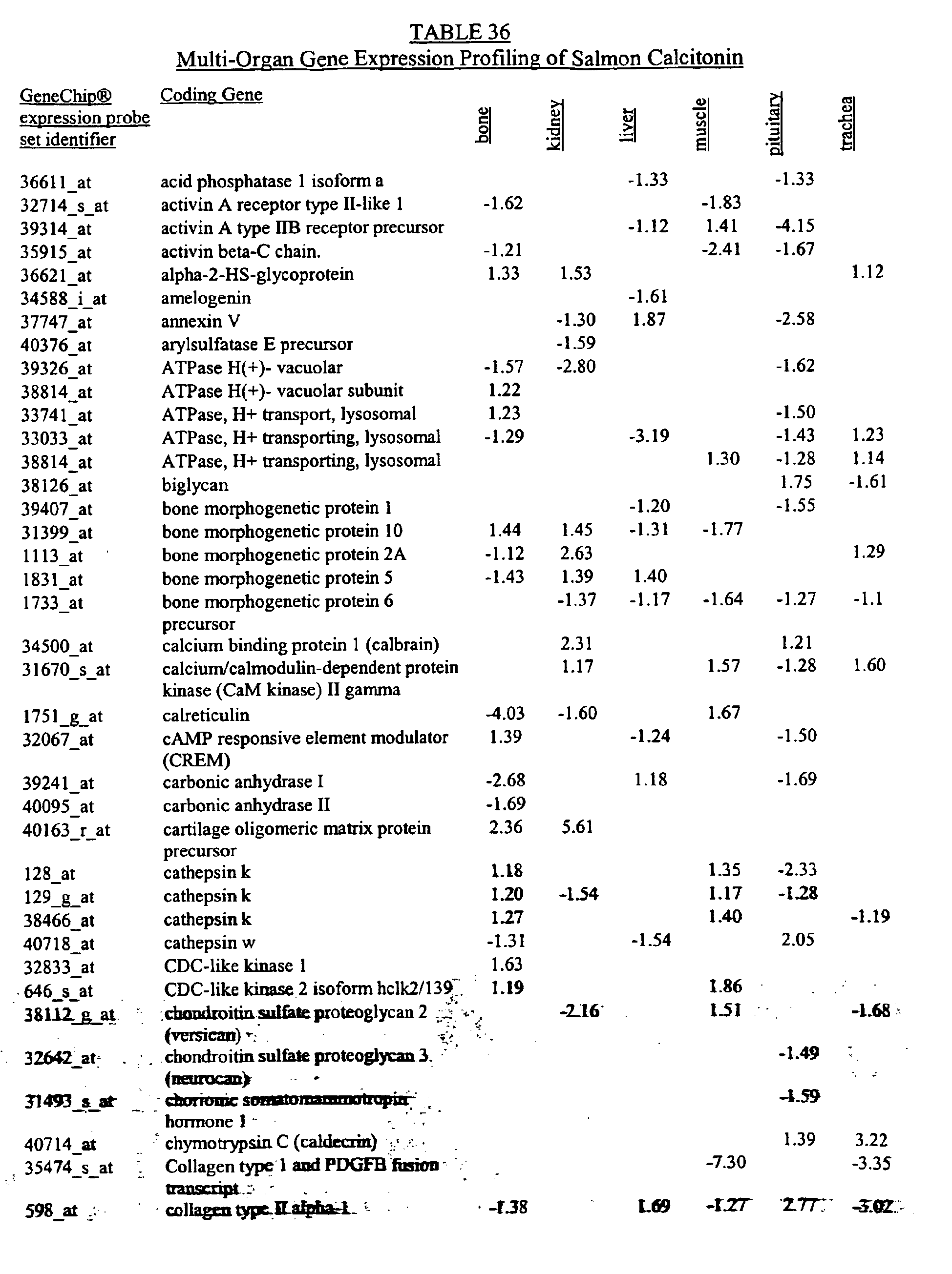

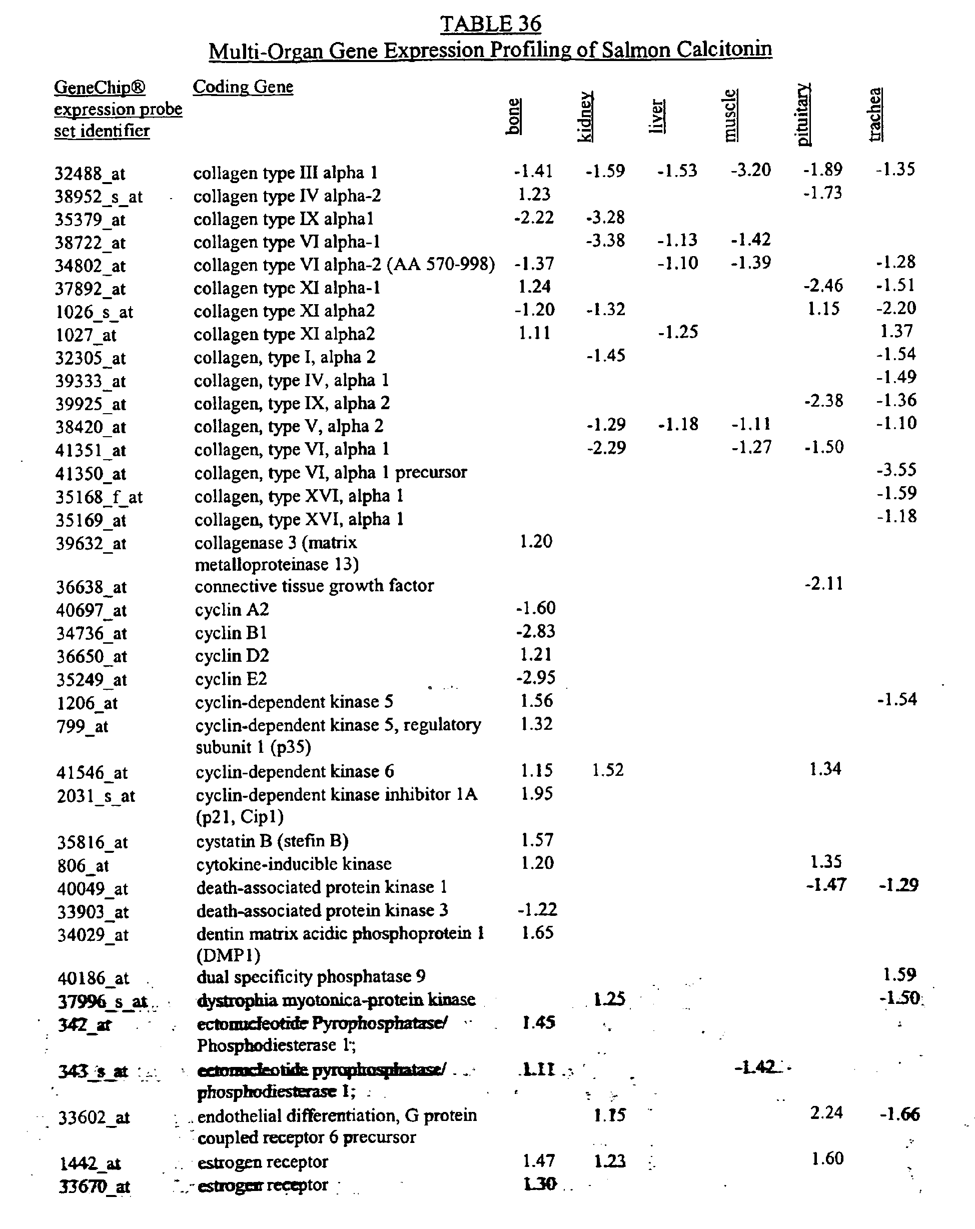

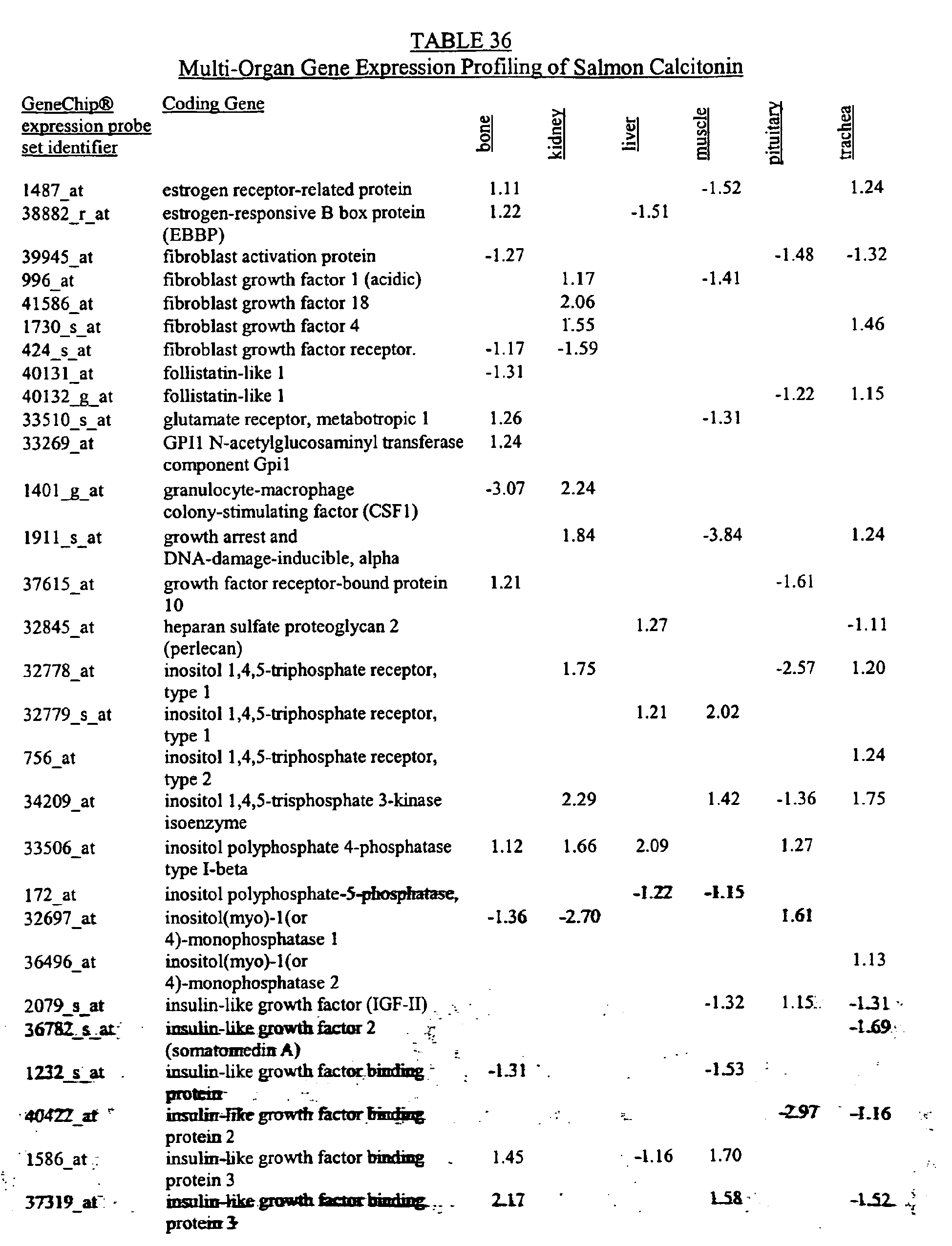

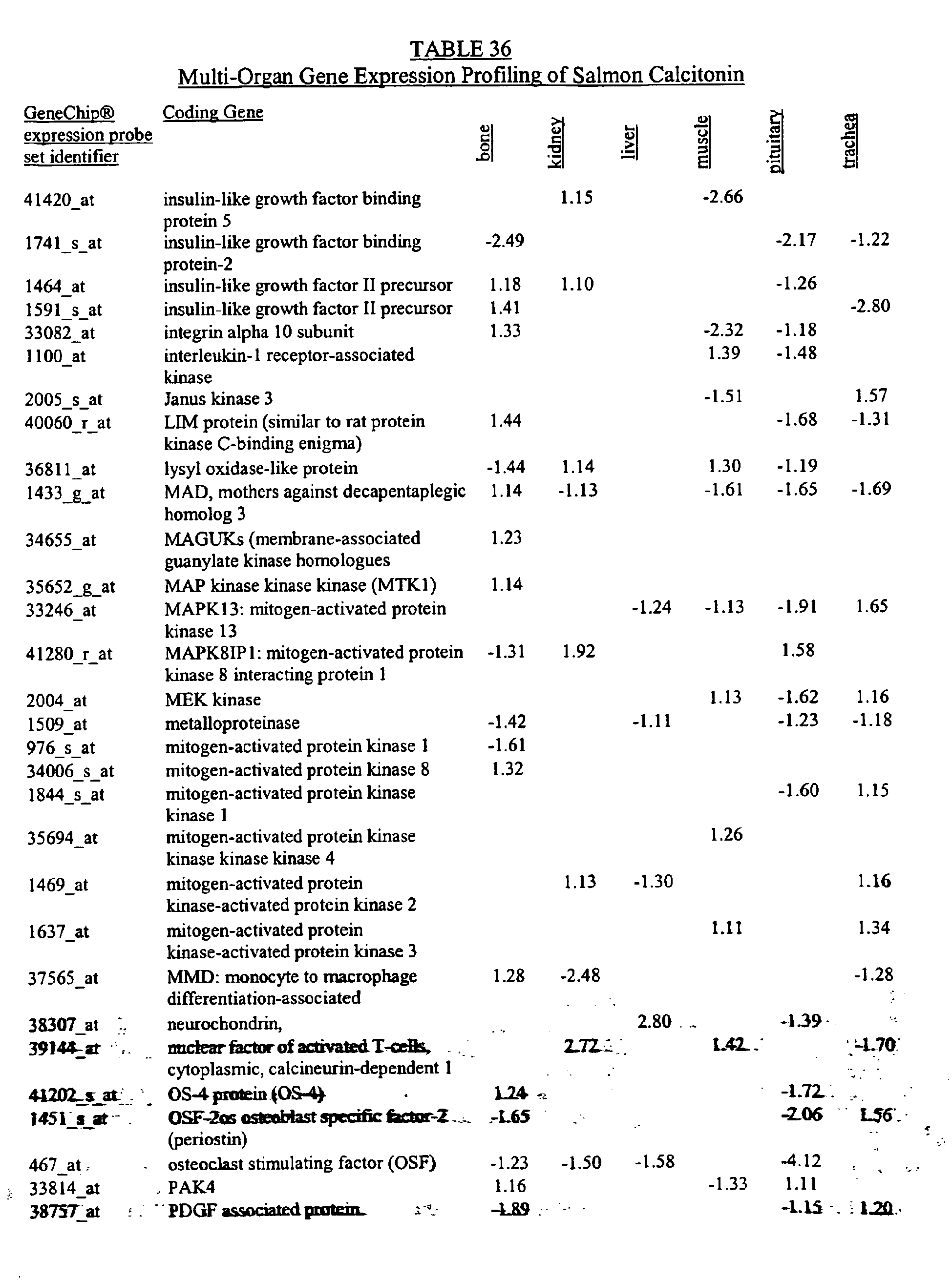

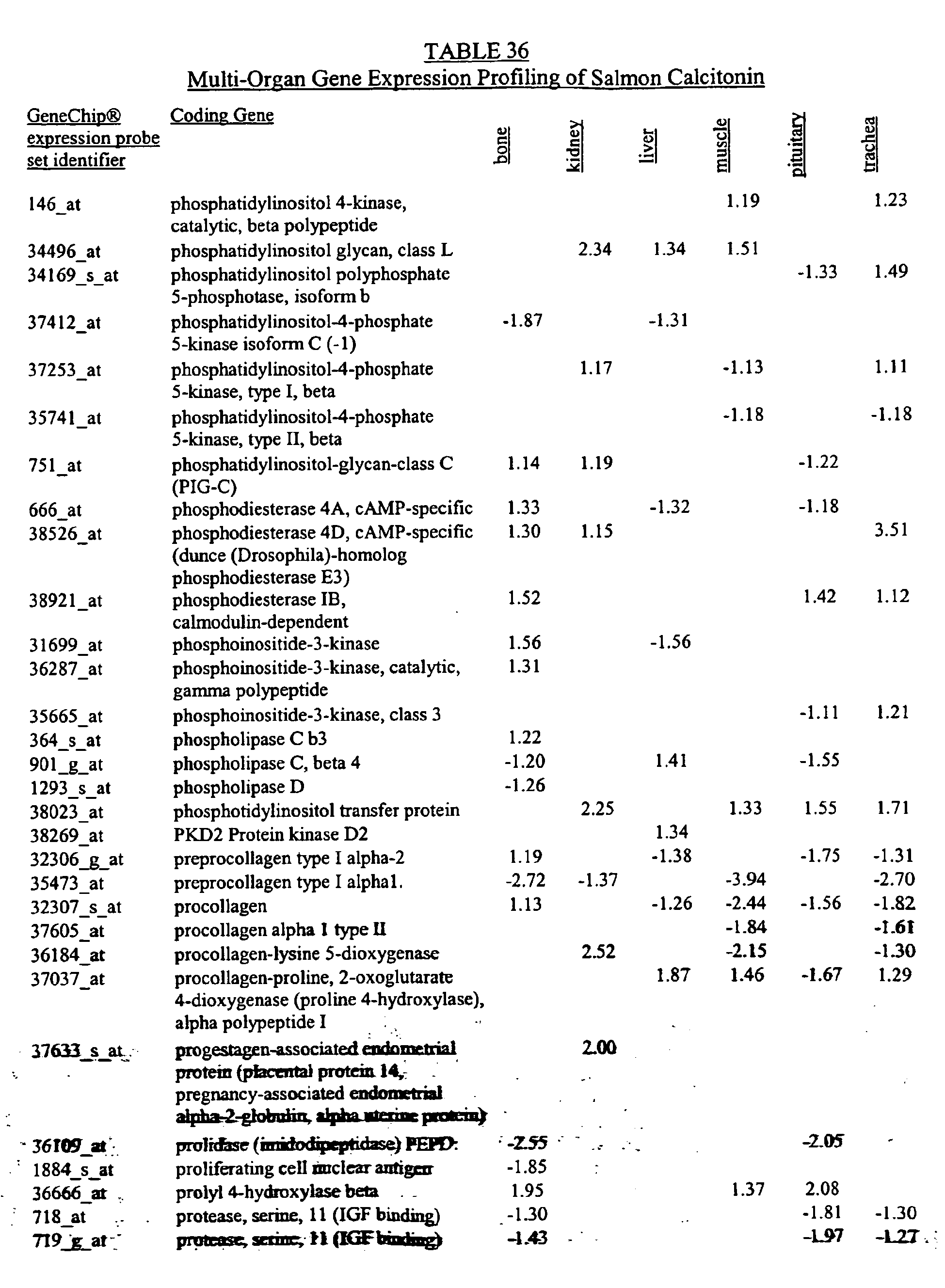

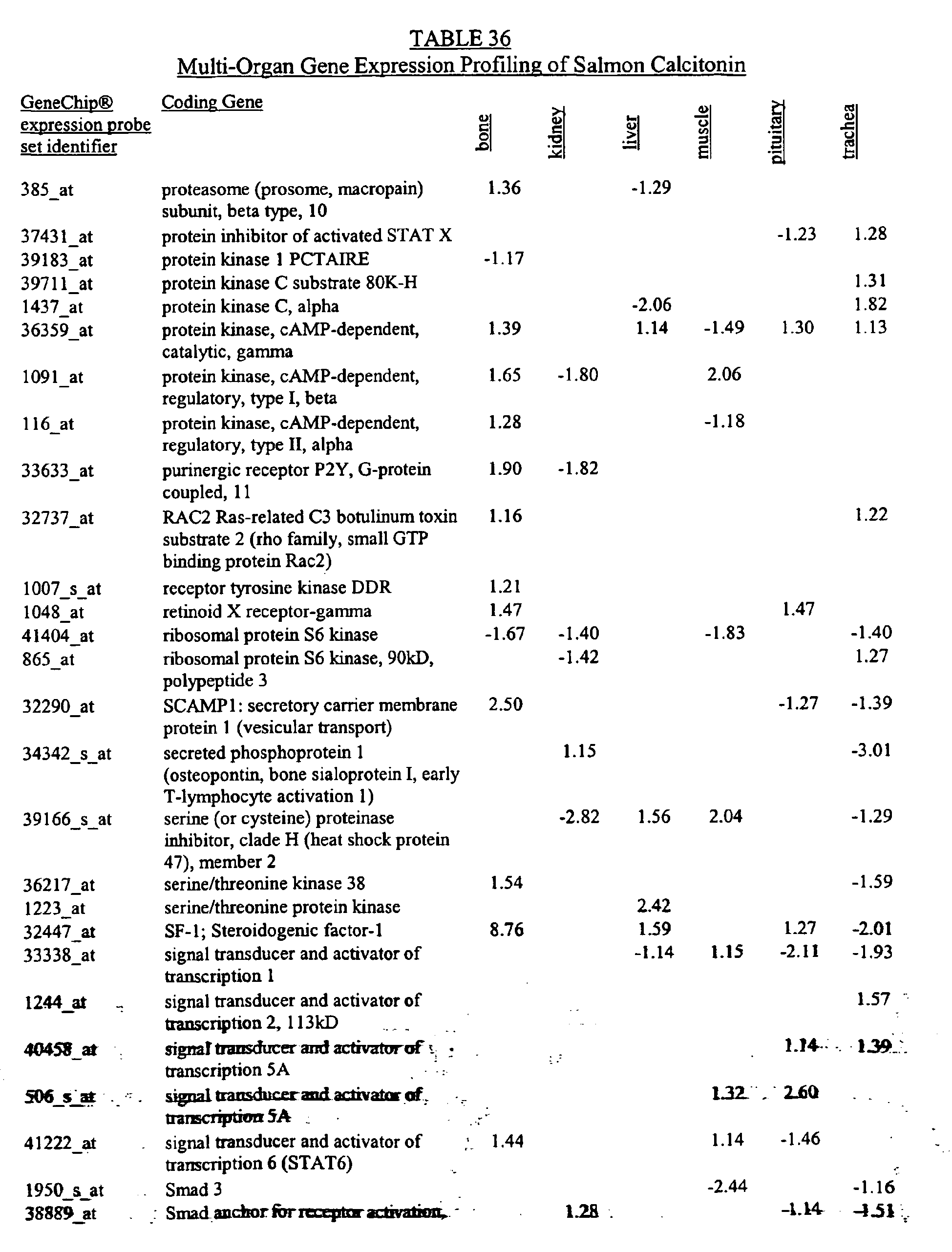

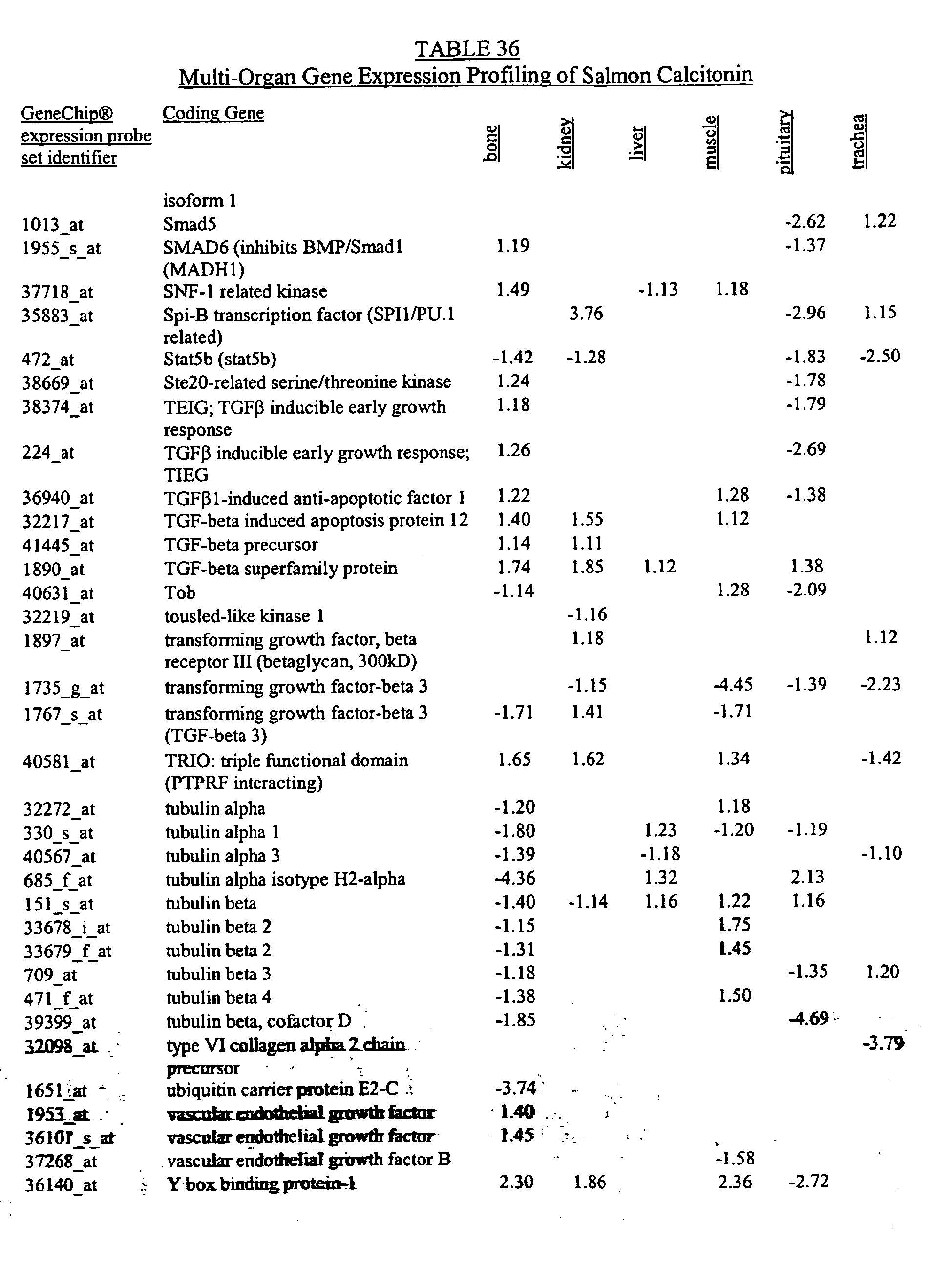

In addition, the effect of PTS893 was assessed in bone.

**TABLE 37**

| Gene-Profiling Analysis of Salmon Calcitonin and PTS893 in Bone | | | |
|---|---|---|---|
| GeneChip® | Coding Gene | Fold Increase | Fold Increase |
| Expression Probe | | Salmon | PTS893 |
| Set Identifier | | Calcitonin | |
| 38909 at | 25-hydroxyvitamin D3 1-alpha-hydroxylase | | -1.14 |
| 32714_s_at | activin A receptor type II-like 1 | -1.62 | |
| 35915 at | activin beta-C chain. | -1.21 | |
| 39279_at | activin type II receptor | | 1.24 |
| 39383_at | adenylate cyclase 6, isoform a | | -1.22 |
| 38965_at | aggrecan 1 | | 2.03 |
| 39206_s_at | aggrecan 1 | | 1.41 |
| 36621_at | alpha-2-HS-glycoprotein | 1.33 | |
| 34589_f_at | Amelogenin | 1.10 | -3.10 |
| 39326_at | ATPase H(+) vacuolar | -1.57 | -1.19 |
| 38814_at | ATPase H(+) vacuolar | 1.22 | |
| 33741_at | ATPase, H+ transport, lysosomal | 1.23 | |
| 33033_at | ATPase, H+ transporting, lysosomal | -1.29 | -1.17 |
| 40328_at | bHLH transcription factor | | 2.57 |
| 39407_at | bone morphogenetic protein 1 | | 1.16 |
| 31399_at | bone morphogenetic protein 10 | 1.44 | 1.20 |
| 1113_at | bone morphogenetic protein 2A | -1.12 | -1.13 |
| 40367_at | bone morphogenetic protein 2A | | -1.18 |
| 1114_at | bone morphogenetic protein 2B or BMP4 | | -1.70 |
| 1831_at | bone morphogenetic protein 5 | -1.43 | -1.60 |
| 1733_at | bone morphogenetic protein 6 precursor | | 1.27 |
| 40333_at | bone morphogenetic protein-4 (hBMP-4) | | -1.42 |
| 34847_s_at | calcium/calmodulin-dependent protein kinase (CaM kinase)II | | 1.13 |
| | beta | | |
| 33935_at | calcyclin binding protein | | 1.41 |
| 1751_g_at | Calreticulin | -4.03 | |
| 32067_at | cAMP responsive element modulator (CREM) | 1.39 | 2.75 |
| 39241_at | carbonic anhydrase I | -2.68 | |
| 40095_at | carbonic anhydrase II | -1.69 | |
| 40163_r_at | cartilage oligomeric matrix protein precursor | 2.36 | |
| 128_at | cathepsin k | 1.18 | |
| 129_g_at | cathepsin k | 1.20 | |
| 38466_at | cathepsin k | 1.27 | |
| 40718_at | cathepsin w | -1.31 | |
| 32833_at | CDC-like kinase 1 | 1.63 | |
| 646_s_at_ | CDC-like kinase 2 isoform helk2/139. | 1.19 | |
| 34163_at | chondroitin sulfate proteoglycan 6 | | -1.18 |
| 598_at | collagen type II alpha-1 | -1.38 | -1.19 |
| 32488_at | collagen type III alpha 1 | -1.41 | |
| 38952_s at | collagen type IV alpha-2 | 1.23 | 1.44 |
| 35379_at | collagen type IX alpha 1 | -2.22 | |
| 34802_at | collagen type VI alpha-2 (AA 570-998) | -1.37 | |
| 38566_at | collagen type X alpha-1 | | 1.67 |
| 37892_at | collagen type XI alpha-1 | 1.24 | 1.18 |

| Expression | Probe | Salmon | PTS893 |
|---|---|---|---|
| Set Identifier | | Calcitonin | |
| 1026_s_at | collagen type XI alpha2 | -1.20 | |
| 1027_at | collagen type XI alpha2 | 1.11 | |
| 39632_at | collagenase 3 (matrix metalloproteinase 13) | 1.20 | |
| 36638_at | connective tissue growth factor. | | -1.32 |
| 1943_at | cyclin A | | -1.74 |
| 40697_at | cyclin A2 | -1.60 | -1.39 |
| 34736_at | cyclin B1 | -2.83 | |
| 39251_at | cyclin C | | -2.03 |
| 1983_at | cyclin D2 | | -1.28 |
| 36650_at | cyclin D2 | 1.21 | |
| 35249_at | cyclin E2 | -2.95 | |
| 1649_at | cyclin G 1 interacting protein | | 1.31 |
| 1913_at | cyclin G2 | | -1.29 |
| 160024_at | cyclin-dependent kinase (CDC2-like) 10 PISSLRE | | 1.53 |
| 1942_s_at | cyclin-dependent kinase 4 | | -1.22 |
| 1206_at | cyclin-dependent kinase 5 | 1.56 | |
| 40549_at | cyclin-dependent kinase 5 | | -1.40 |
| 799_at | cyclin-dependent kinase 5, regulatory subunit 1 (p35) | 1.32 | |
| 41546_at | cyclin-dependent kinase 6 | 1.15 | |
| 2031_s_at | cyclin-dependent kinase inhibitor 1A (p21, Cip1) | 1.95 | |
| 1787_at | cyclin-dependent kinase inhibitor 1C | | 1.18 |
| 38673_s_at | cyclin-dependent kinase inhibitor 1C | | 1.13 |
| 39545_at | cyclin-dependent kinase inhibitor 1C | | 1.24 |
| 1797_at | cyclin-dependent kinase inhibitor 2D (p19, inhibits CDK4) | | -1.21 |
| 35816_at | cystatin B (stefin B) | 1.57 | |
| 806_at | cytokine-inducible kinase | 1.20 | |
| 40049_at | death-associated protein kinase 1 | | -1.30 |
| 33903_at | death-associated protein kinase 3 | -1.22 | -7.73 |
| 34029_at | dentin matrix acidic phosphoprotein 1 (DMP1) | 1.65 | |
| 38059_g_at | dermatopontin | | 1.72 |
| 343_s_at | ectonucleotide pyrophosphatase/ phosphodiesterase | 1.11 | |
| 342_at | ectonucleotide Pyrophosphatase/ Phosphodiesterase | 1.45 | |
| 1442_at | estrogen receptor | 1.47 | |
| 33670_at | estrogen receptor | 1.30 | |
| 1487_at | estrogen receptor-related protein | 1.11 | |
| 38882_r_at | estrogen-responsive B box protein (EBBP) | 1.22 | |
| 38902_r_at | estrogen-responsive B box protein (EBBP) | | 1.23 |
| 39945_at | fibroblast activation protein | -127 | |
| 424_s_at | fibroblast growth factor receptor. | -I.I7 | |
| 466_at | general_transciption factor II, | | 1.34 |
| 1102_s_at | glucocorticoid receptor alpha | | 1.43 |
| 33510_s_at | glutamate receptor, metabotropic 1 | 1.26 | 1.23 |
| 33269_at | GPI1 N-acetylglucosaminyl transferase component Gpi1 | 1.24 | 1.21 |
| 41476_at | G-protein alpha subunit 11 | | 1.24 |
| 1401_g at | granulocyte-macrophage colony-stimulating factor (CSF1) | -3.07 | -2.57 |
| 1911_s_at | growth arrest and DNA-damage-inductible protein (gadd45) | | 2.87 |

| Expression Probe | | Salmon | PTS893 |
|---|---|---|---|
| Set Identifier | | Calcitonin | |
| 888_s_at | growth differentiation factor 1 | | -1.43 |
| 37615_at | growth factor receptor-bound protein 10 | 1.21 | |
| 33929_at | heparan sulfate proteoglycan (glypican). | | 2.00 |
| 39757_at | heparan sulfate proteoglycan core protein | | 1.10 |
| 755_at | inositol 1,4,5-trisphosphate receptor type 1 | | 1.27 |
| 33506_at | inositol polyphosphate 4-phosphatase type I-beta | 1.12 | -1.24 |
| 33290_at | inositol polyphosphate 5-phosphatase (5ptase) | | -1.20 |
| 32697_at | inositol(myo)-1(or 4)-monophosphatase 1 | -1.36 | |
| 1975_s_at | insulin-like growth factor 1 | | -1.41 |
| 1501_at | insulin-like growth factor 1 (somatomedin C) | | -1.12 |
| 1232_s_at | insulin-like growth factor binding protein | -1.31 | |
| 40422_at | insulin-like growth factor binding protein 2 | | -1.27 |
| 1586_at | insulin-like growth factor binding protein 3 | 1.45 | |
| 37319_at | insulin-like growth factor binding protein 3 | 2.17 | |
| 1737_s_at | insulin-like growth factor binding protein 4 | | 1.13 |
| 41420_at | insulin-like growth factor binding protein 5 | | 1.18 |
| 1396_at | insulin-like growth factor binding protein 5 | | 1.62 |
| 1678_g_at | insulin-like growth factor binding protein 5 | | 1.44 |
| 38650_at | insulin-like growth factor binding protein 5 | | 1.53 |
| 1741_s_at | insulin-like growth factor binding protein-2 | -2.49 | -2.11 |
| 1464_at | insulin-like growth factor II precursor | 1.18 | |
| 1591_s_at | insulin-like growth factor II precursor | 1.41 | 1.31 |
| 39781_at | insulin-like growth factor-binding protein 4 | | 1.16 |
| 33082_at | integrin alpha 10 subunit | 1.33 | |
| 35131_at | integrin-binding sialoprotein (bone sialoprotein, bone sialoprotein II) | | 1.15 |
| 40060_r_at | LIM protein (similar to rat protein kinase C-binding enigma) | 1.44 | 1.32 |
| 36184_at | lysyl hydroxylase (PLOD) procollagen-lysine, 2-oxoglutarate 5 dioxygenase | | -1.40 |
| 34795_at | lysyl hydroxylase isoform 2 (PLOD2) | | 1.49 |
| 36811_at | lysyl oxidase-like protein | -1.44 | |
| 1433_g_at | MAD, mothers against decapentaplegic homolog 3 | 1.14 | 1.73 |
| 34655_at | MAGUKs (membrane-associated guanylate kinase homologues | 1.23 | |
| 36179_at | MAP kinase activated protein kinase 2 | | 1.18 |
| 35652_g_at | MAP kinase kinase kinase (MTK1) | 1.14 | |
| 41279_f_at | MAPK8IP1 Mitogen-activated protein kinase 8 interacting protein 1 | | 1.25 |
| 41280_r_at | MAPK8IP1: mitogen-activated protein kinase 8 interacting protein 1 | -131 | -1.31 |
| 1509_at | Metalloproteinase | -1.42 | |
| 976_s_at | mitogen-activated protein kinase1 | -1.61 | 1.12 |
| 34006_s_at | mitogen-activated protein kinase 8 | 1.32 | |
| 1439_s_at | mitogen-activated protein kinase-activated protein kinase 2 | | 1.78 |
| 37565_at | MMD: monocyte to macrophage differentiation-associated | 1.28 | 1.30 |
| 38369_at | myeloid differentiation primary response gene (88) | | -1.10 |
| 1052_s_at | NF-IL6-beta protein | | 1.30 |
| 36472_at | N-myc and STAT interacter- | | -1.35 |
| 38354_at | nuclear factor NF-IL6 (AA 1-345) | | 1.92 |
| 33106_at | nuclear orphan receptor LXR-alpha nuclear receptor subfamily | | 3.29 |
| 1, | group H, member 3 | | |
| 33381_at | nuclear receptor co-activator | | 1.11 |
| 279_at | nuclear receptor subfamily 4, group A, member 1 | | 2.30 |
| 280_g_at | nuclear receptor subfamily 4, group A, member 1 | | 3.08 |
| 37623_at | nuclear receptor subfamily 4, group A, member 2 Member of the steroid/thyroid hormone receptor family | | 27.72 |
| 547_s_at | nuclear receptor subfamily 4, group A, member 2 Member of the steroid/thyroid hormone receptor family | | 26.77 |
| 190_at | nuclear receptor subfamily 4, group A, member 3 Member of steroid/thyroid receptor family of nuclear hormone receptors | | 5.45 |
| 41202_s_at | OS-4 protein (OS-4) | 1.24 | |
| 1451_s_at | OSF-2os osteoblast specific factor-2 (periostin) | -1.65 | |
| 38822_at | O-sialoglycoprotein endopeptidase | | 2.43 |
| 467_at | osteoclast stimulating factor (OSF | -1.23 | |
| 35107_at | osteoprotegerin ligand | | 3.33 |
| 33814_at | PAK4 protein | 1.16 | |
| 38757_at | PDGF associated protein. | -1.89 | |
| 40253_at | phosphatidylinositol 4-kinase (NPIK-C). 1.77 | | |
| 37412_at | phosphatidylinositol-4-phosphate 5-kinase isoform C (-1) | -1.87 | |
| 751_at | phosphatidylinositol-glycan-class C (PIG-C) | 1.14 | -1.25 |
| 666_at | phosphodiesterase 4A, cAMP-specific | 1.33 | 1.30 |
| 38526_at | phosphodiesterase 4D, cAMP-specific | 1.30 | 3.53 |
| 38921_at | phosphodiesterase IB, calmodulin-dependent | 1.52 | |
| 38944_at | phosphodiesterase IB, calmodulin-dependent | | 1.17 |
| 32029_at | phosphoinositide dependent protein kinase-1 (3) | | 1.16 |
| 31699_at | phosphoinositide-3-kinase | 1.56 | 1.16 |
| 1085_s_at | phospholipase C | | -1.14 |
| 364_s_at | phospholipase C b3 | 1.22 | |
| 901_g_at | phospholipase C, beta 4 | -1.20 | |
| 1293_s_at | phospholipase D | -1.26 | |
| 32306_g_at | preprocollagen type I alpha-2 | 1.19 | |
| 35473_at | preprocollagen type I alpha1. | -2.72 | |
| 38951_at | PRKCQ Protein kinase C, theta | | 1.43 |
| 32307_s_at | procollagen | 1.13 | |
| 34494_at | procollagen I-N proteinase. | | 1.92 |
| 37605_at | procollagen type II alpha1 | | 1.91. |
| 36109_at | prolidase (imidodipeptidase) PEPD | -2.55 | |
| 1884_s_at | proliferating cell nuclear antigen | -1.85 | |
| 34390_at | prolyl 4-hydroxylase alpha (II) subunit | | 1.19 |
| 37037_at | prolyl 4-hydroxylase alpha subunit | | 1.20 |
| 36666_at | prolyl 4-hydroxylase beta | 1.95 | |
| 36533_at | prostacyclin synthase | | 1.20 |
| 718_at | protease, serine, 11 (IGF binding) | | -1.30 |
| 719_g_at | protease, serine, 11 (IGF binding) | -1.43 | |
| 385_at | proteasome (prosome, macropain) subunit, beta type, 10 | 1.36 | |
| 39183_at | protein kinase 1 PCTAIRE | -1.17 | |
| 37698_at | protein kinase A (PRKA) anchor protein 1 | | 1.29 |
| 397711_at | protein kinase C substrate 80K-H | | 1.13 |
| 39161_at | protein kinase Njmu-R1 | | 1.21 |
| 35348_at | protein kinase, AMP-activated, beta 1 non-catalytic subunit | | 2.10 |
| 36359_at | protein kinase, cAMP-dependent, catalytic, gamma | 1.39 | |
| 546_at | protein kinase, cAMP-dependent, catalytic, inhibitor alpha | | 1.14 |
| 227_g_at | protein kinase, cAMP-dependent, regulatory, type I, alpha | | 1.18 |
| 41768_at | protein kinase, cAMP-dependent, regulatory, type I, alpha | | 1.15 |
| 1091_at | protein kinase, cAMP-dependent, regulatory, type I, beta | 1.65 | |
| 116_at | protein kinase, cAMP-dependent, regulatory, type II, alpha | 1.28 | |
| 33633_at | purinergic receptor P2Y, G-protein coupled, 11 | 1.90 | |
| 32737_at | RAC2 Ras-related C3 botulinum toxin substrate 2 (rho family, small GTP binding protein Rac2) | 1.16 | |
| 40299_at | RE2 G-protein coupled receptor | | 1.24 |
| 35668_at | receptor (calcitonin) activity modifying protein 1 RAMP 1 | | 1.34 |
| 40696_at | receptor (TNFRSF)-interacting serine-threonine kinase 1 | | 1.12 |
| 1007_s_at | receptor tyrosine kinase DDR | 1.21 | |
| 37701_at | regulator of G-protein signalling 2, 24kD | | 2.06 |
| 1048_at | retinoid X receptor-gamma | 1.47 | 1.34 |
| 36217_at | serine/threonine kinase 38 | 1.54 | |
| 41544_at | serum-inducible kinase | | 1.16 |
| 32447_at | SF-1; Steroidogenic factor-1 | 8.76 | |
| 36487_at | short stature homeobox 2, | | -1.46 |
| 41222_at | signal transducer and activator of transcription 6 (STAT6) | 1.44 | |
| 1955_s_at | SMAD6 (inhibits BMP/Smad1 (MADH1) signalling) | 1.19 | |
| 37718_at | SNF-1 related kinase | 1.49 | 1.19 |
| 35883_at | Spi-B | | -2.80 |
| 1244_at | Stat2 | | -1.12 |
| 506_s_at | Stat5A | | 1.16 |
| 38994_at | STAT-induced STAT inhibitor-2 | | 1.25 |
| 38669_at | Ste20-related serine/threonine kinase | 1.24 | 1.65 |
| 37152_at | steroid hormone receptor superfamily | | 1.19 |
| 35844_at | syndecan 4 | | 1.37 |
| 38374_at | TEIG; TGFB inducible early growth response | 1.18 | |
| 38427_at | TEIG; TGFB inducible early growth response | | 1.38 |
| 32080_at | tetracycline transporter-like protein | | 1.41 |
| 224_at | TGFB inducible early growth response; TIEG | 1.26 | |
| 36940_at | TGFBI-induced anti-apoptotic factor 1 | 1.22 | 1.60 |
| 32217_at | TGF-beta induced apotosis protein 12 | 1.40 | |
| 41445_at | TGF-beta precursor | 1.14 | |
| 1890_at | TGF-beta superfamily protein | 1.74 | |
| 40631_at | Tob | -1.14 | 1.59 |
| 39358_at | transcriptional co-repressor nuclear receptor co-repressor 2 | | 1.42 |
| 1385_at | transforming growth factor induced protein | | 1.36 |
| 1830_s_at | transforming growth factor-beta | | 1.17 |
| 1767_s_at | transforming growth factor-beta 3 (TGF-beta 3) | -1.71 | -1.63 |
| 40581_at | TRIO: triple functional domain (PTPRF interacting) | 1.65 | 1.56 |
| 32272_at | tubulin alpha | -1.20 | |
| 685_f_at | tubulin alpha isotype H2-alpha | -4.36 | -1.79 |
| 330_s_at | tubulin alpha, 1, | -1.80 | -1.15 |
| 151_s_at | tubulin beta | -1.40 | |
| 39399_at | tubulin beta cofactor D | -1.85 | |
| 471_f_at | tubulin beta, 4 | -1.38 | |
| 40567_at | tubulin, alpha 3 | -1.39 | |
| 709_at | tubulin, beta 3 | -1.18 | |
| 33678_i_at | tubulin, beta, 2 | -1.15 | |
| 33679_f_at | tubulin, beta, 2 | -1.31 | |
| 1651_at | ubiquitin carrier protein E2-C | -3.74 | -1.22 |
| 32548_at | unactive progesterone receptor | | -1.33 |
| 1953_at | vascular endothelial growth factor | 1.40 | 1.20 |
| 36101_s_at | vascular endothelial growth factor | 1.45 | 1.44 |
| 36140_at | Y box binding protein-1 | 2.30 | 5.49 |
| - numbers = fold | down-regulated | | |
| + numbers = fold | up-regulated | | |

*Real-time PCR.* Based on the DNA microarray data a set of transcripts was chosen for quantitative analysis by real time-PCR (RT-PCR).

Briefly, the method exploits the SyBr Green dye which intercalates into double stranded DNA. Accumulation of PCR products is detected directly by monitoring the increase in fluorescence of the SyBr Green dye. Reactions are characterised by the point in time during cycling when amplification of a PCR product is first detected rather than the amount of PCR product accumulated after a fixed number of cycles. The higher the starting copy number of nucleic acid target, the sooner a significant increase in fluorescence is observed.

From each RNA sample, cDNA was made using an Applied Biosystem kit (Applied Biosystems # N808-0234) following the recommendation of the manufacturer. The PCR mixture was prepared using the SyBr Green Universal PCR Master Mix (Applied Biosystems # 4309155) as follows: 5 µl cDNA template, 400 nM of each primer, 0.2 mM deoxynucleotide triphosphates, 1 mM MgCl2 and 0.5 U Taq DNA polymerase, 5 µl SyBr. Green PCR buffer and RNase free water up to a final volume of 50 µl. The PCR was performed using the ABI Prism 7700 Sequence Detection System, after a step at 95°C for 10 min, the step-cycle program was performed for a total of 40 cycles as follows: 95°C for 30 s, 60°C for 1 min. A negative control was included: PCR reaction mixture with water in place of the cDNA sample.

The initial template concentration was determined based on the threshold cycle. The threshold cycle is the PCR cycle at which fluorescence is first detected above background and has been shown to be inversely proportional to the number of target copies present in the sample. Quantification was performed by calculating the unknown target concentration relative to an absolute standard and by normalizing to a validated endogenous control such as a housekeeping gene (β-action). Results are presented as percentage of control, once the ratio between the numbers of molecule for the gene of interest divided by the number of molecule for beta-actin has been calculated.

Based on the DNA microarray data the following set of transcripts was chosen for quantitative analysis by RT-PCR: adhesion receptor CD44, angiopoietin, bone morphogenetic protein 5, carbonic anhydrase II, cartilage oligomeric matrix protein, cathepsin K, osteopontin, pre-pro-alpha-2 type I collagen, Spi-B and Y-box binding protein.

**TABLE 38**

| Real Time PCR Results | | | |
|---|---|---|---|
| GeneChip® Expression | Coding Gene | Treatment Effect Salmon | Treatment Effect |
| Probe Set Identifier | | Calcitonin (% respect to control) | PTS893 (% respect to control) |
| 1372_at | adhesion receptor CD44 | No change | No change |
| 1929_at | angiopoietin-1 | No change | No change |
| 1831_at | bone morphogenetic protein 5 | +16 | +18 |
| 40095_at | carbonic anhydrase II | - 60 | No change |
| 40161_at | cartilage oligomeric matrix protein | +34.23 | No change |
| 128_at | cathepsin K | +67.2 | No change |
| 2092_s_at | osteopontin | No change | No change |
| 32306_g_at | pre-pro-alpha-2 type I collagen | +38 | +62 |
| 35883_at | Spi-B | -44 | -18 |
| 36140_at | Y-box binding protein (bone) | +14 | +26 |
| 36140_at | Y-box binding protein (kidney) | +15 | n.a. |
| 36140_at | Y-box binding protein (muscle) | -26 | n.a. |

| | | | |
|---|---|---|---|
| n.a.: not applicable | | | |

RT-PCR confirmed in most of the cases the changes observed in the gene profiling analysis, as it was the case for bone morphogenetic protein 5, carbonic anhydrase II, cathepsin K, cartilage oligomeric matrix protein, pre-pro-alpha-2 type I collagen, Spi-B and Y-Box binding protein. No changes were however detected in the level of expression of adhesion receptor CD44, angiopoietin-1 and osteopontin.

*Analysis.* Calcitonin is known to exert an effect on the differentiation, survival and resorptive activity of osteoclasts, resulting in a decreased osteoclastic activity. Pondel M, Intl. J. Exp. Pathol. 81 (6): 405-22 (2000). These effects could be reconstructed by multiorgan gene profiling (TABLE 39).

**TABLE 39**

| Effects on Osteoclasts | | | |
|---|---|---|---|
| Function | Coding genes | Salmon Calcitonin | PTS893 |
| Osteoclast | PU.1 (SPI1) | B, K, P, T | B |
| determination, survival | | | |
| and differentiation | Granulocyte to macrophage | B, K | B |
| | colony-stimulating factor (CSF1) | | |
| | Monocyte to macrophage differentiation | B, K, T | B |
| | associate (MMD) | | |
| | Osteoclast stimulating factor 1 (Autocrine | B, K, L, P | |
| | stimulation of osteoclast resorptive activity) | | |
| Bone resorption by | H+ ATP-ases | ALL | B |
| osteoclast | Carbonic anhydrase I, II. | B, L, P | |
| | Cathepsin K | ALL | |
| | ODF/OPGL: osteoprotegerin ligand | | B |
| Osteoclast motility | Tubulins | ALL | |
| | PAK4 protein | B, M, P | |

| | | | |
|---|---|---|---|
| Multiorgan gene expression profiling in salmon calcitonin treated animals. Organs where changes in expression were seen are displayed. B= bone; K= kidney; M= muscle; P= pituitary; L= liver; T= trachea. | | | |

PU.1 is involved in the initial stages of osteoclastogenesis. Tondravi MM et al., Nature 386(6620): 81-4 (1997). CSF-1 is imperative for macrophage maturation; it binds to its receptor c-*fms* on early osteoclast precursors, providing signals required for their survival and proliferation. Teitelbaum SL, Science 289(5484):1504-1508 (2000).

Interestingly, PTS893 also regulates the genes implicated in osteoclast differentiation and survival, SPI1, CSF-1 and MMD. This osteoclast regulation has not been previously described.

Salmon calcitonin was shown to regulate the expression of the gene coding for osteoclast stimulating factor (OSF), which is an intracellular protein produced by osteoclasts that indirectly induces osteoclast formation and bone resorption. Reddy S et al., J. Cell Physiol. 177 (4): 636-45 (1998). This would imply an autocrine effect of salmon calcitonin in the regulation of the osteoclast function, which is described here for the first time.

In addition salmon calcitonin seems to exert a paracrine regulation of the osteoclast resorptive activity, through the regulation of cystatin expression in the osteoblast. Carbonic anhydrase I, II, H⁺-ATPases and cathepsin K are the main effectors for dissolving bone mineral and matrix degradation. Blair HC et al., Biochem. (2002). Regulation of tubulins and PAK4 genes can be related to the effect of calcitonin on osteoclast motility PAK 4. Zaidi M et al., Bone 30(5): 655-63 (2002); Jaffer ZM & Chernoff J, Intl. J. Biochem. Cell Biol. 34(7): 713-7 (2002).

These results show modulating effects of calcitonin on genes affecting the direct, autocrine, paracrine and endocrine regulation of the osteoblast function (TABLE 40). These data support the hypothesis that attributes a bone anabolic effect to calcitonin.

**TABLE 40**

| Effects on Osteoblasts | | | |
|---|---|---|---|
| Function | Coding Gene | Salmon Calcitonin | PTS893 |
| Antagonists of cathepsins; | Cystatins | B | |
| antiresorptive activity | | | |
| Autocrine/paracrine | Alpha-2-HS-Glycoprotein | B, K, T | |
| regulation of osteoblast | | | |
| function | Bone Morphogenetic Proteins | ALL | B |
| | Fibroblast Growth Factors | B, K, M, P, T | B |
| | IL6/LIF | | B |
| | Insulin-like Growth Factors | ALL | B |
| | TGFs | B, K, M, P | B |
| | Tob | B, M, P | B |
| | Vascular Endothelial Growth Factor | B, M | X |
| Endocrine regulation of | Activin | B, L, M, P | B |
| osteoblast function | | | |
| | Estrogen receptor | ALL | |
| | Retinoic receptor X | B, P | B |
| | Steroidogenic factor | B, L, P, T | |
| | nuclear receptors (steroid/thyroid family) | | B |
| Transcription factor that | Y-box binding protein | B, K, M, P | B |
| regulates collagen type 1 | | | |
| synthesis | | | |

| | | | |
|---|---|---|---|
| Multiorgan gene expression profiling in salmon calcitonin treated animals. Organs where changes in expression were seen are displayed. B= bone; K= kidney; M= muscle; P= pituitary; L= liver; T= trachea. | | | |

Three families of growth factors, the transforming growth factor betas (TGF-βs), insulin-like growth factors (IGFs), and bone morphogenetic proteins (BMPs), are considered to be principal local regulators of osteogenesis. Bone morphogenetic proteins are thought to have their major effects on early precursor bone cell replication and osteoblast commitment. In contrast, TGB-βs are thought to be the most potent inducers of committed bone cell replication and osteoblast matrix production, while IGFs appear to integrate and extend the effect of both factors. McCarthy TL et al., Crit. Rev. Oral Biol. Med. 11(4): 409-22 (2000). These results support the fact that both salmon calcitonin and PTS893 are able to regulate these local and systemic factors implicated in bone metabolism. The fact that salmon calcitonin regulates α2-HS glycoprotein (AHSG), which blocks TGF-β-dependent signalling in osteoblastic cells, also supports this role. Mice lacking AHSG display growth plate defects, increased bone formation with age, and enhanced cytokine-dependent osteogenesis. Szweras M et al., J. Biol. Chem., 277(22): 19991-19997 (2002).

Salmon calcitonin and PTS893 were also shown to modulate the expression of the genes coding for vascular endothelial growth factor (VEGF). VEGF is known for playing a key role in normal and pathological angiogenesis. The critical role of angiogenesis for successful osteogenesis during the endochondral ossification is well documented. VEGF indirectly induces proliferation and differentiation of osteoblasts by stimulating endothelial cells to produce osteoanabolic growth factors. Wang DS et al., Endocrinology 138(7): 2953-62 (1997). In addition, VEGF stimulates chemotactic migration of primary human osteoblasts, suggesting a functional role in bone formation and remodeling. Mayr-Wohlfahrt U et al., Bone 30 (3): 472-7 (2002).

The effects of parathyroid hormone on osteoblast for mediating both bone resorption and formation have been widely described. Swarthout JT et al., Gene 282(1-2):1-17 (2002). It was here possible to confirm the effect of PTS893 on cytokines like interleukin 6 (IL-6), which mediates the paracrine activation of osteoclast differentiation and activity. Greenfield EM et al., Life Sci. 65:1087-102 (1999). PTS893 also produced a strong up-regulation on nuclear receptors (steroid/thyroid family).

Both calcitonin and parathyroid hormone receptors belong to the G-protein receptor superfamily. After receptor stimulation, signal transduction is mediated by adenylate cyclase/cAMP/protein kinase, phospholipase C, phospholipase D, and MAPK (as a late effecter) pathways in the case of calcitonin, and by adenylate cyclase and phospholipase C in the case of parathyroid hormone. Gene profiling analysis allowed the reconstruction of these pathways, showing genes that were modulated by the treatment and that are localised at different levels of the signal transduction pathway.

**TABLE 41**

| Effects on Signal Transduction and Cell Cycle | | | |
|---|---|---|---|
| Function | Coding Gene | Salmon Calcitonin | PTS893 |
| Signal transduction. | Adenylate cyclase | | B |
| | Calcyclin binding protein | | B |
| | Calreticulin | B, K, M | |
| | CREM | B, L, P | B |
| | CDC Kinase | B, M | |
| | MAPK | ALL | B |
| | Protein kinases | ALL | |
| | Phosphatidylinositol pathway | ALL | B |
| | Phosphodiesterase (IB, 4A, 4B) | ALL | B |
| | Phospholipase (C, D) | ALL | B |
| | PCNA | B | |
| | SMAD pathway | ALL | B |
| | STAT pathway | ALL | B |
| Cell cycle | Cyclins (A, A2, B1, C, D2, E2, G1, G2) | B | B |
| | Cyclin-dependent kinases 5, 6, 10 | B, K, P, T | B |
| | Cyclin-dependent kinases inhibitor 1A, 1C, 2D) | B | B |

| | | | |
|---|---|---|---|
| Multiorgan gene expression profiling in salmon calcitonin treated animals. Organs where changes in expression were seen are displayed. B= bone; K= kidney; M= muscle; P= pituitary; L= liver; T= trachea. | | | |

Bone morphogenetic protein (BMP) controls osteoblast proliferation and differentiation through Smad proteins. Tob, a member of the emerging family of antiproliferative proteins, is a negative regulator of BMP/Smad signalling in osteoblasts. Smad pathway as well as Tob as one of their regulators were also identified as genes modulated by the sCT and PTS893 treatment, in agreement with the hypothesised effect of both compounds on BMP regulation of bone remodelling. Within this context, both compounds seem to exert a direct influence on cell cycle, since changes in cyclins and cyclin-related proteins could be also observed.

Both compounds regulate also synthesis and degradation of extracellular matrix components (TABLE 42).

**TABLE 42**

| Effects on Extracellular Matrix | | | |
|---|---|---|---|
| Function | Coding Gene | Salmon Calcitonin | PTS893 |
| Cell attachment. Signal | Integrins | B, M, P | B |
| transduction. | | | |
| Collagen digestion | Collagenase | B | |
| | Matrix metalloproteinases I, II | B, L, P, T | |
| Collagen synthesis | Procollagen endopeptidase/proteinase | | B |
| | Lysyl hydroxylase | | B |
| Extracellular matrix | Aggrecan | | B |
| component | Cartilage Oligomeric Matrix Protein Precursor | B, K, | |
| | Collagen type I, type II, type III, type IV, type V, | ALL | B |
| | type VI, type IX, type X, type XI, type XIII, type | | |
| | XIV, type XV, and/or type XVI) | | |
| | Chondroitin sulphate proteoglycan | K, M, T | B |
| | Dermatopontin | | B |
| | Heparan sulphate proteoglycan | L,T | B |
| | Syndecan | | B |

| | | | |
|---|---|---|---|
| Multiorgan gene expression profiling in salmon calcitonin-treated animals. Organs where changes in expression were seen are displayed. B= bone; K= kidney; M= muscle; P= pituitary; L= liver; T= trachea. | | | |

Of particular interest is the regulation of the Y-Box binding protein (YB-1), which appears to be modulated by both treatments and in four out of six organs analysed in the salmon calcitonin group. YB-1 is a protein that interacts with a TGF-β response element in the distal region of the collagen alpha 1(I) gene. YB-1 protein activates the collagen promoter and translocates into the nucleus during TGF-β addition to fibroblasts, suggesting a role for this protein in TGF- β signalling. Sun W et al., Matrix Biol. 20(8): 527-41 (2001).

In addition, salmon calcitonin and PTS893 regulated some aspects of the mineralization of the bone extracellular matrix, since changes in amelogenin, dentin and ectonucleotide pyrophosphatases were observed.

**TABLE 43**

| Effects on Mineralization and Visualisation | | | |
|---|---|---|---|
| Function | Coding Gene | Salmon Calcitonin | PTS893 |
| Cement component | Amelogenin | B, L | B |
| Mineral matrix protein | Dentin | B | B |
| Enzyme for synthesis of | Ectonucleotide pyrophosphatases | B, M | |
| inorganic Pi | | | |
| Growth factor | VEGF | B, M | B |
| vascularization | | | |

| | | | |
|---|---|---|---|
| Multiorgan gene expression profiling in salmon calcitonin treated animals. Organs where changes in expression were seen are displayed. B= bone; K= kidney; M= muscle; P= pituitary; L= liver; T= trachea. | | | |

### EXAMPLE VII

### GENE EXPRESSION PROFILING OF COMPOUND OF FORMULA (II)

The compound of formula (II), 7-hydroxy-4-[4-(2-pyrrolidin-1-yl-ethoxy)-benzyl]-3-(2,4-dichloro-phenyl)-chromen-2-one, as shown:

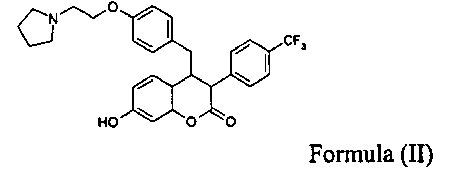

The compound of formula (II) was developed for the treatment of bone-resorbing diseases generally, including osteoporosis, metastatic bone cancer, osteolytic lesions with orthopedic implants, Paget's disease, and bone loss associated with hyperthyroidism. The compound of formula (II) can also be used to treat other conditions associated with IL-6 including various cancers (*e.g*. breast cancer, prostate cancer, colon cancer, endometrial cancer, multiple myeloma, renal cell carcinoma, and cervical carcinoma) and arthritis (*e.g*. adjuvant-, collagen- and antigen-induced arthritis, particularly rhenmatoid arthritis). See, published PCT patent applications WO 96/31206, WO 00/39120, WO 01/49673. The compound of formula (II) has estrogenic, antiestrogenic, antifertility and uterotropic activity.

The purpose of this EXAMPLE is to identify the "compound signature" of the compound of formula (II), to define a strategy for further development. The main concerns are the potential stimulation of the uterus and the induction of deep venous thrombosis (DVT). In addition, a goal of the EXAMPLE is to identify biomarkers for efficacy or risk assessment of the compound of formula (II).

Selective Estrogen Receptor Modulators (SERMs) are selective estrogen receptor modulators that act as agonists or antagonists depending on the target tissue. To increase our understanding of the direct tissue-specific effects of different SERMs and to elucidate the underlying molecular mechanisms, a study was started to perform a comprehensive in vivo gene expression profiling using DNA microarrays.

Gene expression microarray technology allows for the simultaneous detection and measurement of the expression of thousands of genes in a given cell or tissue sample in a single experiment. van de Rijn M & Gilks CB. Histopathology 44:97-108 (2004). This technique has significant advantages over previous methods for measuring gene expression, such as the reverse transcriptase-polymerase chain reaction and Northern blot analysis, which are limited to evaluation of small numbers of genes per experiment.

Because microarray technology enables rapid global gene expression profiling (GEP), it provides a powerful tool for analysis of a wide range of diseases, pharmacogenomic research, high throughput screening in drug discovery, and diagnostic screening for various diseases. Heller MJ. Annual Review of Biomedical Engineering. 4:129-153 (2002). Gene expression profiling can be used to identify the genes whose altered expression may be directly related to causing a particular disease. By systematically evaluating the genes identified by such screens, researchers increase the likelihood of uncovering suitable targets for therapeutic intervention. In addition, the use of gene expression profiling in pharmacogenomics can be useful to identify potential undesirable effects of drug treatment by identifying changes in gene expression. Similarly, gene expression profiling may be useful in identifying patients that have a greater likelihood of achieving a meaningful outcome from a given therapeutic treatment. While methods for detecting changes in gene expression in response to therapy have been used, a method for scoring (or ranking) treatment agents based on their respective gene expression profilings has not been described.

Tamoxifen, a selective estrogen receptor modulator (SERM), is a nonsteroidal hormonally active agent that has agonistic and antagonistic effects in different target tissues; it is an antagonist in breast tissue and an agonist in the uterus. Kiang DT & Kennedy BJ, Ann Intern Med. 87:687-690 (1997); Jordan VC & Allen KE, Eur. J. Cancer 16:239-251 (1980); Jordan VC et al., Breast Cancer Res Treat. 10:31-35 (1987); Gottardis MM et al., Cancer Res. 48:812-815 (1998); Fornander T et al., Lancet 1:117-120 (1989). Tamoxifen is an effective antitumour agent that is used in the treatment of estrogen-mediated breast cancer and as a chemopreventative agent; it decreases the risk of both invasive and noninvasive breast cancer. However, tamoxifen increases the risk for uterine hyperplasia and cancer through its proestrogenic effects on the endometrium. These undesired effects led to a search for SERMs with better safety profiles. Raloxifene, a second generation SERM that has a more favorable therapeutic and safety profile than tamoxifen, was subsequently developed. Cohen FJ et al., Obstet. Gynecol. 95:104-110 (2000); Ring J et al. (The Desloratadine Study Group) Int J Dermatol. 40:1-5 (2001); Fugere P et al., Am J Obstet Gynecol. 182:568-574 (2000). Raloxifene has a reduced antagonistic effect on the endometrium compared with tamoxifen is not associated with endometrial cancer risk.

Because various SERMs exhibit differential estrogenic effects on the uterus, we used this treatment effect as a model system to test and validate a new, unique method of scoring the relative pharmacological and toxicological activity of different therapeutic agents according to their gene expression profilings. Gene expression profiling has been used for dissecting the molecular mechanisms of physiology and disease in the uterus using tissue cultures, transgenic mice, and normal rats. However, few studies have been performed in primates and little is known of the gene expression profiling in monkey uterine tissue. Ace CI & Okulicz WC, Reprod Biol Endocrinol. 2:54(2004); Marvanova M et al., FASEB J. 17:929-931 (2003); Zou J et al., Genome Biol. 3:research0020.1-research0020.13 (2002). Furthermore, no studies have incorporated a method for scoring (or ranking) therapeutic agents based on their respective gene expression profilings. If such a ranking system where available, it could have broad utility for a wide spectrum of therapeutic areas, and represent a significant advance for the discovery and development of new pharmacological entities. In the current EXAMPLE, changes in uterine gene expression profilings were explored in normal and OVX cynomolgus monkeys following tamoxifen and raloxifene treatment; results are compared with those obtained after treatment with estradiol. The estrogenic potency of each agent was then attributed a score based in its effect on uterine gene expression.

*Animals.* Female sexually mature cynomolgus monkeys *(Macaca fascicularis* obtained from R.C. Hartelust BV, Tilburg, the Netherlands) approximately 48 months old were subjected to surgery (ovarectomy [OVX] or sham) 10 weeks prior to treatment and were treated for parasitic arthropods and helminthes, subjected to a tuberculin testing, and acclimated to the treatment facility for at least 14 days before beginning treatment.

All animals received tap water, filtered with a 0.22 µm filter, *ad libitum.* Approximately 180 g of OWM pelleted diet (Dietex France, SDS, Saint Gratien, France) was administered daily to each animal at least one hour after dosing except on the last day of treatment where the animals were fasted. Each animal received two fruits or vegetables daily.

*Surgical Procedures.* The ovariectomized cynomolgus monkey *(Macaca fascicularis),* which has been validated as a useful model predictive of outcomes in human clinical trials of estrogen and SERMs (Cline JM et al., Toxicol Pathol. 29:84-90 (2001)), was used as a model of estrogen deficiency. Animals were ovariectomized approximately 10 weeks before the first day of treatment. The ovaries were removed following anesthesia with a combined intramuscular injection of xylazine (Rompun^{®}: 0.4 mL/animal, Bayer Pharma Division Santé Animale, Puteaux, France) and ketamine hydrochloride (Imalgène^{®}: 0.6 mL/kg, Mérial, Lyon, France). Sham-operated animals were subjected to the same surgical procedure, except for the removal of ovaries.

*Estradiol Assay.* Estradiol serum levels were determined in each animal approximately 2 weeks after surgery in order to verify the effect of ovarectomy. Venous blood samples (approximately 1.5 mL) of unfasted animals were collected in tubes without anticoagulant and analyzed using radioimmunoassay (Sorin, Ecole Nationale Vétérinaire de Lyon, France).

*Treatment Protocol.* Ovariectomized and sham-operated animals were divided into groups of 4 and treated as outlined below.

**TABLE 44**

| Surgery | Drug | Dose (mg/kg/day PO) |
|---|---|---|
| Sham | Vehicle control | -- |
| OVX | Vehicle control¹ | -- |
| OVX | Estradiol² | 0.4 |
| OVX | Tamoxifen³ | 10.0 |
| OVX | Raloxifene³ | 10.0 |

| | | |
|---|---|---|
| ¹An aqueous solution of 0.5% carboxymethylcellulose was used as a vehicle. ²Ethinyl estradiol was prepared in corn oil. ³Tamoxifen and raloxifene were prepared in an aqueous solution of 0.5% carboxymethylcellulose. | | |

*Tissue Preparation.* Gene expression was determined in the pituitary and uterus at the end of drug therapy. Tissues to be analyzed (pituitary glands and uterus) were excised, snap frozen in liquid nitrogen, and stored at -80°C until RNA extraction was performed.

*DNA Microarray Analysis.* Total RNA was obtained by acid guanidinium isothiocyanate-phenol-chloroform extraction (Trizol; Invitrogen Life Technologies, San Diego, CA, USA) and purified on an affinity resin column (RNeasy; Qiagen, Hilden, Germany) according to manufacturer instructions. Chomczynski P & Sacchi N. Anal Biochem. 1987;162:156-159. DNA microarray experiments were conducted as recommended by the manufacturer of the GeneChip system (Affymetrix, Inc. 2002) and as previously described. Lockhart DJ, Dong H, Byrne MC, et al. Nat Biotechnol. 1996;14:1675-1680.

Previous studies have proven the validity of cross-species DNA chip analysis (Hacia JG et al. Nat Genet 18:155-158 (1998)), therefore, the human gene expression probe arrays HGU133A (Affymetrix, Santa Clara, CA, USA) containing 22,283 probe sets interrogating primarily annotated human genes were used. One GeneChip was used per tissue, per animal. The resulting image files (dat files) were processed using the Microarray Analysis Suite 5 (MAS5) software (Affymetrix, Inc.). Tab-delimited files were obtained containing data regarding signal intensity (Signal) and categorical expression level measurement (Absolute Call).

*Quantification of Drug Potency Relative to Specific Drug Action; GEP Scoring Axis.* To define a reference axis representing the desired pharmacological/toxicological action, we used *a priori* knowledge of 2 groups representing or approximating the extremes of the condition under study. These consisted of the OVX group (OVX untreated animals representing the one extreme) and OVX + estradiol (OVX animals treated with estradiol, an agent know to exercise a well-defined action, representing the other extreme). These two groups were used to quantitatively scale the axis. Other compounds were then positioned on this axis whereby the drugs could be ranked with regard to specific aspects of a pharmacological/toxicological action.

*Selection of Features.* Generally, the data derived from an individual microarray, proteomic, or metabolite profiling experiment can be considered as a fingerprint. However, in practical terms, many of the features (e.g. genes) do not represent relevant information and only confound the analysis of the outcomes of such experiments. Thus, selection and elimination of the appropriate features is an important aspect of this methodology and the quantitative nature of this approach depends on the features used to define the axis of drug action.

In the case of a well-defined condition, it is possible to invoke *a priori* knowledge on the selection of features. If the molecular biology of the specific drug action is known, the features (*e.g*. genes, metabolites) to be used for the definition of the axis can be defined *a priori.* In the case of a less-well-defined condition, it is possible to use univariate filters (in this case, gene intensity >50, ≥1.5-fold change in expression vs. controls [OVX], and statistical significance and statistical methods to identify and exclude non-informative features.

*Data Pretreatment.* Issues of transformation, centering (variable-wise), and scaling of the data were considered. In the case of microarrays, the data were log10-transformed and probe set-wise centered. Generally, no scaling was applied. When using only a small number of biologically identified features, these were scaled to unit variance.

*Principal Component Analysis.* To calculate the axis of drug action, a principal component axis (PCA) was performed using only the 2 treatment groups that were at the extremes (the untreated OVX animals vs. the estrodiol-treated OVX animals). Massart DL et al., Handbook for Chemometrics and Qualimetrics, Part a. (Elsevier; 1997); Jolliffe IT. Principal Component Analysis. 2nd Edition (Springer; New York, NY, 2002). Only 1 principal component (PC) was calculated, which was a weighted linear combination of all features in the input data pointing in the direction of maximum variance. The weights of the probe sets in this linear combination were called "loadings" and their absolute value is a measure of their importance in explaining the difference between the 2 groups. Every measurement in the experiment (*e.g*. microarray) could then be represented by its coordinate (score) on the PC. Alternatively, to obtain an even better separation between the groups, the PLS-DA (Partial-Least-Squares Discriminate Analysis) method with a 0/1 dummy variable as response vector was used. Barker M & Rayens W. Journal of Chemometrics 17:166-173 (2004). Massart DL et al., Handbook of Chemometrics and Qualimetrics, Part b. (Elsevier; 1997). The next step was identification of the features that were important for the definition of the axis. This was based on the magnitude of their loadings and their statistical significance using error estimates based on cross-validation and/or normal probability plots of the loadings. Hunter WG & Hunter JS. Statistics for Experimenters: an Introduction to Design, Data Analysis, and Model Building. 1st Edition (Wiley; 1978). This enabled calculation of a new PCA model using only the important features.

If a pharmacological or toxicological action affected multiple biochemical pathways that were represented by grossly different numbers of features in the measurement, block scaling was used. Eriksson L et al., Multi- and Megavariate Data Analysis - Principles and Applications. (Umetrics Academy; 2001). This attributed the same influence to all pathways (groups of features) in the calculation of the PCA model. Hierarchical PCA could be applied to identify the most important pathways of drug action. Eriksson L et al., J Comput Aided Mol Des. 16:711-726 (2002).

*Applying the PCA Model.* Using the loadings of the final PCA model the scores of all measurements in the other treatment groups (tamoxifen and raloxifene) were calculated. The medians (or means) of the scores of the two extreme groups were used to scale the axis of drug action (0 - 100%). The score (estrogenic potency) of a treatment (drug/dose) was expressed on this scale for individuals or group averages (median or mean).

*Statistical Analyses.* Score variances were used to estimate confidence intervals (Sachs L. Angewandte Statistik, 10. (Springer; Berlin, Germany, 2002)), and significant differences between groups were assessed with various statistical tests (*e.g*. t-test, u-test). Zar JH. Biostatistical Analysis. 4th Edition (Prentice Hall; 1998).

*Comparative GEPs Following Estradiol, Tamoxifen, and Raloxifene Treatment. Pituitary Gland.* Ovariectomy induced the expected significant up-regulation of the genes encoding luteinising hormone (LH) and follicle-stimulating hormone (FSH) in the pituitary gland (TABLE 45).

**TABLE 45**

| Affect of Ovariectomy on Luteinising Hormone and Follicle-Stimulating Hormone in the Pituitary Gland | | | | | | |
|---|---|---|---|---|---|---|
| | | | Fold change relative to OVX | | | |
| | *OVX (average)* | *SD* | *Sham* | *EE* | *Tamoxifen* | *Raloxifene* |
| Luteinising hormone | 1385 | 420 | 0.1 | 0.1 | 0.4 | 1.3 |
| Follicle stimulating | | | | | | |
| hormone | 4964 | 971 | 0.2 | <0.1 | 0.4 | 0.8 |

| | | | Fold expression change to SHAM control | | | |
|---|---|---|---|---|---|---|
| | *Sham Control (average)* | *SD* | *OVX* | *EE* | *Tamoxifen* | *Raloxifene* |
| Luteinising hormone | 184 | 141 | 7.5 | 0.5 | 3.2 | 9.8 |
| Follicle stimulating | | | | | | |
| hormone | 807 | 290 | 6.1 | 0.1 | 2.3 | 5.2 |

| | | | | | | |
|---|---|---|---|---|---|---|
| OVX, ovariectomized; EE, ethinyl estradiol Data derived from 4 animals/group (1 DNA chip/animal). | | | | | | |

Compared with sham surgery, the transcript levels were 7.6- and 6.2-fold greater (LH and FSH, respectively) in untreated OVX animals. Administration of estradiol reversed the effects of ovariectomy, and decreased the expression of LH and FSH transcripts 16.4- and 73.4-fold, respectively, well below normal physiological values. Tamoxifen had a weak estrogenic effect, downregulating pituitary LH and FSH gene expression by 2.4- and 2.7-fold in OVX monkeys. Raloxifene had a minimal effect on gonadotropin expression, reducing LH and FSH transcripts by 1.2- to 1.3-fold.

*Uterus.* The effect on uterine gene expression was subdivided by grouping genes in different logical categories; signal transduction, growth factors, extracellular matrix, and as a group, genes involved in cell-cycle, transport, and redox.

*Signal transduction inducers.* Overall, an increased expression of genes encoding signal transduction proteins was observed following estradiol treatment in OVX monkeys (TABLE 46).

**TABLE 46**

| Comparative GEP for Signal Transduction Inducers in the Uterus Following Treatment with Estradiol, | | | | | | |
|---|---|---|---|---|---|---|
| Tamoxifen, and Raloxifene in Ovariectomized Monkeys | | | | | | |
| | | | Fold change relative to OVX | | | |
| | *OVX (average)* | *SD* | *Sham* | *EE* | *Tamoxifen* | *Raloxifene* |
| Progesterone receptor | 101 | 54 | 3.0 | 9.7 | 4.5 | 1.9 |
| LIM and senescent cell antigen-like | | | | | | |
| domains 1 | 196 | 50 | 2.6 | 3.7 | 2.9 | 2.2 |
| Wingless-type MMTV integration site | | | | | | |
| family, | | | | | | |
| member 2B | 34 | 19 | 1.8 | 2.6 | 1.9 | 1.4 |
| Secreted frizzled-related protein 1 | 222 | 61 | 2.0 | 7.1 | 2.2 | 1.6 |
| Integrin, beta 5 | 68 | 49 | 1.7 | 1.7 | 1.5 | 1.5 |
| Secreted frizzled-related protein 4 | 931 | 251 | 2.2 | 4.2 | 2.9 | 0.8 |
| Phospholipid transfer protein | 716 | 383 | 1.6 | 2.0 | 1.4 | 1.2 |
| Cadherin 11, type 2, OB-cadherin | | | | | | |
| (osteoblast) | 486 | 104 | 1.6 | 3.4 | 3.2 | 1.3 |
| Wingless-type MMTV integration site | | | | | | |
| family, | | | | | | |
| member 16 | 58 | 61 | 1.1 | 1.1 | 1.1 | 0.9 |
| Phosphatase and tensin homolog (mutated in | | | | | | |
| multiple advanced cancers 1) | 173 | 50 | 1.6 | 1.8 | 1.6 | 1.1 |
| Protein tyrosine kinase 9 | 84 | 6 | 1.6 | 2.1 | 1.7 | 1.2 |
| Four and a half LIM domains 2 | 175 | 68 | 1.5 | 3.0 | 1.7 | 0.9 |
| Dishevelled associated activator of | | | | | | |
| morphogenesis 1 | 351 | 39 | 1.6 | 3.1 | 1.2 | 0.9 |
| Calcium channel, voltage-dependent, beta 2 | | | | | | |
| subunit | 158 | 36 | 0.5 | 0.6 | 0.5 | 0.8 |
| Catenin (cadherin-associated protein), | | | | | | |
| alpha-like 1 | 158 | 62 | 0.5 | 0.5 | 0.7 | 0.8 |
| LDL receptor adaptor protein | 81 | 25 | 0.7 | 0.6 | 1.2 | 1.0 |
| Insulin receptor | 520 | 90 | 0.6 | 0.8 | 0.7 | 0.9 |
| Mitogen-activated protein kinase kinase | | | | | | |
| kinase 4 | 670 | 146 | 0.6 | 0.6 | 0.6 | 0.8 |

| Tamoxifen, and Raloxifene in Ovariectomized Monkevs | | | | | | |
|---|---|---|---|---|---|---|
| | | | Fold change relative to SHAM control | | | |
| | *Sham Control (average)* | *SD* | *OVX* | *EE* | *Tamoxifen* | *Ralolxifene* |
| Progesterone receptor | 308 | 258 | 0.3 | 3.2 | 1.5 | 0.6 |
| LIM and senescent cell antigen-like | | | | | | |
| domains 1 | 508 | 269 | 0.4 | 1.4 | 1.1 | 0.8 |
| Wingless-type MMTV integration site | | | | | | |
| family, | | | | | | |
| member 2B | 59 | 11 | 0.6 | 1.5 | 1.1 | 0.8 |
| Secreted frizzled-related protein I | 441 | 78 | 0.5 | 3.6 | 1.1 | 0.8 |
| Integrin, beta 5 | 117 | 45 | 0.6 | 1.0 | 0.9 | 0.9 |
| Secreted frizzled-related protein 4 | 2018 | 995 | 0.5 | 1.9 | 1.4 | 0.4 |
| Phospholipid transfer protein | 1140 | 473 | 0.6 | 1.3 | 0.9 | 0.7 |
| Cadherin 11, type 2, OB-cadherin | | | | | | |
| (osteoblast) | 795 | 204 | 0.6 | 2.1 | 1.9 | 0.8 |
| Wingless-type MMTV integration site | | | | | | |
| family, | | | | | | |
| member 16 | 67 | 18 | 0.9 | 0.9 | 1.0 | 0.8 |
| Phosphatase and tensin homolog (mutated in | | | | | | |
| multiple advanced cancers 1) | 269 | 30 | 0.6 | 1.2 | 1.0 | 0.7 |
| Protein tyrosine kinase 9 | 133 | 14 | 0.6 | 1.3 | 1.1 | 0.8 |
| Four and a half LIM domains 2 | 268 | 90 | 0.7 | 1.9 | 1.1 | 0.6 |
| Dishevelled associated activator of | | | | | | |
| morphogenesis 1 | ·550 | 136 | 0.6 | 2.0 | 0.8 | 0.6 |
| Calcium channel, voltage-dependent, beta 2 | | | | | | |
| subunit | 82 | 24 | 1.9 | 1.1 | 0.9 | 1.5 |
| Catenin (cadherin-associated protein), | | | | | | |
| alpha-like 1 | 81 | 15 | 1.9 | 1.0 | 1.5 | 1.5 |
| LDL receptor adaptor protein | 58 | 36 | 1.4 | 0.8 | 1.6 | 1.4 |
| Insulin receptor | 333 | 66 | 1.6 | 1.2 | 1.2 | 1.4 |
| Mitogen-activated protein kinase kinase | | | | | | |
| kinase 4 | 431 | 53 | 1.6 | 0.9 | 1.0 | 1.2 |

| | | | | | | |
|---|---|---|---|---|---|---|
| OVX, ovariectomized; EE, ethinyl estradiol Data derived from 4 animals/group (1 DNA chip/animal). | | | | | | |

Progesterone receptor, secreted frizzled-related protein 1, secreted frizzled-related protein 4, Dishevelled associated activator of morphogenesis 1, wingless-type MMTV integration site (family, member 2B; wnt2B), and integrin (beta 5) were increased 9.8-, 7.1-, 4.2-, 3.1-, and 2.6, and 1.7 fold, respectively, compared with untreated OVX controls. These same genes were up regulated by 1.2- to 4.5-fold following tamoxifen therapy. In contrast raloxifene therapy was associated with more modest changes in gene expression for these same signal transduction proteins (0.9- to 1.9-fold). The remaining genes examined were all downregulated by estradiol, tamoxifen (except for LDL receptor adaptor protein), and raloxifine.

*Growth factors.* Estradiol increased the expression of several genes encoding growth factors (GFs) as outlined in TABLE 47.

**TABLE 47**

| Comparative GEP for Cell Cycle Genes in the Uterus Following Treatment with Estradiol, Tamoxifen, and | | | | | | |
|---|---|---|---|---|---|---|
| Raloxifene in Ovariectomized Monkeys | | | | | | |
| | | | Fold change relative to OVX | | | |
| | *OVX (average)* | *SD* | *Sham* | *EE* | *Tamoxifen* | *Raloxifene* |
| CDC28 protein kinase regulatory subunit 2 | 58 | 15 | 1.7 | 3.8 | 3.1 | 0.8 |
| G1 to S phase transition 1 | 227 | 45 | 1.2 | 1.6 | 1.6 | 0.9 |
| Testis Enhanced Gene Transcript (Bax | | | | | | |
| Inhibitor 1) | 1516 | 492 | 1.0 | 1.8 | 1.5 | 1.1 |
| H2A histone family, member Y | 482 | 94 | 1.2 | 1.4 | 1.7 | 1.0 |
| H3 histone, family 3A | 9692 | 447 | 1.1 | 1.4 | 1.4 | 1.0 |
| H3 histone, family 3A | 9495 | 1003 | 1.0 | 1.5 | 1.4 | 0.9 |
| Histone H2A.F/Z variant | 890 | 89 | 1.0 | 1.4 | 1.5 | 1.0 |
| Cyclin D1 (PRAD1: parathyroid | | | | | | |
| adenomatosis 1) | 54 | 17 | 0.9 | 2.4 | 1.8 | 1.2 |
| Cyclin-dependent kinase inhibitor 1C (p57, | | | | | | |
| Kip2) | 224 | 69 | 0.9 | 0.5 | 0.7 | 0.9 |
| Cyclin-dependent kinase inhibitor 1C (p57, | | | | | | |
| Kip2) | 762 | 194 | 0.9 | 0.5 | 0.5 | 1.1 |
| Cyclin-dependent kinase inhibitor 1C (p57, | | | | | | |
| Kip2) | 527 | 142 | 0.9 | 0.4 | 0.5 | 1.2 |

| | | | Fold change relative to SHAM control | | | |
|---|---|---|---|---|---|---|
| | *SHAM Control (average)* | *SD* | *OVX* | *EE* | *Tamoxifen* | *Raloxifene* |
| CDC28 protein kinase regulatory subunit 2 | 96 | 66 | 0.6 | 2.3 | 1.9 | 0.5 |
| G1 to S phase transition 1 | 277 | 87 | 0.8 | 1.3 | 1.3 | 0.7 |
| Testis Enhanced Gene Transcript (Bax | | | | | | |
| Inhibitor 1) | 1579 | 341 | 1.0 | 1.8 | 1.5 | 1.1 |
| H2A histone family, member Y | 598 | 96 | 0.8 | 1.2 | 1.4 | 0.8 |
| H3 histone, family 3A | 10239 | 888 | 0.9 | 1.4 | 1.3 | 1.0 |
| H3 histone, family 3A | 9408 | 555 | 1.0 | 1.5 | 1.4 | 0.9 |
| Histone H2A.F/Z variant | 925 | 67 | 1.0 | 1.4 | 1.4 | 1.0 |
| Cyclin D1 (PRAD1: parathyroid | | | | | | |
| adenomatosis 1) | 49 | 22 | 1.1 | 2.7 | 2.0 | 1.3 |
| Cyclin-dependent kinase inhibitor 1C (p57, | | | | | | |
| Kip2) | 201 | 57 | 1.1 | 0.6 | 0.8 | 1.0 |
| Cyclin-dependent kinase inhibitor 1C (p57, | | | | | | |
| Kip2) | 660 | 213 | 1.2 | 0.5 | 0.6 | 1.3 |
| Cyclin-dependent kinase inhibitor 1C (p57, | | | | | | |
| Kip2) | 459 | 119 | 1.1 | 0.5 | 0.6 | 1.4 |

| | | | | | | |
|---|---|---|---|---|---|---|
| OVX, ovariectomized; EE, ethinyl estradiol Data derived from 4 animals/group (1 DNA chip/animal). | | | | | | |

The most prominent effects were observed for insulin-like GF binding protein 2 (36kDa) (7.1-fold increase), and insulin-like GF 1 (somatomedin C) (5.3- to 6.9-fold increase), compared with OVX controls. Genes encoding these growth factors were also upregulated by 2.7- and 3.7- to 4.8-fold following tamoxifen therapy. Raloxifene caused minor increases of (approximately 2-fold, similar to the sham group.

Estradiol also upregulated insulin-like growth factor binding protein 5, latent transforming growth factor beta binding protein 1, and cysteine-rich angiogenic inducer 61 by 5.2-, 3.6-, and 3.5-fold, respectively, compared with OVX controls. In contrast tamoxifen and raloxifene were associated with only modest changes in genes encoding these growth factors, similar to the levels observed in the sham group (TABLE 46).

*Extracellular matrix.* A number of diverse extracellular matrix proteins were upregulated by estradiol as outlined in TABLE 47. Collagen type I alpha 2, collagen type III alpha 1, collagen type I alphal, and collagen type IV alpha.1 were upregulated by estradiol treatment 13.2-, 6.8-, 6.6-, and 4.3-fold, respectively compared with OVX controls. Tamoxifen therapy also upregulated genes encoding these matrix proteins by 6.1-, 3.4-, 3.5-, and 3.2-fold, respectively. For the majority of the rest of the genes in this category, after treatment with tamoxifen or raloxifene, overall expression was brought back up to levels not that dissimilar to sham controls.

*Transport, redox, and cell cycle.* Estradiol increased expression of a variety of transport genes (solute carrier family 2 [facilitated glucose/fructose transporter]; member 5 ATPase; Na+/K+ transporting, beta 3 polypeptide; solute carrier family 2 [facilitated glucose/fructose transporter], member 5; solute carrier family 25 [mitochondrial carrier; adenine nucleotide translocator], member 5; ATPase, H+ transporting, lysosomal 38kDa, V0 subunit d isoform 1; ATP synthase, H+ transporting, mitochondrial F0 complex, subunit c [subunit 9] isoform 3; solute carrier family 22 [organic cation transporter], member 1-like ATP synthase, H+ transporting, mitochondrial F0 complex, subunit c [subunit 9] isoform 3; chloride intracellular channel 4) by 2- to 7-fold; genes encoding redox proteins (SET domain bifurcated 1, peroxiredoxin 1, and cytochrome c oxidase subunits VIa and VIIa) by approximately 2- to 3-fold; and genes encoding cell cycle proteins (CDC28 protein kinase regulatory subunit 2) by up to 4-fold in estradiol treated animals compared with OVX controls. Estrodial treatment downregulated expression of the gene for the redox protein, thioredoxin interacting protein, 3-fold and the gene for the cell cycle protein, cyclin-dependent kinase inhibitor 1C (p57, Kip2), by 2- to 4-fold compared with OVX control. Although slightly less potent, tamoxifen exerted similar effects whereas raloxifene had little or no effect on any of the transporter, redox, and cell cycle genes studied.

*Estrogen-induced genes.* Several estrogen-induced genes (brain creatine kinase; cytoplasmic dynein; hippocalcin-like 1; light polypeptide 1; brain prostaglandin D2 synthase 21kDa; hexokinase 1; and osteoblast cadherin 11, type 2) were upregulated by estradiol by 3- to 10-fold compared to OVX control However, the significance of these changes to uterine physiology and pathology has yet to be defined.

*Ranking of Drug Activity According to Uterine GEP.* To determine the relative estrogenic potency and score for a given estrogenic treatment, a potency axis was constructed by assigning a median GEP score of "0" for OVX controls (no estrogen) and a median GEP score of "100" for estradiol treated animals (maximum estradiol effect). The respective GEP scores for tamoxifen and raloxifene were plotted to determine their relative estrogenic potencies.

Application of different filtering parameters-gene intensity >50, statistically different change from OVX controls, and genes showing >1.5-fold changes from OVX control values-permitted ranking of the relative treatment potencies according to defined gene expression profiling (GEP) criteria. These approaches yielded more useful information than unsupervised GEP data analysis.

Of the 22,283 probes assayed per chip 225 individual genes showed statistically significant greater than 1.5-fold changes in expression level compared with OVX controls. Considering only this group of genes, tamoxifen showed a strong estrogenic response with a score of 59 and raloxifene had a weak estrogenic score of 23. In contrast, sham animals exhibit a baseline estrogenic score of 48.

When considering fold changes alone and disregarding statistical significance, 673 genes showed greater than 1.5-fold change in expression level compared with OVX controls. Tamoxifen exhibited a strong estradiol effect, with a score of 71, raloxifene still had a weak estrogenic score of 34, and baseline for sham animals was 66.

A further subanalysis was done using 123 genes that showed statistically significant greater than 1.5-fold changes in expression and are thought to have a role in uterine function. According to this analysis, tamoxifen retained a high estrogenic score of 69, raloxifene still had a weak estrogenic score of 20, and the sham baseline was 44. Using this approach, further subanalysis of specific gene groups showed that the estrogenic effects of tamoxifen (scores of 61 to 87) were consistently greater than those of raloxifene (scores of -11 to 32) in each category.

**TABLE 48**

| Comparative GEP for Transport Proteins in the Uterus Following Treatment with Estradiol, Tamoxifen, | | | | | | |
|---|---|---|---|---|---|---|
| and Raloxifene in Ovariectomized Monkeys | | | | | | |
| | | | Fold change relative to OVX | | | |
| | *OVX (average)* | *SD* | *Sham* | *EE* | *Tamoxifen* | *Raloxifene* |
| Solute carrier family 2 (facilitated | | | | | | |
| glucose/fructose transporter), member 5 | 171 | 101 | 1.9 | 4.4 | 2.2 | 1.2 |
| ATPase, Na+/K+ transporting, beta 3 | | | | | | |
| polypeptide | 1147 | 1058 | 1.8 | 2.2 | 2.1 | 0.9 |
| Solute carrier family 2 (facilitated | | | | | | |
| glucose/fructose transporter), member 5 | 176 | 103 | 1.7 | 3.5 | 2.1 | 1.0 |
| Solute carrier family 25 (mitochondrial | | | | | | |
| carrier; adenine nucleotide translocator), | | | | | | |
| member 5 | 923 | 463 | 1.5 | 2.5 | 2.2 | 1.1 |
| ATPase, H+ transporting, lysosomal 38kda, | | | | | | |
| V0 subunit d isoform 1 | 465 | 50 | 1.2 | 1.9 | 1.2 | 1.1 |
| ATP synthase, H+ transporting, | | | | | | |
| mitochondrial F0 complex, subunit c | | | | | | |
| (subunit 9) isoform 3 | 811 | 121 | 1.3 | 2.0 | 2.0 | 1.2 |
| Solute carrier family 22 (organic cation | | | | | | |
| transporter), member 1-like | 92 | 49 | 1.2 | 1.9 | 1.8 | 1.0 |
| ATP synthase, H+ transporting, | | | | | | |
| mitochondrial F0 complex, subunit c | | | | | | |
| (subunit 9) isoform 3 | 1007 | 188 | 1.3 | 2.0 | 1.9 | 1.2 |
| Chloride intracellular channel 4 | 287 | 185 | 1.8 | 1.7 | 1.9 | 1.4 |

| | | Fold change relative to SHAM control | | | | |
|---|---|---|---|---|---|---|
| | *SHAM Control (average)* | *SD* | *OVX* | *EE* | *Tamoxifen* | *Raloxifene* |
| Solute carrier family 2 (facilitated | | | | | | |
| glucose/fructose transporter), member 5 | 317 | 105 | 0.5 | 2.4 | 1.2 | 0.7 |
| ATPase, Na+/K+ transporting, beta 3 | | | | | | |
| polypeptide | 2028 | 2519 | 0.6 | 1.2 | 1.2 | 0.5 |
| Solute carrier family 2 (facilitated | | | | | | |
| glucose/fructose transporter), member 5 | 291 | 134 | 0.6 | 2.1 | 1.3 | 0.6 |
| Solute carrier family 25 (mitochondrial | | | | | | |
| carrier; adenine nucleotide translocator), | | | | | | |
| member 5 | 1351 | 518 | 0.7 | 1.7 | 1.5 | 0.8 |
| ATPase, H+ transporting, lysosomal 38kda, | | | | | | |
| V0 subunit d isoform 1 | 545 | 80 | 0.9 | 1.6 | 1.0 | 1.0 |
| ATP synthase, H+ transporting, | | | | | | |
| mitochondrial F0 complex, subunit c | | | | | | |
| (subunit 9) isoform 3 | 1045 | 276 | 0.8 | 1.6 | 1.6 | 0.9 |
| Solute carrier family 22 (organic cation | | | | | | |
| transporter), member 1-like | 112 | 41 | 0.8 | 1.6 | 1.5 | 0.8 |
| ATP synthase, H+ transporting, | | | | | | |
| mitochondrial F0 complex, subunit c | | | | | | |
| (subunit 9) isoform 3 | 1355 | 184 | 0.7 | 1.5 | 1.4 | 0.9 |
| Chloride intracellular channel 4 | 522 | 50 | 0.6 | 0.9 | 1.1 | 0.7 |
| OVX, ovariectomized; EE, ethinyl estradiol | | | | | | |
| Data derived from 4 animals/group (1 DNA chip/animal). | | | | | | |

**TABLE 49**

| Comparative GEP for Redox Proteins in the Uterus Following Treatment with Estradiol, Tamoxifen, and Raloxifene in Ovariectomized Monkeys | | | | | | |
|---|---|---|---|---|---|---|
| | | | Fold change relative to OVX | | | |
| | *OVX (average)* | *SD* | *Sham* | *EE* | *Tamoxifen* | *Raloxifene* |
| SET domain, bifurcated 1 | 928 | 171 | 1.3 | 3.0 | 1.9 | 1.1 |
| Peroxiredoxin 1 | 2,575 | 923 | 1.2 | 1.9 | 1.7 | 1.0 |
| Cytochrome c oxidase subunit via | | | | | | |
| polypeptide 1 | 912 | 199 | 1.1 | 1.5 | 1.3 | 1.0 |
| Cytochrome c oxidase subunit viia | | | | | | |
| polypeptide 2 (liver) | 266 | 18 | 1.0 | 1.8 | 1.6 | 1.1 |
| Ubiquinol-cytochrome c reductase, | | | | | | |
| Rieske iron-sulfur polypeptide 1 | 708 | 54 | 1.2 | 1.4 | 1.4 | 1.0 |
| Thioredoxin interacting protein | 5,300 | 644 | 0.6 | 0.3 | 0.6 | 1.0 |

| | | | Fold change relative to SHAM control | | | |
|---|---|---|---|---|---|---|
| | *Sham Control (average)* | *SD* | *OVX* | *EE* | *Tamoxifen* | *Raloxifene* |
| SET domain, bifurcated 1 | 1209 | 238 | 0.8 | 2.3 | 1.4 | 0.8 |
| Peroxiredoxin 1 | 3041 | 787 | 0.8 | 1.6 | 1.4 | 0.8 |
| Cytochrome c oxidase subunit via | | | | | | |
| polypeptide 1 | 1043 | 240 | 0.9 | 1.3 | 1.2 | 0.8 |
| Cytochrome c oxidase subunit viia | | | | | | |
| polypeptide 2 (liver) | 255 | 94 | 1.0 | 1.8 | 1.7 | 1.1 |
| Ubiquinol-cytochrome c reductase, | | | | | | |
| Rieske iron-sulfur polypeptide 1 | 881 | 80 | 0.8 | 1.1 | 1.1 | 0.8 |
| Thioredoxin interacting protein | 3327 | 778 | 1.6 | 0.5 | 0.9 | 1.7 |

| | | | | | | |
|---|---|---|---|---|---|---|
| OVX, ovariectomized; EE, ethinyl estradiol Data derived from 4 animals/group (1 DNA chip/animal). | | | | | | |

**TABLE 50**

| Comparative GEP for Extracellular Matrix Factors in the Uterus Following Treatment with Estradiol, | | | | | | |
|---|---|---|---|---|---|---|
| Tamoxifen, and Raloxifene in Ovariectomized Monkeys | | | | | | |
| | | | Fold change relative to OVX | | | |
| | *OVX (average)* | *SD* | *Sham* | *EE* | *Tamoxifen* | *Raloxifene* |
| Matrix metalloproteinase 19 | 11 | 5 | 5.1 | 6.3 | 1.6 | 0.9 |
| Biglycan | 23 | 28 | 2.7 | 1.9 | 2.0 | 2.4 |
| Fibronectin 1 | 39 | 26 | 2.5 | 1.6 | 2.3 | 2.3 |
| Collagen, type III, alpha 1 (Ehlers-Danlos | | | | | | |
| syndrome type IV, autosomal dominant) | 1084 | 471 | 2.8 | 6.8 | 3.4 | 1.9 |
| Collagen, type I, alpha 2 | 2757 | 929 | 2.8 | 5.1 | 3.7 | 2.2 |
| Collagen, type I, alpha 1 | 3056 | 697 | 2.8 | 6.6 | 3.5 | 1.8 |
| Collagen, type IV, alpha 1 | 182 | 87 | 2.4 | 4.3 | 3.2 | 1.8 |
| Heparan sulfate proteoglycan 2 (perlecan) | 17 | 12 | 2.2 | 3.5 | 2.2 | 1.2 |
| Procollagen C-endopeptidase enhancer | 215 | 97 | 2.1 | 3.2 | 2.3 | 1.7 |
| Collagen, type III, alpha 1 (Ehlers-Danlos | | | | | | |
| syndrome type IV, autosomal dominant) | 3445 | 859 | 2.1 | 4.1 | 2.9 | 2.0 |
| Collagen, type V, alpha 1 | 189 | 82 | 2.1 | 4.0 | 1.3 | 1.2 |
| Secreted protein, acidic, cysteine-rich | | | | | | |
| (osteonectin) | 561 | 172 | 2.1 | 2.9 | 2.2 | 1.7 |
| Fibronectin 1 | 419 | 99 | 2.2 | 2.4 | 2.0 | 2.2 |
| Fibrillin I (Marfan syndrome) | 124 | 57 | 1.9 | 2.2 | 1.9 | 2.1 |
| Serine (or cysteine) proteinase inhibitor, | | | | | | |
| clade H (heat shock protein 47), member | | | | | | |
| 1, (collagen binding protein 1) | 501 | 15 | 2.0 | 3.3 | 2.2 | 1.4 |
| Osteoglycin (osteoinductive factor, | | | | | | |
| mimecan) | 54 | 13 | 2.2 | 2.2 | 1.7 | 1.6 |
| Fibulin 1 | 583 | 413 | 1.8 | 1.6 | 1.9 | 1.4 |
| Fibronectin 1 | 648 | 126 | 2.0 | 1.9 | 1.9 | 1.7 |
| Collagen, type VIII, alpha 2 | 39 | 19 | 1.7 | 2.1 | 1.8 | 1.9 |
| Fibronectin 1 | 586 | 121 | 1.9 | 2.1 | 1.9 | 2.2 |
| Collagen, type VI, alpha 3 | 3428 | 408 | 1.8 | 3.7 | 2.0 | 1.2 |
| Collagen, type IV, alpha 2 | 929 | 335 | 1.7 | 3.3 | 2.2 | 1.4 |
| Fibrillin 1 (Marfan syndrome) | 874 | 453 | 1.6 | 3.5 | 2.1 | 1.5 |
| Collagen, type IV, alpha 1 | 690 | 203 | 1.7 | 3.7 | 2.7 | 1.2 |
| Fibronectin 1 | 757 | 89 | 1.7 | 1.8 | 1.6 | 1.9 |
| SPARC-like 1 (mast9, hevin) | 4971 | 1787 | 1.6 | 2.4 | 1.3 | 1.7 |
| Nidogen (enactin) | 24 | 16 | 1.4 | 3.6 | 1.9 | 2.4 |
| Laminin, beta 1 | 215 | 102 | 1.4 | 1.4 | 1.4 | 1.1 |
| - Procollagen-proline, 2-oxoglurate 4- | | | | | | |
| dioxygenase(proline 4-hydroxylase), | | | | | | |
| alpha polypeptide II | 120 | 49 | 1.5 | 2.6 | 1.6 | 1.2 |
| Chondroitin sulfate proteoglycan 2 | | | | | | |
| (versican) | 248 | 85 | 1.4 | 3.6 | 2.9 | 1.4 |
| Tenascin C (hexabrachion) | 74 | 36 | 1.3 | 1.2 | 3.8 | 1.0 |
| Collagen, type I, alpha 2 | 154 | 34 | 3.2 | 13.2 | 6.1 | 3.2 |
| Chitinase 3-like 1 (cartilage glycoprotein- | | | | | | |
| 39) | 100 | 25 | 1.4 | 3.9 | 1.7 | 1.1 |
| Dermatopontin | 132 | 92 | 1.8 | 1.9 | 1.8 | 1.5 |

| | | | Fold change relative to SHAM control | | | |
|---|---|---|---|---|---|---|
| | *SHAM Control (average)* | *SD* | *OVX* | *EE* | *Tamoxifen* | *Raloxifene* |
| Matrix metalloproteinase 19 | 59 | 27 | 0.2 | 1.2 | 0.3 | 0.2 |
| Biglycan | 61 | 11 | 0.4 | 0.7 | 0.7 | 0.9 |
| Fibronectin 1 | 100 | 31 | 0.4 | 0.6 | 0.9 | 0.9 |
| Collagen, type III, alpha 1 (Ehlers-Danlos | | | | | | |
| syndrome type IV, autosomal dominant) | 2992 | 860 | 0.4 | 2.5 | 1.2 | 0.7 |
| Collagen, type I, alpha 2 | 7758 | 3072 | 0.4 | 1.8 | 1.3 | 0.8 |
| Collagen, type I, alpha 1 | 8502 | 5403 | 0.4 | 2.4 | 1.3 | 0.7 |
| Collagen, type IV, alpha 1 | 436 | 183 | 0.4 | 1.8 | 1.3 | 0.7 |
| Heparan sulfate proteoglycan 2 (perlecan) | 38 | 14 | 0.5 | 1.6 | 1.0 | 0.6 |
| Procollagen C-endopeptidase enhancer | 449 | 97 | 0.5 | 1.5 | 1.1 | 0.8 |
| Collagen, type III, alpha 1 (Ehlers-Danlos | | | | | | |
| syndrome type IV, autosomal dominant) | 7354 | 1454 | 0.5 | 1.9 | 1.4 | 1.0 |
| Collagen, type V, alpha 1 | 394 | 137 | 0.5 | 1.9 | 0.6 | 0.6 |
| Secreted protein, acidic, cysteine-rich | | | | | | |
| (osteonectin) | 1187 | 361 | 0.5 | 1.4 | 1.0 | 0.8 |
| Fibronectin 1 | 920 | 427 | 0.5 | 1.1 | 0.9 | 1.0 |
| Fibrillin 1 (Marfan syndrome) | 236 | 71 | 0.5 | 1.2 | 1.0 | 1.1 |
| Serine (or cysteine) proteinase inhibitor, | | | | | | |
| clade H (heat shock protein 47), member | | | | | | |
| 1, (collagen binding protein 1) | 1011 | 306 | 0.5 | 1.6 | 1.1 | 0.7 |
| Osteoglycin (osteoinductive factor, | | | | | | |
| mimecan) | 120 | 76 | 0.4 | 1.0 | 0.8 | 0.7 |
| Fibulin 1 | 1026 | 469 | 0.6 | 0.9 | 1.1 | 0.8 |
| Fibronectin 1 | 1288 | 483 | 0.5 | 0.9 | 0.9 | 0.9 |
| Collagen, type VIII, alpha 2 | 68 | 18 | 0.6 | 1.2 | 1.1 | 1.1 |
| Fibronectin 1 | 1089 | 456 | 0.5 | 1.1 | 1.0 | 1.2 |
| Collagen, type VI, alpha 3 | 6008 | 1342 | 0.6 | 2.1 | 1.1 | 0.7 |
| Collagen, type IV, alpha 2 | 1594 | 548 | 0.6 | 1.9 | 1.3 | 0.8 |
| Fibrillin 1 (Marfan syndrome) | 1388 | 424 | 0.6 | 22 | 1.3 | 1.0 |
| Collagen, type IV, alpha 1 | 1143 | 276 | 0.6 | 2.2 | 1.6 | 0.7 |

| Comparative GEP for Extracellular Matrix Factors in the Uterus Following Treatment with Estradiol, | | | | | | |
|---|---|---|---|---|---|---|
| Tamoxifen, and Raloxifene in Ovariectomized Monkeys | | | | | | |
| Fibronectin 1 | 1301 | 425 | 0.6 | 1.1 | 0.9 | 1.1 |
| SPARC-like 1 (mast9, hevin) | 8078 | 2034 | 0.6 | 1.5 | 0.8 | 1.1 |
| Nidogen (enactin) | 34 | 15 | 0.7 | 2.6 | 1.4 | 1.7 |
| Laminin, beta 1 | 302 | 48 | 0.7 | 1.0 | 1.0 | 0.8 |
| Procollagen-proline, 2-oxoglutarate 4- | | | | | | |
| dioxygenase (proline 4-hydroxylase), | | | | | | |
| alpha polypeptide II | 184 | 92 | 0.7 | 1.7 | 1.0 | 0.8 |
| Chondroitin sulfate proteoglycan 2 | | | | | | |
| (versican) | 340 | 127 | 0.7 | 2.6 | 2.1 | 1.0 |
| Tenascin C (hexabrachion) | 96 | 34 | 0.8 | 0.9 | 2.9 | 0.8 |
| Collagen, type I, alpha 2 | 493 | 255 | 0.3 | 4.1 | 1.9 | 1.0 |
| Chitinase 3-like 1 (cartilage glycoprotein- | | | | | | |
| 39) | 141 | 59 | 0.7 | 2.8 | 1.2 | 0.8 |
| Dermatopontin | 233 | 43 | 0.6 | 1.1 | 1.0 | 0.8 |

| | | | | | | |
|---|---|---|---|---|---|---|
| OVX, ovariectomized; EE, ethinyl estradiol Data derived from 4 animals/group (1 DNA chip/animal). | | | | | | |

**TABLE 51**

| Comparative GEP for Growth Factors in the Uterus Following Treatment with Estradiol, Tamoxifen, and | | | | | | |
|---|---|---|---|---|---|---|
| Raloxifene in Ovariectomized Monkeys | | | | | | |
| | | | Fold change relative to OVX | | | |
| | *OVX (average)* | *SD* | *Sham* | *EE* | *Tamoxifen* | *Raloxifene* |
| Bone morphogenetic protein 1 | 93 | 24 | 2.2 | 2.9 | 2.2 | 1.4 |
| Bone morphogenetic protein 1 | 79 | 24 | 1.8 | 2.6 | 1.9 | 1.2 |
| Cysteine-rich, angiogenic inducer, 61 | 172 | 80 | 1.8 | 3.5 | 1.6 | 1.7 |
| Fibroblast growth factor 2 (basic) | 34 | 22 | 2.0 | 2.1 | 2.0 | 2.0 |
| Growth associated protein 43 | 37 | 8 | 2.4 | 2.0 | 1.9 | 1.7 |
| Insulin receptor | 520 | 90 | 0.6 | 0.8 | 0.7 | 0.9 |
| Insulin-like growth factor 1 (somatomedin C) | 156 | 48 | 2.7 | 6.9 | 4.6 | 2.2 |
| Insulin-like growth factor 1 (somatomedin C) | 102 | 40 | 2.2 | 5.3 | 3.7 | 1.5 |
| Insulin-like growth factor 1 (somatomedin C) | 310 | 79 | 1.9 | 6.3 | 4.8 | 2.3 |
| Insulin-like growth factor binding protein 2, | | | | | | |
| 36kda | 596 | 77 | 1.6 | 7.1 | 2.7 | 1.1 |
| Insulin-like growth factor binding protein 5 | 299 | 56 | 1.9 | 5.2 | 2.0 | 2.0 |
| Latent transforming growth factor beta | | | | | | |
| binding protein 1 | 181 | 80 | 2.1 | 3.6 | 2.6 | 1.8 |
| Pituitary tumour-transforming 1 interacting | | | | | | |
| protein | 59 | 25 | 2.1 | 1.8 | 1.9 | 1.5 |
| Stem cell growth factor; lymphocyte | | | | | | |
| secreted C-type lectin | 92 | 12 | 1.6 | 2.2 | 1.4 | 1.2 |
| Transforming growth factor, beta-induced, | | | | | | |
| 68kda | 376 | 97 | 2.2 | 1.7 | 2.2 | 1.7 |
| Intercrine-alpha, mrna sequence | 859 | 76 | 1.6 | 1.5 | 1.5 | 1.3 |

| | | | Fold change relative to SHAM control | | | |
|---|---|---|---|---|---|---|
| | *SHAM Control (average)* | *SD* | *OVX* | *EE* | *Tamoxifen* | *Raloxifene* |
| Bone morphogenetic protein 1 | 205 | 57 | 0.5 | 1.3 | 1.0 | 0.6 |
| Bone morphogenetic protein 1 | 143 | 44 | 0.5 | 1.4 | 1.0 | 0.7 |
| Cysteine-rich, angiogenic inducer, 61 | 303 | 200 | 0.6 | 2.0 | 0.9 | 0.9 |
| Fibroblast growth factor 2 (basic) | 67 | 40 | 0.5 | 1.1 | 1.0 | 1.0 |
| Growth associated protein 43 | 88 | 27 | 0.4 | 0.8 | 0.8 | 0.7 |
| Insulin receptor | 333 | 66 | 1.6 | 1.2 | 1.2 | 1.4 |
| Insulin-like growth factor 1 (somatomedin C) | 417 | 68 | 0.4 | 2.6 | 1.7 | 0.8 |
| Insulin-like growth factor 1 (somatomedin C) | 226 | 69 | 0.5 | 2.4 | 1.7 | 0.7 |
| Insulin-like growth factor 1 (somatomedin C) | 581 | 185 | 0.5 | 3.4 | 2.6 | 1.2 |
| Insulin-like growth factor binding protein 2, | | | | | | |
| 36kda | 957 | 104 | 0.6 | 4.4 | 1.7 | 0.7 |
| Insulin-like growth factor binding protein 5 | 562 | 260 | 0.5 | 2.8 | 1.0 | 1.1 |
| Latent transforming growth factor beta | | | | | | |
| binding protein 1 | 389 | 322 | 0.5 | 1.7 | 1.2 | 0.8 |
| Pituitary tumour-transforming 1 interacting | | | | | | |
| protein | 126 | 17 | 0.5 | 0.9 | 0.9 | 0.7 |
| Stem cell growth factor; lymphocyte | | | | | | |
| secreted C-type lectin | 148 | 36 | 0.6 | 1.4 | 0.9 | 0.7 |
| Transforming growth factor, beta-induced, | | | | | | |
| 68kda | 843 | 212 | 0.4 | 0.8 | 1.0 | 0.8 |
| Intercrine-alpha, mrna sequence | 1383 | 258 | 0.6 | 0.9 | 0.9 | 0.8 |

| | | | | | | |
|---|---|---|---|---|---|---|
| OVX, ovariectomized; EE, ethinyl estradiol Data derived from 4 animals/group (1 DNA chip/animal). | | | | | | |

*Discussion.* Previous studies have established that the OVX cynomolgus monkey model of estrogen deficiency is relevant to humans with respect to hormonal effects on the uterus. Cline JM et al., Toxicol Pathol.29:84-90 (2001). Data from these studies showed increased endometrial hyperplasia following treatment with estradiol or tamoxifen, and were confirmed in the present EXAMPLE. Moreover, we showed that in this model, estradiol reversed the effects of OVX on gonadotropin gene upregulation in the pituitary (TABLE 45), providing further evidence that the OVX monkey represents a valid model for evaluating the gene expression profiling (GEP) of estradiol and SERMs.

Overall, the gene expression data reported in the present EXAMPLE correlated with the histopathological effects of estradiol and SERMs on the uterus. Experimental and clinical studies have demonstrated the unequivocal proliferative effects of estrogen and tamoxifen on the uterus and uterine tumour cells. Gottardis MM et al., Cancer Res.48:812-815 (1988). Tamoxifen induced an estrogen-like gene expression profiling in the uterus of OVX monkeys that was consistent with uterine cystic hyperplasia, stromal fibrosis, and increased progesterone receptor expression observed in ovariectomized macaques. Cline JM et al., Toxicol Pathol.29:84-90 (2001). The minor changes in uterine GEPs of OVX monkeys following raloxifene therapy were in accord with studies showing its lack of pathological effects on the human uterus. Cohen FJ et al., Obstet Gynecol.95:104-110 (2000).

In the uterus, the proliferative effects of tamoxifen are mediated in part by GFs. Increased expression of IGF-1 and related binding proteins following estrogen therapy is well documented and may be important for local paracrine stimulation of uterine tissue growth. Norstedt G et al., Acta Endocrinologica (Copenh).120:466-472 (1989); Stygar D et al., Reproductive Biology and Endocrinology.1:40 (2003). Induction of cysteine-rich angiogenic inducer "61" and transforming growth factor beta genes has also been described in response to estrogen, and both growth factors appear to mediate endometrial growth and hyperplasia. Sampath D et al., Endocrinology. 142:2540-2548 (2001); Takahashi T et al., Cell Growth Differ. 5:919-935 (1994); Sartor BM et al., Reprod Toxicol.9:225-231 (1995). These observations support data from the present EXAMPLE in which estradiol increased the expression of these growth factors in the uterus (TABLE 46).

Recent studies using gene expression profiling in human endometrium have identified an array of genes and gene families associated with the implantation process, which involves a complex interaction between the embryo and the maternal endometrium. Locally secreted wnt proteins initiate a cell signaling pathway that involves activation of membrane-associated frizzle receptors (FzRs), which in turn activate Dishevelled (Dvl) and regulation of nuclear gene transcription required for implantation. Frizzle-related proteins (FRPs) modulate wnt signaling at the wnt-FzR-ligand binding site, and secreted FRP4 (also know as FrpHE) inhibits wnt signaling. Although wnt genes are controlled by sex steroids, including estrogen, the relevance of these findings to estrogen-induced uterine growth is not known. Tulac S et al., J Clin Endocrinol Metab. 88:3860-3866 (2003). According to studies in cancer cell lines, wnt2B signaling appears to play a role in tumourigenesis. Katoh M, Int J Mol Med. 8:657-660 (2001); Ricken A et al., Endocrinology. 143:2741-2749 (2002). Moreover, increased expression of secreted FRP1 and secreted FRP4 was observed in human uterine leiomyomas, the uterus of OVX rats administered estradiol, and human proliferative endometrium. Fujita M et al., J Mol Endocrinol. 28:213-223 (2002); Fukuhara K et al., J Clin Endocrinol Metab. 87:1729-1736 (2002). Whereas stromal FRP4, which is upregulated by estrogen, was involved in endometrial proliferation, secreted FRP1 was associated with antiapoptotic and antitumour effects. These studies are relevant to data reported in the present EXAMPLE, which showed that increased uterine expression of genes encoding wnt2B, Dvl, and secreted FRP1 and FRP4 following estradiol therapy (TABLE 46). The data suggest estrogenic effects on the uterus may involve the wnt signaling pathway and its regulation by secreted FRP4. Tulac S et al., J Clin Endocrinol Metab. 88:3860-3866 (2003).

Long-term treatment with conjugated equine estrogens upregulates progesterone receptors in the endometrial stromal tissue of OVX cynomolgus macaques. Wang H et al., Reprod Biol Endocrinol. 1:7 (2003). Estradiol increased progesterone receptor gene expression in the present EXAMPLE (TABLE 46), further validating the OVX monkey model for gene expression profiling.

Estradiol also increased uterine expression of other functional classes of genes, including extracellular matrix proteins (TABLE 47) and genes generally associated with cell proliferation (*e.g*., cell cycle, redox, and transport; TABLE 48). These effects are likely related to estrogen-induced uterine hyperplasia, which is characterized by increased cell proliferation and tissue growth. Cline JM et al., Toxicol Pathol. 29:84-90 (2001).

Analysis of the gene expression profiling (GEP) profiles associated with estradiol and SERMs revealed important differences in the molecular mechanisms through which these agents affect the uterus. Thus, tamoxifen's gene expression profiling suggests, that it acts primarily via estrogenic stimulation of growth factors, signal transduction mechanisms, extracellular matrix protein synthesis, and genes related to cell proliferation in the uterus. In contrast, raloxifene exhibited weak estrogenic activity with little or no effect on these molecular pathways as indicated by its gene expression profiling.

Further analysis of the gene expression profiling (GEP) data involved the development of a novel scoring system for ranking the relative estrogenic effects of tamoxifen and raloxifene. Across each of the seven different classes of genes expressed, tamoxifen ranked as the most estrogenic agent, with scores ranging from 69 to 87, compared with 100 for estradiol. Raloxifene was much less estrogenic by these criteria as shown by its low scores ranging from -11 to 32, compared with 100 for estradiol.

In conclusion, gene expression profiling (GEP) using DNA microarray combined with the novel method of ranking therapeutic agents described here is a powerful technique for defining and predicting the relative pharmacological and toxicological actions of new chemical entities, and represents a new paradigm for drug development in the pharmaceutical industry. Moreover, this procedure has enormous potential for identifying efficacy and safety biomarkers that can be used to refine the drug screening process, reducing the time and costs required for taking a drug from discovery to clinic.

### EXAMPLE VIII

*Introduction and summary.* The aim of this EXAMPLE is to further evaluate the effect of FGF23CTP on angiogenesis as shown in EXAMPLE III by using a hypoxic vascular retinopathy mouse model.

*Methods.* Seven days old C57/B16J mice are put together with their nursing parent in a sealed container ventilated by a mixture of oxygen and compressed air to a final oxygen concentration of 75% ± 2% until day 12. Returned at room air, the animals develop a relative ischemia of the retina with neovascularization starting as early as day 14.

On day 12, when animals returned from the oxygen incubation, a volume of 2.0 µl is injected in the eye using a glass pipette with a diameter of about 150 µm at its tip. The right eye is injected with 6 microgram (µg) FGF23CTP (3 µg / µl) and the left eye of the animal with PBS as individual control.

For the intravitreal injection of a substance at day 12, mice are anesthetized by inhalation of isoflurane. Five days later the animals are sacrificed after perfusion with dextran-florescein and retinal flatmounts are prepared. The coded whole mounts allow to evaluate the vascular changes of the retinal brindly. The proliferation score includes quantification of the proliferation including the papilla, vascular development, vasoconstriction, retinal bleeding and tortuosity of the vessels, features also seen in human retinal disease.

*Animals.* In total, 44 animals are used for injection with FGF23CTP and PBS. Of those, 8 animals died during hyperoxia or after injection, 2 animals are not perfused, 5 animals are paraffin embedded for evaluation of neurons, of which only three could be examined, and from 29 of these animals retinal flatmounts are prepared.

*Evaluation of the angioproliferative changes.* Evaluation of the angioproliferative changes in the retinal preparations is performed in a masked way on coded preparations. Retinopathy scoring system is adapted from Higgins, R. D., et al. (J. AAPOS 3: 114-116 (1999)) that was developed after modification of a scoring system used clinically in the neonatal intensive care unit. The following features are taken into consideration: neovascularisation of the optic disc, blood vessel tufts formation, large clusters of blood vessel tufts, central vasoconstriction and tortuosity of the vessels. Retinal haemorrhages are not taken into account as they may also result from the intraocular injection.

For each criteria, a defined number of points (P) is assigned, which are summed up to a total proliferation score. The higher the score, the worse the hypoxia induced retinopathy. In detail:

Neovascularisation of the optic disc: either 1 or 0 points are assigned in case optic disc proliferation is measured (1 P) or absence (0 P).

Blood vessel tufts formation: the score for blood vessel tufts is determined by dividing every clock hour of the preparation surface in 4 zones: Zone 1 (optic disc zone) is evaluated in addition; Zone 2: one point for every wing (maximal 4 P); Zone 3: one point for every clock hour (maximal 12 P); and Zone 4: one point for every clock hour (maximal 12 P). Thus, a maximum of 28 points is measured provided that no vascular areas exist, and a maximum of 12 points is calculated in case of complete avascularization.

Large clusters of blood vessel tufts: every large cluster of blood vessel tufts covering more than 3 sectors is counted as one point. Thus, maximal 8 points can be measured provided that no avascular areas exist, and maximal 4 points are assignable for complete avascularization.

Central vasoconstriction: 1 point is assigned to a vasoconstriction of zone 1 of greater than 50%; I point is allocated if the vasoconstriction is greater than 50% of every wing of zone 2 and 3. Thus, maximal 1 point can be assigned to zone 1, and maximal 4 points each for zones 2, 3 and 4. Since so far no vasoconstriction is ever observed for zone 4, a maximum of total 9 points (without zone 4) can be allotted.

Tortuosity of the vessels: 2 points are assigned if less than one-third of the vessels are tortuous; if the amount of tortuous blood vessels is between one-third and two-thirds, 4 points are allocated, and in case more than two-thirds of the vessels are tortuous, than 6 points are given.

The theoretical maximum of points is never reached, e.g. Zone 4 never shows avascular zones. Proliferation in avascular zones is never observed. This is in principle a mutual exclusion of criteria; the existence of vascular proliferations has a higher impact on the retinopathy score than large avascular areas. Therefore, the maximum score ever counted with this system is 38 points using a substance which is considered to enhance vascular proliferation.

The proliferation score relating to vascular changes on retinal flatmounts for individual animals treated with FGF23CTP (right eye) and PBS control (left eye) are measured as described above. The severity of retinopathy varies from animal to animal.
TABLE 52 summarizes the determined proliferation score.

**TABLE 52 Proliferation score**

| | right eye | left eye | | right eye | left eye |
|---|---|---|---|---|---|
| Animal No. | FGF23CTP | PBS | Animal No. | FGF23CTP | PBS |
| 146.1 | 4 | 4 | 150.1 | 14 | 16 |
| 146.2 | 13 | 8 | 150.2 | 8 | 26 |
| 146.3 | 8 | 9 | 150.3 | 17 | 24 |
| 146.4 | 3 | 5 | 150.4 | 30 | 29 |
| 146.5 | 6 | 8 | 160.2 | 10 | 7 |
| 146.6 | 1 | 9 | 160.3 | 10 | 7 |
| 146.7 | 2 | 4 | 160.4 | 16 | 20 |
| 146.8 | 5 | 6 | 160.5 | 28 | 28 |
| 147.1 | 9 | 8 | 161.1 | 30 | 15 |
| 147.2 | 8 | 9 | 161.2 | 21 | 33 |
| 147.3 | 2 | 8 | 161.3 | 17 | 22 |
| 147.4 | 11 | 12 | | | |
| 147.5 | 1 | 11 | | | |
| 149.1 | 5 | 26 | | | |
| 149.2 | 14 | 36 | | | |
| 149.3 | 8 | 10 | | | |
| 149.4 | 17 | 12 | | | |
| 149.5 | 10 | 8 | | | |

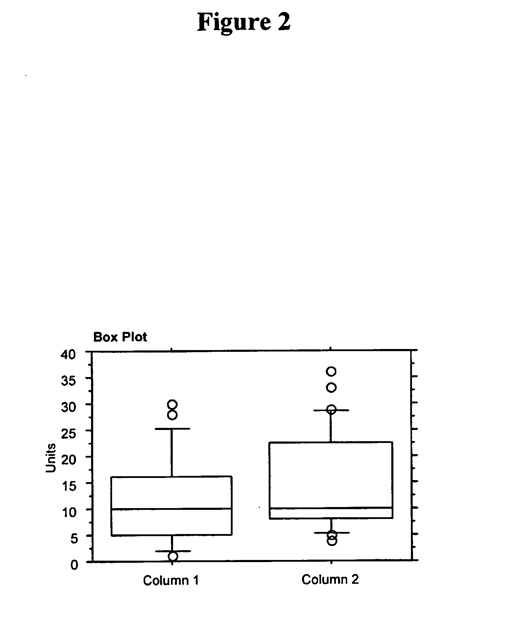

The proliferation scores of all animals are further displayed as box plot in Figure 2. Comparing the individual treated (right eye, FGF23CTP) with control eye (left, PBS)) the effect of FGF23CTP injection reaches statistical significance in a paired t-test with p= 0.04 (TABLE 53).

**TABLE 53 Paired t-test**

| Mean Difference | DF | t-Value | P-Value |
|---|---|---|---|
| -3,172 | 28 | -2,183 | 0,0375 |

The proliferation scores for the separate factors are presented in TABLE 54, and the respective paired t-tests in TABLE 55 to 57.

**TABLE 54 Proliferation scores**

| proliferation sum | | blood vessel tufts | | large clusters of prolif. | | avascular zone | | tortuosity of vessels | |
|---|---|---|---|---|---|---|---|---|---|
| right eye | left eye | right eye | left eye | right eye | left eye | right eye | left eye | right eye | left eye |
| FGF23CTP | PBS | FGF23CTP | PBS | FGF23CTP | PBS | FGF23CTP | PBS | FGF23CTP | PBS |
| 2 | 2 | 2 | 2 | 0 | 0 | 1 | 2 | 1 | 0 |
| 10 | 8 | 10 | 8 | 0 | 0 | 3 | 0 | 0 | 0 |
| 7 | 9 | 6 | 9 | 0 | 0 | 1 | 0 | 0 | 0 |
| 2 | 4 | 2 | 4 | 0 | 0 | 1 | 1 | 0 | 0 |
| 4 | 8 | 4 | 8 | 0 | 0 | 2 | 0 | 0 | 0 |
| 1 | 9 | 1 | 9 | 0 | 0 | 0 | 0 | 0 | 0 |
| 1 | 2 | 1 | 2 | 1 | 1 | 1 | 1 | 0 | 1 |
| 4 | 6 | 3 | 5 | 1 | 0 | 1 | 0 | 0 | 0 |
| 6 | 4 | 6 | 4 | 0 | 0 | 1 | 1 | 2 | 3 |
| 5 | 6 | 5 | 6 | 0 | 0 | 0 | 0 | 3 | 3 |
| 0 | 4 | 0 | 4 | 1 | 1 | 1 | 1 | 1 | 3 |
| 7 | 7 | 7 | 7 | 2 | 2 | 2 | 2 | 2 | 3 |
| 0 | 7 | 0 | 7 | 0 | 0 | 0 | 0 | 1 | 4 |
| 2 | 17 | 2 | 14 | 0 | 2 | 3 | 4 | 0 | 5 |
| 8 | 25 | 7 | 20 | 1 | 5 | 4 | 6 | 2 | 5 |
| 4 | 6 | 4 | 5 | 0 | 1 | 3 | 3 | 1 | 1 |
| 10 | 6 | 10 | 6 | 0 | 0 | 2 | 2 | 5 | 4 |
| 6 | 4 | 6 | 3 | 0 | 0 | 3 | 3 | 1 | 1 |
| 8 | 9 | 7 | 7 | 1 | 2 | 4 | 5 | 2 | 2 |
| 4 | 21 | 4 | 17 | 0 | 4 | 3 | 4 | 1 | 4 |
| 11 | 14 | 10 | 13 | 0 | 1 | 4 | 6 | 2 | 4 |
| 17 | 18 | 14 | 15 | 3 | 3 | 7 | 5 | 6 | 6 |
| 6 | 3 | 5 | 3 | 1 | 0 | 3 | 3 | 1 | 1 |
| 6 | 5 | 6 | 5 | 0 | 0 | 2 | 2 | 2 | 0 |
| 12 | 14 | 7 | 11 | 4 | 3 | 3 | 4 | 1 | 2 |
| 18 | 20 | 14 | 16 | 3 | 4 | 6 | 6 | 4 | 2 |
| 18 | 9 | 13 | 7 | 4 | 2 | 6 | 4 | 6 | 2 |
| 14 | 21 | 11 | 15 | 5 | 3 | 5 | 7 | 2 | 5 |
| 12 | 16 | 9 | 11 | 2 | 5 | 4 | 5 | 1 | 1 |

**TABLE 55 Paired t-test proliferation sum**

| Mean Difference | DF | t-Value | P-Value |
|---|---|---|---|
| -2,724 | 28 | -2,514 | 0,0180 |

**TABLE 56 Paired t-test avascular-zone**

| Mean Difference | DE | t-Value | P-Value |
|---|---|---|---|
| -0,034 | 28 | -0,154 | 0,8791 |

**TABLE 57 Paired t-test tortuosity of the vessels**

| Mean Difference | DF | t-Value | P-Value |
|---|---|---|---|
| -0,517 | 28 | -1,543 | 0,1341 |

*Evaluation of the toxicity.* From the five mice originally planed for evaluation of neuronal damage after injection of FGF23CTP in the right eye and PBS in the left eye, only three are studied due to damage to the lens in one eye and fixation problems in another animal.

The total thickness of the outer nuclear layer, the inner nuclear layer and the ganglion cell layer is measured using a standardized magnification after paraffin embedding and hematoxylin and eosin (HE)-staining. Three sections of each eye are measured at three locations each. TABLE 58 shows the result of the measurements in mm using a x200 magnification. Sections are encoded for evaluation.

**TABLE 58 Neuronal Damage**

| Animal No. | Outer nuclear layer | | Inner nuclear layer | | Ganglion cell layer | |
|---|---|---|---|---|---|---|
| | right | left | right | left | right | left |
| 150-5 | 10,5 | 11 | 6 | 6,5 | 2,5 | 2 |
| | 11 | 9 | 7 | 5 | 3 | 2 |
| | 10,5 | 10 | 5 | 5,5 | 2 | 3 |
| | 10 | 10 | 5,5 | 5 | 2 | 1,5 |
| | 10 | 10,5 | 7 | 6 | 2,5 | 3,5 |
| | 11 | 10 | 6 | 7 | 2 | 2 |
| | 11 | 9 | 6,5 | 5 | 2,5 | 1,5 |
| | 11,5 | 11 | 6,5 | 7 | 2,5 | 2,5 |
| | 10 | 10 | 6,5 | 6 | 2 | 2 |
| 150-7 | 10,5 | 10,5 | 6 | 6 | 1,5 | 2 |
| | 12 | 12 | 7 | 7 | 2 | 2 |
| | 10 | 9 | 6 | 5,5 | 2,5 | 3 |
| | 10 | 11 | 6 | 7 | 2 | 2 |
| | 10 | 11 | 6 | 6 | 2 | 2,5 |
| | 11,5 | 11 | 7 | 7 | 2,5 | 3 |
| | 10 | 11 | 6 | 6,5 | 2 | 2 |
| | 10 | 12,5 | 5,5 | 6 | 2 | 2 |
| | | 10 | | 7 | | 2,5 |
| 150-9 | 10,5 | 10 | 5 | 6 | 2 | 2 |
| | 11 | 10 | 6 | 6 | 2 | 2,5 |
| | 10,5 | 11 | 6 | 5,5 | 2 | 1,5 |
| | 11 | 9 | 5 | 6 | 2 | 2,5 |
| | 11 | 11 | 5 | 5 | 2,5 | 2 |
| | 12 | 11 | 6 | 5,5 | 2,5 | 2,5 |
| | 10 | 12 | 5,5 | 6 | 3 | 2 |
| | 11 | 9 | 5,5 | 6 | 3 | 2,5 |
| | 10 | 11 | 6,5 | 5 | 2 | 1,5 |
| variance | 0,64121279 | 0,9600273 | 0,63245553 | 0,69337525 | 0,38078866 | 0,50636968 |
| average | 10,6346154 | 10,462963 | 6 | 6 | 2,25 | 2,22222222 |

Based on the measurements performed on the three animals, FGF23CTP is found not to induce any neuronal damage in the eye. In detail, there is no marked difference in the thickness of the neuronal layers of the inner nuclear layer, the outer nuclear layer or the ganglion cell layer five days after the injection of either FGF23CTP or PBS.

## Claims

1. A fragment of FGF23, wherein said fragment
a) consists of the sequence of SEQ ID NO 2, or
b) is a functional variant of the polypeptide of SEQ ID NO. 2, having at least 90% sequence-identity with SEQ ID NO 2, derived by conservative amino acid substitution of the amino acid sequence of SEQ ID NO 2.

2. A polypeptide according to claim 1 for use as a medicament.

3. A polypeptide according to claim 1 for the treatment of a disease associated with increased angiogenesis.

4. A polypeptide according to claim 3, wherein the disease associated with increased angiogenesis is selected from the group consisting of retinopathies, age-related macular degeneration, haemangioblastoma, haemangioma, tumors and cancer.

5. A polypeptide according to claim 4, wherein the disease associated with increased angiogenesis is retinopathy.

6. A polypeptide according to claim 5, wherein the disease associated with enhanced angiogenesis is cancer.

7. Use of a polypeptide according to claim 1 for the manufacture of a medicament for treatment of a disease selected from the group consisting of retinopathies, age-related macular degeneration, haemangioblastoma, haemangioma, tumors and cancer.

8. A pharmaceutical composition comprising a polypeptide according to claim 1 and a pharmaceutically-acceptable carrier.

## Patentansprüche

1. Fragment von FGF23, wobei das Fragment
a) aus der Sequenz SEQ ID NR: 2 besteht oder
b) eine funktionelle Variante des Polypeptids der SEQ ID NR: 2 ist, die mindestens 90% Sequenzidentität mit SEQ ID NR: 2 hat und aus einer konservativen Aminosäuresubstitution der Aminosäuresequenz SEQ ID NR: 2 hervorgegangen ist.

2. Polypeptid nach Anspruch 1 für die Verwendung als Arzneimittel.

3. Polypeptid nach Anspruch 1 für die Behandlung einer Krankheit, die mit erhöhter Angiogenese einhergeht.

4. Polypeptid nach Anspruch 3, wobei die Krankheit, die mit erhöhter Angiogenese einhergeht, aus der Gruppe ausgewählt ist, die aus Retinopathien, altersbedingter Makula-Degeneration, Hämangioblastom, Hämangiom, Tumoren und Krebs besteht.

5. Polypeptid nach Anspruch 4, wobei es sich bei der Krankheit, die mit erhöhter Angiogenese einhergeht, um Retinopathie handelt.

6. Polypeptid nach Anspruch 5, wobei es sich bei der Krankheit, die mit erhöhter Angiogenese einhergeht, um Krebs handelt.

7. Verwendung eines Polypeptids nach Anspruch 1 zur Herstellung eines Arzneimittels zur Behandlung einer Krankheit, die aus der Gruppe ausgewählt ist, die aus Retinopathien, altersbedingter Makula-Degeneration, Hämangioblastom, Hämangiom, Tumoren und Krebs besteht.

8. Pharmazeutische Zusammensetzung, die ein Polypeptid nach Anspruch 1 und einen pharmazeutisch annehmbaren Träger umfasst.

## Revendications

1. Fragment de FGF23, **caractérisé en ce que** ledit fragment
a) est constitué de la SEQ ID NO 2, ou
b) est un variant fonctionnel du polypeptide de SEQ ID NO 2, ayant au moins 90 % d'identité de séquence avec SEQ ID NO 2, dérivé par substitution d'acide aminé conservatrice de la séquence d'acides aminés de SEQ ID NO 2.

2. Polypeptide selon la revendication 1 pour utilisation en tant que médicament.

3. Polypeptide selon la revendication 1 pour le traitement d'une maladie associée à une angiogenèse augmentée.

4. Polypeptide selon la revendication 3, **caractérisé en ce que** la maladie associée à une angiogenèse augmentée est choisie dans le groupe constitué des rétinopathies, dégénérescence maculaire liée à l'âge, hémangioblastome, hémangiome, tumeurs et cancer.

5. Polypeptide selon la revendication 4, **caractérisé en ce que** la maladie associée à une angiogenèse augmentée est une rétinopathie.

6. Polypeptide selon la revendication 5, **caractérisé en ce que** la maladie associée à une angiogenèse augmentée est un cancer.

7. Utilisation d'un polypeptide selon la revendication 1 pour la fabrication d'un médicament pour le traitement d'une maladie choisie dans le groupe constitué des rétinopathies, dégénérescence maculaire liée à l'âge, hémangioblastome, hémangiome, tumeurs et cancer.

8. Composition pharmaceutique comprenant un polypeptide selon la revendication 1 et un véhicule pharmaceutiquement acceptable.
